# EUROPEAN PATENT APPLICATION

(11) **EP 3 321 358 A2**
(43) Date of publication of application: **16.05.2018**
(21) Application number: 17203397.9
(22) Date of filing: 10.05.2013
(51) Int. Cl.: C12N 7/04, A61K 39/15, A61P 31/14, A61P 37/04, C07K 14/14, C12N 15/46, C12N 15/82, C12N 7/01

(54) **ROTAVIRUS-LIKE PARTICLE PRODUCTION IN PLANTS**

(30) Priority: 11.05.2012 US 201261646058 P
(62) Divisional of application: 13787880.7
(71) Applicant: Medicago Inc., Québec, Québec G1V 3V9 (CA); Mitsubishi Tanabe Pharma Corporation, Chuo-ku, Osaka 541-8505 (JP)
(72) Inventor: D'AOUST, Marc-Andre, Québec, Québec G1X 4N4 (CA); LANDRY, Nathalie, St-Jean-Chrysostome, Québec G6Z 1K1 (CA); LAVOIE, Pierre-Olivier, Québec, Québec G1N 2L7 (CA); ARAI, Masaaki, Osaka, Osaka 541-8505 (JP); ASAHARA, Naomi, Osaka, Osaka 541-8505 (JP); MUTEPFA, David Levi Rutendo, Sherwood, Nottinghamshire N5G 3EY (GB); HITZEROTH, Inga Isabel, Vredehoek Cape Town (ZA); RYBICKI, Edward Peter, 7405 Cape Town (ZA)
(74) Representative: Kador & Partner

(57) **Abstract**

A method of producing a virus-like particle (VLP) in a plant is provided. The method comprises introducing a first nucleic acid into the plant, or portion of the plant. The first nucleic acid comprising a first regulatory region active in the plant operatively linked to a nucleotide sequence encoding one or more rotavirus structural protein for example but not limited to rotavirus protein VP2. The nucleotide sequence may further comprise one or more than one amplification element and/or a compartment targeting sequence. A second nucleic acid might be introduced into the plant, or portion of the plant. The second nucleic acid comprises a second regulatory region active in the plant and operatively linked to a nucleotide sequence encoding one or more rotavirus structural protein, for example but not limited to rotavirus protein VP6. Optionally, a third nucleic acid and/ or fourth nucleic acid might be introduced into the plant, or portion of the plant. The third nucleic acid comprises a third regulatory region active in the plant and operatively linked to a nucleotide sequence encoding one or more rotavirus structural protein, for example but not limited to rotavirus protein VP4. The fourth nucleic acid comprises a fourth regulatory region active in the plant and operatively linked to a nucleotide sequence encoding one or more rotavirus structural protein, for example but not limited to rotavirus protein VP7. The plant or portion of the plant is incubated under conditions that permit the expression of the nucleic acids, thereby producing the VLP.

SEQUENCE LISTING

<110>  Medicago Inc.
       Mitsubishi Tanabe Pharma Corporation

<120>  Rotavirus-like Particle  Production in Plants

<130>  V84653WO

<150>  US 61/646,058
<151>  2012-05-11

<160>  59

<170>  PatentIn version 3.5

<210>  1
<211>  884
<212>  PRT
<213>  Nicotiana tabacum

<400>  1

Gly Arg Val Arg Ser Met Ala Tyr Arg Lys Arg Gly Ala Arg Arg Glu
1               5                   10                  15

Ala Asn Leu Asn Asn Asn Asp Arg Met Gln Glu Lys Ile Asp Glu Lys
            20                  25                  30

Gln Asp Ser Asn Lys Ile Gln Leu Ser Asp Lys Val Leu Ser Lys Lys
        35                  40                  45

Glu Glu Ile Val Thr Asp Ser His Glu Glu Val Lys Val Thr Asp Glu
    50                  55                  60

Leu Lys Lys Ser Thr Lys Glu Glu Ser Lys Gln Leu Leu Glu Val Leu
65                  70                  75                  80

Lys Thr Lys Glu Glu His Gln Lys Glu Ile Gln Tyr Glu Ile Leu Gln
                85                  90                  95

Lys Thr Ile Pro Thr Phe Glu Pro Lys Glu Thr Ile Leu Arg Lys Leu
            100                 105                 110

Glu Asp Ile Gln Pro Glu Leu Ala Lys Lys Gln Thr Lys Leu Phe Arg
        115                 120                 125

Ile Phe Glu Pro Lys Gln Leu Pro Ile Tyr Arg Ala Asn Gly Glu Arg
    130                 135                 140

Glu Leu Arg Asn Arg Trp Tyr Trp Lys Leu Lys Lys Asp Thr Leu Pro
145                 150                 155                 160

Asp Gly Asp Tyr Asp Val Arg Glu Tyr Phe Leu Asn Leu Tyr Asp Gln
                165                 170                 175

Val Leu Thr Glu Met Pro Asp Tyr Leu Leu Leu Lys Asp Met Ala Val
            180                 185                 190

Glu Asn Lys Asn Ser Arg Asp Ala Gly Lys Val Val Asp Ser Glu Thr
        195                 200                 205

Ala Ser Ile Cys Asp Ala Ile Phe Gln Asp Glu Glu Thr Glu Gly Ala
    210                 215                 220

Val Arg Arg Phe Ile Ala Glu Met Arg Gln Arg Val Gln Ala Asp Arg
225                 230                 235                 240

Asn Val Val Asn Tyr Pro Ser Ile Leu His Pro Ile Asp Tyr Ala Phe
                245                 250                 255

Asn Glu Tyr Phe Leu Gln His Gln Leu Val Glu Pro Leu Asn Asn Asp
            260                 265                 270

Ile Ile Phe Asn Tyr Ile Pro Glu Arg Ile Arg Asn Asp Val Asn Tyr
        275                 280                 285

Ile Leu Asn Met Asp Arg Asn Leu Pro Ser Thr Ala Arg Tyr Ile Arg
    290                 295                 300

Pro Asn Leu Leu Gln Asp Arg Leu Asn Leu His Asp Asn Phe Glu Ser
305                 310                 315                 320

Leu Trp Asp Thr Ile Thr Thr Ser Asn Tyr Ile Leu Ala Arg Ser Val
                325                 330                 335

Val Pro Asp Leu Lys Glu Leu Val Ser Thr Glu Ala Gln Ile Gln Lys
            340                 345                 350

Met Ser Gln Asp Leu Gln Leu Glu Ala Leu Thr Ile Gln Ser Glu Thr
        355                 360                 365

Gln Phe Leu Thr Gly Ile Asn Ser Gln Ala Ala Asn Asp Cys Phe Lys
    370                 375                 380

Thr Leu Ile Ala Ala Met Leu Ser Gln Arg Thr Met Ser Leu Asp Phe
385                 390                 395                 400

Val Thr Thr Asn Tyr Met Ser Leu Ile Ser Gly Met Trp Leu Leu Thr
                405                 410                 415

Val Val Pro Asn Asp Met Phe Ile Arg Glu Ser Leu Val Ala Cys Gln
            420                 425                 430

Leu Ala Ile Val Asn Thr Ile Ile Tyr Pro Ala Phe Gly Met Gln Arg
        435                 440                 445

Met His Tyr Arg Asn Gly Asp Pro Gln Thr Pro Phe Gln Ile Ala Glu
    450                 455                 460

Gln Gln Ile Gln Asn Phe Gln Val Ala Asn Trp Leu His Phe Val Asn
465                 470                 475                 480

Asn Asn Gln Phe Arg Gln Ala Val Ile Asp Gly Val Leu Asn Gln Val
                485                 490                 495

Leu Asn Asp Asn Ile Arg Asn Gly His Val Ile Asn Gln Leu Met Glu
            500                 505                 510

Ala Leu Met Gln Leu Ser Arg Gln Gln Phe Pro Thr Met Pro Ile Asp
        515                 520                 525

Tyr Lys Arg Ser Ile Gln Arg Gly Ile Leu Leu Leu Ser Asn Arg Leu
    530                 535                 540

Gly Gln Leu Val Asp Leu Thr Arg Leu Leu Ala Tyr Asn Tyr Glu Thr
545                 550                 555                 560

Leu Met Ala Cys Ile Thr Met Asn Met Gln His Val Gln Thr Leu Thr
                565                 570                 575

Thr Glu Lys Leu Gln Leu Thr Ser Val Thr Ser Leu Cys Met Leu Ile
            580                 585                 590

Gly Asn Ala Thr Val Ile Pro Ser Pro Gln Thr Leu Phe His Tyr Tyr
        595                 600                 605

Asn Val Asn Val Asn Phe His Ser Asn Tyr Asn Glu Arg Ile Asn Asp
    610                 615                 620

Ala Val Ala Ile Ile Thr Ala Ala Asn Arg Leu Asn Leu Tyr Gln Lys
625                 630                 635                 640

Lys Met Lys Ala Ile Val Glu Asp Phe Leu Lys Arg Leu Tyr Ile Phe
                645                 650                 655

Asp Val Ser Arg Val Pro Asp Asp Gln Met Tyr Arg Leu Arg Asp Arg
            660                 665                 670

Leu Arg Leu Leu Pro Val Glu Ile Arg Arg Leu Asp Ile Phe Asn Leu
        675                 680                 685

Ile Leu Met Asn Met Asp Gln Ile Glu Arg Ala Ser Asp Lys Ile Ala
    690                 695                 700

Gln Gly Val Ile Ile Ala Tyr Arg Asp Met His Leu Glu Arg Asp Glu
705                 710                 715                 720

Met Tyr Gly Tyr Val Asn Ile Ala Arg Asn Leu Glu Gly Phe Gln Gln
                725                 730                 735

Ile Asn Leu Glu Glu Leu Met Arg Ser Gly Asp Tyr Ala Gln Ile Thr
            740                 745                 750

Asn Met Leu Leu Asn Asn Gln Pro Val Ala Leu Val Gly Ala Leu Pro
        755                 760                 765

Phe Ile Thr Asp Ser Ser Val Ile Ser Leu Ile Ala Lys Leu Asp Ala
    770                 775                 780

Thr Val Phe Ala Gln Ile Val Lys Leu Arg Lys Val Asp Thr Leu Lys
785                 790                 795                 800

Pro Ile Leu Tyr Lys Ile Asn Ser Asp Ser Asn Asp Phe Tyr Leu Val
                805                 810                 815

Ala Asn Tyr Asp Trp Val Pro Thr Ser Thr Thr Lys Val Tyr Lys Gln
            820                 825                 830

Val Pro Gln Gln Phe Asp Phe Arg Asn Ser Met His Met Leu Thr Ser
        835                 840                 845

Asn Leu Thr Phe Thr Val Tyr Ser Asp Leu Leu Ala Phe Val Ser Ala
    850                 855                 860

Asp Thr Val Glu Pro Ile Asn Ala Val Ala Phe Asp Asn Met Arg Ile
865                 870                 875                 880

Met Asn Glu Leu

<210>  2
<211>  380
<212>  PRT
<213>  Nicotiana tabacum

<400> 2

Gly Arg Val Arg Ser Met Ala Ser Leu Ile Tyr Arg Gln Leu Leu Thr
1               5                   10                  15

Asn Ser Tyr Thr Val Glu Leu Ser Asp Glu Ile Asn Thr Ile Gly Ser
            20                  25                  30

Glu Lys Ser Gln Asn Val Thr Ile Asn Pro Gly Pro Phe Ala Gln Thr
        35                  40                  45

Asn Tyr Ala Pro Val Thr Trp Ser His Gly Glu Val Asn Asp Ser Thr
    50                  55                  60

Thr Ile Glu Pro Val Leu Asp Gly Pro Tyr Gln Pro Thr Asn Phe Lys
65                  70                  75                  80

Pro Pro Asn Asp Tyr Trp Ile Leu Leu Asn Pro Thr Asn Gln Gln Val
                85                  90                  95

Val Leu Glu Gly Thr Asn Lys Thr Asp Ile Trp Val Ala Leu Leu Leu
            100                 105                 110

Val Glu Pro Asn Val Thr Asn Gln Ser Arg Gln Tyr Thr Leu Phe Gly
        115                 120                 125

Glu Thr Lys Gln Ile Thr Val Glu Leu Pro Thr Asp Phe Ser Val Ser
    130                 135                 140

Arg Tyr Glu Val Ile Lys Glu Asn Ser Tyr Val Tyr Val Asp Tyr Trp
145                 150                 155                 160

Asp Asp Ser Gln Ala Phe Arg Asn Met Val Tyr Val Arg Ser Leu Ala
                165                 170                 175

Ala Asn Leu Asn Ser Val Lys Cys Ser Gly Gly Asn Tyr Asn Phe Gln
            180                 185                 190

Ile Pro Val Gly Ala Trp Pro Val Met Ser Gly Gly Ala Val Ser Leu
        195                 200                 205

His Phe Ala Gly Val Thr Leu Ser Thr Gln Phe Thr Asp Phe Val Ser
    210                 215                 220

Leu Asn Ser Leu Arg Phe Arg Phe Ser Leu Thr Val Glu Glu Pro Pro
225                 230                 235                 240

Phe Ser Ile Leu Arg Thr Arg Val Ser Gly Leu Tyr Gly Leu Pro Ala

245                 250                 255

Phe Asn Pro Asn Asn Gly His Glu Tyr Tyr Glu Ile Ala Gly Arg Phe
            260                 265                 270

Ser Leu Ile Ser Leu Val Pro Ser Asn Asp Asp Tyr Gln Thr Pro Ile
        275                 280                 285

Met Asn Ser Val Thr Val Arg Gln Asp Leu Glu Arg Gln Leu Gly Asp
    290                 295                 300

Leu Arg Glu Glu Phe Asn Ser Leu Ser Gln Glu Ile Ala Met Thr Gln
305                 310                 315                 320

Leu Ile Asp Leu Ala Leu Leu Pro Leu Asp Met Phe Ser Met Phe Ser
                325                 330                 335

Asn Tyr Gly Ile Thr Arg Ser Gln Ala Leu Asp Leu Ile Arg Ser Asp
            340                 345                 350

Pro Arg Val Leu Arg Asp Phe Ile Asn Gln Asn Asn Pro Ile Ile Lys
        355                 360                 365

Asn Arg Ile Glu Gln Leu Ile Leu Gln Cys Arg Leu
    370                 375                 380

<210>  3
<211>  402
<212>  PRT
<213>  Nicotiana tabacum

<400>  3

Gly Arg Val Arg Ser Met Asp Val Leu Tyr Ser Leu Ser Lys Thr Leu
1               5                   10                  15

Lys Asp Ala Arg Asp Lys Ile Val Glu Gly Thr Leu Tyr Ser Asn Val
            20                  25                  30

Ser Asp Leu Ile Gln Gln Phe Asn Gln Met Ile Ile Thr Met Asn Gly
        35                  40                  45

Asn Glu Phe Gln Thr Gly Gly Ile Gly Asn Leu Pro Ile Arg Asn Trp
    50                  55                  60

Asn Phe Asp Phe Gly Leu Leu Gly Thr Thr Leu Leu Asn Leu Asp Ala
65                  70                  75                  80

Asn Tyr Val Glu Thr Ala Arg Asn Thr Ile Asp Tyr Phe Val Asp Phe

                85                  90                  95

Val Asp Asn Val Cys Met Asp Glu Met Val Arg Glu Ser Gln Arg Asn
            100                 105                 110

Gly Ile Ala Pro Gln Ser Asp Ser Leu Arg Lys Leu Ser Gly Ile Lys
        115                 120                 125

Phe Lys Arg Ile Asn Phe Asp Asn Ser Ser Glu Tyr Ile Glu Asn Trp
    130                 135                 140

Asn Leu Gln Asn Arg Arg Gln Arg Thr Gly Phe Thr Phe His Lys Pro
145                 150                 155                 160

Asn Ile Phe Pro Tyr Ser Ala Ser Phe Thr Leu Asn Arg Ser Gln Pro
                165                 170                 175

Ala His Asp Asn Leu Met Gly Thr Met Trp Leu Asn Ala Gly Ser Glu
            180                 185                 190

Ile Gln Val Ala Gly Phe Asp Tyr Ser Cys Ala Ile Asn Ala Pro Ala
        195                 200                 205

Asn Thr Gln Gln Phe Glu His Ile Val Gln Leu Arg Arg Val Leu Thr
    210                 215                 220

Thr Ala Thr Ile Thr Leu Leu Pro Asp Ala Glu Arg Phe Ser Phe Pro
225                 230                 235                 240

Arg Val Ile Asn Ser Ala Asp Gly Ala Thr Thr Trp Tyr Phe Asn Pro
                245                 250                 255

Val Ile Leu Arg Pro Asn Asn Val Glu Val Glu Phe Leu Leu Asn Gly
            260                 265                 270

Gln Ile Ile Asn Thr Tyr Gln Ala Arg Phe Gly Thr Ile Val Ala Arg
        275                 280                 285

Asn Phe Asp Thr Ile Arg Leu Ser Phe Gln Leu Met Arg Pro Pro Asn
    290                 295                 300

Met Thr Pro Ser Val Ala Ala Leu Phe Pro Asn Ala Gln Pro Phe Glu
305                 310                 315                 320

His His Ala Thr Val Gly Leu Thr Leu Lys Ile Glu Ser Ala Val Cys
                325                 330                 335

Glu Ser Val Leu Ala Asp Ala Ser Glu Thr Met Leu Ala Asn Val Thr
            340                 345                 350

Ser Val Arg Gln Glu Tyr Ala Ile Pro Val Gly Pro Val Phe Pro Pro
        355                 360                 365

Gly Met Asn Trp Thr Asp Leu Ile Thr Asn Tyr Ser Pro Ser Arg Glu
    370                 375                 380

Asp Asn Leu Gln Arg Val Phe Thr Val Ala Ser Ile Arg Ser Met Leu
385                 390                 395                 400

Val Lys

<210>  4
<211>  331
<212>  PRT
<213>  Nicotiana tabacum

<400>  4

Gly Arg Val Arg Cys Met Tyr Gly Ile Glu Tyr Thr Thr Ile Leu Thr
1               5                   10                  15

Phe Leu Ile Ser Ile Val Leu Leu Asn Tyr Ile Leu Lys Ser Leu Thr
            20                  25                  30

Ser Ala Met Asp Phe Ile Ile Tyr Arg Phe Leu Leu Leu Ile Val Ile
        35                  40                  45

Ala Ser Pro Phe Val Lys Thr Gln Asn Tyr Gly Ile Asn Leu Pro Ile
    50                  55                  60

Thr Gly Ser Met Asp Thr Ala Tyr Ala Asn Ser Ser Gln Gln Glu Thr
65                  70                  75                  80

Phe Leu Thr Ser Thr Leu Cys Leu Tyr Tyr Pro Thr Glu Ala Ser Thr
                85                  90                  95

Gln Ile Gly Asp Thr Glu Trp Lys Asp Thr Leu Ser Gln Leu Phe Leu
            100                 105                 110

Thr Lys Gly Trp Pro Thr Gly Ser Val Tyr Phe Lys Glu Tyr Thr Asp
        115                 120                 125

Ile Ala Ser Phe Ser Ile Asp Pro Gln Leu Tyr Cys Asp Tyr Asn Val
    130                 135                 140

Val Leu Met Lys Tyr Asp Ser Thr Leu Glu Leu Asp Met Ser Glu Leu
145                 150                 155                 160

Ala Asp Leu Ile Leu Asn Glu Trp Leu Cys Asn Pro Met Asp Ile Thr
                165                 170                 175

Leu Tyr Tyr Tyr Gln Gln Thr Asp Glu Ala Asn Lys Trp Ile Ser Met
            180                 185                 190

Gly Gln Ser Cys Thr Ile Lys Val Cys Pro Leu Asn Thr Gln Thr Leu
        195                 200                 205

Gly Ile Gly Cys Ile Thr Thr Asn Thr Ala Thr Phe Glu Glu Val Ala
    210                 215                 220

Thr Ser Glu Lys Leu Val Ile Thr Asp Val Val Asp Gly Val Asn His
225                 230                 235                 240

Lys Leu Asp Val Thr Thr Asn Thr Cys Thr Ile Arg Asn Cys Lys Lys
                245                 250                 255

Leu Gly Pro Arg Glu Asn Val Ala Ile Ile Gln Val Gly Gly Ser Asp
            260                 265                 270

Val Leu Asp Ile Thr Ala Asp Pro Thr Thr Ala Pro Gln Thr Glu Arg
        275                 280                 285

Met Met Arg Val Asn Trp Lys Lys Trp Trp Gln Val Phe Tyr Thr Val
    290                 295                 300

Val Asp Tyr Ile Asn Gln Ile Val Gln Val Met Ser Lys Arg Ser Arg
305                 310                 315                 320

Ser Leu Asn Ser Ala Ala Phe Tyr Tyr Arg Val
                325                 330

<210>  5
<211>  22
<212>  DNA
<213>  Artificial sequence

<220>
<223>  Primer VP2F

<400>  5
ttccatggct taccgtaaaa gg                                              22

<210>  6
<211>  32
<212>  DNA

<213>  Artificial sequence

<220>
<223>  Primer VP2R

<400>  6
atgcggccgc aagctcgttc ataatcctca tg                                    32


<210>  7
<211>  22
<212>  DNA
<213>  Artificial Sequence

<220>
<223>  Primer VP4F

<400>  7
ttccatggct tccctcatct ac                                               22


<210>  8
<211>  31
<212>  DNA
<213>  Artificial sequence

<220>
<223>  Primer VP4R

<400>  8
atgcggccgc aagacggcac tggagaatga g                                     31


<210>  9
<211>  21
<212>  DNA
<213>  Artificial sequence

<220>
<223>  Primer VP6F

<400>  9
ttccatggat gtgctctact c                                                21


<210>  10
<211>  28
<212>  DNA
<213>  Artificial sequence

<220>
<223>  Primer VP6R

<400>  10
atgcggccgc cttcacgagc atggaacg                                         28


<210>  11
<211>  22
<212>  DNA
<213>  Artificial sequence

<220>

<223>  Primer VP7F

<400>  11
gtacatgtac ggaatcgagt ac                                              22


<210>  12
<211>  31
<212>  DNA
<213>  Artificial sequence

<220>
<223>  Primer VP7R

<400>  12
atgcggccgc cacacggtag tagaaagcag c                                    31


<210>  13
<211>  2700
<212>  DNA
<213>  Nicotiana tabacum

<400>  13
ggtaccgaat tcggacgcgt tcgttccatg gcttaccgta aaaggggtgc taggcgtgaa      60

gctaacctca acaacaacga taggatgcaa gagaagatcg atgagaagca ggattccaac     120

aagatccagc tctccgataa ggtgctctcc aagaaagaag agatcgttac tgattcccac     180

gaagaggtga aggtgacaga tgagcttaag aagtccacaa aagaagagtc caagcagctc     240

cttgaggtgc tcaagacaaa agaggaacac cagaaagaga tccagtacga gatcctccaa     300

aagactatcc caactttcga gccaaaagag actatcctca ggaagcttga ggatatccag     360

ccagagcttg ctaagaagca gactaagctc ttcaggatct tcgagccaaa gcagctccca     420

atctaccgtg ctaacggtga aagggaactt aggaacaggt ggtactggaa gctcaagaag     480

gatactctcc cagacggtga ttacgatgtg agagagtact tcctcaacct ctacgatcag     540

gtgctcactg agatgccaga ttacctcctc ctcaaggata tggctgtgga gaacaagaac     600

tccagggatg ctggaaaggt ggtggattcc gagactgctt ccatctgtga tgctatcttc     660

caggatgaag agactgaggg tgctgtgagg cgtttcattg ctgagatgag gcagagggtt     720

caggctgata ggaacgtggt gaactaccca tccatcctcc acccaatcga ttacgctttc     780

aacgagtact tccttcagca ccagcttgtg gagccactca acaacgatat catcttcaac     840

tacatcccag agaggattag gaacgacgtt aactacatcc tcaacatgga taggaacctc     900

ccatccactg ctcgttacat caggccaaac ctcctccagg ataggctcaa cctccacgat     960

aacttcgagt ccctctggga tacaatcact acttccaact acattctcgc tcgttccgtg    1020

gtgccagatc tcaaagaact cgtgtccact gaggctcaga tccagaagat gtcccaggat    1080

ctccagcttg aggctctcac tatccagtcc gagactcagt tcctcactgg tatcaactcc    1140

caggctgcta acgattgctt caagactctc attgctgcta tgctctccca gaggactatg    1200

tccctcgatt tcgtgactac taactatatg tccctcatct ccggaatgtg gctcttgact 1260

gtggtgccaa acgatatgtt catccgtgag tcccttgtgg cttgccagct cgctatcgtg 1320

aacactatca tctacccagc tttcggaatg caaaggatgc actaccgtaa cggtgatcca 1380

cagactccat tccagatcgc agagcagcag atccagaact tccaggtggc aaactggctc 1440

cacttcgtga acaacaacca gttcaggcag gctgtgatcg atggtgtgtt gaaccaggtg 1500

ctcaacgata acatccgtaa cggtcacgtg atcaaccagc tcatggaagc tctcatgcaa 1560

ctctccaggc agcagttccc aactatgcct atcgattaca agcgttccat ccagagggga 1620

atcctcctcc tttccaacag gcttggacag ctcgtggatc tcactaggct cctcgcttac 1680

aactacgaga ctctcatggc ttgcatcact atgaacatgc agcacgttca gactctcact 1740

actgagaagc tccagctcac ttccgtgact tccctctgca tgctcatcgg aaacgctact 1800

gtgatcccat ccccacagac actcttccac tactacaacg tgaacgtgaa cttccactcc 1860

aactacaacg agaggatcaa cgatgctgtg gctatcatca ctgctgctaa caggcttaac 1920

ctctaccaaa agaagatgaa ggctatcgtt gaggatttcc tcaagaggct ctacatcttc 1980

gatgtgtcca gggtgccaga tgatcagatg taccgtctta gggataggct taggctcctc 2040

ccagtggaga tcagaaggct cgatatcttc aacctcatcc ttatgaacat ggatcagatc 2100

gagagggctt ccgataagat cgctcagggt gttattatcg cttaccgtga tatgcacctt 2160

gagagggatg agatgtacgg atacgtgaac attgctagga accttgaggg attccagcag 2220

atcaaccttg aagagcttat gcgttccggt gattacgctc agatcactaa catgctcctc 2280

aacaaccagc cagtggctct tgttggtgct ctcccattca tcactgattc ctccgtgatc 2340

tccctcattg ctaagttgga tgctactgtg ttcgctcaga tcgtgaagct caggaaagtg 2400

gatactctca agccaatcct ctacaagatc aactccgatt ccaacgattt ctacctcgtg 2460

gctaactacg attgggtgcc aacttccact acaaaggtgt acaagcaggt gccacagcag 2520

ttcgatttcc gtaactccat gcacatgctc acttccaacc tcactttcac tgtgtactcc 2580

gatctcctcg ctttcgtgtc cgctgatact gtggagccta tcaacgctgt ggctttcgat 2640

aacatgagga ttatgaacga gctttgatga ctcgagggat cctctagaga attcgagctc 2700

<210> 14
<211> 2700
<212> DNA
<213> Nicotiana tabacum

<400> 14
ccatggctta agcctgcgca agcaaggtac cgaatggcat tttccccacg atccgcactt 60

cgattggagt tgttgttgct atcctacgtt ctcttctagc tactcttcgt cctaaggttg 120

ttctaggtcg agaggctatt ccacgagagg ttctttcttc tctagcaatg actaagggtg 180

cttctccact tccactgtct actcgaattc ttcaggtgtt ttcttctcag gttcgtcgag 240

gaactccacg agttctgttt tctccttgtg gtctttctct aggtcatgct ctaggaggtt 300

ttctgatagg gttgaaagct cggttttctc tgataggagt ccttcgaact cctataggtc 360

ggtctcgaac gattcttcgt ctgattcgag aagtcctaga agctcggttt cgtcgagggt 420

tagatggcac gattgccact ttcccttgaa tccttgtcca ccatgacctt cgagttcttc 480

ctatgagagg gtctgccact aatgctacac tctctcatga aggagttgga gatgctagtc 540

cacgagtgac tctacggtct aatggaggag gagttcctat accgacacct cttgttcttg 600

aggtccctac gacctttcca ccacctaagg ctctgacgaa ggtagacact acgatagaag 660

gtcctacttc tctgactccc acgacactcc gcaaagtaac gactctactc cgtctcccaa 720

gtccgactat ccttgcacca cttgatgggt aggtaggagg tgggttagct aatgcgaaag 780

ttgctcatga aggaagtcgt ggtcgaacac ctcggtgagt tgttgctata gtagaagttg 840

atgtagggtc tctcctaatc cttgctgcaa ttgatgtagg agttgtacct atccttggag 900

ggtaggtgac gagcaatgta gtccggtttg gaggaggtcc tatccgagtt ggaggtgcta 960

ttgaagctca gggagaccct atgttagtga tgaaggttga tgtaagagcg agcaaggcac 1020

cacggtctag agtttcttga gcacaggtga ctccgagtct aggtcttcta cagggtccta 1080

gaggtcgaac tccgagagtg ataggtcagg ctctgagtca aggagtgacc atagttgagg 1140

gtccgacgat tgctaacgaa gttctgagag taacgacgat acgagagggt ctcctgatac 1200

agggagctaa agcactgatg attgatatac agggagtaga ggccttacac cgagaactga 1260

caccacggtt tgctatacaa gtaggcactc agggaacacc gaacggtcga gcgatagcac 1320

ttgtgatagt agatgggtcg aaagccttac gtttcctacg tgatggcatt gccactaggt 1380

gtctgaggta aggtctagcg tctcgtcgtc taggtcttga aggtccaccg tttgaccgag 1440

gtgaagcact tgttgttggt caagtccgtc cgacactagc taccacacaa cttggtccac 1500

gagttgctat tgtaggcatt gccagtgcac tagttggtcg agtaccttcg agagtacgtt 1560

gagaggtccg tcgtcaaggg ttgatacgga tagctaatgt tcgcaaggta ggtctcccct 1620

taggaggagg aaaggttgtc cgaacctgtc gagcacctag agtgatccga ggagcgaatg 1680

ttgatgctct gagagtaccg aacgtagtga tacttgtacg tcgtgcaagt ctgagagtga 1740

tgactcttcg aggtcgagtg aaggcactga agggagacgt acgagtagcc tttgcgatga 1800

cactagggta ggggtgtctg tgagaaggtg atgatgttgc acttgcactt gaaggtgagg 1860

ttgatgttgc tctcctagtt gctacgacac cgatagtagt gacgacgatt gtccgaattg 1920

gagatggttt tcttctactt ccgatagcaa ctcctaaagg agttctccga gatgtagaag 1980

ctacacaggt cccacggtct actagtctac atggcagaat ccctatccga atccgaggag 2040

ggtcacctct agtcttccga gctatagaag ttggagtagg aatacttgta cctagtctag 2100

ctctcccgaa ggctattcta gcgagtccca caataatagc gaatggcact atacgtggaa 2160

ctctccctac tctacatgcc tatgcacttg taacgatcct tggaactccc taaggtcgtc 2220

tagttggaac ttctcgaata cgcaaggcca ctaatgcgag tctagtgatt gtacgaggag 2280

ttgttggtcg gtcaccgaga acaaccacga gagggtaagt agtgactaag gaggcactag 2340

agggagtaac gattcaacct acgatgacac aagcgagtct agcacttcga gtcctttcac 2400

ctatgagagt tcggttagga gatgttctag ttgaggctaa ggttgctaaa gatggagcac 2460

cgattgatgc taacccacgg ttgaaggtga tgtttccaca tgttcgtcca cggtgtcgtc 2520

aagctaaagg cattgaggta cgtgtacgag tgaaggttgg agtgaaagtg acacatgagg 2580

ctagaggagc gaaagcacag gcgactatga cacctcggat agttgcgaca ccgaaagcta 2640

ttgtactcct aatacttgct cgaaactact gagctcccta ggagatctct taagctcgag 2700

<210> 15
<211> 2388
<212> DNA
<213> Rotavirus

<400> 15
ggtaccgaat tcggacgcgt tcgttccatg gcttccctca tctaccgtca gttgctcact 60

aactcctaca ctgtggagct ttccgatgag atcaacacta tcggttccga gaagtcccag 120

aacgtgacta tcaacccagg accattcgct cagactaact acgctccagt gacttggtca 180

cacggtgaag tgaacgattc cactactatc gagccagtgc tcgatggacc ataccagcca 240

actaacttca agccaccaaa cgattactgg attctcctca acccaactaa ccagcaggtg 300

gtgcttgagg gaactaacaa gactgatatc tgggtggcac tccttcttgt ggagccaaac 360

gtgactaacc agtccaggca gtacactctc ttcggagaga ctaagcagat cactgtggag 420

aacaacacta acaagtggaa gttcttcgag atgttcaggt ccaacgtgaa cgctgagttc 480

cagcacaaga ggactctcac ttccgataca aagctcgctg gttttatgaa gttctacaac 540

tctgtgtgga ctttccacgg tgaaactcca cacgctacta ctgattactc ctccacttcc 600

aacctttccg aggtggagac tgtgatccac gtggagttct acatcatccc aaggtcccaa 660

gagtctaagt gctccgagta catcaacact ggactcccac caatgcaaaa cactaggaac 720

atcgtgccag tggctttgtc ctctcgttcc gtgacttacc agagggctca ggtgaacgag 780

gatatcatca tctccaagac ttccctctgg aaagagatgc agtacaacag ggatattatc 840

atcaggttca agttcaacaa ctccatcgtg aagctcggag gactcggata caagtggagt 900

gagatctcct tcaaggctgc taactaccag tactcctacc tcagggatgg tgaacaggtg 960

acagctcaca ctacttgctc cgtgaacggt gttaacaact tctcctacaa cggtggttcc 1020

ctcccaactg atttctccgt gtcccgttac gaggtgatca aagagaactc ctacgtttac 1080

gtggattact gggatgattc ccaggctttc aggaacatgg tgtatgttag atccctcgct 1140

gctaacctca actccgtgaa gtgctccggt ggaaactaca acttccagat cccagtggga 1200

gcttggccag tgatgtctgg tggagctgtg tctctccact tcgctggtgt tacactctcc 1260

actcagttca ctgatttcgt gtccctcaac tccctcaggt tcaggttctc cctcactgtg 1320

gaagagccac cattctccat cctcaggact agggtgtccg gactttacgg actcccagct 1380

ttcaacccaa acaacggaca cgagtactac gagatcgctg gacgtttctc ccttatctcc 1440

ctcgtgccat ccaacgatga ttaccagact ccaattatga actccgtgac tgtgaggcag 1500

gatcttgaga ggcagctcgg agatctcagg gaagagttca actccctctc ccaagagatc 1560

gctatgactc agctcatcga tctcgctctc ctcccactcg atatgttctc catgttctct 1620

ggtatcaagt ccactatcga tgtggctaag tctatggtga ctaaggtgat gaagaagttc 1680

aagaagtccg gactcgctac ttccatctcc gagcttactg gatctctctc caacgctgct 1740

tcttctgtgt ctaggtcctc ctccatcagg tccaacatct cctccatctc agtgtggact 1800

gatgtgtccg agcagatcgc tggatcttcc gattccgtgc gtaacatctc cactcagact 1860

tccgctatct ccaagaggct taggctcaga gagatcacta ctcagactga gggaatgaac 1920

ttcgatgata tctccgctgc tgtgctcaag actaagatcg ataggtccac tcacatctcc 1980

ccagatactc tcccagatat catcactgag tcctccgaga agttcatccc aaagcgtgct 2040

taccgtgttc tcaaggatga tgaggtgatg gaagctgatg tggatggaaa gttcttcgct 2100

tacaaagtgg gaactttcga agaggtgcca ttcgatgtgg ataagttcgt ggatctcgtg 2160

actgattccc cagtgatctc cgctatcatc gatttcaaga ctctcaagaa cctcaacgat 2220

aactacggaa tcactaggtc ccaggctctc gatctcatcc gttccgatcc aagggtgctc 2280

agggatttca tcaaccagaa caacccaatc atcaagaaca ggatcgagca gctcattctc 2340

cagtgccgtc tttgatgact cgagggatcc tctagagaat tcgagctc 2388

<210> 16
<211> 2388
<212> DNA
<213> Rotavirus

<400> 16
ccatggctta agcctgcgca agcaaggtac cgaagggagt agatggcagt caacgagtga 60

ttgaggatgt gacacctcga aaggctactc tagttgtgat agccaaggct cttcagggtc 120

ttgcactgat agttgggtcc tggtaagcga gtctgattga tgcgaggtca ctgaaccagt 180

gtgccacttc acttgctaag gtgatgatag ctcggtcacg agctacctgg tatggtcggt 240

tgattgaagt tcggtggttt gctaatgacc taagaggagt tgggttgatt ggtcgtccac 300

cacgaactcc cttgattgtt ctgactatag acccaccgtg aggaagaaca cctcggtttg 360
cactgattgg tcaggtccgt catgtgagag aagcctctct gattcgtcta gtgacacctc 420
ttgttgtgat tgttcacctt caagaagctc tacaagtcca ggttgcactt gcgactcaag 480
gtcgtgttct cctgagagtg aaggctatgt ttcgagcgac caaaatactt caagatgttg 540
agacacacct gaaaggtgcc actttgaggt gtgcgatgat gactaatgag gaggtgaagg 600
ttggaaaggc tccacctctg acactaggtg cacctcaaga tgtagtaggg ttccagggtt 660
ctcagattca cgaggctcat gtagttgtga cctgagggtg gttacgtttt gtgatccttg 720
tagcacggtc accgaaacag gagagcaagg cactgaatgg tctcccgagt ccacttgctc 780
ctatagtagt agaggttctg aagggagacc tttctctacg tcatgttgtc cctataatag 840
tagtccaagt tcaagttgtt gaggtagcac ttcgagcctc ctgagcctat gttcacctca 900
ctctagagga agttccgacg attgatggtc atgaggatgg agtccctacc acttgtccac 960
tgtcgagtgt gatgaacgag gcacttgcca caattgttga agaggatgtt gccaccaagg 1020
gagggttgac taaagaggca cagggcaatg ctccactagt ttctcttgag gatgcaaatg 1080
cacctaatga ccctactaag ggtccgaaag tccttgtacc acatacaatc tagggagcga 1140
cgattggagt tgaggcactt cacgaggcca cctttgatgt tgaaggtcta gggtcaccct 1200
cgaaccggtc actacagacc acctcgacac agagaggtga agcgaccaca atgtgagagg 1260
tgagtcaagt gactaaagca cagggagttg agggagtcca agtccaagag ggagtgacac 1320
cttctcggtg gtaagaggta ggagtcctga tcccacaggc ctgaaatgcc tgagggtcga 1380
aagttgggtt tgttgcctgt gctcatgatg ctctagcgac ctgcaaagag ggaatagagg 1440
gagcacggta ggttgctact aatggtctga ggttaatact tgaggcactg acactccgtc 1500
ctagaactct ccgtcgagcc tctagagtcc cttctcaagt tgagggagag ggttctctag 1560
cgatactgag tcgagtagct agagcgagag gagggtgagc tatacaagag gtacaagaga 1620
ccatagttca ggtgatagct acaccgattc agataccact gattccacta cttcttcaag 1680
ttcttcaggc ctgagcgatg aaggtagagg ctcgaatgac ctagagagag gttgcgacga 1740
agaagacaca gatccaggag gaggtagtcc aggttgtaga ggaggtagag tcacacctga 1800
ctacacaggc tcgtctagcg acctagaagg ctaaggcacg cattgtagag gtgagtctga 1860
aggcgataga ggttctccga atccgagtct ctctagtgat gagtctgact cccttacttg 1920
aagctactat agaggcgacg acacgagttc tgattctagc tatccaggtg agtgtagagg 1980
ggtctatgag agggtctata gtagtgactc aggaggctct tcaagtaggg tttcgcacga 2040
atggcacaag agttcctact actccactac cttcgactac acctaccttt caagaagcga 2100
atgtttcacc cttgaaagct tctccacggt aagctacacc tattcaagca cctagagcac 2160
tgactaaggg gtcactagag gcgatagtag ctaaagttct gagagttctt ggagttgcta 2220

ttgatgcctt agtgatccag ggtccgagag ctagagtagg caaggctagg ttcccacgag 2280

tccctaaagt agttggtctt gttgggttag tagttcttgt cctagctcgt cgagtaagag 2340

gtcacggcag aaactactga gctccctagg agatctctta agctcgag 2388

<210> 17
<211> 1254
<212> DNA
<213> Nicotiana tabacum

<400> 17
ggtaccgaat tcggacgcgt tcgttccatg gatgtgctct actccctctc caagactctc 60

aaggatgcta gggataagat cgtggaggga actctctact ccaacgtttc cgatctcatc 120

cagcagttca accagatgat catcactatg aacggaaacg agttccagac tggtggaatc 180

ggaaacctcc caatcaggaa ctggaacttc gatttcggac tcctcggaac tactctcctc 240

aacctcgatg ctaactacgt ggagactgct aggaacacta tcgattactt cgttgatttc 300

gtggataatg tgtgcatgga tgagatggtt cgtgagtccc agaggaacgg aattgctcca 360

cagtccgatt ccctcaggaa gctctccggt atcaagttca agaggatcaa cttcgataac 420

tcctccgagt acatcgagaa ctggaacctc cagaacagaa ggcagaggac tggattcact 480

ttccacaagc caaacatctt cccatactcc gcttccttca ctctcaacag gtcccagcca 540

gctcacgata acctcatggg aactatgtgg ctcaacgctg gttctgagat ccaggtggca 600

ggattcgatt actcctgcgc tatcaacgct ccagctaaca ctcagcagtt cgagcacatc 660

gttcagctca gaagggtgct cactactgct actatcactc tcctcccaga tgctgagagg 720

ttctccttcc caagggtgat caactccgct gatggtgcta ctacttggta cttcaaccca 780

gtgatcctca ggccaaacaa cgtggaggtg gagttccttc tcaacggaca gatcatcaac 840

acttaccagg ctcgtttcgg aactatcgtg gctaggaact tcgatacaat caggctctcc 900

ttccagctta tgaggccacc aaacatgact ccatccgtgg ctgcactctt cccaaacgca 960

cagccattcg agcaccacgc tactgtggga ctcactctca agatcgagtc cgctgtgtgc 1020

gagtctgtgc tcgctgatgc ttccgagact atgctcgcta acgtgacttc tgtgaggcaa 1080

gagtacgcta tcccagtggg accagtgttt ccaccaggaa tgaactggac tgatctcatc 1140

actaactact ccccatccag agaggataac ctccagaggg tgttcactgt ggcttccatc 1200

cgttccatgc tcgtgaagtg atgactcgag ggatcctcta gagaattcga gctc 1254

<210> 18
<211> 1254
<212> DNA
<213> Nicotiana tabacum

<400> 18

ccatggctta agcctgcgca agcaaggtac ctacacgaga tgagggagag gttctgagag 60

ttcctacgat ccctattcta gcacctccct tgagagatga ggttgcaaag gctagagtag 120

gtcgtcaagt tggtctacta gtagtgatac ttgcctttgc tcaaggtctg accaccttag 180

cctttggagg gttagtcctt gaccttgaag ctaaagcctg aggagccttg atgagaggag 240

ttggagctac gattgatgca cctctgacga tccttgtgat agctaatgaa gcaactaaag 300

cacctattac acacgtacct actctaccaa gcactcaggg tctccttgcc ttaacgaggt 360

gtcaggctaa gggagtcctt cgagaggcca tagttcaagt tctcctagtt gaagctattg 420

aggaggctca tgtagctctt gaccttggag gtcttgtctt ccgtctcctg acctaagtga 480

aaggtgttcg gtttgtagaa gggtatgagg cgaaggaagt gagagttgtc cagggtcggt 540

cgagtgctat tggagtaccc ttgatacacc gagttgcgac caagactcta ggtccaccgt 600

cctaagctaa tgaggacgcg atagttgcga ggtcgattgt gagtcgtcaa gctcgtgtag 660

caagtcgagt cttcccacga gtgatgacga tgatagtgag aggagggtct acgactctcc 720

aagaggaagg gttcccacta gttgaggcga ctaccacgat gatgaaccat gaagttgggt 780

cactaggagt ccggtttgtt gcacctccac ctcaaggaag agttgcctgt ctagtagttg 840

tgaatggtcc gagcaaagcc ttgatagcac cgatccttga agctatgtta gtccgagagg 900

aaggtcgaat actccggtgg tttgtactga ggtaggcacc gacgtgagaa gggtttgcgt 960

gtcggtaagc tcgtggtgcg atgacaccct gagtgagagt tctagctcag gcgacacacg 1020

ctcagacacg agcgactacg aaggctctga tacgagcgat tgcactgaag acactccgtt 1080

ctcatgcgat agggtcaccc tggtcacaaa ggtggtcctt acttgacctg actagagtag 1140

tgattgatga ggggtaggtc tctcctattg gaggtctccc acaagtgaca ccgaaggtag 1200

gcaaggtacg agcacttcac tactgagctc cctaggagat ctcttaagct cgag 1254

<210> 19
<211> 1041
<212> DNA
<213> Nicotiana tabacum

<400> 19
ggtaccgaat tcggacgcgt tcgttgcatg tacggaatcg agtacactac tatcctcact 60

ttcctcatct ccatcgtgct cctcaactac atcctcaagt ccctcacttc cgctatggat 120

ttcatcatct accgtttcct cctcctcatc gtgatcgctt ccccattcgt taagactcag 180

aactacggta tcaacctccc aatcactgga tctatggata ctgcttacgc taactcctcc 240

cagcaagaga ctttcctcac ttccacactc tgcctctact acccaactga ggcatccaca 300

cagatcggag atacagagtg gaaggatact ctctcccagc tcttcctcac taaggatggg 360

ccaactggtt ccgtgtactt caaagagtac actgatatcg cttccttctc catcgatcca 420

cagctctact gcgattacaa cgtggtgctt atgaagtacg attccactct tgagcttgat 480

atgtccgagc ttgctgatct catcctcaac gagtggctct gcaacccaat ggatatcact 540

ctctactact accagcagac tgatgaggct aacaagtgga tctctatggg acagtcctgc 600

actatcaaag tgtgcccact caacactcag actctcggaa tcggatgcat cactactaac 660

actgctactt tcgaggaagt ggctacttcc gagaagctcg tgatcactga tgtggtggat 720

ggtgttaacc acaagctcga tgtgactact aacacatgca caatcaggaa ctgcaagaag 780

ctcggaccaa gggaaaacgt ggctatcatc caagtgggag gttccgatgt gctcgatatc 840

actgctgatc caactactgc tccacagact gagaggatga tgagggtgaa ctggaagaag 900

tggtggcagg ttttctacac tgtggtggat tacatcaacc agatcgttca ggtgatgtcc 960

aagaggtccc gttctctcaa ctccgctgct ttctactacc gtgtgtgatg actcgaggga 1020

tcctctagag aattcgagct c 1041

<210> 20
<211> 1041
<212> DNA
<213> Nicotiana tabacum

<400> 20
ccatggctta agcctgcgca agcaacgtac atgccttagc tcatgtgatg ataggagtga 60

aaggagtaga ggtagcacga ggagttgatg taggagttca gggagtgaag gcgataccta 120

aagtagtaga tggcaaagga ggaggagtag cactagcgaa ggggtaagca attctgagtc 180

ttgatgccat agttggaggg ttagtgacct agatacctat gacgaatgcg attgaggagg 240

gtcgttctct gaaaggagtg aaggtgtgag acggagatga tgggttgact ccgtaggtgt 300

gtctagcctc tatgtctcac cttcctatga gagagggtcg agaaggagtg attccctacc 360

ggttgaccaa ggcacatgaa gtttctcatg tgactatagc gaaggaagag gtagctaggt 420

gtcgagatga cgctaatgtt gcaccacgaa tacttcatgc taaggtgaga actcgaacta 480

tacaggctcg aacgactaga gtaggagttg ctcaccgaga cgttgggtta cctatagtga 540

gagatgatga tggtcgtctg actactccga ttgttcacct agagataccc tgtcaggacg 600

tgatagtttc acacgggtga gttgtgagtc tgagagcctt agcctacgta gtgatgattg 660

tgacgatgaa agctccttca ccgatgaagg ctcttcgagc actagtgact acaccaccta 720

ccacaattgg tgttcgagct acactgatga ttgtgtacgt gttagtcctt gacgttcttc 780

gagcctggtt cccttttgca ccgatagtag gttcaccctc caaggctaca cgagctatag 840

tgacgactag gttgatgacg aggtgtctga ctctcctact actcccactt gaccttcttc 900

accaccgtcc aaaagatgtg acaccaccta atgtagttgg tctagcaagt ccactacagg 960

ttctccaggg caagagagtt gaggcgacga aagatgatgg cacacactac tgagctccct 1020

aggagatctc ttaagctcga g                                                1041

<210>  21
<211>  48
<212>  DNA
<213>  Artificial sequence

<220>
<223>  Primer IF-WA_VP2(opt).s1+3c

<400>  21
aaatttgtcg ggcccatggc ataccggaag agaggagcaa agcgcgaa                      48

<210>  22
<211>  50
<212>  DNA
<213>  Artificial sequence

<220>
<223>  Primer IF-WA_VP2(opt).s1-4r

<400>  22
actaaagaaa ataggccttt aaagctcgtt cattattcgc atattgtcga                    50

<210>  23
<211>  4903
<212>  DNA
<213>  Artificial sequence

<220>
<223>  Construct 1191

<400>  23
tggcaggata tattgtggtg taaacaaatt gacgcttaga caacttaata acacattgcg        60

gacgttttta atgtactgaa ttaacgccga atcccgggct ggtatattta tatgttgtca       120

aataactcaa aaaccataaa agtttaagtt agcaagtgtg tacattttta cttgaacaaa       180

aatattcacc tactactgtt ataaatcatt attaaacatt agagtaaaga aatatggatg       240

ataagaacaa gagtagtgat attttgacaa caattttgtt gcaacatttg agaaaatttt       300

gttgttctct cttttcattg gtcaaaaaca atagagagag aaaaaggaag agggagaata       360

aaaacataat gtgagtatga gagagaaagt tgtacaaaag ttgtaccaaa atagttgtac       420

aaatatcatt gaggaatttg acaaaagcta cacaaataag ggttaattgc tgtaaataaa       480

taaggatgac gcattagaga gatgtaccat tagagaattt ttggcaagtc attaaaaaga       540

aagaataaat tatttttaaa attaaaagtt gagtcatttg attaaacatg tgattattta       600

atgaattgat gaaagagttg gattaaagtt gtattagtaa ttagaatttg gtgtcaaatt       660

taatttgaca tttgatcttt tcctatatat tgccccatag agtcagttaa ctcattttta       720

tatttcatag atcaaataag agaaataacg gtatattaat ccctccaaaa aaaaaaaacg       780

gtatatttac taaaaaatct aagccacgta ggaggataac aggatccccg taggaggata       840

acatccaatc caaccaatca caacaatcct gatgagataa cccactttaa gcccacgcat 900

ctgtggcaca tctacattat ctaaatcaca cattcttcca cacatctgag ccacacaaaa 960

accaatccac atctttatca cccattctat aaaaaatcac actttgtgag tctacacttt 1020

gattcccttc aaacacatac aaagagaaga gactaattaa ttaattaatc atcttgagag 1080

aaaatggaac gagctataca aggaaacgac gctagggaac aagctaacag tgaacgttgg 1140

gatggaggat caggaggtac cacttctccc ttcaaacttc ctgacgaaag tccgagttgg 1200

actgagtggc ggctacataa cgatgagacg aattcgaatc aagataatcc ccttggtttc 1260

aaggaaagct ggggtttcgg gaaagttgta tttaagagat atctcagata cgacaggacg 1320

gaagcttcac tgcacagagt ccttggatct tggacgggag attcggttaa ctatgcagca 1380

tctcgatttt tcggtttcga ccagatcgga tgtacctata gtattcggtt tcgaggagtt 1440

agtatcaccg tttctggagg gtcgcgaact cttcagcatc tctgtgagat ggcaattcgg 1500

tctaagcaag aactgctaca gcttgcccca atcgaagtgg aaagtaatgt atcaagagga 1560

tgccctgaag gtactcaaac cttcgaaaaa gaaagcgagt aagttaaaat gcttcttcgt 1620

ctcctattta taatatggtt tgttattgtt aattttgttc ttgtagaaga gcttaattaa 1680

tcgttgttgt tatgaaatac tatttgtatg agatgaactg gtgtaatgta attcatttac 1740

ataagtggag tcagaatcag aatgtttcct ccataactaa ctagacatga agacctgccg 1800

cgtacaattg tcttatattt gaacaactaa aattgaacat cttttgccac aactttataa 1860

gtggttaata tagctcaaat atatggtcaa gttcaataga ttaataatgg aaatatcagt 1920

tatcgaaatt cattaacaat caacttaacg ttattaacta ctaattttat atcatcccct 1980

ttgataaatg atagtacacc aattaggaag gagcatgctc gcctaggaga ttgtcgtttc 2040

ccgccttcag tttgcaagct gctctagccg tgtagccaat acgcaaaccg cctctccccg 2100

cgcgttggga attactagcg cgtgtcgaca agcttgcatg ccggtcaaca tggtggagca 2160

cgacacactt gtctactcca aaaatatcaa agatacagtc tcagaagacc aaagggcaat 2220

tgagactttt caacaaaggg taatatccgg aaacctcctc ggattccatt gcccagctat 2280

ctgtcacttt attgtgaaga tagtggaaaa ggaaggtggc tcctacaaat gccatcattg 2340

cgataaagga aaggccatcg ttgaagatgc ctctgccgac agtggtccca aagatggacc 2400

cccacccacg aggagcatcg tggaaaaaga agacgttcca accacgtctt caaagcaagt 2460

ggattgatgt gataacatgg tggagcacga cacacttgtc tactccaaaa atatcaaaga 2520

tacagtctca gaagaccaaa gggcaattga gacttttcaa caaagggtaa tatccggaaa 2580

cctcctcgga ttccattgcc cagctatctg tcactttatt gtgaagatag tggaaaagga 2640

aggtggctcc tacaaatgcc atcattgcga taaaggaaag gccatcgttg aagatgcctc 2700

tgccgacagt ggtcccaaag atggaccccc acccacgagg agcatcgtgg aaaaagaaga 2760

cgttccaacc acgtcttcaa agcaagtgga ttgatgtgat atctccactg acgtaaggga 2820

tgacgcacaa tcccactatc cttcgcaaga cccttcctct atataaggaa gttcatttca 2880

tttggagagg tattaaaatc ttaataggtt ttgataaaag cgaacgtggg gaaacccgaa 2940

ccaaaccttc ttctaaactc tctctcatct ctcttaaagc aaacttctct cttgtctttc 3000

ttgcgtgagc gatcttcaac gttgtcagat cgtgcttcgg caccagtaca acgttttctt 3060

tcactgaagc gaaatcaaag atctctttgt ggacacgtag tgcggcgcca ttaaataacg 3120

tgtacttgtc ctattcttgt cggtgtggtc ttgggaaaag aaagcttgct ggaggctgct 3180

gttcagcccc atacattact tgttacgatt ctgctgactt tcggcgggtg caatatctct 3240

acttctgctt gacgaggtat tgttgcctgt acttctttct tcttcttctt gctgattggt 3300

tctataagaa atctagtatt ttctttgaaa cagagttttc ccgtggtttt cgaacttgga 3360

gaaagattgt taagcttctg tatattctgc ccaaatttgt cgggcccgcg gatggcgaaa 3420

aacgttgcga ttttcggctt attgttttct cttcttgtgt tggttccttc tcagatcttc 3480

gcctgcaggc tcctcagcca aaacgacacc cccatctgtc tatccactgg cccctggatc 3540

tgctgcccaa actaactcca tggtgaccct gggatgcctg gtcaagggct atttccctga 3600

gccagtgaca gtgacctgga actctggatc cctgtccagc ggtgtgcaca ccttcccagc 3660

tgtcctgcag tctgacctct acactctgag cagctcagtg actgtcccct ccagcacctg 3720

gcccagcgag accgtcacct gcaacgttgc ccacccggcc agcagcacca aggtggacaa 3780

gaaaattgtg cccagggatt gtggttgtaa gccttgcata tgtacagtcc cagaagtatc 3840

atctgtcttc atcttccccc caaagcccaa ggatgtgctc accattactc tgactcctaa 3900

ggtcacgtgt gttgtggtag acatcagcaa ggatgatccc gaggtccagt tcagctggtt 3960

tgtagatgat gtggaggtgc acacagctca gacgcaaccc cgggaggagc agttcaacag 4020

cactttccgc tcagtcagtg aacttcccat catgcaccag gactggctca atggcaagga 4080

gcgatcgctc accatcacca tcaccatcac catcaccatt aaaggcctat tttctttagt 4140

ttgaatttac tgttattcgg tgtgcatttc tatgtttggt gagcggtttt ctgtgctcag 4200

agtgtgttta ttttatgtaa tttaatttct ttgtgagctc ctgtttagca ggtcgtccct 4260

tcagcaagga cacaaaaaga ttttaatttt attaaaaaaa aaaaaaaaaa agaccgggaa 4320

ttcgatatca agcttatcga cctgcagatc gttcaaacat ttggcaataa agtttcttaa 4380

gattgaatcc tgttgccggt cttgcgatga ttatcatata atttctgttg aattacgtta 4440

agcatgtaat aattaacatg taatgcatga cgttatttat gagatgggtt tttatgatta 4500

gagtcccgca attatacatt taatacgcga tagaaaacaa aatatagcgc gcaaactagg 4560

ataaattatc gcgcgcggtg tcatctatgt tactagatct ctagagtctc aagcttggcg 4620

cgcccacgtg actagtggca ctggccgtcg ttttacaacg tcgtgactgg gaaaaccctg 4680

gcgttaccca acttaatcgc cttgcagcac atcccccttt cgccagctgg cgtaatagcg 4740

aagaggcccg caccgatcgc ccttcccaac agttgcgcag cctgaatggc gaatgctaga 4800

gcagcttgag cttggatcag attgtcgttt cccgccttca gtttaaacta tcagtgtttg 4860

acaggatata ttggcgggta aacctaagag aaaagagcgt tta 4903

<210> 24
<211> 2721
<212> DNA
<213> Artificial sequence

<220>
<223> Expression cassette number 1733

<400> 24
gtcaacatgg tggagcacga cacacttgtc tactccaaaa atatcaaaga tacagtctca 60

gaagaccaaa gggcaattga gacttttcaa caaagggtaa tatccggaaa cctcctcgga 120

ttccattgcc cagctatctg tcactttatt gtgaagatag tggaaaagga aggtggctcc 180

tacaaatgcc atcattgcga taaaggaaag gccatcgttg aagatgcctc tgccgacagt 240

ggtcccaaag atggaccccc acccacgagg agcatcgtgg aaaaagaaga cgttccaacc 300

acgtcttcaa agcaagtgga ttgatgtgat aacatggtgg agcacgacac acttgtctac 360

tccaaaaata tcaaagatac agtctcagaa gaccaaaggg caattgagac ttttcaacaa 420

agggtaatat ccggaaacct cctcggattc cattgcccag ctatctgtca ctttattgtg 480

aagatagtgg aaaaggaagg tggctcctac aaatgccatc attgcgataa aggaaaggcc 540

atcgttgaag atgcctctgc cgacagtggt cccaaagatg gacccccacc cacgaggagc 600

atcgtggaaa aagaagacgt tccaaccacg tcttcaaagc aagtggattg atgtgatatc 660

tccactgacg taagggatga cgcacaatcc cactatcctt cgcaagaccc ttcctctata 720

taaggaagtt catttcattt ggagaggtat taaaatctta ataggttttg ataaaagcga 780

acgtggggaa acccgaacca aaccttcttc taaactctct ctcatctctc ttaaagcaaa 840

cttctctctt gtctttcttg cgtgagcgat cttcaacgtt gtcagatcgt gcttcggcac 900

cagtacaacg ttttctttca ctgaagcgaa atcaaagatc tctttgtgga cacgtagtgc 960

ggcgccatta aataacgtgt acttgtccta ttcttgtcgg tgtggtcttg ggaaaagaaa 1020

gcttgctgga ggctgctgtt cagccccata cattacttgt tacgattctg ctgactttcg 1080

gcgggtgcaa tatctctact tctgcttgac gaggtattgt tgcctgtact tctttcttct 1140

tcttcttgct gattggttct ataagaaatc tagtattttc tttgaaacag agttttcccg 1200

tggttttcga acttggagaa agattgttaa gcttctgtat attctgccca aatttgtcgg 1260

gcccatgtac ggcatcgagt atacaacaat tttaattttc ctgatttcca tcattctgtt 1320

aaactacatc cttaagtccg tgaccagaat tatggattat attatctatc gtagcctcct 1380

catctacgtg gccctttttg ccctgaccag ggcccagaac tatggcctga acttaccaat 1440

caccggttca atggataccg tttacgctaa ttccactcaa gaggggatat ttctgacaag 1500

taccctgtgc ctgtattatc caacagaagc ctctacccag atcaatgatg gggagtggaa 1560

ggatagtctc tcacagatgt tcctaaccaa gggctggccc accggttccg tctacttcaa 1620

ggaatactct agtattgtcg acttctcagt tgacccccag ctttattgcg actacaacct 1680

ggtacttatg aaatacgacc agaacctgga gctggatatg tccgagctgg ctgacctgat 1740

cctcaatgag tggctgtgca accccatgga catcacatta tattactacc agcagtctgg 1800

agaatccaac aagtggatca gtatgggctc aagttgcacc gtgaaggtgt gtcccttgaa 1860

cacccaaatg ctgggcattg gttgtcagac aactaatgtg gattcgtttg aaatggtagc 1920

cgaaaacgag aagctggcta tagtggacgt agtcgatggg attaaccaca agatcaatct 1980

gactaccacc acttgtacca tcagaaactg taaaaagctc ggcccccggg agaacgtcgc 2040

cgtgatccag gtggggggga gcaatgtgct cgacattact gccgacccta ccaccaatcc 2100

acagacggaa cggatgatga gagtcaactg gaagaaatgg tggcaggtct tttataccat 2160

tgtggactac attaaccaga ttgtgcaagt catgagtaaa cggtccagat ccctgaactc 2220

agcagccttc tattatcgcg tttagaggcc tattttcttt agtttgaatt tactgttatt 2280

cggtgtgcat ttctatgttt ggtgagcggt tttctgtgct cagagtgtgt ttattttatg 2340

taatttaatt tctttgtgag ctcctgttta gcaggtcgtc ccttcagcaa ggacacaaaa 2400

agattttaat tttattaaaa aaaaaaaaaa aaaagaccgg gaattcgata tcaagcttat 2460

cgacctgcag atcgttcaaa catttggcaa taaagtttct taagattgaa tcctgttgcc 2520

ggtcttgcga tgattatcat ataatttctg ttgaattacg ttaagcatgt aataattaac 2580

atgtaatgca tgacgttatt tatgagatgg gtttttatga ttagagtccc gcaattatac 2640

atttaatacg cgatagaaaa caaaatatag cgcgcaaact aggataaatt atcgcgcgcg 2700

gtgtcatcta tgttactaga t 2721

<210> 25
<211> 890
<212> PRT
<213> Rotavirus

<400> 25

Met Ala Tyr Arg Lys Arg Gly Ala Lys Arg Glu Asn Leu Pro Gln Gln
1               5                   10                  15

Asn Glu Arg Leu Gln Glu Lys Glu Ile Glu Lys Asp Val Asp Val Thr

20                  25                  30

Met Glu Asn Lys Asn Asn Asn Arg Lys Gln Gln Leu Ser Asp Lys Val
        35                  40                  45

Leu Ser Gln Lys Glu Glu Ile Ile Thr Asp Ala Gln Asp Asp Ile Lys
    50                  55                  60

Ile Ala Gly Glu Ile Lys Lys Ser Ser Lys Glu Glu Ser Lys Gln Leu
65                  70                  75                  80

Leu Glu Ile Leu Lys Thr Lys Glu Asp His Gln Lys Glu Ile Gln Tyr
                85                  90                  95

Glu Ile Leu Gln Lys Thr Ile Pro Thr Phe Glu Ser Lys Glu Ser Ile
            100                 105                 110

Leu Lys Lys Leu Glu Asp Ile Arg Pro Glu Gln Ala Lys Lys Gln Met
        115                 120                 125

Lys Leu Phe Arg Ile Phe Glu Pro Lys Gln Leu Pro Ile Tyr Arg Ala
    130                 135                 140

Asn Gly Glu Lys Glu Leu Arg Asn Arg Trp Tyr Trp Lys Leu Lys Lys
145                 150                 155                 160

Asp Thr Leu Pro Asp Gly Asp Tyr Asp Val Arg Glu Tyr Phe Leu Asn
                165                 170                 175

Leu Tyr Asp Gln Ile Leu Ile Glu Met Pro Asp Tyr Leu Leu Leu Lys
            180                 185                 190

Asp Met Ala Val Glu Asn Lys Asn Ser Arg Asp Ala Gly Lys Val Val
        195                 200                 205

Asp Ser Glu Thr Ala Asn Ile Cys Asp Ala Ile Phe Gln Asp Glu Glu
    210                 215                 220

Thr Glu Gly Val Val Arg Arg Phe Ile Ala Asp Met Arg Gln Gln Val
225                 230                 235                 240

Gln Ala Asp Arg Asn Ile Val Asn Tyr Pro Ser Ile Leu His Pro Ile
                245                 250                 255

Asp His Ala Phe Asn Glu Tyr Phe Leu Asn His Gln Leu Val Glu Pro
            260                 265                 270

Leu Asn Asn Glu Ile Ile Phe Asn Tyr Ile Pro Glu Arg Ile Arg Asn
        275                 280                 285

Asp Val Asn Tyr Ile Leu Asn Met Asp Met Asn Leu Pro Ser Thr Ala
    290                 295                 300

Arg Tyr Ile Arg Pro Asn Leu Leu Gln Asp Arg Leu Asn Leu His Asp
305                 310                 315                 320

Asn Phe Glu Ser Leu Trp Asp Thr Ile Thr Thr Ser Asn Tyr Ile Leu
                325                 330                 335

Ala Arg Ser Val Val Pro Asp Leu Lys Glu Lys Glu Leu Val Ser Thr
            340                 345                 350

Glu Ala Gln Ile Gln Lys Met Ser Gln Asp Leu Gln Leu Glu Ala Leu
        355                 360                 365

Thr Ile Gln Ser Glu Thr Gln Phe Leu Ala Gly Ile Asn Ser Gln Ala
    370                 375                 380

Ala Asn Asp Cys Phe Lys Thr Leu Ile Ala Ala Met Leu Ser Gln Arg
385                 390                 395                 400

Thr Met Ser Leu Asp Phe Val Thr Thr Asn Tyr Met Ser Leu Ile Ser
                405                 410                 415

Gly Met Trp Leu Leu Thr Val Ile Pro Asn Asp Met Phe Leu Arg Glu
            420                 425                 430

Ser Leu Val Ala Cys Glu Leu Ala Ile Ile Asn Thr Ile Val Tyr Pro
        435                 440                 445

Ala Phe Gly Met Gln Arg Met His Tyr Arg Asn Gly Asp Pro Gln Thr
    450                 455                 460

Pro Phe Gln Ile Ala Glu Gln Gln Ile Gln Asn Phe Gln Val Ala Asn
465                 470                 475                 480

Trp Leu His Phe Ile Asn Asn Asn Arg Phe Arg Gln Val Val Ile Asp
                485                 490                 495

Gly Val Leu Asn Gln Thr Leu Asn Asp Asn Ile Arg Asn Gly Gln Val
            500                 505                 510

Ile Asn Gln Leu Met Glu Ala Leu Met Gln Leu Ser Arg Gln Gln Phe
        515                 520                 525

Pro Thr Met Pro Val Asp Tyr Lys Arg Ser Ile Gln Arg Gly Ile Leu
    530                 535                 540

Leu Leu Ser Asn Arg Leu Gly Gln Leu Val Asp Leu Thr Arg Leu Val
545                 550                 555                 560

Ser Tyr Asn Tyr Glu Thr Leu Met Ala Cys Val Thr Met Asn Met Gln
                565                 570                 575

His Val Gln Thr Leu Thr Thr Glu Lys Leu Gln Leu Thr Ser Val Thr
            580                 585                 590

Ser Leu Cys Met Leu Ile Gly Asn Thr Thr Val Ile Pro Ser Pro Gln
        595                 600                 605

Thr Leu Phe His Tyr Tyr Asn Ile Asn Val Asn Phe His Ser Asn Tyr
    610                 615                 620

Asn Glu Arg Ile Asn Asp Ala Val Ala Ile Ile Thr Ala Ala Asn Arg
625                 630                 635                 640

Leu Asn Leu Tyr Gln Lys Lys Met Lys Ser Ile Val Glu Asp Phe Leu
                645                 650                 655

Lys Arg Leu Gln Ile Phe Asp Val Pro Arg Val Pro Asp Asp Gln Met
            660                 665                 670

Tyr Arg Leu Arg Asp Arg Leu Arg Leu Leu Pro Val Glu Arg Arg Arg
        675                 680                 685

Leu Asp Ile Phe Asn Leu Ile Leu Met Asn Met Glu Gln Ile Glu Arg
    690                 695                 700

Ala Ser Asp Lys Ile Ala Gln Gly Val Ile Ile Ala Tyr Arg Asp Met
705                 710                 715                 720

Gln Leu Glu Arg Asp Glu Met Tyr Gly Tyr Val Asn Ile Ala Arg Asn
                725                 730                 735

Leu Asp Gly Tyr Gln Gln Ile Asn Leu Glu Glu Leu Met Arg Thr Gly
            740                 745                 750

Asp Tyr Gly Gln Ile Thr Asn Met Leu Leu Asn Asn Gln Pro Val Ala
        755                 760                 765

Leu Val Gly Ala Leu Pro Phe Val Thr Asp Ser Ser Val Ile Ser Leu
    770                 775                 780

Ile Ala Lys Leu Asp Ala Thr Val Phe Ala Gln Ile Val Lys Leu Arg
785                 790                 795                 800

Lys Val Asp Thr Leu Lys Pro Ile Leu Tyr Lys Ile Asn Ser Asp Ser
                805                 810                 815

Asn Asp Phe Tyr Leu Val Ala Asn Tyr Asp Trp Ile Pro Thr Ser Thr
            820                 825                 830

Thr Lys Val Tyr Lys Gln Val Pro Gln Pro Phe Asp Phe Arg Ala Ser
        835                 840                 845

Met His Met Leu Thr Ser Asn Leu Thr Phe Thr Val Tyr Ser Asp Leu
    850                 855                 860

Leu Ser Phe Val Ser Ala Asp Thr Val Glu Pro Ile Asn Ala Val Ala
865                 870                 875                 880

Phe Asp Asn Met Arg Ile Met Asn Glu Leu
                885                 890

<210>  26
<211>  6745
<212>  DNA
<213>  Artificial sequence

<220>
<223>  Construct 193

<400>  26
tggcaggata tattgtggtg taaacaaatt gacgcttaga caacttaata acacattgcg      60

gacgttttta atgtactgaa ttaacgccga atcccgggct ggtatattta tatgttgtca     120

aataactcaa aaaccataaa agtttaagtt agcaagtgtg tacattttta cttgaacaaa     180

aatattcacc tactactgtt ataaatcatt attaaacatt agagtaaaga aatatggatg     240

ataagaacaa gagtagtgat attttgacaa caattttgtt gcaacatttg agaaaatttt     300

gttgttctct cttttcattg gtcaaaaaca atagagagag aaaaaggaag agggagaata     360

aaaacataat gtgagtatga gagagaaagt tgtacaaaag ttgtaccaaa atagttgtac     420

aaatatcatt gaggaatttg acaaaagcta cacaaataag ggttaattgc tgtaaataaa     480

taaggatgac gcattagaga gatgtaccat tagagaattt ttggcaagtc attaaaaaga     540

aagaataaat tatttttaaa attaaaagtt gagtcatttg attaaacatg tgattattta     600

atgaattgat gaaagagttg gattaaagtt gtattagtaa ttagaatttg gtgtcaaatt     660

taatttgaca tttgatcttt tcctatatat tgccccatag agtcagttaa ctcattttta     720

tatttcatag atcaaataag agaaataacg gtatattaat ccctccaaaa aaaaaaaacg 780

gtatatttac taaaaaatct aagccacgta ggaggataac aggatccccg taggaggata 840

acatccaatc caaccaatca caacaatcct gatgagataa cccactttaa gcccacgcat 900

ctgtggcaca tctacattat ctaaatcaca cattcttcca cacatctgag ccacacaaaa 960

accaatccac atctttatca cccattctat aaaaaatcac actttgtgag tctacacttt 1020

gattcccttc aaacacatac aaagagaaga gactaattaa ttaattaatc atcttgagag 1080

aaaatggaac gagctataca aggaaacgac gctagggaac aagctaacag tgaacgttgg 1140

gatggaggat caggaggtac cacttctccc ttcaaacttc ctgacgaaag tccgagttgg 1200

actgagtggc ggctacataa cgatgagacg aattcgaatc aagataatcc ccttggtttc 1260

aaggaaagct ggggtttcgg gaaagttgta tttaagagat atctcagata cgacaggacg 1320

gaagcttcac tgcacagagt ccttggatct tggacgggag attcggttaa ctatgcagca 1380

tctcgatttt tcggtttcga ccagatcgga tgtacctata gtattcggtt tcgaggagtt 1440

agtatcaccg tttctggagg gtcgcgaact cttcagcatc tctgtgagat ggcaattcgg 1500

tctaagcaag aactgctaca gcttgcccca atcgaagtgg aaagtaatgt atcaagagga 1560

tgccctgaag gtactcaaac cttcgaaaaa gaaagcgagt aagttaaaat gcttcttcgt 1620

ctcctattta taatatggtt tgttattgtt aattttgttc ttgtagaaga gcttaattaa 1680

tcgttgttgt tatgaaatac tatttgtatg agatgaactg gtgtaatgta attcatttac 1740

ataagtggag tcagaatcag aatgtttcct ccataactaa ctagacatga agacctgccg 1800

cgtacaattg tcttatattt gaacaactaa aattgaacat cttttgccac aactttataa 1860

gtggttaata tagctcaaat atatggtcaa gttcaataga ttaataatgg aaatatcagt 1920

tatcgaaatt cattaacaat caacttaacg ttattaacta ctaattttat atcatcccct 1980

ttgataaatg atagtacacc aattaggaag gagcatgctc gcctaggaga ttgtcgtttc 2040

ccgccttcag tttgcaagct gctctagccg tgtagccaat acgcaaaccg cctctccccg 2100

cgcgttggga attactagcg cgtgtcgaga cgcgttgttg ttgtgactcc gaggggttgc 2160

ctcaaactct atcttataac cggcgtggag gcatggaggc aggggtattt tggtcatttt 2220

aatagatagt ggaaaatgac gtggaattta cttaaagacg aagtctttgc gacaaggggg 2280

ggcccacgcc gaatttaata ttaccggcgt ggccccccct tatcgcgagt gctttagcac 2340

gagcggtcca gatttaaagt agaaaatttc ccgcccacta gggttaaagg tgttcacact 2400

ataaaagcat atacgatgtg atggtatttg gtcgacaagc ttgcatgccg gtcaacatgg 2460

tggagcacga cacacttgtc tactccaaaa atatcaaaga tacagtctca gaagaccaaa 2520

gggcaattga gacttttcaa caaagggtaa tatccggaaa cctcctcgga ttccattgcc 2580

cagctatctg tcactttatt gtgaagatag tggaaaagga aggtggctcc tacaaatgcc 2640

atcattgcga taaaggaaag gccatcgttg aagatgcctc tgccgacagt ggtcccaaag 2700

atggaccccc acccacgagg agcatcgtgg aaaaagaaga cgttccaacc acgtcttcaa 2760

agcaagtgga ttgatgtgat aacatggtgg agcacgacac acttgtctac tccaaaaata 2820

tcaaagatac agtctcagaa gaccaaaggg caattgagac ttttcaacaa agggtaatat 2880

ccggaaacct cctcggattc cattgcccag ctatctgtca ctttattgtg aagatagtgg 2940

aaaaggaagg tggctcctac aaatgccatc attgcgataa aggaaaggcc atcgttgaag 3000

atgcctctgc cgacagtggt cccaaagatg gacccccacc cacgaggagc atcgtggaaa 3060

aagaagacgt tccaaccacg tcttcaaagc aagtggattg atgtgatatc tccactgacg 3120

taagggatga cgcacaatcc cactatcctt cgcaagaccc ttcctctata taaggaagtt 3180

catttcattt ggagaggtat taaaatctta ataggttttg ataaaagcga acgtggggaa 3240

acccgaacca aaccttcttc taaactctct ctcatctctc ttaaagcaaa cttctctctt 3300

gtctttcttg cgtgagcgat cttcaacgtt gtcagatcgt gcttcggcac cagtacaacg 3360

ttttctttca ctgaagcgaa atcaaagatc tctttgtgga cacgtagtgc ggcgccatta 3420

aataacgtgt acttgtccta ttcttgtcgg tgtggtcttg ggaaaagaaa gcttgctgga 3480

ggctgctgtt cagccccata cattacttgt tacgattctg ctgactttcg gcgggtgcaa 3540

tatctctact tctgcttgac gaggtattgt tgcctgtact tctttcttct tcttcttgct 3600

gattggttct ataagaaatc tagtattttc tttgaaacag agttttcccg tggttttcga 3660

acttggagaa agattgttaa gcttctgtat attctgccca aatttgtcgg gcccgcggat 3720

ggcgaaaaac gttgcgattt tcggcttatt gttttctctt cttgtgttgg ttccttctca 3780

gatcttcgcc tgcaggctcc tcagccaaaa cgacaccccc atctgtctat ccactggccc 3840

ctggatctgc tgcccaaact aactccatgg tgaccctggg atgcctggtc aagggctatt 3900

tccctgagcc agtgacagtg acctggaact ctggatccct gtccagcggt gtgcacacct 3960

tcccagctgt cctgcagtct gacctctaca ctctgagcag ctcagtgact gtcccctcca 4020

gcacctggcc cagcgagacc gtcacctgca acgttgccca cccggccagc agcaccaagg 4080

tggacaagaa aattgtgccc agggattgtg gttgtaagcc ttgcatatgt acagtcccag 4140

aagtatcatc tgtcttcatc ttccccccaa agcccaagga tgtgctcacc attactctga 4200

ctcctaaggt cacgtgtgtt gtggtagaca tcagcaagga tgatcccgag gtccagttca 4260

gctggtttgt agatgatgtg gaggtgcaca cagctcagac gcaaccccgg gaggagcagt 4320

tcaacagcac tttccgctca gtcagtgaac ttcccatcat gcaccaggac tggctcaatg 4380

gcaaggagcg atcgctcacc atcaccatca ccatcaccat caccattaaa ggcctatttt 4440

ctttagtttg aatttactgt tattcggtgt gcatttctat gtttggtgag cggttttctg 4500

tgctcagagt gtgtttattt tatgtaattt aatttctttg tgagctcctg tttagcaggt 4560

cgtcccttca gcaaggacac aaaaagattt taattttatt aaaaaaaaaa aaaaaaaaga 4620

ccgggaattc gatatcaagc ttatcgacct gcagatcgtt caaacatttg gcaataaagt 4680

ttcttaagat tgaatcctgt tgccggtctt gcgatgatta tcatataatt tctgttgaat 4740

tacgttaagc atgtaataat taacatgtaa tgcatgacgt tatttatgag atgggttttt 4800

atgattagag tcccgcaatt atacatttaa tacgcgatag aaaacaaaat atagcgcgca 4860

aactaggata aattatcgcg cgcggtgtca tctatgttac tagatctcta gagtctcaag 4920

cttggcgcgc cataaaatga ttattttatg aatatatttc attgtgcaag tagatagaaa 4980

ttacatatgt tacataacac acgaaataaa caaaaaaaga caatccaaaa acaaacaccc 5040

caaaaaaaat aatcacttta gataaactcg tatgaggaga ggcacgttca gtgactcgac 5100

gattcccgag caaaaaaagt ctccccgtca cacatatagt gggtgacgca attatcttta 5160

aagtaatcct tctgttgact tgtcattgat aacatccagt cttcgtcagg attgcaaaga 5220

attatagaag ggatcccacc ttttattttc ttcttttttc catatttagg gttgacagtg 5280

aaatcagact ggcaacctat taattgcttc cacaatggga cgaacttgaa ggggatgtcg 5340

tcgatgatat tataggtggc gtgttcatcg tagttggtga aatcgatggt accgttccaa 5400

tagttgtgtc gtccgagact tctagcccag gtggtctttc cggtacgagt tggtccgcag 5460

atgtagaggc tggggtgtcg gattccattc cttccattgt cctggttaaa tcggccatcc 5520

attcaaggtc agattgagct tgttggtatg agacaggatg tatgtaagta taagcgtcta 5580

tgcttacatg gtatagatgg gtttccctcc aggagtgtag atcttcgtgg cagcgaagat 5640

ctgattctgt gaagggcgac acatacggtt caggttgtgg agggaataat ttgttggctg 5700

aatattccag ccattgaagt tttgttgccc attcatgagg gaattcttcc ttgatcatgt 5760

caagatattc ctccttagac gttgcagtct ggataatagt tctccatcgt gcgtcagatt 5820

tgcgaggaga gaccttatga tctcggaaat ctcctctggt tttaatatct ccgtcctttg 5880

atatgtaatc aaggacttgt ttagagtttc tagctggctg gatattaggg tgatttcctt 5940

caaaatcgaa aaaagaagga tccctaatac aaggtttttt atcaagctgg agaagagcat 6000

gatagtgggt agtgccatct tgatgaagct cagaagcaac accaaggaag aaaataagaa 6060

aaggtgtgag tttctcccag agaaactgga ataaatcatc tctttgagat gagcacttgg 6120

gataggtaag gaaaacatat ttagattgga gtctgaagtt cttactagca gaaggcatgt 6180

tgttgtgact ccgaggggtt gcctcaaact ctatcttata accggcgtgg aggcatggag 6240

gcaggggtat tttggtcatt ttaatagata gtggaaaatg acgtggaatt tacttaaaga 6300

cgaagtcttt gcgacaaggg ggggcccacg ccgaatttaa tattaccggc gtggcccccc 6360

cttatcgcga gtgctttagc acgagcggtc cagatttaaa gtagaaaatt tcccgcccac 6420

tagggttaaa ggtgttcaca ctataaaagc atatacgatg tgatggtatt tgactagtgg 6480

cactggccgt cgttttacaa cgtcgtgact gggaaaaccc tggcgttacc caacttaatc 6540

gccttgcagc acatccccct ttcgccagct ggcgtaatag cgaagaggcc cgcaccgatc 6600

gcccttccca acagttgcgc agcctgaatg gcgaatgcta gagcagcttg agcttggatc 6660

agattgtcgt ttcccgcctt cagtttaaac tatcagtgtt tgacaggata tattggcggg 6720

taaacctaag agaaaagagc gttta 6745

<210> 27
<211> 4413
<212> DNA
<213> Artificial sequence

<220>
<223> Expression cassette number 1710

<400> 27
gtcaacatgg tggagcacga cacacttgtc tactccaaaa atatcaaaga tacagtctca 60

gaagaccaaa gggcaattga gacttttcaa caaagggtaa tatccggaaa cctcctcgga 120

ttccattgcc cagctatctg tcactttatt gtgaagatag tggaaaagga aggtggctcc 180

tacaaatgcc atcattgcga taaaggaaag gccatcgttg aagatgcctc tgccgacagt 240

ggtcccaaag atggaccccc acccacgagg agcatcgtgg aaaaagaaga cgttccaacc 300

acgtcttcaa agcaagtgga ttgatgtgat aacatggtgg agcacgacac acttgtctac 360

tccaaaaata tcaaagatac agtctcagaa gaccaaaggg caattgagac ttttcaacaa 420

agggtaatat ccggaaacct cctcggattc cattgcccag ctatctgtca ctttattgtg 480

aagatagtgg aaaaggaagg tggctcctac aaatgccatc attgcgataa aggaaaggcc 540

atcgttgaag atgcctctgc cgacagtggt cccaaagatg gacccccacc cacgaggagc 600

atcgtggaaa aagaagacgt tccaaccacg tcttcaaagc aagtggattg atgtgatatc 660

tccactgacg taagggatga cgcacaatcc cactatcctt cgcaagaccc ttcctctata 720

taaggaagtt catttcattt ggagaggtat taaaatctta ataggttttg ataaaagcga 780

acgtggggaa acccgaacca aaccttcttc taaactctct ctcatctctc ttaaagcaaa 840

cttctctctt gtctttcttg cgtgagcgat cttcaacgtt gtcagatcgt gcttcggcac 900

cagtacaacg ttttctttca ctgaagcgaa atcaaagatc tctttgtgga cacgtagtgc 960

ggcgccatta aataacgtgt acttgtccta ttcttgtcgg tgtggtcttg ggaaaagaaa 1020

gcttgctgga ggctgctgtt cagccccata cattacttgt tacgattctg ctgactttcg 1080

gcgggtgcaa tatctctact tctgcttgac gaggtattgt tgcctgtact tctttcttct 1140

tcttcttgct gattggttct ataagaaatc tagtattttc tttgaaacag agttttcccg 1200

tggttttcga acttggagaa agattgttaa gcttctgtat attctgccca aatttgtcgg 1260

gcccatggca taccggaaga gaggagcaaa gcgcgaaaac ctgccgcaac agaacgagag 1320

actgcaagaa aaagagatag agaaagatgt cgacgtaaca atggaaaaca agaataacaa 1380

taggaaacaa cagctgtccg acaaagttct gtcccagaag gaggaaatta tcactgacgc 1440

ccaggacgat attaaaattg ccggagaaat aaagaagagc tcgaaagaag aatctaaaca 1500

gctgctcgaa attctgaaaa caaaagaaga ccatcagaaa gagattcaat atgaaatttt 1560

gcaaaaaaca atacctacat ttgagtccaa agaaagtatc ctcaagaagc ttgaagacat 1620

aagaccggag caggcaaaaa aacagatgaa actctttcgc attttcgagc caaaacagct 1680

ccctatatat cgcgccaatg gcgagaagga gctacgcaac cggtggtact ggaagttgaa 1740

aaaagacacc ctgccagatg gagattatga cgtccgggag tatttcctca atctctatga 1800

tcagatcctc atcgaaatgc cggactatct gctcctcaag gacatggccg tggagaacaa 1860

aaatagcaga gacgccggca aagttgtcga ctctgagact gccaatattt gtgatgccat 1920

cttccaggat gaggagaccg agggagtcgt ccgtagattc atcgctgata tgcggcaaca 1980

ggtccaggct gatcgtaaca ttgtcaatta cccttccatc cttcacccta ttgatcatgc 2040

attcaatgag tattttctta accaccagtt ggtggagccg ctgaacaatg agataatctt 2100

caattacata ccagagagga taaggaatga cgtgaattac atcctgaaca tggatatgaa 2160

tctgccatct acagccaggt atatcaggcc aaacttgttg caggatagac tgaatcttca 2220

cgataatttt gagtccctgt gggataccat cacaacatcc aactacattc tggccaggtc 2280

cgtcgttccc gatttgaagg agaaggagct ggtctccacc gaagcacaga tccagaaaat 2340

gagccaggac ctgcagctgg aggccctcac tattcagagc gagacacagt ttttagccgg 2400

gattaacagt caggctgcca atgattgttt caagaccctc atagccgcca tgctgtctca 2460

aagaaccatg tctttggact ttgtgaccac gaactatatg agcctaatct ccggaatgtg 2520

gctacttaca gtgattccca acgatatgtt cctccgggag tcactagtgg cctgtgagct 2580

ggcgatcatc aacaccatcg tgtatccagc attcggaatg cagagaatgc attaccggaa 2640

tggcgaccct cagacaccct tccagatcgc agaacagcag atccagaatt tccaggtggc 2700

gaactggctc cattttatta acaataacag attcaggcaa gttgtgattg atggagttct 2760

gaatcagact ctgaacgaca atatacggaa tggacaggtc atcaaccagc tgatggaagc 2820

attgatgcaa ctcagcagac agcagttccc cacgatgcct gtggattaca aacggagcat 2880

ccaacggggc attctgcttc tctccaatag gctggggcag cttgtcgact taacccgact 2940

ggtctcctat aactacgaga cgctaatggc ttgtgtgacc atgaacatgc agcacgtgca 3000

aaccctgaca actgagaagt tgcagctcac ttctgtgact tcgctttgta tgttaattgg 3060

taacacaacc gtgattccgt ccccacagac actgttccac tactacaaca tcaacgtgaa 3120

tttccactcc aattataatg agcggatcaa cgacgccgtc gccataatta ccgcagcaaa 3180

taggctgaat ctttatcaga aaaaaatgaa gtccatagtg gaagactttc tgaaacggct 3240

ccagattttc gacgtaccac gagtgcctga cgaccaaatg tacaggctga gggatcgcct 3300

tcggctctta cccgttgaac ggagacggct tgacatattc aacttgatcc tgatgaatat 3360

ggagcagatc gaacgcgctt ctgataagat tgctcagggg gttatcatcg cataccgaga 3420

tatgcagctg gaacgcgacg agatgtacgg atatgttaat attgcacgga atcttgatgg 3480

ctaccagcaa attaacttgg aggaactcat gcgcaccggt gattacggac aaattacgaa 3540

catgcttctc aacaatcaac ccgttgccct tgtgggtgca ttgcccttcg ttacggactc 3600

atccgtgatc agtctaatcg ccaagctcga cgcaaccgtc ttcgctcaga tagtgaagct 3660

caggaaagtt gacacactga agcccatact gtacaaaata aactcggatt ccaatgactt 3720

ttaccttgtg gccaactacg actggatccc cacaagtaca actaaggtct acaaacaggt 3780

gccacaacca ttcgacttta gagccagcat gcacatgctg acttctaacc ttacgtttac 3840

cgtctactct gacctactgt catttgtttc agcggacacg gtagagccca ttaacgcagt 3900

cgcattcgac aatatgcgaa taatgaacga gctttaaagg cctattttct ttagtttgaa 3960

tttactgtta ttcggtgtgc atttctatgt ttggtgagcg gttttctgtg ctcagagtgt 4020

gtttatttta tgtaatttaa tttctttgtg agctcctgtt tagcaggtcg tcccttcagc 4080

aaggacacaa aaagatttta attttattaa aaaaaaaaaa aaaaaagacc gggaattcga 4140

tatcaagctt atcgacctgc agatcgttca aacatttggc aataaagttt cttaagattg 4200

aatcctgttg ccggtcttgc gatgattatc atataatttc tgttgaatta cgttaagcat 4260

gtaataatta acatgtaatg catgacgtta tttatgagat gggtttttat gattagagtc 4320

ccgcaattat acatttaata cgcgatagaa aacaaaatat agcgcgcaaa ctaggataaa 4380

ttatcgcgcg cggtgtcatc tatgttacta gat 4413

<210> 28
<211> 49
<212> DNA
<213> Artificial sequence

<220>
<223> Primer IF-WA_VP6(opt).s1+3c

<400> 28
aaatttgtcg ggcccatgga ggtcctttat agtctctcca aaacgctga 49

<210> 29
<211> 52
<212> DNA
<213> Artificial sequence

<220>
<223>  Primer IF-WA_VP6(opt).s1-4r

<400>  29
actaaagaaa ataggcctct acttgatcaa catactccgg atagaggcca ca          52


<210>  30
<211>  2934
<212>  DNA
<213>  Artificial sequence

<220>
<223>  Expression cassette number 1713

<400>  30
gtcaacatgg tggagcacga cacacttgtc tactccaaaa atatcaaaga tacagtctca      60

gaagaccaaa gggcaattga gacttttcaa caaagggtaa tatccggaaa cctcctcgga     120

ttccattgcc cagctatctg tcactttatt gtgaagatag tggaaaagga aggtggctcc     180

tacaaatgcc atcattgcga taaaggaaag gccatcgttg aagatgcctc tgccgacagt     240

ggtcccaaag atggaccccc acccacgagg agcatcgtgg aaaaagaaga cgttccaacc     300

acgtcttcaa agcaagtgga ttgatgtgat aacatggtgg agcacgacac acttgtctac     360

tccaaaaata tcaaagatac agtctcagaa gaccaaaggg caattgagac ttttcaacaa     420

agggtaatat ccggaaacct cctcggattc cattgcccag ctatctgtca ctttattgtg     480

aagatagtgg aaaaggaagg tggctcctac aaatgccatc attgcgataa aggaaaggcc     540

atcgttgaag atgcctctgc cgacagtggt cccaaagatg gacccccacc cacgaggagc     600

atcgtggaaa aagaagacgt tccaaccacg tcttcaaagc aagtggattg atgtgatatc     660

tccactgacg taagggatga cgcacaatcc cactatcctt cgcaagaccc ttcctctata     720

taaggaagtt catttcattt ggagaggtat taaaatctta ataggttttg ataaaagcga     780

acgtggggaa acccgaacca aaccttcttc taaactctct ctcatctctc ttaaagcaaa     840

cttctctctt gtctttcttg cgtgagcgat cttcaacgtt gtcagatcgt gcttcggcac     900

cagtacaacg ttttctttca ctgaagcgaa atcaaagatc tctttgtgga cacgtagtgc     960

ggcgccatta aataacgtgt acttgtccta ttcttgtcgg tgtggtcttg ggaaaagaaa    1020

gcttgctgga ggctgctgtt cagccccata cattacttgt tacgattctg ctgactttcg    1080

gcgggtgcaa tatctctact tctgcttgac gaggtattgt tgcctgtact tctttcttct    1140

tcttcttgct gattggttct ataagaaatc tagtattttc tttgaaacag agttttcccg    1200

tggttttcga acttggagaa agattgttaa gcttctgtat attctgccca aatttgtcgg    1260

gcccatggag gtcctttata gtctctccaa aacgctgaag gacgctaggg acaagatcgt    1320

ggagggtaca ctttatagca atgtcagcga cctaatacag cagtttaatc aaatgatcgt    1380

tacaatgaat gggaatgatt tccaaactgg cggtattggt aatctgcccg tgaggaactg    1440

gacattcgat ttcggcctgc tgggcacgac tctccttaat ctcgatgcaa attatgtaga 1500

aaacgccaga acgattatcg agtactttat cgatttcatt gataacgttt gtatggatga 1560

gatggcccgc gagtcacaac ggaacggagt tgctccacag tccgaggccc ttcggaaact 1620

cgccggcatt aagttcaagc gtattaattt cgacaactcc tccgaatata tagagaactg 1680

gaacttgcag aatcgtcgac agagaaccgg cttcgtgttc cataaaccta atatctttcc 1740

gtatagcgcc tcattcaccc tgaataggag tcagcccatg cacgacaacc tcatgggtac 1800

aatgtggctg aatgcgggga gtgaaataca ggtcgccggg ttcgattact cctgtgccat 1860

taatgcaccc gcaaacatcc agcagttcga acatatcgtg caactaagac gggctctcac 1920

gaccgcgaca attacactcc tgcccgacgc cgagcgcttc tcctttcccc gcgtaatcaa 1980

ctcagctgat ggcgccacca cttggttctt caaccctgtt atattgcgcc ctaacaacgt 2040

agaggtggag tttctcttaa acggacagat catcaatacc taccaagcca ggttcggcac 2100

gattattgca agaaatttcg acgctatcag gctgctcttc caactgatga ggccccccaa 2160

tatgactccc gctgtgaacg ctttgtttcc gcaggctcag cctttccagc accacgccac 2220

cgtcggcttg actcttcgaa tagagagcgc ggtctgcgaa tcagtgctgg cagacgccaa 2280

cgagacgctg ctggcaaacg ttaccgccgt gcggcaagag tatgccatcc cagtagggcc 2340

tgtgtttcca cccggcatga actggactga actaattact aactatagcc catccagaga 2400

agacaacttg cagcgggtct tcactgtggc ctctatccgg agtatgttga tcaagtagag 2460

gcctattttc tttagtttga atttactgtt attcggtgtg catttctatg tttggtgagc 2520

ggttttctgt gctcagagtg tgtttatttt atgtaattta atttctttgt gagctcctgt 2580

ttagcaggtc gtcccttcag caaggacaca aaaagatttt aattttatta aaaaaaaaaa 2640

aaaaaaagac cgggaattcg atatcaagct tatcgacctg cagatcgttc aaacatttgg 2700

caataaagtt tcttaagatt gaatcctgtt gccggtcttg cgatgattat catataattt 2760

ctgttgaatt acgttaagca tgtaataatt aacatgtaat gcatgacgtt atttatgaga 2820

tgggttttta tgattagagt cccgcaatta tacatttaat acgcgataga aaacaaaata 2880

tagcgcgcaa actaggataa attatcgcgc gcggtgtcat ctatgttact agat 2934

<210> 31
<211> 397
<212> PRT
<213> Rotavirus

<400> 31

Met Glu Val Leu Tyr Ser Leu Ser Lys Thr Leu Lys Asp Ala Arg Asp
1               5                   10                  15

Lys Ile Val Glu Gly Thr Leu Tyr Ser Asn Val Ser Asp Leu Ile Gln
            20                  25                  30

Gln Phe Asn Gln Met Ile Val Thr Met Asn Gly Asn Asp Phe Gln Thr
        35                  40                  45

Gly Gly Ile Gly Asn Leu Pro Val Arg Asn Trp Thr Phe Asp Phe Gly
    50                  55                  60

Leu Leu Gly Thr Thr Leu Leu Asn Leu Asp Ala Asn Tyr Val Glu Asn
65                  70                  75                  80

Ala Arg Thr Ile Ile Glu Tyr Phe Ile Asp Phe Ile Asp Asn Val Cys
                85                  90                  95

Met Asp Glu Met Ala Arg Glu Ser Gln Arg Asn Gly Val Ala Pro Gln
            100                 105                 110

Ser Glu Ala Leu Arg Lys Leu Ala Gly Ile Lys Phe Lys Arg Ile Asn
        115                 120                 125

Phe Asp Asn Ser Ser Glu Tyr Ile Glu Asn Trp Asn Leu Gln Asn Arg
    130                 135                 140

Arg Gln Arg Thr Gly Phe Val Phe His Lys Pro Asn Ile Phe Pro Tyr
145                 150                 155                 160

Ser Ala Ser Phe Thr Leu Asn Arg Ser Gln Pro Met His Asp Asn Leu
                165                 170                 175

Met Gly Thr Met Trp Leu Asn Ala Gly Ser Glu Ile Gln Val Ala Gly
            180                 185                 190

Phe Asp Tyr Ser Cys Ala Ile Asn Ala Pro Ala Asn Ile Gln Gln Phe
        195                 200                 205

Glu His Ile Val Gln Leu Arg Arg Ala Leu Thr Thr Ala Thr Ile Thr
    210                 215                 220

Leu Leu Pro Asp Ala Glu Arg Phe Ser Phe Pro Arg Val Ile Asn Ser
225                 230                 235                 240

Ala Asp Gly Ala Thr Thr Trp Phe Phe Asn Pro Val Ile Leu Arg Pro
                245                 250                 255

Asn Asn Val Glu Val Glu Phe Leu Leu Asn Gly Gln Ile Ile Asn Thr
            260                 265                 270

Tyr Gln Ala Arg Phe Gly Thr Ile Ile Ala Arg Asn Phe Asp Ala Ile
        275                 280                 285

Arg Leu Leu Phe Gln Leu Met Arg Pro Pro Asn Met Thr Pro Ala Val
    290                 295                 300

Asn Ala Leu Phe Pro Gln Ala Gln Pro Phe Gln His His Ala Thr Val
305                 310                 315                 320

Gly Leu Thr Leu Arg Ile Glu Ser Ala Val Cys Glu Ser Val Leu Ala
                325                 330                 335

Asp Ala Asn Glu Thr Leu Leu Ala Asn Val Thr Ala Val Arg Gln Glu
            340                 345                 350

Tyr Ala Ile Pro Val Gly Pro Val Phe Pro Pro Gly Met Asn Trp Thr
        355                 360                 365

Glu Leu Ile Thr Asn Tyr Ser Pro Ser Arg Glu Asp Asn Leu Gln Arg
    370                 375                 380

Val Phe Thr Val Ala Ser Ile Arg Ser Met Leu Ile Lys
385                 390                 395

<210>  32
<211>  2934
<212>  DNA
<213>  Artificial sequence

<220>
<223>  Expression cassette number 1714

<400>  32
gtcaacatgg tggagcacga cacacttgtc tactccaaaa atatcaaaga tacagtctca      60

gaagaccaaa gggcaattga gacttttcaa caaagggtaa tatccggaaa cctcctcgga     120

ttccattgcc cagctatctg tcactttatt gtgaagatag tggaaaagga aggtggctcc     180

tacaaatgcc atcattgcga taaaggaaag gccatcgttg aagatgcctc tgccgacagt     240

ggtcccaaag atggaccccc acccacgagg agcatcgtgg aaaaagaaga cgttccaacc     300

acgtcttcaa agcaagtgga ttgatgtgat aacatggtgg agcacgacac acttgtctac     360

tccaaaaata tcaaagatac agtctcagaa gaccaaaggg caattgagac ttttcaacaa     420

agggtaatat ccggaaacct cctcggattc cattgcccag ctatctgtca ctttattgtg     480

aagatagtgg aaaaggaagg tggctcctac aaatgccatc attgcgataa aggaaaggcc     540

atcgttgaag atgcctctgc cgacagtggt cccaaagatg gacccccacc cacgaggagc     600

atcgtggaaa aagaagacgt tccaaccacg tcttcaaagc aagtggattg atgtgatatc     660

tccactgacg taagggatga cgcacaatcc cactatcctt cgcaagaccc ttcctctata 720

taaggaagtt catttcattt ggagaggtat taaaatctta ataggttttg ataaaagcga 780

acgtggggaa acccgaacca aaccttcttc taaactctct ctcatctctc ttaaagcaaa 840

cttctctctt gtctttcttg cgtgagcgat cttcaacgtt gtcagatcgt gcttcggcac 900

cagtacaacg ttttctttca ctgaagcgaa atcaaagatc tctttgtgga cacgtagtgc 960

ggcgccatta aataacgtgt acttgtccta ttcttgtcgg tgtggtcttg ggaaaagaaa 1020

gcttgctgga ggctgctgtt cagccccata cattacttgt tacgattctg ctgactttcg 1080

gcgggtgcaa tatctctact tctgcttgac gaggtattgt tgcctgtact tctttcttct 1140

tcttcttgct gattggttct ataagaaatc tagtattttc tttgaaacag agttttcccg 1200

tggttttcga acttggagaa agattgttaa gcttctgtat attctgccca aatttgtcgg 1260

gcccatggag gtcctttata gtctctccaa aacgctgaag gacgctaggg acaagatcgt 1320

ggagggtaca ctttatagca atgtcagcga cctaatacag cagtttaatc aaatgatcgt 1380

tacaatgaat gggaatgatt tccaaactgg cggtattggt aatctgcccg tgaggaactg 1440

gacattcgat ttcggcctgc tgggcacgac tctccttaat ctcgatgcaa attatgtaga 1500

aaacgccaga acgattatcg agtactttat cgatttcatt gataacgttt gtatggatga 1560

gatggcccgc gagtcacaac ggaacggagt tgctccacag tccgaggccc ttcggaaact 1620

cgccggcatt aagttcaagc gtattaattt cgacaactcc tccgaatata tagagaactg 1680

gaacttgcag aatcgtcgac agagaaccgg cttcgtgttc cataaaccta atatctttcc 1740

gtatagcgcc tcattcaccc tgaataggag tcagcccatg cacgacaacc tcatgggtac 1800

aatgtggctg aatgcgggga gtgaaataca ggtcgccggg ttcgattact cctgtgccat 1860

taatgcaccc gcaaacatcc agcagttcga acatatcgtg caactaagac gggctctcac 1920

gaccgcgaca attacactcc tgcccgacgc cgagcgcttc tcctttcccc gcgtaatcaa 1980

ctcagctgat ggcgccacca cttggttctt caaccctgtt atattgcgcc ctaacaacgt 2040

agaggtggag tttctcttaa acggacagat catcaatacc taccaagcca ggttcggcac 2100

gattattgca agaaatttcg acgctatcag gctgctcttc caactgatga ggccccccaa 2160

tatgactccc gctgtgaacg ctttgtttcc gcaggctcag cctttccagc accacgccac 2220

cgtcggcttg actcttcgaa tagagagcgc ggtctgcgaa tcagtgctgg cagacgccaa 2280

cgagacgctg ctggcaaacg ttaccgccgt gcggcaagag tatgccatcc cagtagggcc 2340

tgtgtttcca cccggcatga actggactga actaattact aactatagcc catccagaga 2400

agacaacttg cagcgggtct tcactgtggc ctctatccgg agtatgttga tcaagtagag 2460

gcctattttc tttagtttga atttactgtt attcggtgtg catttctatg tttggtgagc 2520

ggttttctgt gctcagagtg tgtttatttt atgtaattta atttctttgt gagctcctgt 2580

ttagcaggtc gtcccttcag caaggacaca aaaagatttt aattttatta aaaaaaaaaa 2640

aaaaaaagac cgggaattcg atatcaagct tatcgacctg cagatcgttc aaacatttgg 2700

caataaagtt tcttaagatt gaatcctgtt gccggtcttg cgatgattat catataattt 2760

ctgttgaatt acgttaagca tgtaataatt aacatgtaat gcatgacgtt atttatgaga 2820

tgggttttta tgattagagt cccgcaatta tacatttaat acgcgataga aaacaaaata 2880

tagcgcgcaa actaggataa attatcgcgc gcggtgtcat ctatgttact agat 2934

<210> 33
<211> 53
<212> DNA
<213> Artificial sequence

<220>
<223> Primer IF-Rtx_VP4(opt).s1+3c

<400> 33
aaatttgtcg ggcccatggc tagcctgatc tacagacaac tcttgaccaa ttc 53

<210> 34
<211> 55
<212> DNA
<213> Artificial sequence

<220>
<223> Primer IF-Rtx_VP4(opt).s1-4r

<400> 34
actaaagaaa ataggccttc agagtttaca ttgcaggatt aattgctcaa tccta 55

<210> 35
<211> 4068
<212> DNA
<213> Artificial sequence

<220>
<223> Expression cassette number 1731

<400> 35
gtcaacatgg tggagcacga cacacttgtc tactccaaaa atatcaaaga tacagtctca 60

gaagaccaaa gggcaattga gacttttcaa caaagggtaa tatccggaaa cctcctcgga 120

ttccattgcc cagctatctg tcactttatt gtgaagatag tggaaaagga aggtggctcc 180

tacaaatgcc atcattgcga taaaggaaag gccatcgttg aagatgcctc tgccgacagt 240

ggtcccaaag atggaccccc acccacgagg agcatcgtgg aaaaagaaga cgttccaacc 300

acgtcttcaa agcaagtgga ttgatgtgat aacatggtgg agcacgacac acttgtctac 360

tccaaaaata tcaaagatac agtctcagaa gaccaaaggg caattgagac ttttcaacaa 420

agggtaatat ccggaaacct cctcggattc cattgcccag ctatctgtca ctttattgtg 480

aagatagtgg aaaaggaagg tggctcctac aaatgccatc attgcgataa aggaaaggcc 540

atcgttgaag atgcctctgc cgacagtggt cccaaagatg gacccccacc cacgaggagc 600

atcgtggaaa aagaagacgt tccaaccacg tcttcaaagc aagtggattg atgtgatatc 660

tccactgacg taagggatga cgcacaatcc cactatcctt cgcaagaccc ttcctctata 720

taaggaagtt catttcattt ggagaggtat taaaatctta ataggttttg ataaaagcga 780

acgtggggaa acccgaacca aaccttcttc taaactctct ctcatctctc ttaaagcaaa 840

cttctctctt gtctttcttg cgtgagcgat cttcaacgtt gtcagatcgt gcttcggcac 900

cagtacaacg ttttctttca ctgaagcgaa atcaaagatc tctttgtgga cacgtagtgc 960

ggcgccatta aataacgtgt acttgtccta ttcttgtcgg tgtggtcttg ggaaaagaaa 1020

gcttgctgga ggctgctgtt cagccccata cattacttgt tacgattctg ctgactttcg 1080

gcgggtgcaa tatctctact tctgcttgac gaggtattgt tgcctgtact tctttcttct 1140

tcttcttgct gattggttct ataagaaatc tagtattttc tttgaaacag agttttcccg 1200

tggttttcga acttggagaa agattgttaa gcttctgtat attctgccca aatttgtcgg 1260

gcccatggct agcctgatct acagacaact cttgaccaat tcatattctg tggatcttca 1320

tgacgaaatc gagcagattg ggtccgagaa gacccagaac gtgaccatca accctggacc 1380

ttttgctcag acccgctatg cccctgtgaa ttgggatcac ggagaaatca acgacagtac 1440

gaccgtcgaa cccattctgg acgggccata ccaacccacc accttcaccc cacctaatga 1500

ttattggatt ttaatcaact ccaacacaaa cggagtggtc tacgagtcca ctaataactc 1560

cgatttttgg accgccgttg tagccatcga gccacacgtc aatcctgtcg atcgccagta 1620

tatgatattc ggcgagtcca aacagtttaa cgtttccaat gacagcaaca aatggaagtt 1680

tctggagatg tttcgcagct cctctcagaa cgaattctat aatagacgga cccttacctc 1740

cgatacacga ctcgtgggta tttttaagta cggcggcagg gtgtggacat ttcacggtga 1800

aacccctcga gcaaccactg actccagtag cactgcaaac ctgaacaata tatctattac 1860

catccacagc gaattctaca taatcccaag atctcaggaa agtaagtgta acgaatatat 1920

caacaacgga ctccccccaa ttcagaatac acggaacgtg gtgcctctcc cactcagttc 1980

tcggtctatc cagtataaga gagcacaagt gaatgaggac attattgtga gcaagactag 2040

cctttggaaa gaaatgcagt acaacagaga cattatcatc cggtttaagt ttgggaactc 2100

tatcgtgaag atgggcggcc tggggtacaa atggtcagaa atctcatata aagccgccaa 2160

ctatcagtat aactacttga gagacggcga gcaggtaacc gcccacacaa catgctctgt 2220

caacggcgtt aataacttta gctacaacgg aggcttcctt cccaccgact tcggtatcag 2280

ccggtatgaa gtcatcaagg aaaattctta tgtgtacgta gattactggg atgatagcaa 2340

agcgttccgc aacatggtgt atgttaggag cctggctgct aatctcaatt ctgtgaagtg 2400

tactggtgga tcatattatt tctcaattcc cgtgggggct tggccagtca tgaatggcgg 2460

ggcagtctcc ctccattttg ctggcgtgac gttgagcact cagtttaccg atttcgtgtc 2520

tctgaactcc ctgaggttcc ggttttccct tactgtcgac gagcccccat tcagcattct 2580

gcgtacaaga actgtcaacc tctacgggtt acctgccgcg aatccaaaca acggcaatga 2640

atactatgaa atttcgggcc gcttctcttt gataagtctg gtaccaacta atgacgacta 2700

tcagacaccc atcatgaaca gcgtgactgt cagacaggac ctggaaagac aacttacaga 2760

tctgcgggaa gaattcaatt ctctcagtca ggagattgca atggcccaat tgatagatct 2820

tgccctactg cctctcgata tgtttagtat gttctccggc atcaaatcaa ctatagatct 2880

gacaaagagc atggctactt ctgtgatgaa gaagttcagg aaatcaaaac ttgccacgag 2940

catatcagaa atgacgaact ctctgagtga tgcagcatca tcagcgtcac gcaacgtttc 3000

cattcggtcg aatctcagcg ccatcagcaa ctggacaaac gtgtccaacg acgtcagcaa 3060

cgtgaccaac tccttgaacg atatttctac ccagacgtca acgatcagta agaaactccg 3120

cttgaaagaa atgatcaccc agactgaggg aatgtctttc gacgacattt ccgccgccgt 3180

gctaaaaacc aaaatcgata tgtctactca gatcggcaag aacactctgc cggatatcgt 3240

aaccgaagcc tccgaaaagt ttatccctaa gcgcagctac agaatattga aagatgacga 3300

ggtcatggag atcaacacag aagggaagtt cttcgcttat aagatcaaca cctttgacga 3360

ggttccgttt gacgtcaata agtttgcaga gctcgtgaca gatagtccag tgatttctgc 3420

catcattgac tttaagactt tgaagaacct gaacgacaac tatggaataa cacggaccga 3480

agcgttgaac ctcattaagt ccaatcccaa tatgttgcgc aatttcatta accagaacaa 3540

tccaatcata agaaatagga ttgagcaatt aatcctgcaa tgtaaactct gaaggcctat 3600

tttctttagt ttgaatttac tgttattcgg tgtgcatttc tatgtttggt gagcggtttt 3660

ctgtgctcag agtgtgttta ttttatgtaa tttaatttct ttgtgagctc ctgtttagca 3720

ggtcgtccct tcagcaagga cacaaaaaga ttttaatttt attaaaaaaa aaaaaaaaaa 3780

agaccgggaa ttcgatatca agcttatcga cctgcagatc gttcaaacat ttggcaataa 3840

agtttcttaa gattgaatcc tgttgccggt cttgcgatga ttatcatata atttctgttg 3900

aattacgtta agcatgtaat aattaacatg taatgcatga cgttatttat gagatgggtt 3960

tttatgatta gagtcccgca attatacatt taatacgcga tagaaaacaa aatatagcgc 4020

gcaaactagg ataaattatc gcgcgcggtg tcatctatgt tactagat 4068

<210> 36
<211> 775
<212> PRT
<213> Rotavirus

<400> 36

Met Ala Ser Leu Ile Tyr Arg Gln Leu Leu Thr Asn Ser Tyr Ser Val
1               5                   10                  15

Asp Leu His Asp Glu Ile Glu Gln Ile Gly Ser Glu Lys Thr Gln Asn
            20                  25                  30

Val Thr Ile Asn Pro Gly Pro Phe Ala Gln Thr Arg Tyr Ala Pro Val
        35                  40                  45

Asn Trp Asp His Gly Glu Ile Asn Asp Ser Thr Thr Val Glu Pro Ile
    50                  55                  60

Leu Asp Gly Pro Tyr Gln Pro Thr Thr Phe Thr Pro Pro Asn Asp Tyr
65                  70                  75                  80

Trp Ile Leu Ile Asn Ser Asn Thr Asn Gly Val Val Tyr Glu Ser Thr
                85                  90                  95

Asn Asn Ser Asp Phe Trp Thr Ala Val Val Ala Ile Glu Pro His Val
            100                 105                 110

Asn Pro Val Asp Arg Gln Tyr Met Ile Phe Gly Glu Ser Lys Gln Phe
        115                 120                 125

Asn Val Ser Asn Asp Ser Asn Lys Trp Lys Phe Leu Glu Met Phe Arg
    130                 135                 140

Ser Ser Ser Gln Asn Glu Phe Tyr Asn Arg Arg Thr Leu Thr Ser Asp
145                 150                 155                 160

Thr Arg Leu Val Gly Ile Phe Lys Tyr Gly Gly Arg Val Trp Thr Phe
                165                 170                 175

His Gly Glu Thr Pro Arg Ala Thr Thr Asp Ser Ser Ser Thr Ala Asn
            180                 185                 190

Leu Asn Asn Ile Ser Ile Thr Ile His Ser Glu Phe Tyr Ile Ile Pro
        195                 200                 205

Arg Ser Gln Glu Ser Lys Cys Asn Glu Tyr Ile Asn Asn Gly Leu Pro
    210                 215                 220

Pro Ile Gln Asn Thr Arg Asn Val Val Pro Leu Pro Leu Ser Ser Arg
225                 230                 235                 240

Ser Ile Gln Tyr Lys Arg Ala Gln Val Asn Glu Asp Ile Ile Val Ser
                245                 250                 255

Lys Thr Ser Leu Trp Lys Glu Met Gln Tyr Asn Arg Asp Ile Ile Ile
            260                 265                 270

Arg Phe Lys Phe Gly Asn Ser Ile Val Lys Met Gly Gly Leu Gly Tyr
        275                 280                 285

Lys Trp Ser Glu Ile Ser Tyr Lys Ala Ala Asn Tyr Gln Tyr Asn Tyr
    290                 295                 300

Leu Arg Asp Gly Glu Gln Val Thr Ala His Thr Thr Cys Ser Val Asn
305                 310                 315                 320

Gly Val Asn Asn Phe Ser Tyr Asn Gly Gly Phe Leu Pro Thr Asp Phe
                325                 330                 335

Gly Ile Ser Arg Tyr Glu Val Ile Lys Glu Asn Ser Tyr Val Tyr Val
            340                 345                 350

Asp Tyr Trp Asp Asp Ser Lys Ala Phe Arg Asn Met Val Tyr Val Arg
        355                 360                 365

Ser Leu Ala Ala Asn Leu Asn Ser Val Lys Cys Thr Gly Gly Ser Tyr
    370                 375                 380

Tyr Phe Ser Ile Pro Val Gly Ala Trp Pro Val Met Asn Gly Gly Ala
385                 390                 395                 400

Val Ser Leu His Phe Ala Gly Val Thr Leu Ser Thr Gln Phe Thr Asp
                405                 410                 415

Phe Val Ser Leu Asn Ser Leu Arg Phe Arg Phe Ser Leu Thr Val Asp
            420                 425                 430

Glu Pro Pro Phe Ser Ile Leu Arg Thr Arg Thr Val Asn Leu Tyr Gly
        435                 440                 445

Leu Pro Ala Ala Asn Pro Asn Asn Gly Asn Glu Tyr Tyr Glu Ile Ser
    450                 455                 460

Gly Arg Phe Ser Leu Ile Ser Leu Val Pro Thr Asn Asp Asp Tyr Gln
465                 470                 475                 480

Thr Pro Ile Met Asn Ser Val Thr Val Arg Gln Asp Leu Glu Arg Gln
                485                 490                 495

Leu Thr Asp Leu Arg Glu Glu Phe Asn Ser Leu Ser Gln Glu Ile Ala
                500                 505                 510

Met Ala Gln Leu Ile Asp Leu Ala Leu Leu Pro Leu Asp Met Phe Ser
            515                 520                 525

Met Phe Ser Gly Ile Lys Ser Thr Ile Asp Leu Thr Lys Ser Met Ala
    530                 535                 540

Thr Ser Val Met Lys Lys Phe Arg Lys Ser Lys Leu Ala Thr Ser Ile
545                 550                 555                 560

Ser Glu Met Thr Asn Ser Leu Ser Asp Ala Ala Ser Ser Ala Ser Arg
                565                 570                 575

Asn Val Ser Ile Arg Ser Asn Leu Ser Ala Ile Ser Asn Trp Thr Asn
            580                 585                 590

Val Ser Asn Asp Val Ser Asn Val Thr Asn Ser Leu Asn Asp Ile Ser
        595                 600                 605

Thr Gln Thr Ser Thr Ile Ser Lys Lys Leu Arg Leu Lys Glu Met Ile
    610                 615                 620

Thr Gln Thr Glu Gly Met Ser Phe Asp Asp Ile Ser Ala Ala Val Leu
625                 630                 635                 640

Lys Thr Lys Ile Asp Met Ser Thr Gln Ile Gly Lys Asn Thr Leu Pro
                645                 650                 655

Asp Ile Val Thr Glu Ala Ser Glu Lys Phe Ile Pro Lys Arg Ser Tyr
            660                 665                 670

Arg Ile Leu Lys Asp Asp Glu Val Met Glu Ile Asn Thr Glu Gly Lys
        675                 680                 685

Phe Phe Ala Tyr Lys Ile Asn Thr Phe Asp Glu Val Pro Phe Asp Val
    690                 695                 700

Asn Lys Phe Ala Glu Leu Val Thr Asp Ser Pro Val Ile Ser Ala Ile
705                 710                 715                 720

Ile Asp Phe Lys Thr Leu Lys Asn Leu Asn Asp Asn Tyr Gly Ile Thr
                725                 730                 735

Arg Thr Glu Ala Leu Asn Leu Ile Lys Ser Asn Pro Asn Met Leu Arg
            740                 745                 750

Asn Phe Ile Asn Gln Asn Asn Pro Ile Ile Arg Asn Arg Ile Glu Gln
        755                 760                 765

Leu Ile Leu Gln Cys Lys Leu
    770                 775

<210>  37
<211>  51
<212>  DNA
<213>  Artificial sequence

<220>
<223>  Primer IF-Rtx_VP7(opt).s1+3c

<400>  37
aaatttgtcg ggcccatgta cggcatcgag tatacaacaa ttttaatttt c                51

<210>  38
<211>  54
<212>  DNA
<213>  Artificial sequence

<220>
<223>  Primer IF-Rtx_VP7(opt).s1-4r

<400>  38
actaaagaaa ataggcctct aaacgcgata atagaaggct gctgagttca ggga             54

<210>  39
<211>  326
<212>  PRT
<213>  Rotavirus

<400>  39

Met Tyr Gly Ile Glu Tyr Thr Thr Ile Leu Ile Phe Leu Ile Ser Ile
1               5                   10                  15

Ile Leu Leu Asn Tyr Ile Leu Lys Ser Val Thr Arg Ile Met Asp Tyr
            20                  25                  30

Ile Ile Tyr Arg Ser Leu Leu Ile Tyr Val Ala Leu Phe Ala Leu Thr
        35                  40                  45

Arg Ala Gln Asn Tyr Gly Leu Asn Leu Pro Ile Thr Gly Ser Met Asp
    50                  55                  60

Thr Val Tyr Ala Asn Ser Thr Gln Glu Gly Ile Phe Leu Thr Ser Thr
65                  70                  75                  80

Leu Cys Leu Tyr Tyr Pro Thr Glu Ala Ser Thr Gln Ile Asn Asp Gly
                85                  90                  95

Glu Trp Lys Asp Ser Leu Ser Gln Met Phe Leu Thr Lys Gly Trp Pro
            100                 105                 110

Thr Gly Ser Val Tyr Phe Lys Glu Tyr Ser Ser Ile Val Asp Phe Ser
        115                 120                 125

Val Asp Pro Gln Leu Tyr Cys Asp Tyr Asn Leu Val Leu Met Lys Tyr
    130                 135                 140

Asp Gln Asn Leu Glu Leu Asp Met Ser Glu Leu Ala Asp Leu Ile Leu
145                 150                 155                 160

Asn Glu Trp Leu Cys Asn Pro Met Asp Ile Thr Leu Tyr Tyr Tyr Gln
                165                 170                 175

Gln Ser Gly Glu Ser Asn Lys Trp Ile Ser Met Gly Ser Ser Cys Thr
            180                 185                 190

Val Lys Val Cys Pro Leu Asn Thr Gln Met Leu Gly Ile Gly Cys Gln
        195                 200                 205

Thr Thr Asn Val Asp Ser Phe Glu Met Val Ala Glu Asn Glu Lys Leu
    210                 215                 220

Ala Ile Val Asp Val Val Asp Gly Ile Asn His Lys Ile Asn Leu Thr
225                 230                 235                 240

Thr Thr Thr Cys Thr Ile Arg Asn Cys Lys Lys Leu Gly Pro Arg Glu
                245                 250                 255

Asn Val Ala Val Ile Gln Val Gly Gly Ser Asn Val Leu Asp Ile Thr
            260                 265                 270

Ala Asp Pro Thr Thr Asn Pro Gln Thr Glu Arg Met Met Arg Val Asn
        275                 280                 285

Trp Lys Lys Trp Trp Gln Val Phe Tyr Thr Ile Val Asp Tyr Ile Asn
    290                 295                 300

Gln Ile Val Gln Val Met Ser Lys Arg Ser Arg Ser Leu Asn Ser Ala
305                 310                 315                 320

Ala Phe Tyr Tyr Arg Val
                325

<210>  40

<211>  60
<212>  DNA
<213>  Artificial sequence

<220>
<223>  Primer IF-Rtx_VP7(opt).s2+4c

<400>  40
tctcagatct tcgcccagaa ctatggcctg aacttaccaa tcaccggttc aatggatacc        60


<210>  41
<211>  4897
<212>  DNA
<213>  Artificial sequence

<220>
<223>  Construct 1192

<400>  41
tggcaggata tattgtggtg taaacaaatt gacgcttaga caacttaata acacattgcg        60

gacgttttta atgtactgaa ttaacgccga atcccgggct ggtatattta tatgttgtca       120

aataactcaa aaaccataaa agtttaagtt agcaagtgtg tacattttta cttgaacaaa       180

aatattcacc tactactgtt ataaatcatt attaaacatt agagtaaaga aatatggatg       240

ataagaacaa gagtagtgat attttgacaa caattttgtt gcaacatttg agaaaatttt       300

gttgttctct cttttcattg gtcaaaaaca atagagagag aaaaaggaag agggagaata       360

aaaacataat gtgagtatga gagagaaagt tgtacaaaag ttgtaccaaa atagttgtac       420

aaatatcatt gaggaatttg acaaaagcta cacaaataag ggttaattgc tgtaaataaa       480

taaggatgac gcattagaga gatgtaccat tagagaattt ttggcaagtc attaaaaaga       540

aagaataaat tatttttaaa attaaaagtt gagtcatttg attaaacatg tgattattta       600

atgaattgat gaaagagttg gattaaagtt gtattagtaa ttagaatttg gtgtcaaatt       660

taatttgaca tttgatcttt tcctatatat tgccccatag agtcagttaa ctcattttta       720

tatttcatag atcaaataag agaaataacg gtatattaat ccctccaaaa aaaaaaaacg       780

gtatatttac taaaaaatct aagccacgta ggaggataac aggatccccg taggaggata       840

acatccaatc caaccaatca caacaatcct gatgagataa cccactttaa gcccacgcat       900

ctgtggcaca tctacattat ctaaatcaca cattcttcca cacatctgag ccacacaaaa       960

accaatccac atctttatca cccattctat aaaaaatcac actttgtgag tctacacttt      1020

gattcccttc aaacacatac aaagagaaga gactaattaa ttaattaatc atcttgagag      1080

aaaatggaac gagctataca aggaaacgac gctagggaac aagctaacag tgaacgttgg      1140

gatggaggat caggaggtac cacttctccc ttcaaacttc ctgacgaaag tccgagttgg      1200

actgagtggc ggctacataa cgatgagacg aattcgaatc aagataatcc ccttggtttc      1260

aaggaaagct ggggtttcgg gaaagttgta tttaagagat atctcagata cgacaggacg      1320

gaagcttcac tgcacagagt ccttggatct tggacgggag attcggttaa ctatgcagca 1380

tctcgatttt tcggtttcga ccagatcgga tgtacctata gtattcggtt tcgaggagtt 1440

agtatcaccg tttctggagg gtcgcgaact cttcagcatc tctgtgagat ggcaattcgg 1500

tctaagcaag aactgctaca gcttgcccca atcgaagtgg aaagtaatgt atcaagagga 1560

tgccctgaag gtactcaaac cttcgaaaaa gaaagcgagt aagttaaaat gcttcttcgt 1620

ctcctattta taatatggtt tgttattgtt aattttgttc ttgtagaaga gcttaattaa 1680

tcgttgttgt tatgaaatac tatttgtatg agatgaactg gtgtaatgta attcatttac 1740

ataagtggag tcagaatcag aatgtttcct ccataactaa ctagacatga agacctgccg 1800

cgtacaattg tcttatattt gaacaactaa aattgaacat cttttgccac aactttataa 1860

gtggttaata tagctcaaat atatggtcaa gttcaataga ttaataatgg aaatatcagt 1920

tatcgaaatt cattaacaat caacttaacg ttattaacta ctaattttat atcatcccct 1980

ttgataaatg atagtacacc aattaggaag gagcatgctc gcctaggaga ttgtcgtttc 2040

ccgccttcag tttgcaagct gctctagccg tgtagccaat acgcaaaccg cctctccccg 2100

cgcgttggga attactagcg cgtgtcgaca agcttgcatg ccggtcaaca tggtggagca 2160

cgacacactt gtctactcca aaaatatcaa agatacagtc tcagaagacc aaagggcaat 2220

tgagactttt caacaaaggg taatatccgg aaacctcctc ggattccatt gcccagctat 2280

ctgtcacttt attgtgaaga tagtggaaaa ggaaggtggc tcctacaaat gccatcattg 2340

cgataaagga aaggccatcg ttgaagatgc ctctgccgac agtggtccca aagatggacc 2400

cccacccacg aggagcatcg tggaaaaaga agacgttcca accacgtctt caaagcaagt 2460

ggattgatgt gataacatgg tggagcacga cacacttgtc tactccaaaa atatcaaaga 2520

tacagtctca gaagaccaaa gggcaattga gacttttcaa caaagggtaa tatccggaaa 2580

cctcctcgga ttccattgcc cagctatctg tcactttatt gtgaagatag tggaaaagga 2640

aggtggctcc tacaaatgcc atcattgcga taaaggaaag gccatcgttg aagatgcctc 2700

tgccgacagt ggtcccaaag atggaccccc acccacgagg agcatcgtgg aaaaagaaga 2760

cgttccaacc acgtcttcaa agcaagtgga ttgatgtgat atctccactg acgtaaggga 2820

tgacgcacaa tcccactatc cttcgcaaga cccttcctct atataaggaa gttcatttca 2880

tttggagagg tattaaaatc ttaataggtt ttgataaaag cgaacgtggg gaaacccgaa 2940

ccaaaccttc ttctaaactc tctctcatct ctcttaaagc aaacttctct cttgtctttc 3000

ttgcgtgagc gatcttcaac gttgtcagat cgtgcttcgg caccagtaca acgttttctt 3060

tcactgaagc gaaatcaaag atctctttgt ggacacgtag tgcggcgcca ttaaataacg 3120

tgtacttgtc ctattcttgt cggtgtggtc ttgggaaaag aaagcttgct ggaggctgct 3180

gttcagcccc atacattact tgttacgatt ctgctgactt tcggcgggtg caatatctct 3240

acttctgctt gacgaggtat tgttgcctgt acttctttct tcttcttctt gctgattggt 3300

tctataagaa atctagtatt ttctttgaaa cagagttttc ccgtggtttt cgaacttgga 3360

gaaagattgt taagcttctg tatattctgc ccaaatttgt cgggcccatg gcgaaaaacg 3420

ttgcgatttt cggcttattg ttttctcttc ttgtgttggt tccttctcag atcttcgccg 3480

cggctcctca gccaaaacga cacccccatc tgtctatcca ctggcccctg gatctgctgc 3540

ccaaactaac tccatggtga ccctgggatg cctggtcaag ggctatttcc ctgagccagt 3600

gacagtgacc tggaactctg gatccctgtc cagcggtgtg cacaccttcc cagctgtcct 3660

gcagtctgac ctctacactc tgagcagctc agtgactgtc ccctccagca cctggcccag 3720

cgagaccgtc acctgcaacg ttgcccaccc ggccagcagc accaaggtgg acaagaaaat 3780

tgtgcccagg gattgtggtt gtaagccttg catatgtaca gtcccagaag tatcatctgt 3840

cttcatcttc cccccaaagc ccaaggatgt gctcaccatt actctgactc ctaaggtcac 3900

gtgtgttgtg gtagacatca gcaaggatga tcccgaggtc cagttcagct ggtttgtaga 3960

tgatgtggag gtgcacacag ctcagacgca accccgggag gagcagttca acagcacttt 4020

ccgctcagtc agtgaacttc ccatcatgca ccaggactgg ctcaatggca aggagcgatc 4080

gctcaccatc accatcacca tcaccatcac cattaaaggc ctattttctt tagtttgaat 4140

ttactgttat tcggtgtgca tttctatgtt tggtgagcgg ttttctgtgc tcagagtgtg 4200

tttattttat gtaatttaat ttctttgtga gctcctgttt agcaggtcgt cccttcagca 4260

aggacacaaa aagattttaa ttttattaaa aaaaaaaaaa aaaaagaccg ggaattcgat 4320

atcaagctta tcgacctgca gatcgttcaa acatttggca ataaagtttc ttaagattga 4380

atcctgttgc cggtcttgcg atgattatca tataatttct gttgaattac gttaagcatg 4440

taataattaa catgtaatgc atgacgttat ttatgagatg ggtttttatg attagagtcc 4500

cgcaattata catttaatac gcgatagaaa acaaaatata gcgcgcaaac taggataaat 4560

tatcgcgcgc ggtgtcatct atgttactag atctctagag tctcaagctt ggcgcgccca 4620

cgtgactagt ggcactggcc gtcgttttac aacgtcgtga ctgggaaaac cctggcgtta 4680

cccaacttaa tcgccttgca gcacatcccc ctttcgccag ctggcgtaat agcgaagagg 4740

cccgcaccga tcgcccttcc caacagttgc gcagcctgaa tggcgaatgc tagagcagct 4800

tgagcttgga tcagattgtc gtttcccgcc ttcagtttaa actatcagtg tttgacagga 4860

tatattggcg ggtaaaccta agagaaaaga gcgttta 4897

<210> 42
<211> 2643
<212> DNA
<213> Artificial sequence

<220>
<223>  Expression cassette number 1735

<400>  42
gtcaacatgg tggagcacga cacacttgtc tactccaaaa atatcaaaga tacagtctca      60

gaagaccaaa gggcaattga gacttttcaa caaagggtaa tatccggaaa cctcctcgga     120

ttccattgcc cagctatctg tcactttatt gtgaagatag tggaaaagga aggtggctcc     180

tacaaatgcc atcattgcga taaaggaaag gccatcgttg aagatgcctc tgccgacagt     240

ggtcccaaag atggaccccc acccacgagg agcatcgtgg aaaaagaaga cgttccaacc     300

acgtcttcaa agcaagtgga ttgatgtgat aacatggtgg agcacgacac acttgtctac     360

tccaaaaata tcaaagatac agtctcagaa gaccaaaggg caattgagac ttttcaacaa     420

agggtaatat ccggaaacct cctcggattc cattgcccag ctatctgtca ctttattgtg     480

aagatagtgg aaaaggaagg tggctcctac aaatgccatc attgcgataa aggaaaggcc     540

atcgttgaag atgcctctgc cgacagtggt cccaaagatg gacccccacc cacgaggagc     600

atcgtggaaa aagaagacgt tccaaccacg tcttcaaagc aagtggattg atgtgatatc     660

tccactgacg taagggatga cgcacaatcc cactatcctt cgcaagaccc ttcctctata     720

taaggaagtt catttcattt ggagaggtat taaaatctta ataggttttg ataaaagcga     780

acgtggggaa acccgaacca aaccttcttc taaactctct ctcatctctc ttaaagcaaa     840

cttctctctt gtctttcttg cgtgagcgat cttcaacgtt gtcagatcgt gcttcggcac     900

cagtacaacg ttttctttca ctgaagcgaa atcaaagatc tctttgtgga cacgtagtgc     960

ggcgccatta aataacgtgt acttgtccta ttcttgtcgg tgtggtcttg ggaaaagaaa    1020

gcttgctgga ggctgctgtt cagccccata cattacttgt tacgattctg ctgactttcg    1080

gcgggtgcaa tatctctact tctgcttgac gaggtattgt tgcctgtact tctttcttct    1140

tcttcttgct gattggttct ataagaaatc tagtattttc tttgaaacag agttttcccg    1200

tggttttcga acttggagaa agattgttaa gcttctgtat attctgccca aatttgtcgg    1260

gcccatggcg aaaaacgttg cgattttcgg cttattgttt tctcttcttg tgttggttcc    1320

ttctcagatc ttcgcccaga actatggcct gaacttacca atcaccggtt caatggatac    1380

cgtttacgct aattccactc aagaggggat atttctgaca agtaccctgt gcctgtatta    1440

tccaacagaa gcctctaccc agatcaatga tggggagtgg aaggatagtc tctcacagat    1500

gttcctaacc aagggctggc ccaccggttc cgtctacttc aaggaatact ctagtattgt    1560

cgacttctca gttgaccccc agctttattg cgactacaac ctggtactta tgaaatacga    1620

ccagaacctg gagctggata tgtccgagct ggctgacctg atcctcaatg agtggctgtg    1680

caaccccatg gacatcacat tatattacta ccagcagtct ggagaatcca acaagtggat    1740

cagtatgggc tcaagttgca ccgtgaaggt gtgtcccttg aacacccaaa tgctgggcat 1800

tggttgtcag acaactaatg tggattcgtt tgaaatggta gccgaaaacg agaagctggc 1860

tatagtggac gtagtcgatg ggattaacca caagatcaat ctgactacca ccacttgtac 1920

catcagaaac tgtaaaaagc tcggcccccg ggagaacgtc gccgtgatcc aggtgggggg 1980

gagcaatgtg ctcgacatta ctgccgaccc taccaccaat ccacagacgg aacggatgat 2040

gagagtcaac tggaagaaat ggtggcaggt cttttatacc attgtggact acattaacca 2100

gattgtgcaa gtcatgagta aacggtccag atccctgaac tcagcagcct tctattatcg 2160

cgtttagagg cctatttctt ttagtttgaa tttactgtta ttcggtgtgc atttctatgt 2220

ttggtgagcg gttttctgtg ctcagagtgt gtttatttta tgtaatttaa tttctttgtg 2280

agctcctgtt tagcaggtcg tcccttcagc aaggacacaa aaagatttta attttattaa 2340

aaaaaaaaaa aaaaaagacc gggaattcga tatcaagctt atcgacctgc agatcgttca 2400

aacatttggc aataaagttt cttaagattg aatcctgttg ccggtcttgc gatgattatc 2460

atataatttc tgttgaatta cgttaagcat gtaataatta acatgtaatg catgacgtta 2520

tttatgagat gggtttttat gattagagtc ccgcaattat acatttaata cgcgatagaa 2580

aacaaaatat agcgcgcaaa ctaggataaa ttatcgcgcg cggtgtcatc tatgttacta 2640

gat 2643

<210> 43
<211> 300
<212> PRT
<213> Rotavirus

<400> 43

Met Ala Lys Asn Val Ala Ile Phe Gly Leu Leu Phe Ser Leu Leu Val
1               5                   10                  15

Leu Val Pro Ser Gln Ile Phe Ala Gln Asn Tyr Gly Leu Asn Leu Pro
            20                  25                  30

Ile Thr Gly Ser Met Asp Thr Val Tyr Ala Asn Ser Thr Gln Glu Gly
        35                  40                  45

Ile Phe Leu Thr Ser Thr Leu Cys Leu Tyr Tyr Pro Thr Glu Ala Ser
    50                  55                  60

Thr Gln Ile Asn Asp Gly Glu Trp Lys Asp Ser Leu Ser Gln Met Phe
65                  70                  75                  80

Leu Thr Lys Gly Trp Pro Thr Gly Ser Val Tyr Phe Lys Glu Tyr Ser
                85                  90                  95

Ser Ile Val Asp Phe Ser Val Asp Pro Gln Leu Tyr Cys Asp Tyr Asn
            100                 105                 110

Leu Val Leu Met Lys Tyr Asp Gln Asn Leu Glu Leu Asp Met Ser Glu
        115                 120                 125

Leu Ala Asp Leu Ile Leu Asn Glu Trp Leu Cys Asn Pro Met Asp Ile
    130                 135                 140

Thr Leu Tyr Tyr Tyr Gln Gln Ser Gly Glu Ser Asn Lys Trp Ile Ser
145                 150                 155                 160

Met Gly Ser Ser Cys Thr Val Lys Val Cys Pro Leu Asn Thr Gln Met
                165                 170                 175

Leu Gly Ile Gly Cys Gln Thr Thr Asn Val Asp Ser Phe Glu Met Val
            180                 185                 190

Ala Glu Asn Glu Lys Leu Ala Ile Val Asp Val Val Asp Gly Ile Asn
        195                 200                 205

His Lys Ile Asn Leu Thr Thr Thr Thr Cys Thr Ile Arg Asn Cys Lys
    210                 215                 220

Lys Leu Gly Pro Arg Glu Asn Val Ala Val Ile Gln Val Gly Gly Ser
225                 230                 235                 240

Asn Val Leu Asp Ile Thr Ala Asp Pro Thr Thr Asn Pro Gln Thr Glu
                245                 250                 255

Arg Met Met Arg Val Asn Trp Lys Lys Trp Trp Gln Val Phe Tyr Thr
            260                 265                 270

Ile Val Asp Tyr Ile Asn Gln Ile Val Gln Val Met Ser Lys Arg Ser
        275                 280                 285

Arg Ser Leu Asn Ser Ala Ala Phe Tyr Tyr Arg Val
    290                 295                 300

<210>  44
<211>  4068
<212>  DNA
<213>  Artificial sequence

<220>
<223>
Expression cassette number 1730

<400>  44

gtcaacatgg tggagcacga cacacttgtc tactccaaaa atatcaaaga tacagtctca 60

gaagaccaaa gggcaattga gacttttcaa caaagggtaa tatccggaaa cctcctcgga 120

ttccattgcc cagctatctg tcactttatt gtgaagatag tggaaaagga aggtggctcc 180

tacaaatgcc atcattgcga taaaggaaag gccatcgttg aagatgcctc tgccgacagt 240

ggtcccaaag atggaccccc acccacgagg agcatcgtgg aaaaagaaga cgttccaacc 300

acgtcttcaa agcaagtgga ttgatgtgat aacatggtgg agcacgacac acttgtctac 360

tccaaaaata tcaaagatac agtctcagaa gaccaaaggg caattgagac ttttcaacaa 420

agggtaatat ccggaaacct cctcggattc cattgcccag ctatctgtca ctttattgtg 480

aagatagtgg aaaaggaagg tggctcctac aaatgccatc attgcgataa aggaaaggcc 540

atcgttgaag atgcctctgc cgacagtggt cccaaagatg gacccccacc cacgaggagc 600

atcgtggaaa aagaagacgt tccaaccacg tcttcaaagc aagtggattg atgtgatatc 660

tccactgacg taagggatga cgcacaatcc cactatcctt cgcaagaccc ttcctctata 720

taaggaagtt catttcattt ggagaggtat taaaatctta ataggttttg ataaaagcga 780

acgtggggaa acccgaacca aaccttcttc taaactctct ctcatctctc ttaaagcaaa 840

cttctctctt gtctttcttg cgtgagcgat cttcaacgtt gtcagatcgt gcttcggcac 900

cagtacaacg ttttctttca ctgaagcgaa atcaaagatc tctttgtgga cacgtagtgc 960

ggcgccatta aataacgtgt acttgtccta ttcttgtcgg tgtggtcttg ggaaaagaaa 1020

gcttgctgga ggctgctgtt cagccccata cattacttgt tacgattctg ctgactttcg 1080

gcgggtgcaa tatctctact tctgcttgac gaggtattgt tgcctgtact tctttcttct 1140

tcttcttgct gattggttct ataagaaatc tagtattttc tttgaaacag agttttcccg 1200

tggttttcga acttggagaa agattgttaa gcttctgtat attctgccca aatttgtcgg 1260

gcccatggct agcctgatct acagacaact cttgaccaat tcatattctg tggatcttca 1320

tgacgaaatc gagcagattg ggtccgagaa gacccagaac gtgaccatca accctggacc 1380

ttttgctcag acccgctatg cccctgtgaa ttgggatcac ggagaaatca acgacagtac 1440

gaccgtcgaa cccattctgg acgggccata ccaacccacc accttcaccc cacctaatga 1500

ttattggatt ttaatcaact ccaacacaaa cggagtggtc tacgagtcca ctaataactc 1560

cgatttttgg accgccgttg tagccatcga gccacacgtc aatcctgtcg atcgccagta 1620

tatgatattc ggcgagtcca aacagtttaa cgtttccaat gacagcaaca aatggaagtt 1680

tctggagatg tttcgcagct cctctcagaa cgaattctat aatagacgga cccttacctc 1740

cgatacacga ctcgtgggta tttttaagta cggcggcagg gtgtggacat ttcacggtga 1800

aacccctcga gcaaccactg actccagtag cactgcaaac ctgaacaata tatctattac 1860

catccacagc gaattctaca taatcccaag atctcaggaa agtaagtgta acgaatatat 1920

caacaacgga ctccccccaa ttcagaatac acggaacgtg gtgcctctcc cactcagttc 1980

tcggtctatc cagtataaga gagcacaagt gaatgaggac attattgtga gcaagactag 2040

cctttggaaa gaaatgcagt acaacagaga cattatcatc cggtttaagt ttgggaactc 2100

tatcgtgaag atgggcggcc tggggtacaa atggtcagaa atctcatata aagccgccaa 2160

ctatcagtat aactacttga gagacggcga gcaggtaacc gcccacacaa catgctctgt 2220

caacggcgtt aataacttta gctacaacgg aggcttcctt cccaccgact tcggtatcag 2280

ccggtatgaa gtcatcaagg aaaattctta tgtgtacgta gattactggg atgatagcaa 2340

agcgttccgc aacatggtgt atgttaggag cctggctgct aatctcaatt ctgtgaagtg 2400

tactggtgga tcatattatt tctcaattcc cgtgggggct tggccagtca tgaatggcgg 2460

ggcagtctcc ctccattttg ctggcgtgac gttgagcact cagtttaccg atttcgtgtc 2520

tctgaactcc ctgaggttcc ggttttccct tactgtcgac gagcccccat tcagcattct 2580

gcgtacaaga actgtcaacc tctacgggtt acctgccgcg aatccaaaca acggcaatga 2640

atactatgaa atttcgggcc gcttctcttt gataagtctg gtaccaacta atgacgacta 2700

tcagacaccc atcatgaaca gcgtgactgt cagacaggac ctggaaagac aacttacaga 2760

tctgcgggaa gaattcaatt ctctcagtca ggagattgca atggcccaat tgatagatct 2820

tgccctactg cctctcgata tgtttagtat gttctccggc atcaaatcaa ctatagatct 2880

gacaaagagc atggctactt ctgtgatgaa gaagttcagg aaatcaaaac ttgccacgag 2940

catatcagaa atgacgaact ctctgagtga tgcagcatca tcagcgtcac gcaacgtttc 3000

cattcggtcg aatctcagcg ccatcagcaa ctggacaaac gtgtccaacg acgtcagcaa 3060

cgtgaccaac tccttgaacg atatttctac ccagacgtca acgatcagta agaaactccg 3120

cttgaaagaa atgatcaccc agactgaggg aatgtctttc gacgacattt ccgccgccgt 3180

gctaaaaacc aaaatcgata tgtctactca gatcggcaag aacactctgc cggatatcgt 3240

aaccgaagcc tccgaaaagt ttatccctaa gcgcagctac agaatattga aagatgacga 3300

ggtcatggag atcaacacag aagggaagtt cttcgcttat aagatcaaca cctttgacga 3360

ggttccgttt gacgtcaata agtttgcaga gctcgtgaca gatagtccag tgatttctgc 3420

catcattgac tttaagactt tgaagaacct gaacgacaac tatggaataa cacggaccga 3480

agcgttgaac ctcattaagt ccaatcccaa tatgttgcgc aatttcatta accagaacaa 3540

tccaatcata agaaatagga ttgagcaatt aatcctgcaa tgtaaactct gaaggcctat 3600

tttctttagt ttgaatttac tgttattcgg tgtgcatttc tatgtttggt gagcggtttt 3660

ctgtgctcag agtgtgttta ttttatgtaa tttaatttct ttgtgagctc ctgtttagca 3720

ggtcgtccct tcagcaagga cacaaaaaga ttttaatttt attaaaaaaa aaaaaaaaaa 3780

agaccgggaa ttcgatatca agcttatcga cctgcagatc gttcaaacat ttggcaataa 3840

agtttcttaa gattgaatcc tgttgccggt cttgcgatga ttatcatata atttctgttg 3900

aattacgtta agcatgtaat aattaacatg taatgcatga cgttatttat gagatgggtt 3960

tttatgatta gagtcccgca attatacatt taatacgcga tagaaaacaa aatatagcgc 4020

gcaaactagg ataaattatc gcgcgcggtg tcatctatgt tactagat 4068

<210> 45
<211> 2673
<212> DNA
<213> Rotavirus

<400> 45
atggcatacc ggaagagagg agcaaagcgc gaaaacctgc cgcaacagaa cgagagactg 60

caagaaaaag agatagagaa agatgtcgac gtaacaatgg aaaacaagaa taacaatagg 120

aaacaacagc tgtccgacaa agttctgtcc cagaaggagg aaattatcac tgacgcccag 180

gacgatatta aaattgccgg agaaataaag aagagctcga aagaagaatc taaacagctg 240

ctcgaaattc tgaaaacaaa agaagaccat cagaaagaga ttcaatatga aattttgcaa 300

aaaacaatac ctacatttga gtccaaagaa agtatcctca agaagcttga agacataaga 360

ccggagcagg caaaaaaaca gatgaaactc tttcgcattt tcgagccaaa acagctccct 420

atatatcgcg ccaatggcga gaaggagcta cgcaaccggt ggtactggaa gttgaaaaaa 480

gacaccctgc cagatggaga ttatgacgtc cgggagtatt tcctcaatct ctatgatcag 540

atcctcatcg aaatgccgga ctatctgctc ctcaaggaca tggccgtgga gaacaaaaat 600

agcagagacg ccggcaaagt tgtcgactct gagactgcca atatttgtga tgccatcttc 660

caggatgagg agaccgaggg agtcgtccgt agattcatcg ctgatatgcg gcaacaggtc 720

caggctgatc gtaacattgt caattaccct tccatccttc accctattga tcatgcattc 780

aatgagtatt ttcttaacca ccagttggtg gagccgctga acaatgagat aatcttcaat 840

tacataccag agaggataag gaatgacgtg aattacatcc tgaacatgga tatgaatctg 900

ccatctacag ccaggtatat caggccaaac ttgttgcagg atagactgaa tcttcacgat 960

aattttgagt ccctgtggga taccatcaca acatccaact acattctggc caggtccgtc 1020

gttcccgatt tgaaggagaa ggagctggtc tccaccgaag cacagatcca gaaaatgagc 1080

caggacctgc agctggaggc cctcactatt cagagcgaga cacagttttt agccgggatt 1140

aacagtcagg ctgccaatga ttgtttcaag accctcatag ccgccatgct gtctcaaaga 1200

accatgtctt tggactttgt gaccacgaac tatatgagcc taatctccgg aatgtggcta 1260

cttacagtga ttcccaacga tatgttcctc cgggagtcac tagtggcctg tgagctggcg 1320

atcatcaaca ccatcgtgta tccagcattc ggaatgcaga gaatgcatta ccggaatggc 1380

gaccctcaga cacccttcca gatcgcagaa cagcagatcc agaatttcca ggtggcgaac 1440

tggctccatt ttattaacaa taacagattc aggcaagttg tgattgatgg agttctgaat 1500

cagactctga acgacaatat acggaatgga caggtcatca accagctgat ggaagcattg 1560

atgcaactca gcagacagca gttccccacg atgcctgtgg attacaaacg gagcatccaa 1620

cggggcattc tgcttctctc caataggctg gggcagcttg tcgacttaac ccgactggtc 1680

tcctataact acgagacgct aatggcttgt gtgaccatga acatgcagca cgtgcaaacc 1740

ctgacaactg agaagttgca gctcacttct gtgacttcgc tttgtatgtt aattggtaac 1800

acaaccgtga ttccgtcccc acagacactg ttccactact acaacatcaa cgtgaatttc 1860

cactccaatt ataatgagcg gatcaacgac gccgtcgcca taattaccgc agcaaatagg 1920

ctgaatcttt atcagaaaaa aatgaagtcc atagtggaag actttctgaa acggctccag 1980

attttcgacg taccacgagt gcctgacgac caaatgtaca ggctgaggga tcgccttcgg 2040

ctcttacccg ttgaacggag acggcttgac atattcaact tgatcctgat gaatatggag 2100

cagatcgaac gcgcttctga taagattgct cagggggtta tcatcgcata ccgagatatg 2160

cagctggaac gcgacgagat gtacggatat gttaatattg cacggaatct tgatggctac 2220

cagcaaatta acttggagga actcatgcgc accggtgatt acggacaaat tacgaacatg 2280

cttctcaaca atcaacccgt tgcccttgtg ggtgcattgc ccttcgttac ggactcatcc 2340

gtgatcagtc taatcgccaa gctcgacgca accgtcttcg ctcagatagt gaagctcagg 2400

aaagttgaca cactgaagcc catactgtac aaaataaact cggattccaa tgacttttac 2460

cttgtggcca actacgactg gatccccaca agtacaacta aggtctacaa acaggtgcca 2520

caaccattcg actttagagc cagcatgcac atgctgactt ctaaccttac gtttaccgtc 2580

tactctgacc tactgtcatt tgtttcagcg gacacggtag agcccattaa cgcagtcgca 2640

ttcgacaata tgcgaataat gaacgagctt taa 2673

<210> 46
<211> 1194
<212> DNA
<213> Rotavirus

<400> 46
atggaggtcc tttatagtct ctccaaaacg ctgaaggacg ctagggacaa gatcgtggag 60

ggtacacttt atagcaatgt cagcgaccta atacagcagt ttaatcaaat gatcgttaca 120

atgaatggga atgatttcca aactggcggt attggtaatc tgcccgtgag gaactggaca 180

ttcgatttcg gcctgctggg cacgactctc cttaatctcg atgcaaatta tgtagaaaac 240

gccagaacga ttatcgagta ctttatcgat ttcattgata acgtttgtat ggatgagatg 300

gcccgcgagt cacaacggaa cggagttgct ccacagtccg aggcccttcg gaaactcgcc 360

ggcattaagt tcaagcgtat taatttcgac aactcctccg aatatataga gaactggaac 420

ttgcagaatc gtcgacagag aaccggcttc gtgttccata aacctaatat ctttccgtat 480

agcgcctcat tcaccctgaa taggagtcag cccatgcacg acaacctcat gggtacaatg 540

tggctgaatg cggggagtga aatacaggtc gccgggttcg attactcctg tgccattaat 600

gcacccgcaa acatccagca gttcgaacat atcgtgcaac taagacgggc tctcacgacc 660

gcgacaatta cactcctgcc cgacgccgag cgcttctcct ttccccgcgt aatcaactca 720

gctgatggcg ccaccacttg gttcttcaac cctgttatat tgcgccctaa caacgtagag 780

gtggagtttc tcttaaacgg acagatcatc aatacctacc aagccaggtt cggcacgatt 840

attgcaagaa atttcgacgc tatcaggctg ctcttccaac tgatgaggcc ccccaatatg 900

actcccgctg tgaacgcttt gtttccgcag gctcagcctt tccagcacca cgccaccgtc 960

ggcttgactc ttcgaataga gagcgcggtc tgcgaatcag tgctggcaga cgccaacgag 1020

acgctgctgg caaacgttac cgccgtgcgg caagagtatg ccatcccagt agggcctgtg 1080

tttccacccg gcatgaactg gactgaacta attactaact atagcccatc cagagaagac 1140

aacttgcagc gggtcttcac tgtggcctct atccggagta tgttgatcaa gtag 1194

<210> 47
<211> 2328
<212> DNA
<213> Artificial sequence

<220>
<223> Optimized coding sequence of Rotavirus A VP4 from strain
RVA/Vaccine/USA/Rotarix-A41CB052A/1988/G1P1A[8]

<400> 47
atggctagcc tgatctacag acaactcttg accaattcat attctgtgga tcttcatgac 60

gaaatcgagc agattgggtc cgagaagacc cagaacgtga ccatcaaccc tggacctttt 120

gctcagaccc gctatgcccc tgtgaattgg gatcacggag aaatcaacga cagtacgacc 180

gtcgaaccca ttctggacgg gccataccaa cccaccacct tcaccccacc taatgattat 240

tggattttaa tcaactccaa cacaaacgga gtggtctacg agtccactaa taactccgat 300

ttttggaccg ccgttgtagc catcgagcca cacgtcaatc ctgtcgatcg ccagtatatg 360

atattcggcg agtccaaaca gtttaacgtt tccaatgaca gcaacaaatg gaagtttctg 420

gagatgtttc gcagctcctc tcagaacgaa ttctataata gacggaccct tacctccgat 480

acacgactcg tgggtatttt taagtacggc ggcagggtgt ggacatttca cggtgaaacc 540

cctcgagcaa ccactgactc cagtagcact gcaaacctga acaatatatc tattaccatc 600

cacagcgaat tctacataat cccaagatct caggaaagta agtgtaacga atatatcaac 660

aacggactcc ccccaattca gaatacacgg aacgtggtgc ctctcccact cagttctcgg 720

tctatccagt ataagagagc acaagtgaat gaggacatta ttgtgagcaa gactagcctt 780

tggaaagaaa tgcagtacaa cagagacatt atcatccggt ttaagtttgg gaactctatc 840

gtgaagatgg gcggcctggg gtacaaatgg tcagaaatct catataaagc cgccaactat 900

cagtataact acttgagaga cggcgagcag gtaaccgccc acacaacatg ctctgtcaac 960

ggcgttaata actttagcta caacggaggc ttccttccca ccgacttcgg tatcagccgg 1020

tatgaagtca tcaaggaaaa ttcttatgtg tacgtagatt actgggatga tagcaaagcg 1080

ttccgcaaca tggtgtatgt taggagcctg gctgctaatc tcaattctgt gaagtgtact 1140

ggtggatcat attatttctc aattcccgtg ggggcttggc cagtcatgaa tggcggggca 1200

gtctccctcc attttgctgg cgtgacgttg agcactcagt ttaccgattt cgtgtctctg 1260

aactccctga ggttccggtt ttcccttact gtcgacgagc ccccattcag cattctgcgt 1320

acaagaactg tcaacctcta cgggttacct gccgcgaatc caaacaacgg caatgaatac 1380

tatgaaattt cgggccgctt ctctttgata agtctggtac caactaatga cgactatcag 1440

acacccatca tgaacagcgt gactgtcaga caggacctgg aaagacaact tacagatctg 1500

cgggaagaat tcaattctct cagtcaggag attgcaatgg cccaattgat agatcttgcc 1560

ctactgcctc tcgatatgtt tagtatgttc tccggcatca aatcaactat agatctgaca 1620

aagagcatgg ctacttctgt gatgaagaag ttcaggaaat caaaacttgc cacgagcata 1680

tcagaaatga cgaactctct gagtgatgca gcatcatcag cgtcacgcaa cgtttccatt 1740

cggtcgaatc tcagcgccat cagcaactgg acaaacgtgt ccaacgacgt cagcaacgtg 1800

accaactcct tgaacgatat ttctacccag acgtcaacga tcagtaagaa actccgcttg 1860

aaagaaatga tcacccagac tgagggaatg tctttcgacg acatttccgc cgccgtgcta 1920

aaaaccaaaa tcgatatgtc tactcagatc ggcaagaaca ctctgccgga tatcgtaacc 1980

gaagcctccg aaaagtttat ccctaagcgc agctacagaa tattgaaaga tgacgaggtc 2040

atggagatca acacagaagg gaagttcttc gcttataaga tcaacacctt tgacgaggtt 2100

ccgtttgacg tcaataagtt tgcagagctc gtgacagata gtccagtgat ttctgccatc 2160

attgacttta agactttgaa gaacctgaac gacaactatg gaataacacg gaccgaagcg 2220

ttgaacctca ttaagtccaa tcccaatatg ttgcgcaatt tcattaacca gaacaatcca 2280

atcataagaa ataggattga gcaattaatc ctgcaatgta aactctga 2328

<210> 48
<211> 981
<212> DNA
<213> Rotavirus

<400> 48
atgtacggca tcgagtatac aacaatttta attttcctga tttccatcat tctgttaaac 60

tacatcctta agtccgtgac cagaattatg gattatatta tctatcgtag cctcctcatc 120

tacgtggccc tttttgccct gaccagggcc cagaactatg gcctgaactt accaatcacc 180

ggttcaatgg ataccgttta cgctaattcc actcaagagg ggatatttct gacaagtacc 240

ctgtgcctgt attatccaac agaagcctct acccagatca atgatgggga gtggaaggat 300

agtctctcac agatgttcct aaccaagggc tggcccaccg gttccgtcta cttcaaggaa 360

tactctagta ttgtcgactt ctcagttgac ccccagcttt attgcgacta caacctggta 420

cttatgaaat acgaccagaa cctggagctg gatatgtccg agctggctga cctgatcctc 480

aatgagtggc tgtgcaaccc catggacatc acattatatt actaccagca gtctggagaa 540

tccaacaagt ggatcagtat gggctcaagt tgcaccgtga aggtgtgtcc cttgaacacc 600

caaatgctgg gcattggttg tcagacaact aatgtggatt cgtttgaaat ggtagccgaa 660

aacgagaagc tggctatagt ggacgtagtc gatgggatta accacaagat caatctgact 720

accaccactt gtaccatcag aaactgtaaa aagctcggcc cccgggagaa cgtcgccgtg 780

atccaggtgg gggggagcaa tgtgctcgac attactgccg accctaccac caatccacag 840

acggaacgga tgatgagagt caactggaag aaatggtggc aggtctttta taccattgtg 900

gactacatta accagattgt gcaagtcatg agtaaacggt ccagatccct gaactcagca 960

gccttctatt atcgcgttta g 981

<210> 49
<211> 903
<212> DNA
<213> Rotavirus

<400> 49
atggcgaaaa acgttgcgat tttcggctta ttgttttctc ttcttgtgtt ggttccttct 60

cagatcttcg cccagaacta tggcctgaac ttaccaatca ccggttcaat ggataccgtt 120

tacgctaatt ccactcaaga ggggatattt ctgacaagta ccctgtgcct gtattatcca 180

acagaagcct ctacccagat caatgatggg gagtggaagg atagtctctc acagatgttc 240

ctaaccaagg gctggcccac cggttccgtc tacttcaagg aatactctag tattgtcgac 300

ttctcagttg acccccagct ttattgcgac tacaacctgg tacttatgaa atacgaccag 360

aacctggagc tggatatgtc cgagctggct gacctgatcc tcaatgagtg gctgtgcaac 420

cccatggaca tcacattata ttactaccag cagtctggag aatccaacaa gtggatcagt 480

atgggctcaa gttgcaccgt gaaggtgtgt cccttgaaca cccaaatgct gggcattggt 540

tgtcagacaa ctaatgtgga ttcgtttgaa atggtagccg aaaacgagaa gctggctata 600

gtggacgtag tcgatgggat taaccacaag atcaatctga ctaccaccac ttgtaccatc 660

agaaactgta aaaagctcgg cccccgggag aacgtcgccg tgatccaggt gggggggagc 720

aatgtgctcg acattactgc cgaccctacc accaatccac agacggaacg gatgatgaga 780

gtcaactgga agaaatggtg gcaggtcttt tataccattg tggactacat taaccagatt 840

gtgcaagtca tgagtaaacg gtccagatcc ctgaactcag cagccttcta ttatcgcgtt 900

tag 903

<210> 50
<211> 2331
<212> DNA
<213> Rotavirus

<400> 50
atggcttcgc tcatttatag acaattgctc acgaattctt atacagtaga tttatccgat 60

gagatacaag agattggatc aactaaatca caaaatgtca caattaatcc tggaccattt 120

gcgcaaacag gttatgctcc agttaactgg ggacctggag aaattaatga ttctacgaca 180

gttgaaccat tgctggatgg gccttatcaa ccaacgacat tcaatccacc agtcgattat 240

tggatgttac tggctccaac gacacctggc gtaattgttg aaggtacaaa taatacagat 300

agatggttag ccacaatttt aatcgagcca aatgttcagt ctgaaaatag aacttacact 360

atatttggta ttcaagaaca attaacggta tccaatactt cacaagacca gtggaaattt 420

attgatgtcg taaaaacaac tgcaaatgga agtataggac aatatggacc attactatcc 480

agtccgaaat tatatgcagt tatgaagcat aatgaaaaat tatatacata tgaaggacag 540

acacctaacg ctaggacagc acattattca acaacgaatt atgattctgt taatatgact 600

gctttttgtg acttttatat aattcctaga tctgaagagt ctaaatgtac ggaatacatt 660

aataatggat taccaccaat acaaaatact agaaatgttg taccattatc gttgactgct 720

agagatgtaa tacactatag agctcaagct aatgaagata ttgtgatatc caagacatca 780

ttgtggaaag aaatgcaata taatagagat ataactatta gatttaaatt tgcaaataca 840

attataaaat caggagggct gggatataag tggtcagaaa tatcatttaa gccagcgaat 900

tatcaataca catatactcg tgatggtgaa gaagttaccg cacatactac ttgttcagtg 960

aatggcgtta atgacttcag ttttaatgga ggatatttac caactgattt tgttgtatct 1020

aaatttgaag taattaaaga gaattcatac gtctatatcg attactggga tgattcacaa 1080

gcatttcgta acgtggtgta tgtccgatcg ttagcagcaa acttgaattc agttatgtgt 1140

actggaggca gctataattt tagtctacca gttggacaat ggcctgtttt aactggggga 1200

gcagtttctt tacattcagc tggtgtaaca ctatctactc aatttacaga tttcgtatca 1260

ttaaattcat taagatttag atttagacta gctgtcgaag aaccacactt taaactgact 1320

agaactagat tagatagatt gtatggtctg cctgctgcag atccaaataa tggtaaagaa 1380

tattatgaaa ttgctggacg attttcactt atatcattag tgccatcaaa tgatgactat 1440

cagactccta tagcaaactc agttactgta cgacaagatt tagaaaggca gttaggagaa 1500

ctaagagaag agtttaacgc tttgtctcaa gaaattgcaa tgtcgcagtt aatcgattta 1560

gcgcttctac cattagatat gttctcaatg ttttctggca ttaaaagtac tattgatgct 1620

gcaaaatcaa tggctactaa tgttatgaaa aaattcaaaa agtcaggatt agcgaattca 1680

gtttcaacac tgacagattc tttatcagac gcagcatcat caatatcaag aggttcatct 1740

atacgttcga ttggatcttc agcatcagca tggacggatg tatcaacaca aataactgat 1800

atatcgtcat cagtaagttc agtttcgaca caaacgtcaa ctatcagtag aagattgaga 1860

ctaaaggaaa tggcaacaca aactgagggt atgaattttg atgatatatc agcggctgtt 1920

ttgaagacta agatagataa atcgactcaa atatcaccaa acacaatacc tgacattgtt 1980

actgaagcat cggaaaaatt cataccaaat agggcttacc gtgttataaa caacgatgat 2040

gtgtttgaag ctggaattga tggaaaattt tttgcttata aagtggatac atttgaggaa 2100

ataccatttg atgtacaaaa attcgctgac ttagttacag attctccagt aatatccgct 2160

ataattgatt ttaaaacact taaaaatttg aacgataatt acggcattac taagcaacaa 2220

gcatttaatc ttttaagatc tgacccaaga gttttacgtg aattcattaa tcaggacaat 2280

cctataatta gaaatagaat tgaacaactg attatgcaat gcaggttgtg a 2331

<210> 51
<211> 2331
<212> DNA
<213> Artificial sequence

<220>
<223> Optimized coding sequence of Rotavirus A VP4 from strain
RVA/Simian-tc/ZAF/SA11-H96/1958/G3P5B[2

<400> 51
atggcttcat tgatatatcg ccagttgctg actaatagct atactgtgga tttgtcagac 60

gaaatccagg aaataggatc cacaaagagt cagaacgtga ccataaaccc cggaccgttc 120

gcccagactg ggtatgcccc cgtaaactgg ggccccggcg agattaacga cagcaccacc 180

gtggagccac tgctggatgg accctaccaa cccactactt ttaatcctcc agtggactac 240

tggatgttgt tggctcccac gacacctggt gtaattgtag agggcaccaa caataccgat 300

cgctggctgg cgacaatact gatagaaccc aacgtgcagt ccgagaacag aacctatacc 360

attttcggca tccaggaaca gctaaccgtg agcaatacga gccaggacca gtggaagttt 420

atcgatgtag tgaaaactac ggccaatgga tctatcgggc aatacgggcc gctgctgtcc 480

tcacctaagc tctacgccgt gatgaaacat aatgagaaac tgtacactta cgagggccaa 540

acccccaatg ccagaactgc ccactacagt acaaccaact atgactcggt gaacatgaca 600

gcgttctgtg atttttatat tattccaaga tcagaagaat ccaagtgtac tgagtacatc 660

aacaatggac ttccacccat ccagaacact cgaaatgtcg tcccactgtc tctaactgct 720

cgggatgtga tccactatcg cgcccaagct aatgaggata tagtcatttc aaagacgagc 780

ttatggaagg aaatgcagta taacagagac atcacaatca ggttcaagtt cgccaatact 840

attattaagt ccgggggact ggggtacaaa tggagtgaga tcagttttaa gcccgctaac 900

tatcagtaca cctatactcg cgacggcgaa gaggtaaccg cccacacaac ttgctcggtt 960

aatggcgtga acgattttag cttcaacggg ggctacctgc ctactgattt cgtggtgagc 1020

aagtttgaag tcatcaagga aaattcctac gtgtatattg actactggga tgatagccag 1080

gccttccgaa atgttgtgta tgttagatca ctggccgcaa accttaattc agtcatgtgc 1140

acaggaggtt cttacaattt tagtcttccc gtcgggcagt ggccagtgct cacagggggc 1200

gctgtgagct tgcattccgc cggagtcacc ttgagtactc agttcacaga ctttgtgtct 1260

ctgaatagcc taaggttcag gtttagactt gcagtagaag agcctcactt taagctcact 1320

cgtacgaggc tggatcggct gtacggcctg ccggccgctg atcccaataa cggcaaggaa 1380

tattacgaga tagccgggag attttcgctg atcagtctgg tgccgtcaaa cgatgattac 1440

cagaccccaa ttgccaacag tgtcactgtc aggcaagatc tggagagaca acttggcgag 1500

ctgagagagg agttcaacgc cctgtctcaa gagatcgcaa tgtctcagct cattgacctg 1560

gccctgttac ccctcgacat gttctcaatg ttctccggca taaaatccac tatcgacgct 1620

gcaaagtcca tggccacaaa tgtgatgaag aagtttaaga agagcggtct ggcaaatagc 1680

gtgtctacgc tgaccgatag tttgtcggat gccgccagtt ccattagccg tggatccagc 1740

attaggtcca ttggctcttc cgcctctgct tggactgacg tgagtacaca gataactgac 1800

atttcctctt ctgtctccag tgtgagcaca caaacttcca cgatatcaag acgactgagg 1860

ctcaaagaga tggcaacgca aacggaaggt atgaattttg atgacatcag cgccgcagtt 1920

ttgaagacaa agatcgataa aagcactcaa attagcccca atacgatccc tgacattgtg 1980

actgaggcat ctgaaaagtt cattcccaac cgtgcttatc gggtcattaa caatgatgat 2040

gtcttcgagg ccggcatcga tggcaagttt tttgcttata aagtggatac cttcgaggag 2100

attcctttcg atgtacagaa gtttgctgac ctcgtaacgg atagcccagt gataagcgcc 2160

attatagact tcaaaacatt gaaaaatttg aacgataatt atggtattac caagcagcag 2220

gcttttaact tgttaagatc tgaccctcgc gtgctcagag agtttattaa ccaggacaac 2280

cccatcatca gaaacaggat cgagcagctg attatgcagt gtcgcctgta a 2331

<210> 52
<211> 981
<212> DNA
<213> Artificial sequence

<220>

<223>  Coding sequence of Rotavirus A VP7 from strain
      RVA/Simian-tc/ZAF/SA11-H96/1958/G3P5B[2]

<400>  52
atgtatggta ttgaatatac cacagttcta acctttctga tatcgattat tctactaaat      60

tacatactta aatcattaac tagaataatg gactttataa tttatagatt tctttttata     120

attgtgatat tgtcaccatt tctcagagca caaaattatg gtattaatct tccaatcaca     180

ggctccatgg acactgcata cgctaattca acgcaagaag aaacattcct cacttctaca     240

ctttgcctat attatccgac tgaggctgcg actgaaataa acgataattc atggaaagac     300

acactgtcac aactatttct tacgaaaggg tggccaactg gatccgtata ttttaaagaa     360

tatactaaca ttgcatcgtt ttctgttgat ccgcagttgt attgtgatta taacgtagta     420

ctaatgaaat atgacgcgac gttgcaattg gatatgtcag aacttgcgga tctaatatta     480

aacgaatggt tgtgtaatcc aatggatatt actctgtatt attatcagca aactgacgaa     540

gcgaataaat ggatatcaat gggctcatca tgtacaatta aagtatgtcc acttaataca     600

caaactcttg gaattggatg cttgacaact gatgctacaa cttttgaaga agttgcgaca     660

gctgaaaagt tggtaattac tgacgtggtt gatggcgtta atcataagct ggatgtcaca     720

acagcaacgt gtactattag aaactgtaag aaattgggac caagagaaaa cgtagccgtt     780

atacaagttg gtggttctga catcctcgat ataactgctg atccaactac tgcaccacag     840

acagaacgga tgatgcgaat taactggaaa aaatggtggc aagtttttta tactgtagta     900

gactatgtag atcagataat acaagttatg tccaaaagat caagatcact aaattcagca     960

gcattttatt acagagtgta g                                               981


<210>  53
<211>  981
<212>  DNA
<213>  Rotavirus

<400>  53
atgtacggaa tcgagtatac caccgttctg acatttctta ttagtattat cctcttgaac      60

tatattctga agtcacttac ccggataatg gattttatta tatataggtt tctgttcatc     120

attgtaattc tgagcccttt cctgagggcc cagaattacg gcataaacct accaatcacc     180

ggttctatgg ataccgctta tgctaactct acacaagagg agacattcct cacatcaacc     240

ctatgcctgt actatccgac tgaagcagcc acagagataa acgataactc ttggaaagat     300

acattgagcc agctcttcct gactaaggga tggcccaccg gatcggtcta ctttaaggag     360

tacacaaaca tcgcaagttt cagcgtggat ccccagctgt attgtgatta taacgttgtg     420

ctgatgaaat acgacgcaac cctccagctt gacatgagcg agttggcaga cctaatcctc     480

aatgagtggc tgtgtaaccc aatggatata acactgtact attatcagca gaccgatgaa     540

gcaaacaaat ggatttcaat gggaagcagc tgtaccatca aagtttgtcc tctcaacacc 600

caaactctcg gcatagggtg tctgaccaca gacgctacta cctttgaaga agttgcgacc 660

gcggaaaagc tggttatcac agatgtggta gatggcgtta accacaaatt ggacgtaacc 720

acagcaacat gcacaattag gaactgcaag aagctaggac ccagggaaaa cgtagccgtc 780

atccaagtgg gcggcagtga catcctagac atcaccgcag acccaacaac agcaccacaa 840

accgagagga tgatgcgcat taattggaag aaatggtggc aggtgtttta cactgtcgtt 900

gactatgtgg accagatcat tcaggtgatg agcaagcgga gtcgctcatt gaatagtgct 960

gccttttatt acagagtcta a 981

<210> 54
<211> 981
<212> DNA
<213> Artificial sequence

<220>
<223> Optimized coding sequence of Rotavirus A VP7

<400> 54
atgtacggca tcgagtatac aacaatttta attttcctga tttccatcat tctgttaaac 60

tacatcctta agtccgtgac cagaattatg gattatatta tctatcgtag cctcctcatc 120

tacgtggccc tttttgccct gaccagggcc cagaactatg gcctgaactt accaatcacc 180

ggttcaatgg ataccgttta cgctaattcc actcaagagg ggatatttct gacaagtacc 240

ctgtgcctgt attatccaac agaagcctct acccagatca atgatgggga gtggaaggat 300

agtctctcac agatgttcct aaccaagggc tggcccaccg gttccgtcta cttcaaggaa 360

tactctagta ttgtcgactt ctcagttgac ccccagcttt attgcgacta caacctggta 420

cttatgaaat acgaccagaa cctggagctg gatatgtccg agctggctga cctgatcctc 480

aatgagtggc tgtgcaaccc catggacatc acattatatt actaccagca gtctggagaa 540

tccaacaagt ggatcagtat gggctcaagt tgcaccgtga aggtgtgtcc cttgaacacc 600

caaatgctgg gcattggttg tcagacaact aatgtggatt cgtttgaaat ggtagccgaa 660

aacgagaagc tggctatagt ggacgtagtc gatgggatta accacaagat caatctgact 720

accaccactt gtaccatcag aaactgtaaa aagctcggcc cccgggagaa cgtcgccgtg 780

atccaggtgg gggggagcaa tgtgctcgac attactgccg accctaccac caatccacag 840

acggaacgga tgatgagagt caactggaag aaatggtggc aggtctttta taccattgtg 900

gactacatta accagattgt gcaagtcatg agtaaacggt ccagatccct gaactcagca 960

gccttctatt atcgcgttta g 981

<210> 55
<211> 50

<212>  DNA
<213>  Artificial sequence

<220>
<223>  Primer IF-TrSP+Rtx_VP7(opt).s1+3c

<400>  55
aaatttgtcg ggcccatgga ttatattatc tatcgtagcc tcctcatcta                50


<210>  56
<211>  54
<212>  DNA
<213>  Artificial sequence

<220>
<223>  Primer IF-Rtx_VP7(opt).s1-4r

<400>  56
actaaagaaa ataggcctct aaacgcgata atagaaggct gctgagttca ggga          54


<210>  57
<211>  981
<212>  DNA
<213>  Artificial sequence

<220>
<223>  Optimized coding sequence of Rotavirus A VP7 from strain
       RVA/Vaccine/USA/Rotarix-A41CB052A/1988/G1P1A[8]

<400>  57
atgtacggca tcgagtatac aacaatttta attttcctga tttccatcat tctgttaaac      60

tacatcctta agtccgtgac cagaattatg gattatatta tctatcgtag cctcctcatc     120

tacgtggccc tttttgccct gaccagggcc cagaactatg gcctgaactt accaatcacc     180

ggttcaatgg ataccgttta cgctaattcc actcaagagg ggatatttct gacaagtacc     240

ctgtgcctgt attatccaac agaagcctct acccagatca atgatgggga gtggaaggat     300

agtctctcac agatgttcct aaccaagggc tggcccaccg gttccgtcta cttcaaggaa     360

tactctagta ttgtcgactt ctcagttgac ccccagcttt attgcgacta caacctggta     420

cttatgaaat acgaccagaa cctggagctg gatatgtccg agctggctga cctgatcctc     480

aatgagtggc tgtgcaaccc catggacatc acattatatt actaccagca gtctggagaa     540

tccaacaagt ggatcagtat gggctcaagt tgcaccgtga aggtgtgtcc cttgaacacc     600

caaatgctgg gcattggttg tcagacaact aatgtggatt cgtttgaaat ggtagccgaa     660

aacgagaagc tggctatagt ggacgtagtc gatgggatta accacaagat caatctgact     720

accaccactt gtaccatcag aaactgtaaa aagctcggcc cccgggagaa cgtcgccgtg     780

atccaggtgg gggggagcaa tgtgctcgac attactgccg accctaccac caatccacag     840

acggaacgga tgatgagagt caactggaag aaatggtggc aggtctttta taccattgtg     900

gactacatta accagattgt gcaagtcatg agtaaacggt ccagatccct gaactcagca     960

gccttctatt atcgcgttta g                                              981

<210>  58
<211>  2634
<212>  DNA
<213>  Artificial sequence

<220>
<223>  Expression cassette number 1734

<400>  58
gtcaacatgg tggagcacga cacacttgtc tactccaaaa atatcaaaga tacagtctca      60

gaagaccaaa gggcaattga gacttttcaa caaagggtaa tatccggaaa cctcctcgga     120

ttccattgcc cagctatctg tcactttatt gtgaagatag tggaaaagga aggtggctcc     180

tacaaatgcc atcattgcga taaaggaaag gccatcgttg aagatgcctc tgccgacagt     240

ggtcccaaag atggaccccc acccacgagg agcatcgtgg aaaaagaaga cgttccaacc     300

acgtcttcaa agcaagtgga ttgatgtgat aacatggtgg agcacgacac acttgtctac     360

tccaaaaata tcaaagatac agtctcagaa gaccaaaggg caattgagac ttttcaacaa     420

agggtaatat ccggaaacct cctcggattc cattgcccag ctatctgtca ctttattgtg     480

aagatagtgg aaaaggaagg tggctcctac aaatgccatc attgcgataa aggaaaggcc     540

atcgttgaag atgcctctgc cgacagtggt cccaaagatg gacccccacc cacgaggagc     600

atcgtggaaa aagaagacgt tccaaccacg tcttcaaagc aagtggattg atgtgatatc     660

tccactgacg taagggatga cgcacaatcc cactatcctt cgcaagaccc ttcctctata     720

taaggaagtt catttcattt ggagaggtat taaaatctta ataggttttg ataaaagcga     780

acgtggggaa acccgaacca aaccttcttc taaactctct ctcatctctc ttaaagcaaa     840

cttctctctt gtctttcttg cgtgagcgat cttcaacgtt gtcagatcgt gcttcggcac     900

cagtacaacg ttttctttca ctgaagcgaa atcaaagatc tctttgtgga cacgtagtgc     960

ggcgccatta aataacgtgt acttgtccta ttcttgtcgg tgtggtcttg ggaaaagaaa    1020

gcttgctgga ggctgctgtt cagccccata cattacttgt tacgattctg ctgactttcg    1080

gcgggtgcaa tatctctact tctgcttgac gaggtattgt tgcctgtact tctttcttct    1140

tcttcttgct gattggttct ataagaaatc tagtattttc tttgaaacag agttttcccg    1200

tggttttcga acttggagaa agattgttaa gcttctgtat attctgccca aatttgtcgg    1260

gcccatggat tatattatct atcgtagcct cctcatctac gtggcccttt ttgccctgac    1320

cagggcccag aactatggcc tgaacttacc aatcaccggt tcaatggata ccgtttacgc    1380

taattccact caagagggga tatttctgac aagtaccctg tgcctgtatt atccaacaga    1440

agcctctacc cagatcaatg atggggagtg gaaggatagt ctctcacaga tgttcctaac    1500

caagggctgg cccaccggtt ccgtctactt caaggaatac tctagtattg tcgacttctc 1560

agttgacccc cagctttatt gcgactacaa cctggtactt atgaaatacg accagaacct 1620

ggagctggat atgtccgagc tggctgacct gatcctcaat gagtggctgt gcaaccccat 1680

ggacatcaca ttatattact accagcagtc tggagaatcc aacaagtgga tcagtatggg 1740

ctcaagttgc accgtgaagg tgtgtccctt gaacacccaa atgctgggca ttggttgtca 1800

gacaactaat gtggattcgt ttgaaatggt agccgaaaac gagaagctgg ctatagtgga 1860

cgtagtcgat gggattaacc acaagatcaa tctgactacc accacttgta ccatcagaaa 1920

ctgtaaaaag ctcggccccc gggagaacgt cgccgtgatc caggtggggg ggagcaatgt 1980

gctcgacatt actgccgacc ctaccaccaa tccacagacg gaacggatga tgagagtcaa 2040

ctggaagaaa tggtggcagg tcttttatac cattgtggac tacattaacc agattgtgca 2100

agtcatgagt aaacggtcca gatccctgaa ctcagcagcc ttctattatc gcgtttagag 2160

gcctattttc tttagtttga atttactgtt attcggtgtg catttctatg tttggtgagc 2220

ggttttctgt gctcagagtg tgtttatttt atgtaattta atttctttgt gagctcctgt 2280

ttagcaggtc gtcccttcag caaggacaca aaaagatttt aattttatta aaaaaaaaaa 2340

aaaaaaagac cgggaattcg atatcaagct tatcgacctg cagatcgttc aaacatttgg 2400

caataaagtt tcttaagatt gaatcctgtt gccggtcttg cgatgattat catataattt 2460

ctgttgaatt acgttaagca tgtaataatt aacatgtaat gcatgacgtt atttatgaga 2520

tgggttttta tgattagagt cccgcaatta tacatttaat acgcgataga aaacaaaata 2580

tagcgcgcaa actaggataa attatcgcgc gcggtgtcat ctatgttact agat 2634

<210> 59
<211> 297
<212> PRT
<213> Rotavirus

<400> 59

Met Asp Tyr Ile Ile Tyr Arg Ser Leu Leu Ile Tyr Val Ala Leu Phe
1               5                   10                  15

Ala Leu Thr Arg Ala Gln Asn Tyr Gly Leu Asn Leu Pro Ile Thr Gly
            20                  25                  30

Ser Met Asp Thr Val Tyr Ala Asn Ser Thr Gln Glu Gly Ile Phe Leu
        35                  40                  45

Thr Ser Thr Leu Cys Leu Tyr Tyr Pro Thr Glu Ala Ser Thr Gln Ile
    50                  55                  60

Asn Asp Gly Glu Trp Lys Asp Ser Leu Ser Gln Met Phe Leu Thr Lys

65                  70                  75                  80

Gly Trp Pro Thr Gly Ser Val Tyr Phe Lys Glu Tyr Ser Ser Ile Val
                85                  90                  95

Asp Phe Ser Val Asp Pro Gln Leu Tyr Cys Asp Tyr Asn Leu Val Leu
            100                 105                 110

Met Lys Tyr Asp Gln Asn Leu Glu Leu Asp Met Ser Glu Leu Ala Asp
        115                 120                 125

Leu Ile Leu Asn Glu Trp Leu Cys Asn Pro Met Asp Ile Thr Leu Tyr
    130                 135                 140

Tyr Tyr Gln Gln Ser Gly Glu Ser Asn Lys Trp Ile Ser Met Gly Ser
145                 150                 155                 160

Ser Cys Thr Val Lys Val Cys Pro Leu Asn Thr Gln Met Leu Gly Ile
                165                 170                 175

Gly Cys Gln Thr Thr Asn Val Asp Ser Phe Glu Met Val Ala Glu Asn
            180                 185                 190

Glu Lys Leu Ala Ile Val Asp Val Val Asp Gly Ile Asn His Lys Ile
        195                 200                 205

Asn Leu Thr Thr Thr Thr Cys Thr Ile Arg Asn Cys Lys Lys Leu Gly
    210                 215                 220

Pro Arg Glu Asn Val Ala Val Ile Gln Val Gly Gly Ser Asn Val Leu
225                 230                 235                 240

Asp Ile Thr Ala Asp Pro Thr Thr Asn Pro Gln Thr Glu Arg Met Met
                245                 250                 255

Arg Val Asn Trp Lys Lys Trp Trp Gln Val Phe Tyr Thr Ile Val Asp
            260                 265                 270

Tyr Ile Asn Gln Ile Val Gln Val Met Ser Lys Arg Ser Arg Ser Leu
        275                 280                 285

Asn Ser Ala Ala Phe Tyr Tyr Arg Val
    290                 295

## Description

### FIELD OF INVENTION

This invention relates to producing rotavirus structural proteins in plants. More specifically, the present invention relates to producing virus-like particles comprising rotavirus structural protein in plants.

### BACKGROUND OF THE INVENTION

Rotavirus infection is a global problem mainly affecting children under the age of five. It results in severe gastroenteritis and in worst cases death.

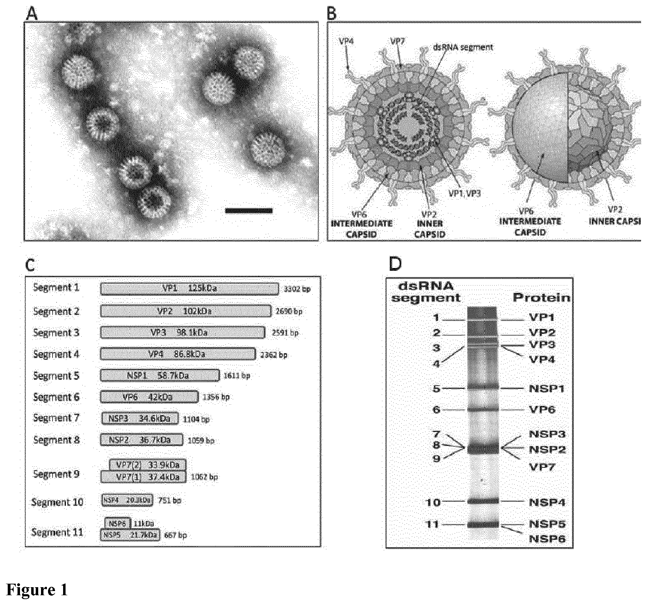

Rotaviruses are members of the Reoviridae family of viruses (genus Rotavirus) that affect the gastrointestinal system and respiratory tract. The name is derived from the wheel like appearance of virions when viewed by negative contrast electron microscopy (Figure 1a; prior art). The rotavirus is usually globular shape and is named after the outer and inner shells or double-shelled capsid structure of the same. The outer capsid is about 70 nm, and inner capsid is about 55 nm in diameter, respectively. The double-shelled capsid of the rotavirus surrounds the core including the inner protein shell and genome. The genome of the rotavirus consists of double stranded RNA segments encoding at least 11 rotavirus proteins.

The dsRNA codes for six structural proteins (VP) and six non-structural proteins (NSP) (Figure 1c; prior art). The structural proteins comprise VP1, VP2, VP3, VP4, VP6 and VP7 (Figure 1b; prior art). Three concentric layers are formed by the assembly of VP2, VP6 and VP7 respectively, with VP4 forming "spikes" on the surface of the virus structure. The NSPs are synthesized in infected cells and function in various parts of the replication cycle or interact with some of the host proteins to influence pathogenesis or the immune response to infection (Greenberg and Estes, 2009).

VP2 is a 102 kDa protein and is the most abundant protein of the viral core. It forms the inner-most structural protein layer and provides a scaffold for the correct assembly of the components and transcription enzymes of the viral core (Lawton, 2000). VP1, the largest viral protein at 125 kDa, acts as an RNA-dependent polymerase for rotavirus, creating a core replication intermediate, and associates with VP2 at its icosahedral vertices (Varani and Allain, 2002; Vende et al., 2002). VP3, a 98 kDa protein, is also directly associated with the viral genome, acting as an mRNA capping enzyme that adds a 5' cap structure to viral mRNAs. Together, VP1 and VP3 form a complex that is attached to the outer 5-fold vertices of the VP2 capsid layer (Angel, 2007). VP6 is a 42 kDa protein which forms the middle shell of the viral core, is the major capsid protein and accounts for more than 50% of the total protein mass of the virion (Gonzalez et al., 2004; Estes, 1996). It is required for gene transcription and may have a role in encapsulation of the rotavirus RNA by anchoring VP1 to VP2 in the core, as seen in bluetongue virus, another member of the Reoviridae family. It also determines the classification of rotaviruses into five groups (A to E) with group A most commonly affecting humans (Palombo, 1999). VP6 in rotavirus group A has at least four subgroups (SG), which depend on the presence or absence of SG specific epitopes: SG I, SG II, SG (I+II) and SG non-(I+II). Groups B and C lack a common group A antigen but are also known to infect humans, while group D only affects animals e.g chickens and cows (Thongprachum, 2010).

The two outer capsid proteins VP7, a 37 kDa glycoprotein (G) and the 87 kDa protease sensitive VP4 (P), define the virus' serotypes. These two proteins induce neutralizing antibody responses and are thus used to classify rotavirus serotypes into a dual nomenclature system, depending on the G-P antigen combination (e.g. G1 P[8] or G2 P[4]) (Sanchez-Padilla et al., 2009). The VP4 protein dimerizes to form 60 spikes on the outer shell of the virus, which are directly involved in the initial stages of host cell entry. The spike protein contains a cleavage site at amino acid (aa) position 248. Upon infection, it is cleaved by the protease trypsin to produce VP5 (529 aa, 60 kDa) and VP8 (246 aa, 28 kDa) (Denisova et al., 1999). This process enhances virus infectivity (cell attachment and invasion of host cell) and stabilizes the spike structure (Glass, 2006). The VP7 glycoprotein forms the third or outside layer of the virus. At present, 27 G and 35 P genotypes are known (Greenberg and Estes, 2009). VP4 and VP7 are the major antigens involved in virus neutralization and are important targets for vaccine development (Dennehy, 2007).

In infected mammalian cells, rotaviruses undergo a unique mode of morphogenesis to form the complete triple-layered VP2/6/4/7 viral particles (Lopez et al., 2005). The triple-layer capsid is a very stable complex which enables faecal-oral transmission and delivery of the virus into the small intestine where it infects non-dividing differentiated enterocytes near the tips of the villi (Greenberg and Estes, 2009). Firstly, the intact virus attaches to sialic acid-independent receptors via 60 VP4 dimer spikes on the surface of the virus (Lundgren and Svensson, 2001). The 60 VP4 dimer spikes on the surface of the virus allow the virus to attach to these cell receptors. VP4 is susceptible to proteolytic cleavage by trypsin which results in a conformational change that exposes additional attachment sites on the surface of the glycoprotein for interaction with a series of co-receptors.

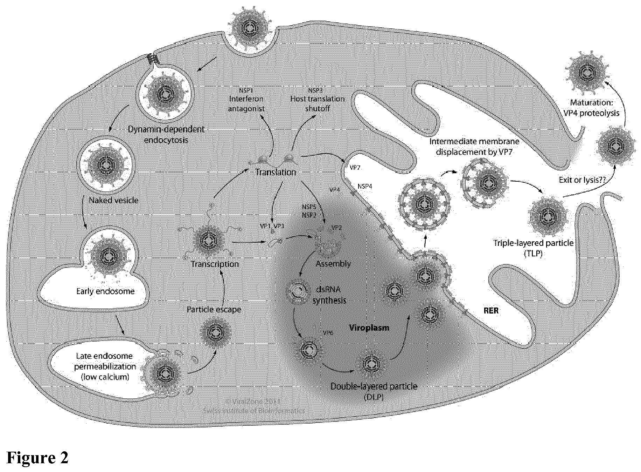

The multi-step attachment and entry process is, however, not clearly understood but the virus is delivered across the host's plasma membrane. The VP7 outer capsid shell which is also involved in the entry process, is removed in the process and double-layered particles (DLP) are delivered into the cell cytoplasm in vesicles (Figure 2; prior art). The DLP escapes from the vesicle and goes into non-membrane bound cytoplasmic inclusions. Early transcription of the genome by VP1 begins in particles so that dsRNA is never exposed to the cytoplasm. RNA replication and core formation takes place in these non-membrane-bound cytoplasmic inclusions. The nascent (+) RNAs are then transported into the cytoplasm and serve as templates for viral protein synthesis. VP4 is produced in the cytosol and transported to the rough endoplasmic reticulum (RER), and VP7 is secreted into the RER. VP2 and VP6 are produced and assemble in the cytosol in virosomes and subsequently bud into the RER compartments, receiving a transient membrane envelope in the process (Lopez et al., 2005; Tian et al., 1996). In the RER, the transient envelopes of the viral particles are removed and replaced by VP4 and VP7 protein monomers, with critical involvement of rotaviral glycoprotein NSP4 (Tian et al., 1996; Lopez et al., 2005; Gonzalez et al., 2000). NSP4 functions as an intracellular receptor in the ER membrane and binds newly made subviral particles and probably also the spike protein VP4 (Tian et al., 1996). NSP4 is also toxic to humans and is the causative agent of the diarrhea. The complete, mature particles are subsequently transferred from the RER through the Golgi apparatus to the plasma membrane for secretion (Lopez et al., 2005).

A variety of different approaches have been taken to generate a rotavirus vaccine suitable to protect human populations from the various serotypes of rotavirus. These approaches include various Jennerian approaches, use of live attenuated viruses, use of virus-like particles, nucleic acid vaccines and viral sub-units as immunogens. At present there are two oral vaccines available on the market, however, these have low efficacy in some developing countries due to strain variation and presence of other pathogens.

U.S. Pat. Nos. 4,624,850, 4,636,385, 4,704,275, 4,751,080, 4,927,628, 5,474,773, and 5,695,767, each describe a variety of rotavirus vaccines and/or methods of preparing the same. A commonality shared by the members of this group is that each of these vaccines relies on the use of whole viral particles to create the ultimate rotavirus vaccines. Given the long standing need for an effective, multivalent vaccine, it is clear that this body of work has been only partially successful in addressing the need for such a vaccine.

Departing from traditional methods of vaccine generation, advances in the field of molecular biology have permitted the expression of individual rotavirus proteins. Crawford et al. (J Virol. 1994 September; 68(9): 5945-5952) cloned VP2, VP4, VP6, and VP7 coding for the major capsid protein into the baculovirus expression system and expressed each protein in insect cells. Co-expression of different combinations of the rotavirus major structural proteins resulted in the formation of stable virus-like particles (VLPs). The co-expression of VP2 and VP6 alone or with VP4 resulted in the production of VP2/6 or VP2/4/6 VLPs, which were similar to double-layered rotavirus particles. Co-expression of VP2, VP6, and VP7, with or without VP4, produced triple-layered VP2/6/7 or VP2/4/6/7 VLPs, which were similar to native infectious rotavirus particles. The VLPs maintained the structural and functional characteristics of native particles, as determined by electron microscopic examination of the particles, the presence of non-neutralizing and neutralizing epitopes on VP4 and VP7, and hemagglutination activity of the VP2/4/6/7 VLPs.

Vaccine candidates generated from virus-like particles of different protein compositions have shown potential as subunit vaccines. O'Neal et al. ("Rotavirus Virus-like Particles Administered Mucosally Induce Protective Immunity," J. Virology, 71(11):8707-8717 (1997)) showed that VLPs containing VPs 2 and 6 or VPs 2, 6, and 7 when administered to mice with and without the addition of cholera toxin induced protective immunity in immunized mice, although protection was more effective when the VLPs were administered with cholera toxin (CT).

Core-like particles (CLP) and VLPs have also been used to immunize cows Fernandez, et al., ("Passive Immunity to Bovine Rotavirus in Newborn Calves Fed Colostrum Supplements From Cows Immunized with Recombinant SA11 rotavirus core-like particle (CLP) or virus-like particle (VLP) vaccines," Vaccine, 16(5):507-516 (1998)). In this study the ability of CLPs and VLPs to create passive immunity was studied. This group concluded that VLPs were more effective than CLPs in inducing passive immunity.

Plants are increasingly being used for large-scale production of recombinant proteins. For example US 2003/0175303 discloses the expression of recombinant rotavirus structural protein VP6, VP2, VP4 or VP7 in stable transformed tomato plants.

Saldana et al. expressed VP2 and VP6 in the cytoplasm of tomato plants using a cauliflower mosaic virus (CaMV) 35S promoter and recombinant A. tumefaciens (Saldana et al., 2006). Electron microscopy studies showed that a small proportion of the particles had assembled into 2/6 VLPs. A protective immune response was detected in mice and this may have to some extent been contributed by the non-assembled VPs. Individual proteins have been shown to elicit immune responses in mice, as in the case of VP8 and VP6 (Zhou et al., 2010).

Matsumura et al., (2002) were first to report bovine rotavirus A VP6 expression and assembly in transgenic potato plants. In their study, they used transgenic potato plants regulated by a cauliflower mosaic virus (CaMV) 35S promoter and recombinant Agrobacterium tumefaciens carrying the VP6 gene. The protein was expressed, purified and immunogenic studies performed. Immune-response in adult mice showed presence of VP6 antibodies in the sera. However, they did not show evidence of assembled VP6 proteins. It may have been simple monomers or trimers that could elicit an immune response in mice. Another group's work showed VP6 assembly in Nicotiana benthamiana using a potato virus X (PVX) vector (O'Brien et al., 2000). When the VP6 protein was expressed in plants, it was discovered that it only assembled when fused to the PVX protein rods. Once cleavage occurred, VP6 assembled into icosahedral VLPs as seen in a similar study of HIV-PVX by Marusic et al., (2001). This result probably suggests that rotavirus proteins may require an additional factor or enhancement in order to form VLPs.

Production of VLP is a challenging task, as both the synthesis and assembly of one or more recombinant proteins are required. This is the case for VLP of rotavirus which is an RNA virus with a capsid formed by 1860 monomers of four different proteins. For VLP production the simultaneous expression and assembly of two to three recombinant proteins is necessary. These comprise 120 molecules of VP2 (inner layer), 780 molecules of VP6 (middle layer) and 780 molecules of the glycoprotein VP7 (outer layer), ultimately forming a double or triple-layered particle. In addition, the production of most VLP requires the simultaneous expression and assembly of several recombinant proteins, which - for the case of rotavirus like particle (RLP) - needs to occur in a single host cell.

A more recent study showed the successful expression of codon-optimized human rotavirus VP6 in Chenopodium amaranticolor using a Beet black scorch virus (BBSV) mediated expression system. The protein was engineered as a replacement to the coat protein open reading frame of BBSV. Oral immunization of female BALB/c mice with the plant based VP6 protein induced high titers of anti-VP6 mucosal IgA and serum IgG (Zhou et al., 2010). The group, however, did not mention whether the VP6 proteins assembled into VLPs or particles.

Rotavirus VP7 has also been successfully expressed in tobacco plants and was shown to maintain its neutralizing immune response in mice (Yu and Langridge, 2001). Another study using transgenic potato plants to express VP7 showed that the VP7 gene was stable over 50 generations in the transformed plants. VP7 protein from the 50th generation induced both protective and neutralizing antibodies in adult mice (Li et al., 2006).

Yang et al. (Yang Y M, Li X, Yang H, et al. 2011) co-expressed three rotavirus capsid proteins VP2, VP6 and VP7 of group A RV (P[8]G1) in tobacco plants and expression levels of these proteins, as well as formation of rotavirus-like particles and immunogenicity were studied. VLPs were purified from transgenic tobacco plants and analyzed by electron microscopy and Western blot. Yang et al. results indicate that the plant derived VP2, VP6 and VP7 protein self-assembled into 2/6 or 2/6/7 rotavirus like particle with a diameter of 60-80 nm.

### SUMMARY OF THE INVENTION

The present invention relates to producing rotavirus structural proteins in plants. More specifically, the present invention also relates to producing virus-like particles comprising rotavirus structural protein in plants.

According to the present invention there is provided a method (A) of producing a rotavirus-likeparticle (RLP) in a plant comprising:
a) introducing a first nucleic acid comprising a first regulatory region active in the plant operatively linked to a first nucleotide sequence encoding a first rotavirus structural protein, a second nucleic acid comprising a second regulatory region active in the plant operatively linked to a second nucleotide sequence encoding a second rotavirus structural protein and a third nucleic acid comprising a third regulatory region active in the plant operatively linked to a third nucleotide sequence encoding a third rotavirus structural protein into the plant, portion of a plant or plant cell,
b) incubating the plant, portion of a plant or plant cell under conditions that permit the transient expression of the first, second and third nucleic acid, thereby producing the RLP.

Furthermore a fourth nucleic acid comprising a fourth regulatory region active in the plant and operatively linked to a fourth nucleotide sequence encoding a fourth rotavirus structural protein may be introduced into the plant, portion of a plant or plant cell in step a), and is expressed when incubating the plant, portion of a plant or plant cell in step b).

In the method (A) as described above the first rotavirus structural protein may be VP2, the second rotavirus structural protein may be VP6 and the third rotavirus structural protein may be VP4 or VP7. Furthermore, the fourth rotavirus structural protein may be VP7 or VP4. The VP4 may be processed or cleaved to produce VP5 and VP8. Cleavage of VP4 may be performed using a protease, for example, trypsin, a trypsin-like protease, a serine protease, a chymotrypsin-like protease, or a subtilisin. The protease may be co-expressed within the plant.

The present invention also provides a method (B) of producing a rotavirus-like particle (RLP) comprising:
a) introducing a nucleic acid comprising a regulatory region active in the plant operatively linked to a first nucleotide sequence encoding one or more rotavirus structural protein, into the plant, portion of a plant or plant cell,
b) incubating the plant, portion of a plant or plant cell under conditions that permit the transient expression of the first nucleic acid, thereby producing the RLP.

The method (B) as described above may further comprise introducing in (step a) a second nucleic acid comprising a second regulatory region active in the plant and operatively linked to a second nucleotide sequence encoding one or more rotavirus structural protein and expressing the a second nucleic acid when incubating the plant, portion of a plant or plant cell in step b).

The method (B) as described above may further comprise introducing in (step a) a third nucleic acid comprising a third regulatory region active in the plant and operatively linked to a third nucleotide sequence encoding one or more rotavirus structural protein is introduced into the plant, portion of a plant or plant cell in step a), and expressing the third nucleic acid when incubating the plant, portion of a plant or plant cell in step b).

Furthermore, in method (A) or (B) an additional nucleic acid may be expressed in the plant, portion of a plant or plant cell, and wherein the additional nucleic acid comprises a regulatory region active in the plant operatively linked to a nucleotide sequence encoding a suppressor of silencing.

The codon usage of the nucleotide sequence may be adjusted to preferred human codon usage, increased GC content or a combination thereof.

The rotavirus structural protein may comprise a truncated, native or a non-native signal peptide. The non-native signal peptide may be a protein disulfide isomerase signal (PDI) peptide.

The first, second, third or fourth nucleotide sequence or a combination thereof may be operatively linked to a Cowpea Mosaic Virus (CPMV) regulatory region.

The method (A) or (B) as described above may further comprise the steps of:
c) harvesting the plant, portion of a plant or plant cell, and
d) purifying the RLPs from the plant, portion of a plant or plant cell.

During the step of harvesting or purifying in method (A) or (B), VP4 may be processed or cleaved to produce VP5 and VP8 using trypsin, a trypsin-like protease, a serine protease, a chymotrypsin-like protease, subtilisin.

The RLPs may range size from 70-100 nm and may purified in the presence of calcium.

The present invention provides a RLP produced by the methods (A) or (B) as described above. The RLP produced may comprise at least aVP4 rotavirus structural protein. The VP4 may be cleaved into VP5 and VP8 using a protease for example, trypsin, a trypsin-like protease, a serine protease, a chymotrypsin-like protease, subtilisin. The protease may be co-expressed within the plant or added during harvesting, purification, or both. Furthermore the RLP produced by the method (A) or (B) may be a double layered RLP and/or a triple layered RLP.

Furthermore, nucleotide sequences are provided. The nucleotide sequence encoding VP2 may comprise from 80% to 100% identity with a nucleotide sequence as defined by SEQ ID NO:13, SEQ ID NO:14, or SEQ ID NO: 45. The nucleotide sequence encoding VP6 may comprise from 80% to 100% identity with a nucleotide sequence as defined by SEQ ID NO:17, SEQ ID NO:18 or SEQ ID NO:46. The nucleotide sequence encoding VP7 may comprise from 80% to 100% identity with a nucleotide sequence as defined by SEQ ID NO: 19, 20, 48, 49, 52, 53, 54 or 57. And the nucleotide sequence encoding VP4 may comprise from 80% to 100% identity with a nucleotide sequence as defined by SEQ ID NO: 15, 16, 47, 50, or 51. In addition VP2 may be encoded by an amino acid sequence comprising from 80% to 100% identity with the amino acid sequence defined by SEQ ID NO:1 or SEQ ID NO: 25. VP6 may be encoded by an amino acid sequence comprising from 80% to 100% identity with the amino acid sequence defined by SEQ ID NO:3 or SEQ ID NO: 31. VP7 may be encoded by an amino acid sequence comprising from 80% to 100% identity with the amino acid sequence defined by SEQ ID NO: 4, 39, 43 or 59. VP4 may be encoded by an amino acid sequence comprising from 80% to 100% identity with the amino acid sequence defined by SEQ ID NO: 2 or SEQ ID NO: 36. 33. The one or more rotavirus structural protein may be VP2, VP4, VP6 and/or VP7. The VP4 may be processed to VP5 and VP8. The one or more rotavirus structural protein may be selected from rotavirus strain G9 P[6], rotavirus A WA strain, rotavirus A vaccine USA/Rotarix-A41CB052A/1988/G1P1A[8] strain, and rotavirus SA11 strain.

In method (A) as described above the first, second or third nucleic acid sequence or a combination thereof may comprise a regulatory region active in the plant operatively linked to one or more than one comovirus enhancer, to one or more than one amplification element and to a nucleotide sequence encoding a rotavirus structural protein, and wherein a fourth nucleic acid encoding a replicase may be introduced into the plant, portion of a plant or plant cell.

In method (B) as described above the first, second, third or fourth nucleic acid sequence or a combination thereof may comprise a regulatory region active in the plant operatively linked to one or more than one comovirus enhancer, to one or more than one amplification element and to a nucleotide sequence encoding a rotavirus structural protein, and wherein a fifth nucleic acid encoding a replicase may be introduced into the plant, portion of a plant or plant cell.

In addition, according to the present invention there is provided a method (C) of producing a rotavirus-likeparticle (RLP) in a plant comprising:
a) introducing a nucleic acid comprising a regulatory region active in the plant operatively linked to a nucleotide sequence encoding one or more rotavirus structural protein, into the plant, or portion of the plant,
b) incubating the plant, portion of the plant under conditions that permit the transient expression of the first nucleic acid, thereby producing the RLP.

Furthermore, a second nucleic acid may be introduced into the plant or portion of the plant, the second nucleic acid comprises a second regulatory region that is active in the plant and operatively linked to a second nucleotide sequence encoding one or more rotavirus structural protein and wherein the second nucleic acid is expressed when incubating the plant or portion of the plant in step b).

Furthermore a third nucleotide sequence may be introduced into the plant or portion of the plant, the third nucleic acid comprises a third regulatory region that is active in the plant and operatively linked to a third nucleotide sequence encoding one or more rotavirus structural protein and wherein the third nucleic acid is expressed when incubating the plant or portion of the plant in step b).

The method (C) as described above may further comprising a step of harvesting the plant and extracting the RLPs.

The one or more rotavirus structural protein of method (C) may be rotavirus protein VP2, VP4 or VP6. The one or more rotavirus structural protein encoded by the first or second nucleotides sequence may be VP2 or VP6. The one or more rotavirus structural protein encoded by the third nucleotides sequence may be VP4. The VP4 may be cleaved to produce VP5 and VP8.

The first, second or third nucleotide sequence may further encode, comprise, or encode and comprise, one or more than one compartment targeting sequence and/or an amplification element. The one or more compartment targeting sequence directs the one or more rotavirus structural protein to the endoplasmatic reticulum (ER), chloroplast, plastid or apoplast of the plant cell.

The present invention also provides a method (D) of producing a rotavirus-likeparticle (RLP) comprising,
a) providing a plant or portion of a plant comprising a nucleic acid comprising a regulatory region active in the plant operatively linked to a nucleotide sequence encoding one or more rotavirus structural protein;
b) incubating the plant, portion of the plant or plant cell under conditions that permit the transient expression of the nucleic acid, thereby producing the RLP.

Furthermore the plant or portion of the plant of method (D) may further comprise;
i) a second nucleic acid comprising a second regulatory region active in the plant and operatively linked to a second nucleotide sequence encoding one or more rotavirus structural protein or,
ii) a second and third nucleic acid, wherein the second nucleic acid comprises a second regulatory region active in the plant and operatively linked to a second nucleotide sequence encoding one or more rotavirus structural protein and the third nucleic acid comprises a third regulatory region active in the plant and operatively linked to a third nucleotide sequence encoding one or more rotavirus structural protein,
wherein the second or the second and third nucleic acids are expressed when incubating the plant or portion of the plant in step b).

The one or more structural protein in method (D) may be rotavirus protein VP2, VP4 or VP6. The one or more rotavirus structural protein encoded by the first or second nucleotides sequence may be VP2 or VP6. The one or more rotavirus structural protein encoded by the third nucleotides sequence may be VP4. The VP4 may be cleaved into VP5 and VP8 using a protease for example, trypsin, a trypsin-like protease, a serine protease, a chymotrypsin-like protease, subtilisin. The protease may be co-expressed within the plant or added during harvesting, purification, or both.

The present invention provides a RLP produced by the methods (A), method (B), method (C), method (D), or a combination thereof, as described above. The RLP may comprise one or more rotavirus structural protein that may comprises comprise plant-specific N-glycans, or modified N-glycans.

The present invention includes a composition comprising an effective dose of the RLP made by the method (A), method (B), method (C), method (D), or a combination thereof as just described, for inducing an immune response, and a pharmaceutically acceptable carrier.

The present invention also includes a method of inducing immunity to a rotavirus infection in a subject, comprising administering the RLP as just described, to the subject. The RLP may be administered to a subject orally, intradermally, intranasally, intramusclarly, intraperitoneally, intravenously, or subcutaneously.

The present invention also provides plant matter comprising a RLP produced by the method (A), method (B), method (C), method (D), or a combination thereof, as described above. The plant matter may be used in inducing immunity to a rotavirus virus infection in a subject. The plant matter may also be admixed as a food supplement.

In the methods as described above (methods A, B, C or D) the plant or portion of the plant may further be administered with, or may further comprise, another nucleic acid sequence encoding a suppressor of silencing.

Furthermore, the present invention also provides a method (E) of producing rotavirus structural protein in plant comprising
a) introducing a nucleic acid comprising a regulatory region active in the plant operatively linked to a nucleotide sequence encoding one or more rotavirus structural protein, into the plant, or portion of the plant;
b) incubating the plant or portion of the plant under conditions that permit the transient expression of the nucleic acid, thereby producing the one or more rotavirus structural protein.

This summary of the invention does not necessarily describe all features of the invention.

### BRIEF DESCRIPTION OF THE DRAWINGS

These and other features of the invention will become more apparent from the following description in which reference is made to the appended drawings wherein:
**FIGURE 1** shows rotavirus structure and gene-protein assignment . (A) Transmission electron microscopy of rotavirus particles (bar represents 100nm). (B) Organization of the virus capsid proteins comprising inner, intermediate and outer. (C) Rotavirus dsRNA segments arranged as per size and function. The dsRNA can be separated polyacrylamide gel electrophoresis (D). Proteins in (C) are indicated by dsRNA segments in (D). Images from Crawford et al.,1997 (A), Swiss Institute of Bioinformatics, 2008 (B) and Greenberg and Estes, 2009 (D).
**Figure 2** shows rotavirus cell entry and replication. When rotavirus enters a cell, VP4 and VP7 are lost, forming a double layered particle (DLP). Transcription of the dsRNA commences resulting in translation of VP2, VP4, VP6 and VP7. Progeny cores with replicase activity are produced in virus factories (also called viroplasms). Late transcription occurs in these progeny cores. At the periphery of virus factories, these core are coated with VP6, forming immature DLPs that bud across the membrane of the endoplasmic reticulum, acquiring a transient lipid membrane which is modified with the ER resident viral glycoproteins NSP4 and VP7; these enveloped particles also contain VP4. As the particles move towards the interior of the ER cisternae, the transient lipid membrane and the nonstructural protein NSP4 are lost, while the virus surface proteins VP4 and VP7 rearrange to form the outermost virus protein layer, yielding mature infectious triple-layered particles (see Swiss Institute of Bioinformatics (ViralZone): viralzone.expasy.org/viralzone/all_by_species/107.html)

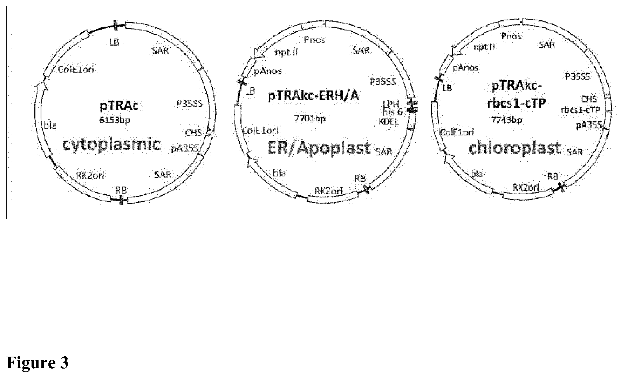

**FIGURE 3** shows Agrobacterium vectors pTRAc, pTRAkc-rbcs1-cTP and pTRAkc-ERH. P35SS, CaMV 35S promoter with duplicated transcriptional enhancer;; CHS, chalcone synthase 5' untranslated region; pA35S, CaMV 35S polyadenylation signal; SAR, scaffold attachment region of the tobacco Rb7 gene; LB and RB, the left and right borders for T-DNA integration; ColE1ori, origin of replication for E. coli; RK2ori, origin of replication for Agrobacterium; bla, ampicillin/carbenicillin-resistance bla gene; LPH, signal-peptide sequence from the murine mAb24 heavy chain; his6, 6 × His tag sequence; SEKDEL, ER-retention signal sequence; rbcs1-cTP, chloroplast-transit peptide sequence of a Rubisco small-subunit gene (rbcS1) from Solanum tuberosum; npt II, kanamycin resistance npt II gene; Pnos and pAnos, promoter and polyadenylation signal of the nopaline synthase gene (Maclean et al., 2007).
**Figure 4** shows an overview of rotavirus cloning and infiltration procedure
**Figure 5** shows an apoplast protein extraction procedure. (A) Illustration of the plant cell and location of the apoplast. VP proteins are expressed in the cytosol and targeted to the apoplast (red arrow). (B) - After time trial, plant leaf is vacuum infiltrated with PBS (1) and placed in a perforated spin column (2) then centrifuged in a 2ml Eppendorf tube to collect sap (3).
**Figure 6** shows a western blots of expression of rotavirus VP6 protein in plant leaf cell compartments over 7 days. Mouse anti-rotavirus VP6 antibody (1:5000) was used to probe the membranes. (+) and (-) indicates expression with or without silencing suppressor respectively. The red lines indicate the position of VP6 proteins in the various samples analysed (∼ 40 kDa). Expression and extraction efficiency of VP6 was best in the cytoplasm.
**Figure 7** shows a western blot showing the individual expression of his-tagged rotavirus proteins at day 3 in the cytoplasm of N. benthamiana plant leaves. +ve - bacterial expressed rotavirus VP2; M - molecular weight marker; VP - rotavirus capsid protein. VP7 infiltration resulted in yellowing of leaves (b).

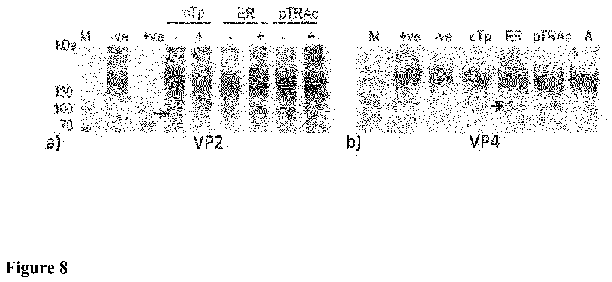

**Figure 8** shows expression of rotavirus VP2 (a) and VP4 (b) targeted to various N. benthamiana plant leaf cell compartments at day 3. The respective chicken anti-rotavirus serum (1:2000) for VP2 and VP4 were used for probing the proteins. cTp - chloroplasts; ER - endoplasmic reticulum; pTRAc - cytoplasm; A - apoplast; Negative control (-ve) - plants infiltrated with silencing suppressor only; Postive control (+ve) in (a) - bacterial expressed VP2, (b) - bacterial expressed VP4; (- and +) with or without silencing suppressor; M - molecular weight marker. The arrows indicate the position of the protein bands in question.
**Figure 9** shows western blot analysis of day 3 extracts of co-expressed VP2/6/4 in the cytoplasm of N. benthamiana leaves. Proteins were probed with a mixture of chicken anti-rotavirus serum (anti-VP2 (1/5000) and anti-VP4 (1/5000)) and mouse anti-VP6 antibody (1:5000). Infiltration of recombinant Agrobacterium was done with silencing suppressor. Negative control (-ve) - whole plants infiltrated with silencing suppressor only; M - molecular weight marker.
**Figure 10** shows electron micrographs of day 3 cytoplasm extracted rotavirus proteins stained with uranyl acetate. (a) Negative protein sample extract with silencing suppressor; (b) VP6 protein extract; (c) VP2/6 protein extract and (d) VP2/6/4 protein extract. Bars = 200 nm. All RLPs detected were between 70 - 100 nm in diameter. Arrow in (b) indicates VP6 sheath/mat. Arrow in (c) indicates an example of aRLP. All proteins were expressed in the presence of a silencing suppressor. All were captured with mouse-anti VP6 antibody (1:2000).
**Figure 11** shows sucrose gradient purification of co-expressed VP2/6 and VP2/6/4 (a). Dot blots of sucrose gradient purified VP2/6 (b) and VP2/6/4 (c). Protein extracts were loaded on a sucrose gradient (10 - 60 %) and ultracentrifuged. Fractions were analysed by probing with (b) mouse anti-VP6 antibody (1:5000) and (c) chicken anti-VP2 and VP4 serum (1:5000).

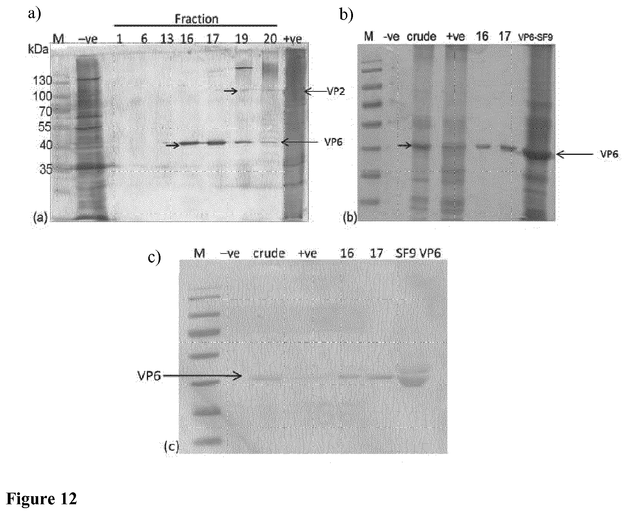

**Figure 12** shows western blot analysis of VP2/6 fractions (a), SDS-PAGE coomassie stained gel photograph of fractions VP2/6 fractions 16 and 17 (b), and western blot analysis of fractions 16 and 17 (c). Mouse anti-VP6 (1:5000) and chicken anti-VP2 serum (1:5000) was used in the western blot (a) and only mouse anti-VP6 (1:5000) in (c). Negative control (- ve) in (a) and (c) - bacterial expressed VP4, and in (b) - plants infiltrated with silencing suppressor and sucrose gradient purified; crude - non-purified VP2/6 extract; Positive control (+ve) in (a) - bacterial expressed VP2, (b) and (c) - plant expressed VP6; VP6-SF9 - VP6 protein of known concentration expressed in SF9 insect cells. Arrows indicate protein bands of interest.
**Figure 13** shows total soluble protein assay on fractions of sucrose density gradient purified VP2/6. (a) - IgG standard curve, (b) - absorbance readings of fractions taken at 750 nm. Points of interest: fractions 16 to 19.
**Figure 14** shows sucrose density gradient analysis of cytoplasm co-expressed VP2/6/4 fractions. Raw absorbance readings at 750nm were taken to verify the protein peaks previously detected on the dot blot of VP2/6/4.
**Figure 15** shows transmission electron micrographs of sucrose density gradient purified VP2/6 particles. Both (a) and (b) show two different sections viewed on the copper grid. All RLPs detected were between 70 - 100 nm in diameter. Samples were captured with mouse-anti VP6 antibody (1:2000). Bars represent 200 nm.

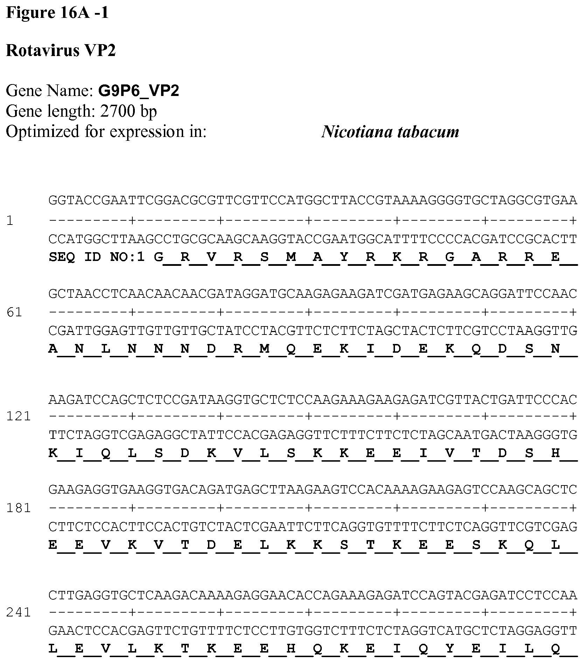

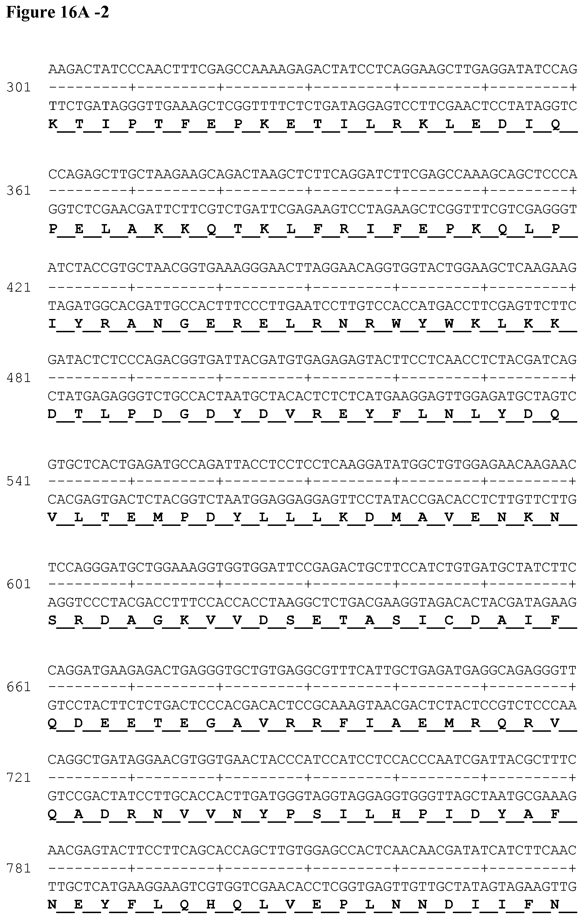

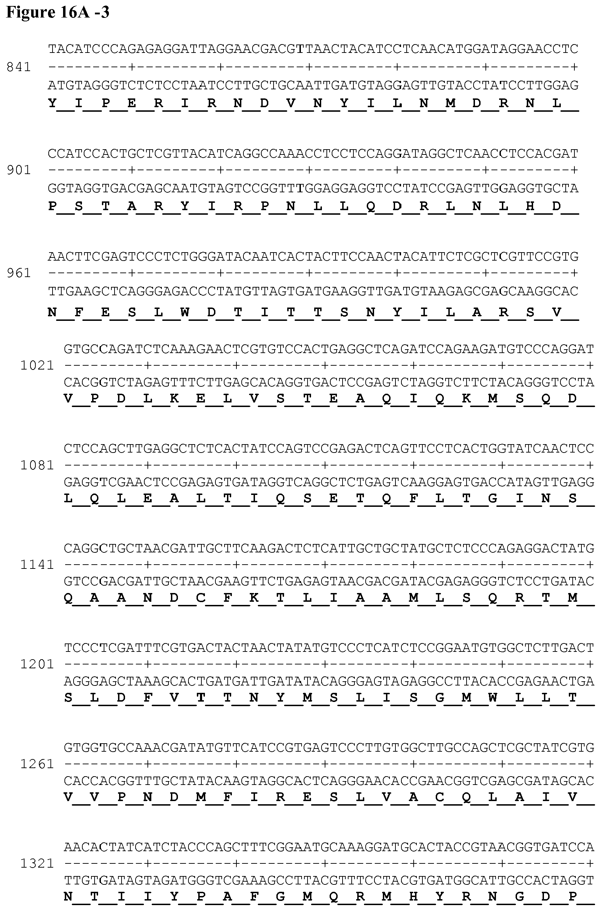

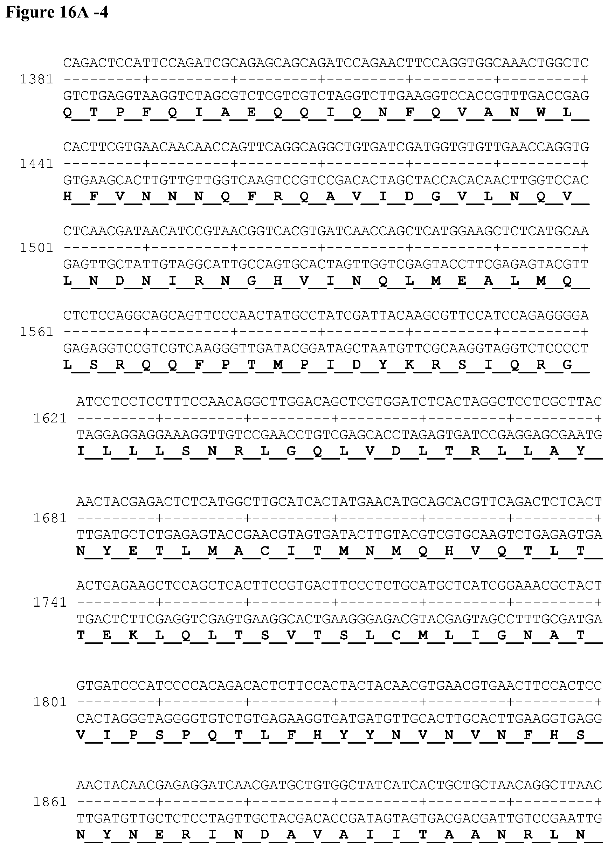

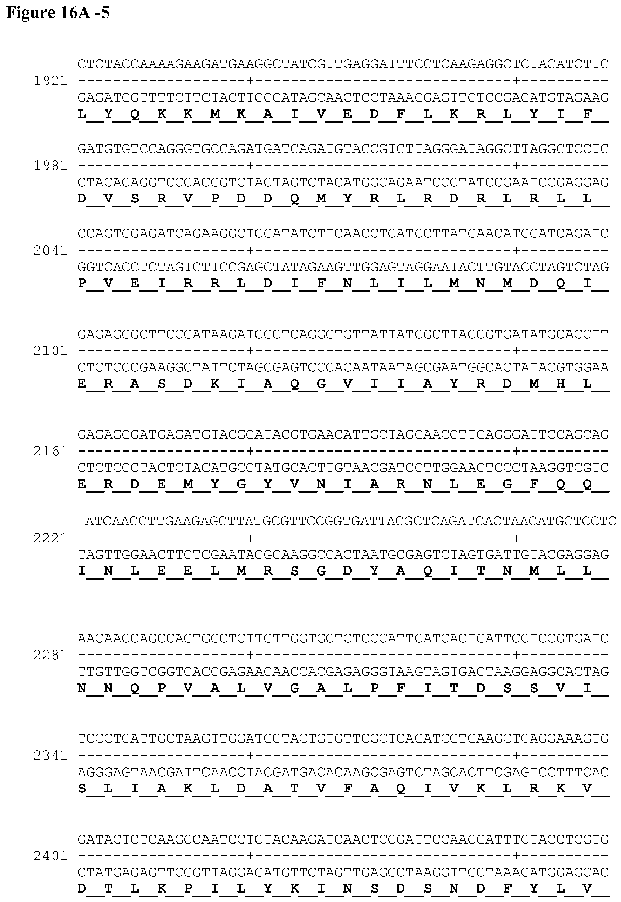

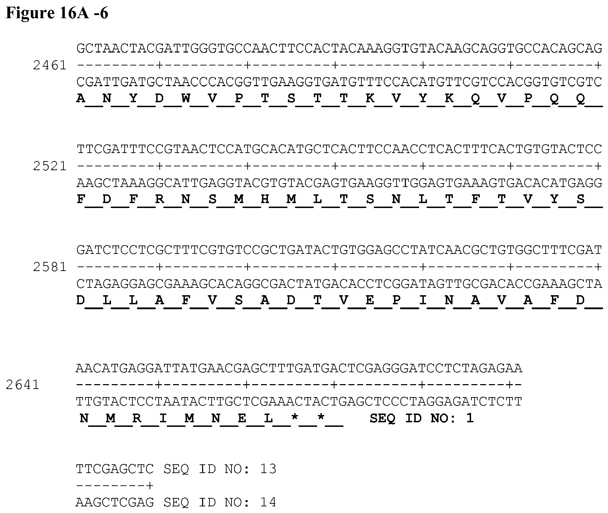

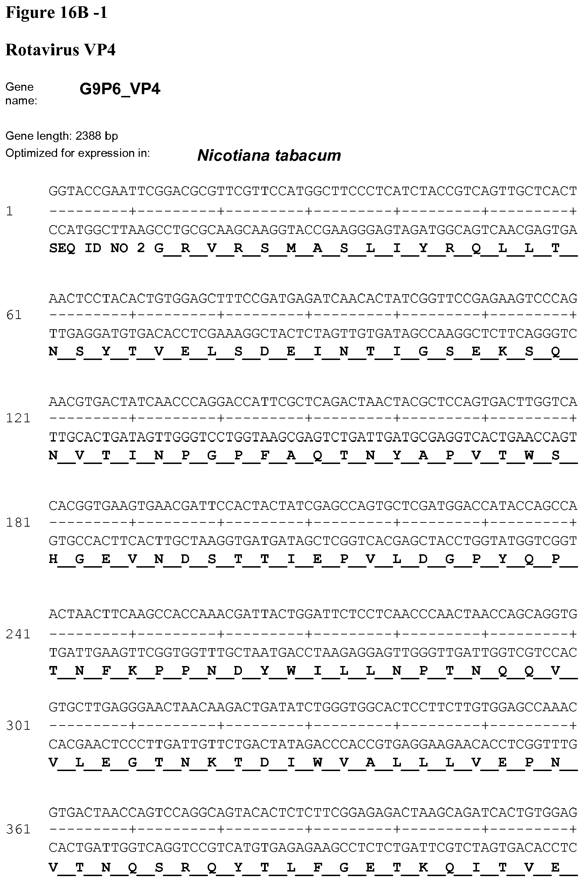

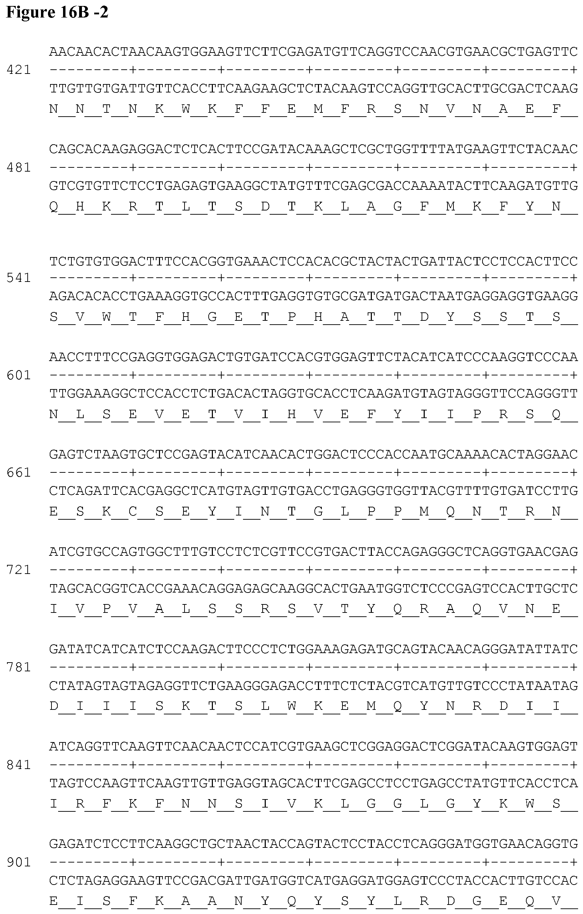

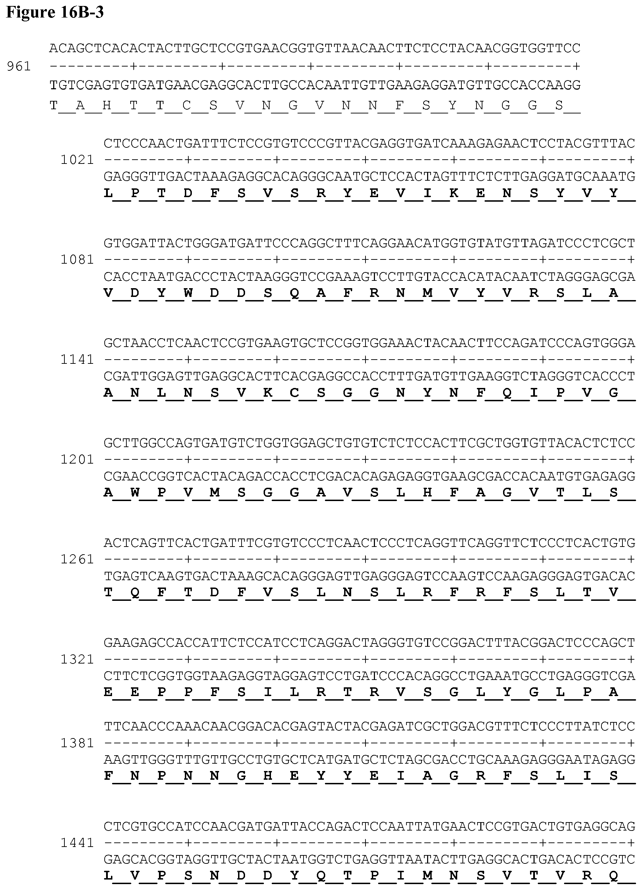

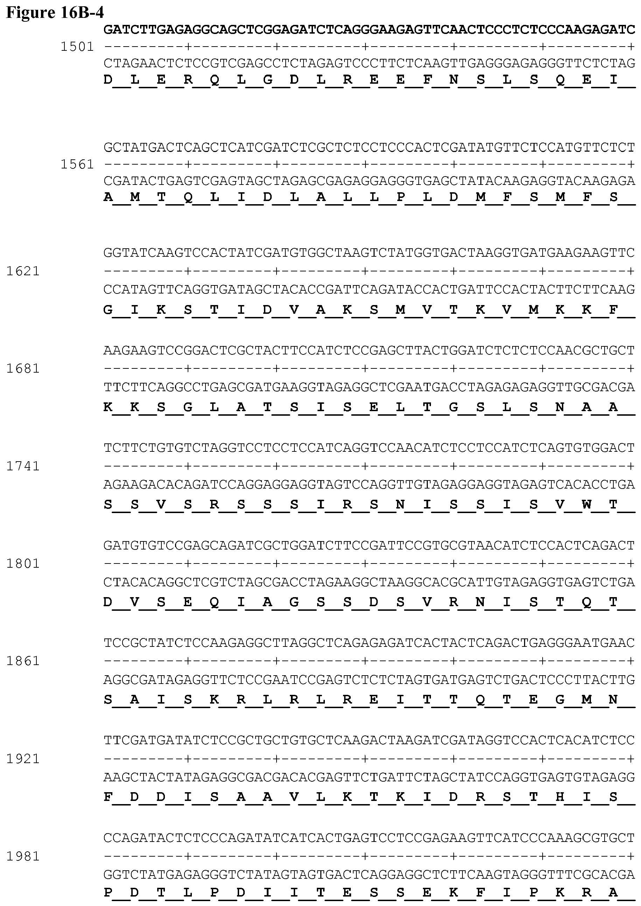

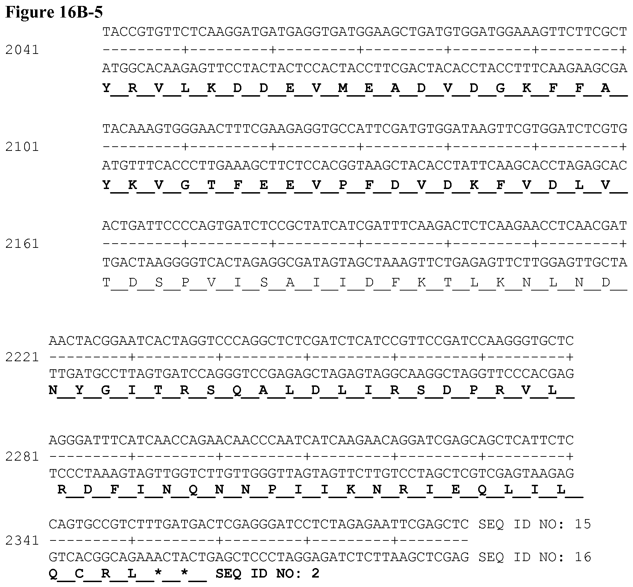

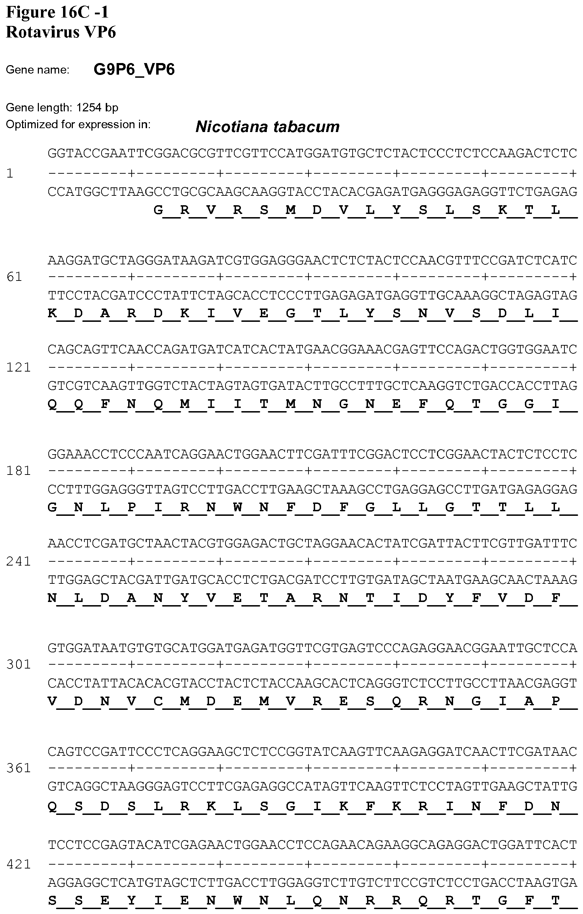

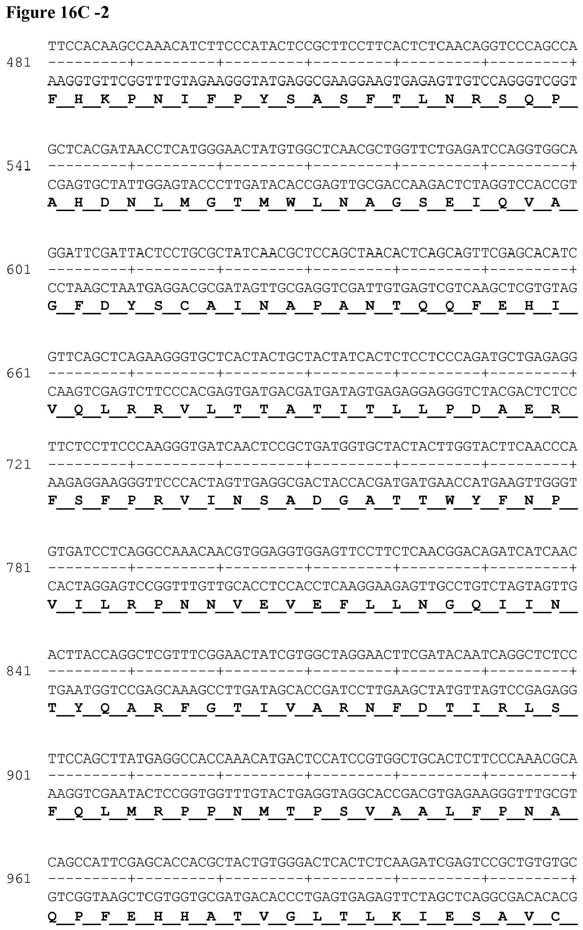

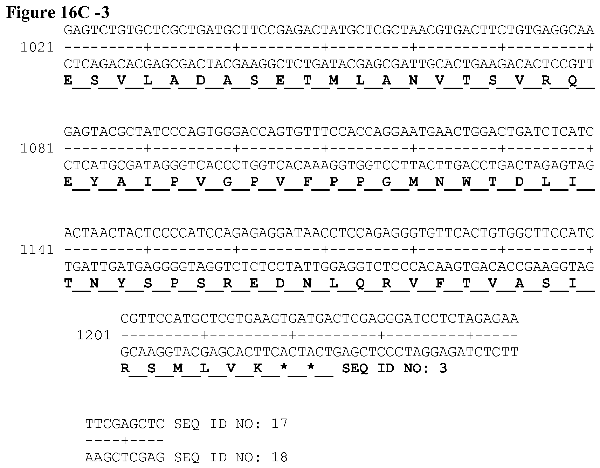

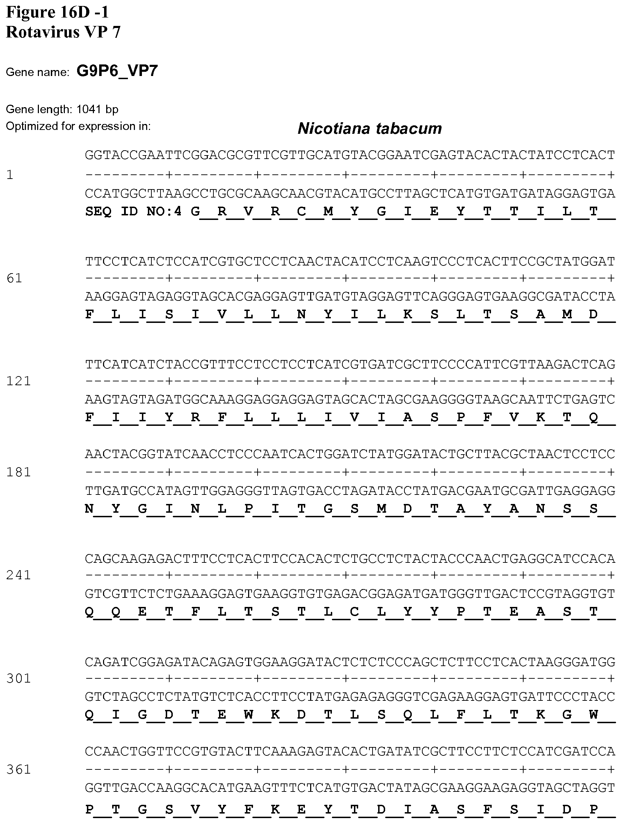

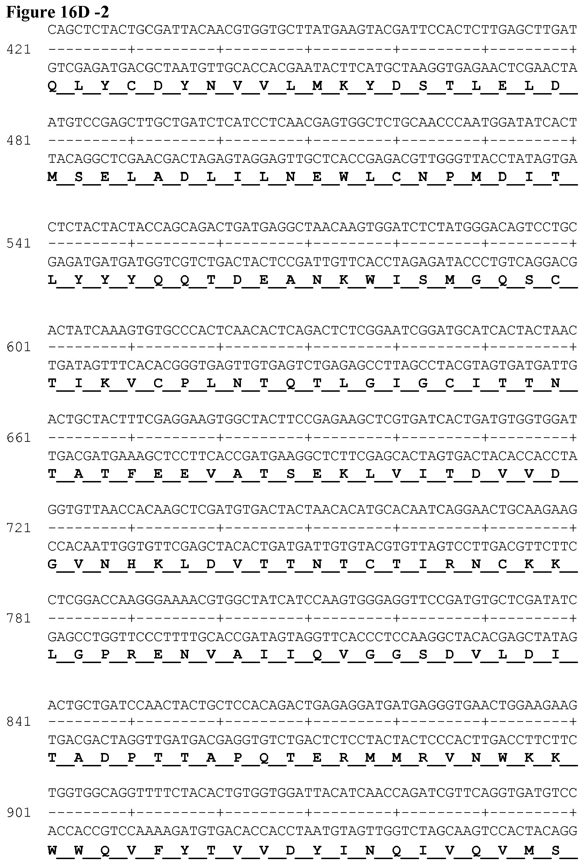

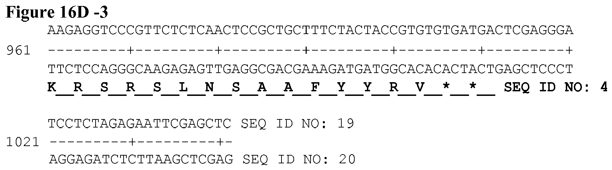

**Figure 16a** shows amino acid sequence (SEQ ID NO:1) and nucleotide sequences of Rotavirus VP2 (SEQ ID NO:13 and 14). **Figure 16b** shows amino acid sequence (SEQ ID NO:2) and nucleotide sequences of Rotavirus VP4 (SEQ ID No: 15 and 16). **Figure 16c** shows amino acid sequence (SEQ ID NO: 3) and nucleotide sequences of Rotavirus VP6 (SEQ ID NO: 17 and 18). **Figure 16d** shows amino acid sequence (SEQ ID NO:4) and nucleotide sequences of Rotavirus VP7 (SEQ ID NO: 19 and 20).
**Figure 17A** shows nucleotide sequence of primer IF-WA_VP2(opt).s1+3c (SEQ ID NO: 21). **Figure 17B** shows nucleotide sequence of primer IF-WA_VP2(opt).s1-4r (SEQ ID NO: 22). **Figure 17C** shows a schematic representation of construct 1191. SacII and StuI restriction enzyme sites used for plasmid linearization are annotated on the representation.
**Figure 18** shows nucleotide sequence (SEQ ID NO: 23) of construct 1191 from left to right t-DNA borders (underlined). 2X35S/CPMV-HT/NOS with Plastocyanine-P19-Plastocyanine silencing inhibitor expression cassette.
**Figure 19** shows nucleotide sequence encoding VP2(opt) from Rotavirus A WA strain (SEQ ID NO:45).
**Figure 20** shows amino acid sequence of VP2 from Rotavirus A WA strain (SEQ ID NO: 25).
**Figure 21** shows a schematic representation of construct number 1710.
**Figure 22A** shows a schematic representation of construct 193. SacII and StuI restriction enzyme sites used for plasmid linearization are annotated on the representation. **Figure 22B** shows nucleotide sequence of construct 193 (SEQ ID NO: 26). Construct 193 is shown from left to right t-DNA borders (underlined). 2X35S/CPMV-HT/NOS into BeYDV(m)+Replicase amplification system with Plastocyanine-P19-Plastocyanine silencing inhibitor expression cassette.
**Figure 23** shows nucleotide sequence of expression cassette 1710 (SEQ ID NO: 27). Expression cassette number 1710 is shown from 2X35S promoter to NOS terminator. VP2(opt) from Rotavirus A WA strain is underlined.
**Figure 24** shows a schematic representation of construct number 1711.
**Figure 25A** shows nucleotide sequence of primer IF-WA_VP6(opt).s1+3c (SEQ ID NO:28). **Figure 25B** shows nucleotide sequence of primer IF-WA_VP6(opt).s1-4r (SEQ ID NO: 29). **Figure 25c** shows expression cassette number 1713 from 2X35S promoter to NOS terminator (SEQ ID NO: 30). VP6(opt) from Rotavirus A WA strain is underlined. Figure 25d shows nucleotide sequence encoding VP6(opt) from Rotavirus A WA strain (SEQ ID NO: 46)
**Figure 26** shows the amino acid sequence of VP6 from Rotavirus A WA strain (SEQ ID NO: 31).
**Figure 27** shows the schematic representation of construct number 1713.
Figure 28 shows the nucleotide sequence of expression cassette number 1714 from 2X35S promoter to NOS terminator (SEQ ID NO:32). VP6(opt) from Rotavirus A WA strain is underlined.
**Figure 29** shows a schematic representation of construct number 1714.
**Figure 30A** shows the nucleotide sequence of primer IF-Rtx_VP4(opt).s1+3c (SEQ ID NO: 33). Figure 30B shows the nucleotide sequence of primer IF-Rtx_VP4(opt).s1-4r (SEQ ID NO: 34).
**Figure 31A** shows the nucleotide sequence of expression cassette number 1731 from 2X35S promoter to NOS terminator (SEQ ID NO: 35). VP4(opt) from Rotavirus A Rotarix strain is underlined. **Figure 31B** shows optimized coding sequence of Rotavirus A VP4 from strain RVA/Vaccine/USA/Rotarix-A41CB052A/1988/G1P1A[8] (SEQ ID NO: 47). **Figure 31C** shows the nucleotide sequence of expression cassette number 1730 from 2X35S promoter to NOS terminator (SEQ ID NO: 44). VP4(opt) from Rotavirus A Rotarix strain is underlined.
**Figure 32** shows amino acid sequence of VP4 from Rotavirus A Rotarix strain (SEQ ID NO: 36).
**Figure 33A** shows a schematic representation of construct number 1730. Figure 33B shows a schematic representation of construct number 1731.

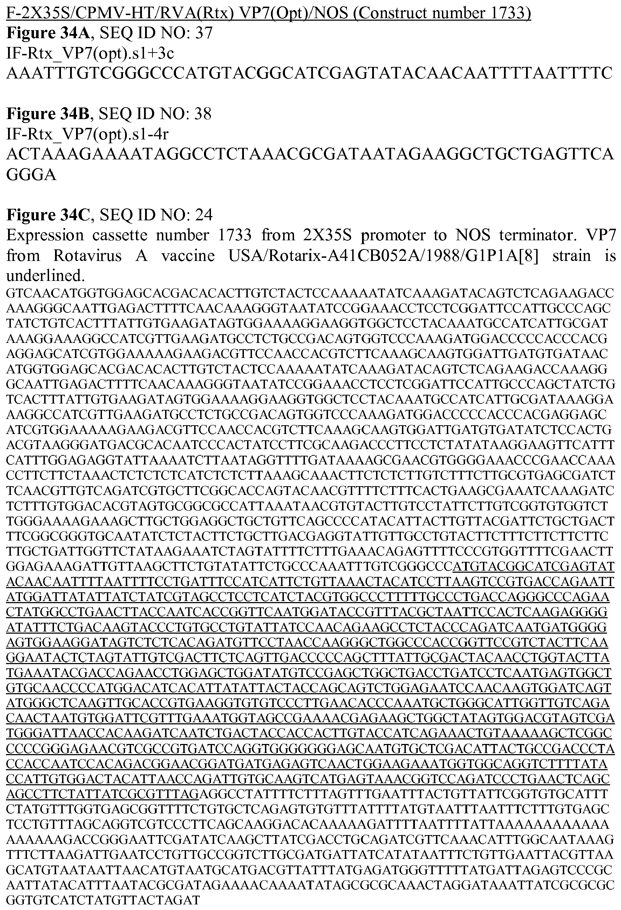

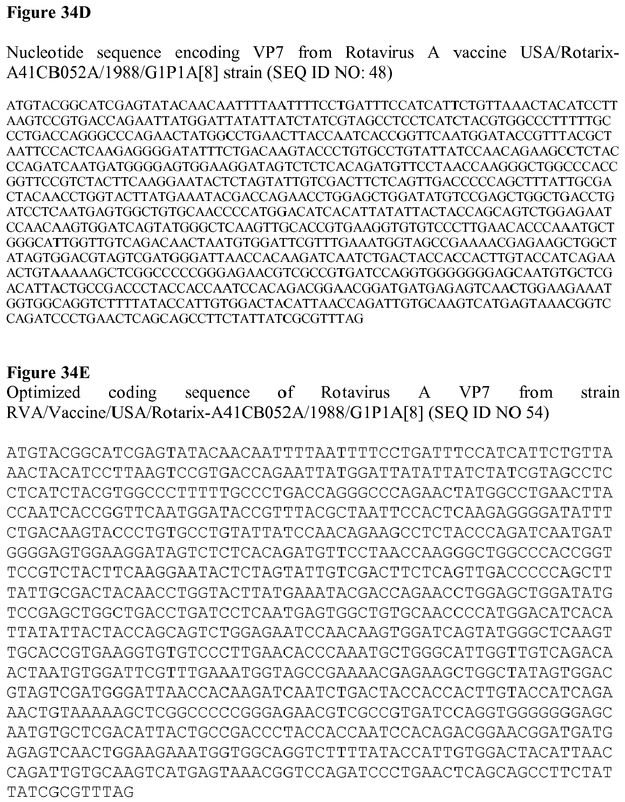

**Figure 34A** shows the nucleotide sequence of primer IF-Rtx_VP7(opt).s1+3c (SEQ ID NO: 37). **Figure 34B** shows the nucleotide sequence of primer IF-Rtx_VP7(opt).s1-4r (SEQ ID NO: 38). **Figure 34C** shows the nucleotide sequence of expression cassette number 1733 from 2X35S promoter to NOS terminator. VP7 from Rotavirus A vaccine USA/Rotarix-A41CB052A/1988/G1P1A[8] strain is underlined (SEQ ID NO: 24). **Figure 34D** shows nucleotide sequence encoding VP7 from Rotavirus A vaccine USA/Rotarix-A41CB052A/1988/G1P1A[8] strain (SEQ ID NO: 48). Figure 34E shows the optimized coding sequence of Rotavirus A VP7 from strain RVA/Vaccine/USA/Rotarix-A41CB052A/1988/G1P1A[8] (SEQ ID NO 54).
**Figure 35** shows the amino acid sequence of VP7 from Rotavirus A vaccine USA/Rotarix-A41CB052A/1988/G1P1A[8] strain (SEQ ID NO: 39).
**Figure 36** shows a schematic representation of construct number 1733.
**Figure 37** shows the nucleotide sequence of primer IF-Rtx_VP7(opt).s2+4c (SEQ ID NO: 40).
**Figure 38** shows a schematic representation of construct 1192. SacII and StuI restriction enzyme sites used for plasmid linearization are annotated on the representation.
**Figure 39** shows the nucleotide sequence of construct 1192 from left to right t-DNA borders (underlined) (SEQ ID NO: 41). 2X35S/CPMV-HT/PDISP/NOS with Plastocyanine-P19-Plastocyanine silencing inhibitor expression cassette are shown.
**Figure 40A** shows the nucleotide sequence of expression cassette number 1735 from 2X35S promoter to NOS terminator (SEQ ID NO: 42). PDISP/VP7(opt) from Rotavirus A vaccine USA/Rotarix-A41CB052A/1988/G1P1A[8] strain is underlined. **Figure 40B** Nucleotide sequence encoding PDISP/VP7(opt) from Rotavirus A vaccine USA/Rotarix-A41CB052A/1988/G1P1A[8] strain (SEQ ID NO: 49).
**Figure 41** shows amino acid sequence of PDISP/VP7 from Rotavirus A vaccine USA/Rotarix-A41CB052A/1988/G1P1A[8] strain (SEQ ID NO: 43).
**Figure 42** shows a schematic representation of construct number 1735.

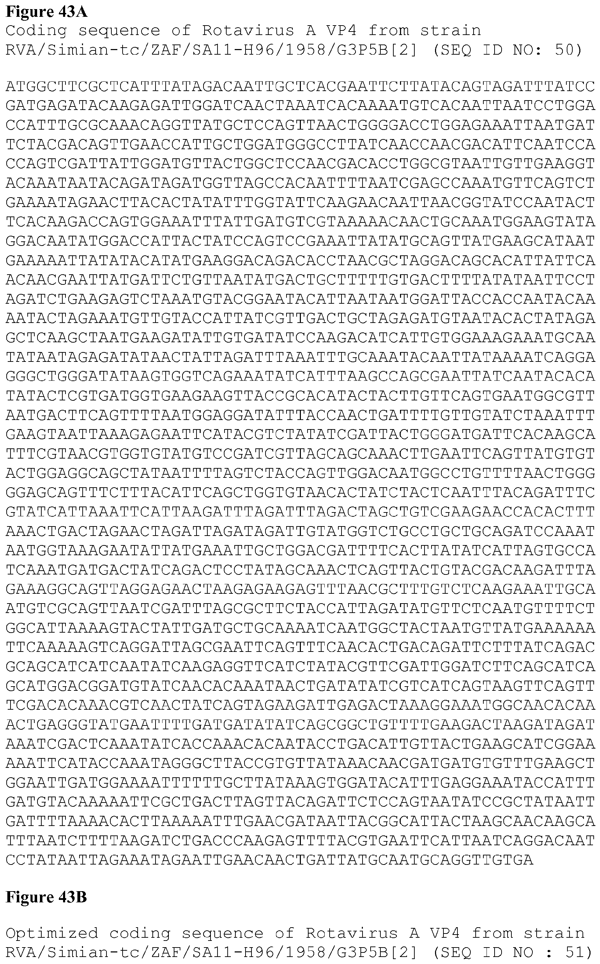

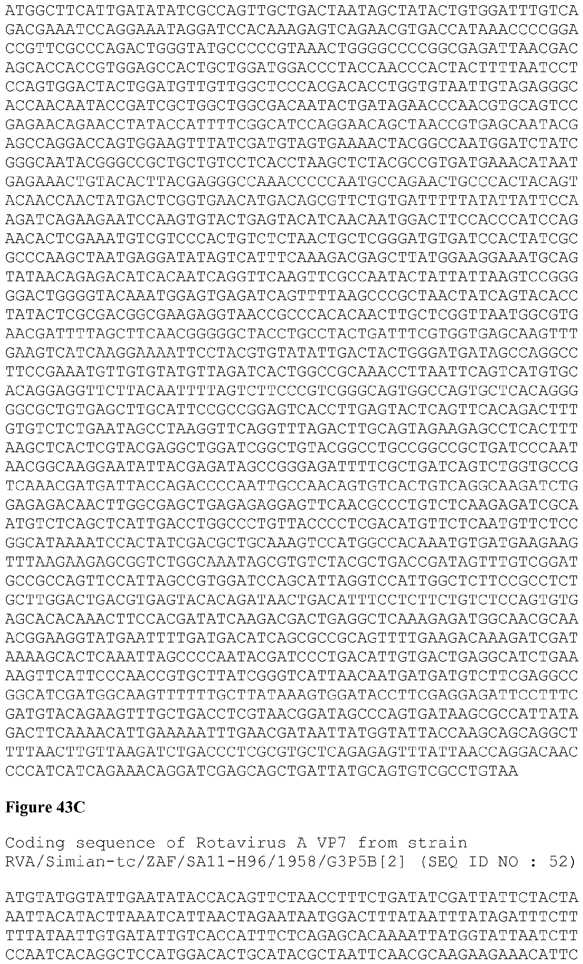

**Figure 43A** shows the coding sequence of Rotavirus A VP4 from strain RVA/Simian-tc/ZAF/SA11-H96/1958/G3P5B[2] (SEQ ID NO: 50). **Figure 43B** shows the optimized coding sequence of Rotavirus A VP4 from strain RVA/Simian-tc/ZAF/SA11-H96/1958/G3P5B[2] (SEQ ID NO : 51). **Figure 43C** shows the coding sequence of Rotavirus A VP7 from strain RVA/Simian-tc/ZAF/SA11-H96/1958/G3P5B[2] (SEQ ID NO : 52). **Figure 43D** shows optimized coding sequence of Rotavirus A VP7 from strain RVA/Simian-tc/ZAF/SA11-H96/1958/G3P5B[2] (SEQ ID NO :53).

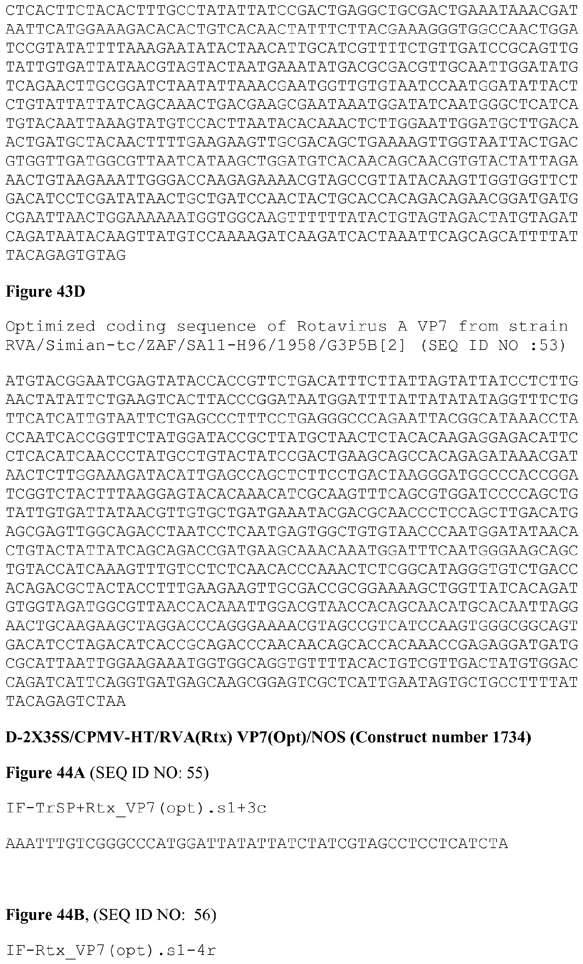

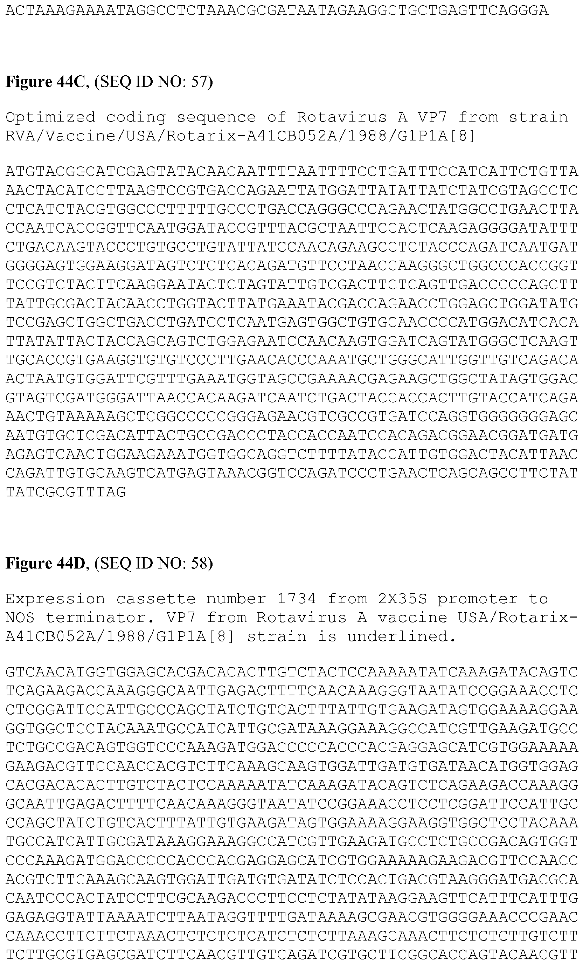

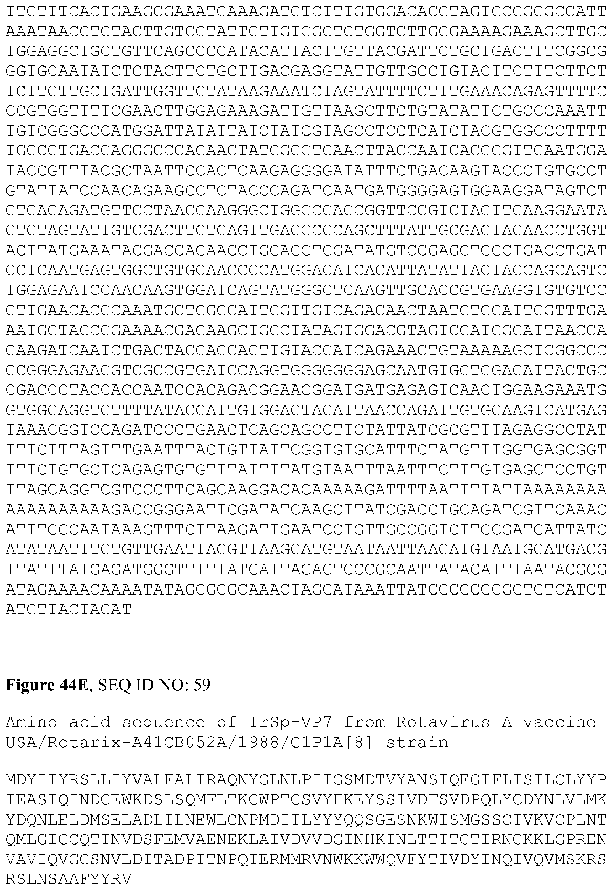

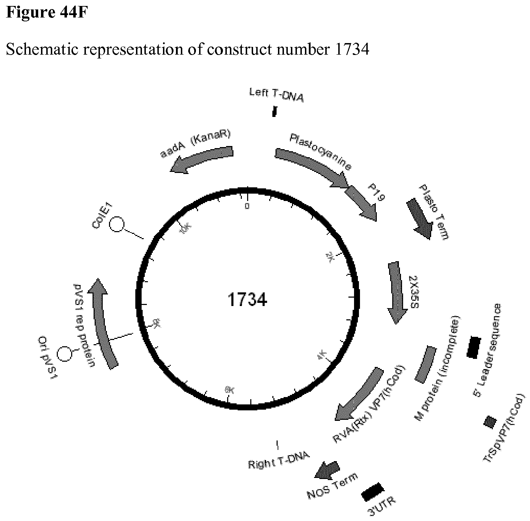

**Figure 44A** shows the nucleotide sequence of primer IF-TrSP+Rtx_VP7(opt).s1+3c (SEQ ID NP: 55). **Figure 44B** shows the nucleotide sequence of primer IF-Rtx_VP7(opt).s1-4r (SEQ ID NO: 56). **Figure 44C** shows the nucleotide sequence of optimized coding sequence of Rotavirus A VP7 from strain RVA/Vaccine/USA/Rotarix-A41CB052A/1988/G1P1A[8] (SEQ ID NO: 57). **Figure 44D** shows the nucleotide sequence of expression cassette number 1734 from 2X35S promoter to NOS terminator (SEQ ID NO 58). VP7 from Rotavirus A vaccine USA/Rotarix-A41CB052A/1988/G1P1A[8] strain is underlined. **Figure 44E** shows the amino acid sequence of TrSp-VP7 from Rotavirus A vaccine USA/Rotarix-A41CB052A/1988/G1P1A[8] strain (SEQ ID NO: 59). **Figure 44F** shows the schematic representation of construct number 1734.
**Figure 45** shows purification of rotavirus-like particles comprising VP2 and VP6 by iodixanol density gradient centrifugation. **Figure 45A** Coomassie-stained SDS-PAGE analysis of the load prior to centrifugation and fractions 1 to 10 (fraction 1 being at the bottom of the tube). Position of the rotavirus antigens are shown by arrows. **Figure 45B** Western blot analysis of the same fractions as in (A) using a rabbit polyclonal anti-rotavirus antibody. Figure 45C Western blot analysis of the same fractions as in (A) using a rabbit polyclonal anti-VP2 antibody.
**Figure 46** shows purification of rotavirus-like particles comprising VP2, VP6 and VP7 by iodixanol density gradient centrifugation. **Figure 46A** Coomassie-stained SDS-PAGE analysis of the load prior to centrifugation and fractions 1 to 10 (fraction 1 being at the bottom of the tube). Position of the rotavirus antigens are shown by arrows. **Figure 46B** Western blot analysis of the same fractions as in (A) using a rabbit polyclonal anti-rotavirus antibody. **Figure 46C** Western blot analysis of the same fractions as in (A) using a rabbit polyclonal anti-VP7 antibody.
**Figure 47** shows purification of rotavirus-like particles comprising VP2, VP4, VP6 and VP7 by iodixanol density gradient centrifugation. **Figure 47A** Coomassie-stained SDS-PAGE analysis of the load prior to centrifugation and fractions 1 to 10 (fraction 1 being at the bottom of the tube). Position of the rotavirus antigens are shown by arrows. **Figure 47B** Western blot analysis of the same fractions as in (A) using a rabbit polyclonal anti-rotavirus antibody. **Figure 47C** Western blot analysis of the same fractions as in (A) using a rabbit polyclonal anti-VP7 antibody.
**Figure 48** shows assessment of VP4 content in purified rotavirus-like particles comprising VP2, VP4, VP6 and VP7 by anti-VP4 specific ELISA.
**Figure 49** shows cryo-electron microscopy image of purified rotavirus-like particles comprising VP2 and VP6 (left panel) and VP2, VP4, VP6 and VP7 (right panel).

### DETAILED DESCRIPTION

The following description is of a preferred embodiment.

The present invention relates to virus-like particles (VLPs) comprising one or more rotavirus structural protein (i.e. a rotavirus like particle, rotavirus VLP or RLP), and methods of producing rotavirus-like particle (RLPs) in plants. The rotavirus like particle (RLP) may therefore comprise one or more rotavirus structural protein. The RLP may be double layered or triple layered.

The present invention in part provides a method of producing a rotavirus-like particle (RLP) in a plant. The method may comprise introducing one or more nucleic acid comprising a regulatory region active in the plant operatively linked to a nucleotide sequence encoding one or more rotavirus structural protein into the plant, or portion of the plant. Followed by incubating the plant or portion of the plant under conditions that permit the transient expression of the nucleic acids, thereby producing the RLP.

Furthermore, the present invention in part provides a method of producing a rotavirus-like particle (RLP) vaccine candidate in a plant. The method may comprise introducing a first nucleic acid comprising a first regulatory region active in the plant operatively linked to a first nucleotide sequence encoding a first rotavirus structural protein, a second nucleic acid comprising a second regulatory region active in the plant operatively linked to a second nucleotide sequence encoding a second rotavirus structural protein and a third nucleic acid comprising a third regulatory region active in the plant operatively linked to a third nucleotide sequence encoding a third rotavirus structural protein into the plant, portion of the plant or plant cell. Followed by incubating the plant, portion of the plant or plant cell under conditions that permit the transient expression of the first, second and third nucleic acid, thereby producing the RLP. The RLP may be single, double or triple layered.

The "rotavirus structural protein" may refer to all or a portion of a rotavirus structural protein sequence isolated from rotavirus, present in any naturally occurring or variant rotavirus strain or isolate. Thus, the term rotavirus structural protein and the like include naturally occurring rotavirus structural protein sequence variants produced by mutation during the virus life-cycle or produced in response to selective pressure (e.g., drug therapy, expansion of host cell tropism or infectivity, etc.). As one of skill in the art appreciates, such rotavirus structural protein sequences and variants thereof may be also produced using recombinant techniques.

Furthermore, structural proteins may include capsid proteins such for example VP2 and VP6 and/or surface proteins such for example VP4. The structural protein may further include for example VP7.

Non-limiting examples of rotavirus structural protein are rotavirus protein VP2, VP4, VP6 and VP7, and a fragment of VP2, VP4, VP6 and VP7. Non-limiting examples of VP2, VP4, VP6 and VP7, or fragments of VP2, VP4, VP6 and VP7 protein that may be used according to the present invention include those VP2, VP4 VP6 and VP7 protein from rotavirus strain G9 P[6], rotavirus A WA strain, rotavirus A vaccine USA/Rotarix-A41CB052A/1988/G1P1A[8] strain and rotavirus SA11 strain.

An example of a VP2 structural protein, which is not to be considered limiting, is set forth in the amino acid sequence of SEQ ID NO: 1 and SEQ ID NO:25. Furthermore, the VP2 structural protein may comprise the sequence set forth in SEQ ID NO: 1, SEQ ID NO:25, or a sequence having at least about 90-100% sequence similarity thereto, including any percent similarity within these ranges, such as 91, 92, 93, 94, 95, 96, 97, 98, 99% sequence similarity thereto. In addition, a VP2 structural protein may be encoded by a nucleotide sequence as set forth in SEQ ID NO:13, 14, 25, or 45 or a sequence having at least about 80-100% sequence similarity thereto, including any percent similarity within these ranges, such as 81, 82, 83, 84, 85, 86, 87, 88, 89, 90, 91, 92, 93, 94, 95, 96, 97, 98, 99% sequence similarity thereto.

An example of a VP4 structural protein, which is not to be considered limiting, is set forth in the amino acid sequence of SEQ ID NO: 2 and SEQ ID NO: 36. Furthermore, the VP4 structural protein may comprise the sequence set forth in SEQ ID NO: 2, SEQ ID NO: 36 or a sequence having at least about 90-100% sequence similarity thereto, including any percent similarity within these ranges, such as 91, 92, 93, 94, 95, 96, 97, 98, 99% sequence similarity thereto. In addition, a VP4 structural protein may be encoded by a nucleotide sequence as set forth in SEQ ID NO: 15, 16, 47, 50 or 51 or a sequence having at least about 80-100% sequence similarity thereto, including any percent similarity within these ranges, such as 81, 82, 83, 84, 85, 86, 87, 88, 89, 90, 91, 92, 93, 94, 95, 96, 97, 98, 99% sequence similarity thereto.

An example of a VP6 structural protein, which is not to be considered limiting, is set forth in the amino acid sequence of SEQ ID NO: 3 and SEQ ID NO: 31. Furthermore, the VP6 structural protein may comprise the sequence set forth in SEQ ID NO: 3, SEQ ID NO: 31 or a sequence having at least about 90-100% sequence similarity thereto, including any percent similarity within these ranges, such as 91, 92, 93, 94, 95, 96, 97, 98, 99% sequence similarity thereto. In addition, a VP6 structural protein may be encoded by a nucleotide sequence as set forth in SEQ ID NO:17, 18, or 46 or a sequence having at least about 80-100% sequence similarity thereto, including any percent similarity within these ranges, such as 81, 82, 83, 84, 85, 86, 87, 88, 89, 90, 91, 92, 93, 94, 95, 96, 97, 98, 99% sequence similarity thereto.

An example of a VP7 structural protein, which is not to be considered limiting, is set forth in the amino acid sequence of SEQ ID NO: 4, SEQ ID NO: 39, SEQ ID NO: 43, and SEQ ID NO: 47. Furthermore, the VP7 structural protein may comprise the sequence set forth in SEQ ID NO: 4, SEQ ID NO: 39 and SEQ ID NO: 43, or a sequence having at least about 90-100% similarity thereto, including any percent similarity within these ranges, such as 91, 92, 93, 94, 95, 96, 97, 98, 99% sequence similarity thereto. In addition, a VP7 structural protein may be encoded by a nucleotide sequence as set forth in SEQ ID NO:19, 20, 48, 49, 52, 53 or 54 or a sequence having at least about 80-100% sequence similarity thereto, including any percent similarity within these ranges, such as 81, 82, 83, 84, 85, 86, 87, 88, 89, 90, 91, 92, 93, 94, 95, 96, 97, 98, 99% sequence similarity thereto.

Amino acid sequence similarity or identity may be computed by using the BLASTP and TBLASTN programs which employ the BLAST (basic local alignment search tool) 2.0 algorithm. Techniques for computing amino acid sequence similarity or identity are well known to those skilled in the art, and the use of the BLAST algorithm is described in ALTSCHUL et al. (1990, J Mol. Biol. 215: 403-410) and ALTSCHUL et al. (1997, Nucleic Acids Res. 25: 3389-3402).

The term "virus-like particle" (VLP), or "virus-like particles" or "VLPs" refers to structures that self-assemble and comprise one or more structural proteins such as for example rotavirus structural protein, for example but not limited to VP2, VP4, VP6 and/or VP7 structural protein. VLPs comprising rotavirus structural protein maybe also be referred to "rotavirus VLP", "rotavirus -like particle (RVLP)", "rotavirus -like particle (RLP)" , "rotavirus -like particle", "RVLP" or "RLP" . VLPs or RLPs are generally morphologically and antigenically similar to virions produced in an infection, but lack genetic information sufficient to replicate and thus are non-infectious. VLPs may be produced in suitable host cells including plant host cells. Following extraction from the host cell and upon isolation and further purification under suitable conditions, VLPs may be purified as intact structures. The RLP may be a single-, double or triple-layered RLP. Single-layered RLPs may be obtained by expressing one rotavirus structural protein, such as VP2 or VP6. Double-layered RLPs may be obtained by expressing two rotavirus structural proteins, such as for example by co-expressing both VP2 and VP6, with or without VP4. Triple-layered RLPs may be obtained by the simultaneous expression of at least three rotavirus structural proteins for example the co-expression of VP2, VP6 and VP7, with or without VP4. Co-expression of VP4 results in a particle with spikes that resembles native rotavirus. VP4 may be processed or cleaved to produce VP5 and VP8. This processing may take place within the host using endogenous proteases, or by co-expressing a suitable protease, for example, trypsin, a trypsin-like protease, a serine protease, a chymotrypsin-like protease, subtilisin. Alternatively, VP4 may be processed to produce VP5 and VP8 by adding a sutiibale protease, for example, trypsin, a trypsin-like protease, a serine protease, a chymotrypsin-like protease, subtilisin during any step of the RLP extraction procedure, or after RLP purification.

Each of the rotavirus structural proteins has different characteristics and size, and is required in different amounts for assembly into RLP. The term "rotavirus VLP", "rotavirus virus-like particle (RVLP)", "rotavirus virus-like particle (RLP)" , "rotavirus virus-like particle", "RVLP" or "RLP" refers to a virus-like particle (VLP) comprising one or more rotavirus structural proteins. Example of rotavirus structural proteins may include, but are not limited to VP2, VP4 (or VP5 and VP8) VP6 and VP7 structural protein.

The present invention also provides for a method of producing RLPs in a plant, wherein a first nucleic acid (a first nucleic acid) encoding a first rotavirus structural protein, for example a VP2 or VP6 protein, is co-expressed with a second nucleic acid encoding a second rotavirus structural protein, for example a VP6 or VP2 protein. Furthermore, a third nucleic acid encoding a third rotavirus structural protein, for example VP4 or VP7 may be co-expressed with the first and second nucleic acid so that the first, the second nucleic acids and third nucleic acids are co-expressed in the plant. The first nucleic acid, second nucleic acid and third nucleic acid, may be introduced into the plant in the same step, or may be introduced to the plant sequentially. The VP4 may be processed or cleaved to produce VP5 and VP8 within the host by co-expressing a nucleic acid encoding a suitable protease, for example, trypsin, a trypsin-like protease, a serine protease, a chymotrypsin-like protease, subtilisin. Alternatively, VP4 may be processed during any step of RLP extraction, or after RLP purification by adding a satiable protease, for example, trypsin, a trypsin-like protease, a serine protease, a chymotrypsin-like protease, subtilisin.

Furthermore, a plant that expresses a first nucleic acid encoding a first rotavirus structural protein, a second nucleic acid encoding a second rotavirus structural protein and a third nucleic acid encoding a third rotavirus structural protein may be further transformed with a fourth nucleic acid encoding a fourth rotavirus structural protein, for example a VP7 or VP4 protein, so that the first, the second nucleic acids, third and fourth nucleic acids are co-expressed in the plant. The VP4 may be processed or cleaved to produce VP5 and VP8 within the host by co-expressing a nucleic acid encoding a suitable protease, for example, trypsin, a trypsin-like protease, a serine protease, a chymotrypsin-like protease, subtilisin. Alternatively, VP4 may be processed during any step of RLP extraction, or after RLP purification by adding a satiable protease, for example, trypsin, a trypsin-like protease, a serine protease, a chymotrypsin-like protease, subtilisin.

Furthermore, a first plant expressing the first nucleic acid encoding one or more rotavirus structural protein, for example a VP2 or VP6 protein, may be crossed with a second plant expressing the second nucleic acid encoding one or more rotavirus structural protein for example but not limited to VP6 or VP2 protein, to produce a progeny plant (third plant) that co-expresses the first and second nucleic acids encoding VP2 and VP6 or VP6 and VP2, respectively. Furthermore, the third plant expressing the first and second nucleic acids encoding VP2 and VP6 or VP6 and VP2, respectively, may be crossed with a fourth plant expressing the third nucleic acid encoding one or more rotavirus structural protein for example but not limited to VP4 or VP7 , to produce a further progeny plant (fifth plant) that co-expresses the first, second and third nucleic acids encoding VP2, VP6, and VP4 or VP7 respectively. The VP4 may be processed or cleaved to produce VP5 and VP8 within the plant using host a protease, or by co-expressing a nucleic acid encoding a suitable protease, for example, trypsin, a trypsin-like protease, a serine protease, a chymotrypsin-like protease, subtilisin within one of the first, second, third or fourth plants. Alternatively, VP4 may be processed during any step of RLP extraction, or after RLP purification by adding a satiable protease, for example, trypsin, a trypsin-like protease, a serine protease, a chymotrypsin-like protease, subtilisin.

As described in more detail below, RLPs may be produced in a plant by expressing a nucleic acid (a first nucleic acid) encoding one or more rotavirus structural protein, for example but not limited to VP2, VP6 or VP7. A second nucleic acid encoding a second rotavirus structural protein, for example but not limited to VP7, VP6 or VP2 might be co-expressed in the plant. Furthermore, a third nucleic acid encoding a third rotavirus structural protein, for example but not limited to VP6, VP7 or VP2 might be co-expressed in the plant. The nucleic acid, second nucleic acid and third nucleic acid may be introduced to the plant in the same step, or they may be introduced to the plant sequentially. The nucleic acid, second nucleic acid and third nucleic acid may be introduced in the plant in a transient manner, or in a stable manner.

Furthermore, a plant that expresses a first nucleic acid encoding a first rotavirus structural protein, for example a VP2 protein, may be transformed with a second nucleic acid encoding a second rotavirus structural protein, for example but not limited to VP6 or VP7 so that both the first and the second nucleic acids are co-expressed in the plant. The plant might be further transformed with a third nucleic acid encoding a third rotavirus structural protein, for example but not limited to VP7 or VP6.

Alternatively, a plant that expresses a VP6 or VP7 protein, (second nucleic acid) may be transformed with the first nucleic acid encoding the VP2 protein, so that both the first and the second nucleic acids are co-expressed in the plant. The plant might be further transformed with a third nucleic acid encoding a third rotavirus structural protein, for example but not limited to VP7 or VP6.

In addition, a plant expressing a first and second nucleic acid encoding a first and second rotavirus structural protein for example a VP2 and VP6 protein, may be transformed with a third nucleic acid encoding a third rotavirus structural protein example VP4 or VP7. The VP4 may be processed or cleaved to produce VP5 and VP8 by co-expressing a nucleic acid encoding a suitable protease, for example, trypsin, a trypsin-like protease, a serine protease, a chymotrypsin-like protease, subtilisin. Alternatively, VP4 may be processed during any step of RLP extraction, or after RLP purification by adding a satiable protease, for example, trypsin, a trypsin-like protease, a serine protease, a chymotrypsin-like protease, subtilisin.

The present invention also provides a method of producing RLPs in a plant that involves introducing one or more nucleic acid encoding one or more rotavirus structural protein operatively linked to a regulatory region active in the plant, and one or more than one compartment targeting sequence and/or an amplification elements, into the plant, portion of the plant or plant cell. The plant, portion of the plant or plant cell is then incubated under conditions that permit the expression of the one or more nucleic acid, thereby producing the RLPs. The one or more rotavirus structural protein may be VP2, VP4 (or VP5 and VP8), VP6, VP7 a fragment of the VP2, VP4 (or VP5 and VP8), VP6, VP7 or a combination thereof.

The present invention further provides for a RLP comprising one or more rotavirus structural protein for example but not limited to VP2, VP4 (or VP5 and VP8), VP6, VP7 or a combination thereof. The RLP may be produced by one or more of the methods as provided by the present invention.

The occurrence of RLPs may be detected using any suitable method for example density gradient centrifugation or size exclusion chromatography. RLPs may be assessed for structure and size by, for example electron microscopy, or by size exclusion chromatography.

For size exclusion chromatography, total soluble proteins may be extracted from plant tissue by homogenizing (Polytron) sample of frozen-crushed plant material in extraction buffer, and insoluble material removed by centrifugation. Precipitation with ice cold acetone or PEG may also be of benefit. The soluble protein is quantified, and the extract passed through a SephacrylTM column, for example a SephacrylTM S500 column. Blue Dextran 2000 may be used as a calibration standard. Following chromatography, fractions may be further analyzed by immunoblot to determine the protein complement of the fraction.

The separated fraction may be for example a supernatant (if centrifuged, sedimented, or precipitated), or a filtrate (if filtered), and is enriched for proteins, or suprastructure proteins, such as for example nanotubes, nanospheres or higher-order, higher molecular weight, particles such as single-layered (sl), double-layered (dl) or triple-layered (tl) RLPs.

The separated fraction may be further processed to isolate, purify, concentrate or a combination thereof, the proteins, suprastructure proteins or higher-order particles by, for example, additional centrifugation steps, precipitation, chromatographic steps (e.g. size exclusion, ion exchange, affinity chromatography), tangential flow filtration, or a combination thereof. The presence of purified proteins, suprastructure proteins or higher-order particles such as RLPs, may be confirmed by, for example, native or SDS-PAGE, Western analysis using an appropriate detection antibody, capillary electrophoresis, electron microscopy, or any other method as would be evident to one of skill in the art.

The RLP's produced according to the present invention may be purified, partially purified from a plant, portion of a plant or plant matter, or may be administered as an oral vaccine, using methods as know to one of skill in the art.

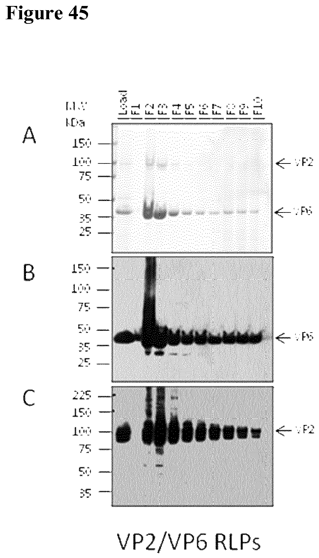

RLP purification may involve gradient centrifugation, for example sucrose, iodixanol, OptiPrep™ or cesium chloride (CsCl) density gradients may be used to purify or partially purify the RLPs from transformed plant biomass. As shown for example in Figure 45, an iodixanol step gradient or iodixanol continuous gradient might be used to purify the RLP and/or expressed rotavirus structural proteins.

Calcium (Ca2+) concentration has been shown to be important for the triple-layer particle (TLP) to double layer particle (DLP) transformation and is strain dependent (see for example Martin et al. Journal of Virology, Jan 2002, which is incorporated herein by reference). Complete loss of the outer-capsid proteins from TLPs (TLP decapsidation) takes place in the nanomolar range of [Ca2+]. Therefore the purification and/or extraction of RLP may be performed in the presence of calcium, and the step of gradient centrifugation may be performed in the presence of calcium, for example in the present of CaC12. The concentration of CaC12 maybe between for example, 1 mM and 1000 mM, or any amount there between, such as 2, 3, 4, 5, 6, 7, 8, 9, 10, 11, 12, 13, 14, 15, 20, 25, 30, 40, 50, 100, 150, 200, 250, 300, 350, 400, 450, 500, 50, 600, 650, 700, 750, 800, 850, 900, 950 mM or any amount therebetween.

The plants, or plant fragments may be minimally processed. By the term "minimal processing" it is meant plant matter, for example, a plant or portion thereof comprising a protein of interest and /or the RLP which is partially purified to yield a plant extract, homogenate, fraction of plant homogenate or the like (i.e. minimally processed). Partial purification may comprise, but is not limited to disrupting plant cellular structures thereby creating a composition comprising soluble plant components, and insoluble plant components which may be separated for example, but not limited to, by centrifugation, filtration or a combination thereof. In this regard, proteins secreted within the extracellular space of leaf or other tissues could be readily obtained using vacuum or centrifugal extraction, or tissues could be extracted under pressure by passage through rollers or grinding or the like to squeeze or liberate the protein free from within the extracellular space. Minimal processing could also involve preparation of crude extracts of soluble proteins, since these preparations would have negligible contamination from secondary plant products. Further, minimal processing may involve aqueous extraction of soluble protein from leaves, followed by precipitation with any suitable salt. Other methods may include large scale maceration and juice extraction in order to permit the direct use of the extract. The RLPs may be purified or extracted using any suitable method for example mechanical or biochemical extraction.

The one or more rotavirus structural protein may be synthesized at an amount up to 2 g per kilogram of plant fresh weight. For example, the amount of synthesized structural protein maybe between 1 and 2 g per kilogram of fresh weight, or any amount there between, such as 1.0, 1.1, 1.2, 1.3, 1.4, 1.5, 1.6, 1.7, 1.8, 1.9, 2 g per kilogram of fresh weight or any amount therebetween. For example, the structural protein may be synthesized at an amount up to 1.54 g per kilogram of plant fresh weight.

Furthermore, the RLP may be synthesized at an amount up to 1.5 g per kilogram of plant fresh weight. For example, the amount of synthesized RLP maybe between 0.5 and 1.5 g per kilogram of fresh weight, or any amount there between, such as 0.5, 0.6, 0.7, 0.8, 0.9, 1.0, 1.1, 1.2, 1.3, 1.4, 1.5 g per kilogram of fresh weight. For example, the RLP may be synthesized at an amount of up to 1.1g per kilogram of plant fresh weight.

The size (i.e. the diameter) of the above-defined RLPs, maybe measures for example by dynamic light scattering (DLS) or electron microscope (EM) techniques, is usually between 50 to 110 nm, or any size therebetween. For example, the size of the intact RLP structure may range from about 70 nm to about 110 nm, or any size therebetween, such as 75 nm, 80 nm, 85 nm, 90 nm, 95 nm, 100 nm, 105 nm or any size therebetween.

The present invention further provides a nucleic acid comprising a nucleotide sequence encoding one or more rotavirus structural protein operatively linked to a regulatory region active in a plant. The nucleotide sequence may be optimized for example for human codon usage or plant codon usage. Furthermore one or more rotavirus structural protein may be operatively linked to one or more than one amplification elements. In addition one or more rotavirus structural protein may be operatively linked to one or more than one compartment targeting sequence. The one or more rotavirus structural protein encoded by the nucleotide sequence may be for example VP2, VP4, VP6 or VP7. Furthermore the one or more rotavirus structural protein encoded by the nucleotide sequence may be for example from any rotavirus group A to G, but more preferably from rotavirus group A. Furthermore, the one or more rotavirus structural protein encoded by the nucleotide sequence maybe from any rotavirus strain having a genotype of any combinations of G- and P- types from G1 to G27 and from PI to P34, and more preferably from G1 to G19 and from PI to P27, including, but not limited to G1P[8], G2P[4], G2P[8], G3P[8], G4P[8], G9P[6], G9P[8], rotavirus A WA strain, rotavirus A vaccine USA/Rotarix-A41CB052A/1988/G1P1A[8] strain or rotavirus SA11 strain.

A nucleic acid sequence referred to in the present invention, may be "substantially homologous" , "substantially similar" or "substantially identical" to a sequence, or a compliment of the sequence if the nucleic acid sequence hybridise to one or more than one nucleotide sequence or a compliment of the nucleic acid sequence as defined herein under stringent hybridisation conditions. Sequences are "substantially homologous" "substantially similar" "substantially identical" when at least about 70%, or between 70 to 100%, or any amount therebetween, for example 70, 72, 74, 76, 78, 80, 82, 84, 86, 88, 90, 92, 94, 96, 98, 100%, or any amount therebetween, of the nucleotides match over a defined length of the nucleotide sequence providing that such homologous sequences exhibit one or more than one of the properties of the sequence, or the encoded product as described herein.

For example the present invention provides an isolated polynucleotide comprising a nucleic acid which encodes one or more rotavirus structural protein that is at least 60%, 65%, 70%, 75%, 80%, 85%, 86%, 87%, 88%, 89%, 90%, 91%, 92%, 93%, 94%, 95%, 96%, 97%, 98%, 99% 100% or any amount therebetween identical to sequences as defines for example in SEQ ID NO: 13, 14, 15, 16, 17, 18, 19, 20, 45, 46, 47, 49, 50, 51, 52, 53, 54. The polynucleotide may be human codon optimized by any of the methods known in the art.

Furthermore, the present invention provides RLPS that comprise rotavirus structural proteins that are for example encoded by nucleic acids that are at least 60%, 65%, 70%, 75%, 80%, 85%, 86%, 87%, 88%, 89%, 90%, 91%, 92%, 93%, 94%, 95%, 96%, 97%, 98%, 99% 100% or any amount therebetween identical to sequences as defines for example in SEQ ID NO: 13, 14, 15, 16, 17, 18, 19, 20, 45, 46, 47, 49, 50, 51, 52, 53, 54.

Such a sequence similarity or identity may be determined using a nucleotide sequence comparison program, such as that provided within DNASIS (using, for example but not limited to, the following parameters: GAP penalty 5, #of top diagonals 5, fixed GAP penalty 10, k tuple 2, floating gap 10, and window size 5). However, other methods of alignment of sequences for comparison are well-known in the art for example the algorithms of Smith & Waterman (1981, Adv. Appl. Math. 2:482), Needleman & Wunsch (J. Mol. Biol. 48:443, 1970), Pearson & Lipman (1988, Proc. Nat'l. Acad. Sci. USA 85:2444), and by computerized implementations of these algorithms (GAP, BESTFIT, FASTA, and BLAST, available through the NIH.), or by manual alignment and visual inspection (see, e.g., Current Protocols in Molecular Biology, Ausubel et al., eds. 1995 supplement), or using Southern or Northern hybridization under stringent conditions (see Maniatis et al., in Molecular Cloning (A Laboratory Manual), Cold Spring Harbor Laboratory, 1982). Preferably, sequences that are substantially homologous exhibit at least about 80% and most preferably at least about 90% sequence similarity over a defined length of the molecule.

An example of one such stringent hybridization conditions may be overnight (from about 16-20 hours) hybridization in 4 X SSC at 65°C, followed by washing in 0.1 X SSC at 65°C for an hour, or 2 washes in 0.1 X SSC at 65□C each for 20 or 30 minutes. Alternatively an exemplary stringent hybridization condition could be overnight (16-20 hours) in 50% formamide, 4 X SSC at 42°C, followed by washing in 0.1 X SSC at 65°C for an hour, or 2 washes in 0.1 X SSC at 65□C each for 20 or 30 minutes, or overnight (16-20 hours), or hybridization in Church aqueous phosphate buffer (7% SDS; 0.5M NaPO4 buffer pH 7.2; 10 mM EDTA) at 65°C, with 2 washes either at 50°C in 0.1 X SSC, 0.1% SDS for 20 or 30 minutes each, or 2 washes at 65°C in 2 X SSC, 0.1% SDS for 20 or 30 minutes each for unique sequence regions.

A nucleic acid encoding a rotavirus structural polypeptide may be described as a "rotavirus nucleic acid", a "rotavirus nucleotide sequence", a "rotavirus nucleic acid", or a "rotavirus nucleotide sequence". For example, which is not to be considered limiting, a virus-like particle comprising one or more rotavirus structural protein or rotavirus structural polypeptide, may be described as a "rotavirus VLP", "RVLP" or "RLP".

Many organisms display a bias for use of particular codons to code for insertion of a particular amino acid in a growing peptide chain. Codon preference or codon bias, differences in codon usage between organisms, is afforded by degeneracy of the genetic code, and is well documented among many organisms. Codon bias often correlates with the efficiency of translation of messenger RNA (mRNA), which is in turn believed to be dependent on, inter alia, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. The process of optimizing the nucleotide sequence coding for a heterologously expressed protein can be an important step for improving expression yields. The optimization requirements may include steps to improve the ability of the host to produce the foreign protein.

"Codon optimization" is defined as modifying a nucleic acid sequence for enhanced expression in cells of interest by replacing at least one, more than one, or a significant number, of codons of the native sequence with codons that may be more frequently or most frequently used in the genes of another organism or species. Various species exhibit particular bias for certain codons of a particular amino acid.

The present invention includes synthetic polynucleotide sequences that have been codon optimized for example the sequences have been optimized for human codon usage or plant codon usage. The codon optimized polynucleotide sequences may then be expressed in plants. More specifically the sequences optimized for human codon usage or plant codon usage may be expressed in plants. Without wishing to be bound by theory, it is believed that the sequences optimized for human codon increases the guanine-cytosine content (GC content) of the sequence and improves expression yields in plants.

There are different codon-optimisation techniques known in the art for improving, the translational kinetics of translationally inefficient protein coding regions. These techniques mainly rely on identifying the codon usage for a certain host organism. If a certain gene or sequence should be expressed in this organism, the coding sequence of such genes and sequences will then be modified such that one will replace codons of the sequence of interest by more frequently used codons of the host organism.

The rotavirus structural protein or polypeptide may be expressed in an expression system comprising a viral based, DNA or RNA, expression system, for example but not limited to, a comovirus-based expression cassette and geminivirus-based amplification element.

The expression system as described herein may comprise an expression cassette based on a bipartite virus, or a virus with a bipartite genome. For example, the bipartite viruses may be of the Comoviridae family. Genera of the Comoviridae family include Comovirus, Nepovirus, Fabavirus, Cheravirus and Sadwavirus. Comoviruses include Cowpea mosaic virus (CPMV), Cowpea severe mosaic virus (CPSMV), Squash mosaic virus (SqMV), Red clover mottle virus (RCMV), Bean pod mottle virus (BPMV), Turnip ringspot virus (TuRSV), Broad bean true mosaic virus (BBtMV), Broad bean stain virus (BBSV), Radish mosaic virus (RaMV). Examples of comovirus RNA-2 sequences comprising enhancer elements that may be useful for various aspects of the invention include, but are not limited to: CPMV RNA-2 (GenBank Accession No. NC_003550), RCMV RNA-2 (GenBank Accession No. NC_003738), BPMV RNA-2 (GenBank Accession No. NC_003495), CPSMV RNA-2 (GenBank Accession No.NC_003544), SqMV RNA-2 (GenBank Accession No.NC_003800), TuRSV RNA-2 (GenBank Accession No. NC_013219.1). BBtMV RNA-2 (GenBank Accession No. GU810904), BBSV RNA2 (GenBank Accession No. FJ028650), RaMV (GenBank Accession No. NC_003800).

Segments of the bipartite comoviral RNA genome are referred to as RNA-1 and RNA-2. RNA-1 encodes the proteins involved in replication while RNA-2 encodes the proteins necessary for cell-to-cell movement and the two capsid proteins. Any suitable comovirus-based cassette may be used including CPMV, CPSMV, SqMV, RCMV, or BPMV, for example, the expression cassette may be based on CPMV.

"Expression cassette" refers to a nucleotide sequence comprising a nucleic acid of interest under the control of, and operably (or operatively) linked to, an appropriate promoter or other regulatory elements for transcription of the nucleic acid of interest in a host cell.

The expression systems may also comprise amplification elements from a geminivirus for example, an amplification element from the bean yellow dwarf virus (BeYDV). BeYDV belongs to the Mastreviruses genus adapted to dicotyledonous plants. BeYDV is monopartite having a single-strand circular DNA genome and can replicate to very high copy numbers by a rolling circle mechanism. BeYDV-derived DNA replicon vector systems have been used for rapid high-yield protein production in plants.

As used herein, the phrase "amplification elements" refers to a nucleic acid segment comprising at least a portion of one or more long intergenic regions or long intergenic repeat (LIR) of a geminivirus genome. As used herein, "long intergenic region" or "long intergenic repeat" refers to a region of a long intergenic region that contains a rep binding site capable of mediating excision and replication by a geminivirus Rep protein. In some aspects, the nucleic acid segment comprising one or more LIRs, may further comprises a short intergenic region or small intergenic region (SIR) of a geminivirus genome. As used herein, "short intergenic region" or "small intergenic region" refers to the complementary strand (the short IR (SIR) of a Mastreviruses). Any suitable geminivirus-derived amplification element may be used herein. See, for example, WO2000/20557; WO2010/025285; Zhang X. et al. (2005, Biotechnology and Bioengineering, Vol. 93, 271-279), Huang Z. et al. (2009, Biotechnology and Bioengineering, Vol. 103, 706-714), Huang Z. et al.(2009, Biotechnology and Bioengineering, Vol. 106, 9-17); which are herein incorporated by reference). If more than one LIR is used in the construct, for example two LIRs, then the promoter, CMPV-HT regions and the nucleic acid sequence of interest and the terminator are bracketed by each of the two LIRs. Furthermore, the amplification element might for example originate from the sequence as disclosed in Halley-Stott et al. (2007) Archives of Virology 152: 1237-1240, deposited under Gen Bank accession number DQ458791, which are herein incorporated by reference. The nucleic acid segment comprising LIRs are joined nucleotides 2401 to 2566 and 1 to 128. The nucleic acid segment comprising SIRs are nucleotides 1154 to 1212.

As described herein, co-delivery of bean yellow dwarf virus (BeYDV)-derived vector and a Rep/RepA-supplying vector, by agroinfiltration of Nicotiana benthamiana leaves results in efficient replicon amplification and robust protein production.

A comovirus-based expression cassette and a geminivirus-derived amplification element may be comprised on separate vectors, or the component parts may be included in one vector. If two vectors are used, the first and second vectors may be introduced into a plant cell simultaneously, or separately.

A viral replicase may also be included in the expression system as described herein to increase expression of the nucleic acid of interest. An non-limiting example of a replicase is a BeYDV replicase (pREP110) encoding BeYDV Rep and RepA (C2/C1; Huang et al., 2009, Biotechnol. Bioeng. 103, 706-714; which is incorporated herein by reference). Another non-limiting example of a replicase is disclosed in Halley-Stott et al. (2007) Archives of Virology 152: 1237-1240, deposited under Gen Bank accession number DQ458791, which are herein incorporated by reference. The nucleic acid segment comprising C1:C2 gene are nucleotides 1310 to 2400.

By "co-expressed" it is meant that two or more than two nucleotide sequences are expressed at about the same time within the plant, and within the same tissue of the plant. However, the nucleotide sequences need not be expressed at exactly the same time. Rather, the two or more nucleotide sequences are expressed in a manner such that the encoded products have a chance to interact. The two or more than two nucleotide sequences can be co-expressed using a transient expression system, where the two or more sequences are introduced within the plant at about the same time under conditions that both sequences are expressed. Alternatively, a platform plant comprising one of the nucleotide sequences may be transformed in a stable manner, with an additional sequence encoding the protein of interest for example one or more rotavirus structural protein, introduced into the platform plant in a transient manner.

Correct folding of the protein may be important for stability of the protein, formation of multimers, formation of RLPs and function. Folding of a protein may be influenced by one or more factors, including, but not limited to, the sequence of the protein, the relative abundance of the protein, the degree of intracellular crowding, the availability of cofactors that may bind or be transiently associated with the folded, partially folded or unfolded protein. Furthermore the compartment or sub-compartment within the plant where the protein is expressed may influence the expression levels and folding of the protein.

The expression of the one or more rotavirus structural protein may be targeted to specific plant cell compartment and/or sub-compartments by agroinfiltration in transgenic plants. The compartment or sub-compartments may be for example plastids, endoplasmic reticule (ER), chloroplast or apoplast. Without wishing to be bound by theory, compartment or sub-compartments targeting may increased protein accumulation into the targeted compartment or sub-compartments over cytoplasmic accumulation. Compartment or sub-compartment accumulation may protect protein from degradation by proteases present in the cytoplasm and/or allow it to accumulate to higher concentration without affecting the function of the plant cell.

Therefore, the expression cassette or vector may be adapted to direct the vector or rotavirus structural protein or polypeptide expressed from the vector to the desired compartment or sub-compartment in the plant.

For example the expression cassette or vector may be adapted to target plastids by causing an expressed rotavirus structural protein or polypeptide to include a portion capable of interacting with the thylakoid membranes of the plastids, in particular the transfer mechanism of the thylakoid membranes. This interaction may cause the rotavirus structural protein or polypeptide to be imported into the plastid from the cytoplasm where it is expressed. Without wishing to be bound by theory, the mechanism of importation from the cytoplasm may be important for proper folding of the proteins. It will be appreciated that the expression cassette or vector may be adapted to target the plastids themselves to become transformed and expression of the rotavirus structural protein or polypeptide may occur wholly within the plastid.

By the term "targeting sequence" it is meant that the targeting sequences may be included in the vector or expression cassette. Such targeting sequences may be translated into a peptide which directs the vector or product thereof to the desired compartment or sub-compartment in the plant, such as a plastid. For example, plastid signal peptides (also referred to as "plastid transit peptides" in the art) for targeting proteins into plastids are known in the art. A non limiting example of a plastid transit peptide that may be used is that of rbcs1-cTP. As suitable example of a chloroplast-transit peptide sequence is the Rubisco small-subunit gene (rbcS1), for example, from Solanum tuberosum .

Therefore, the rotavirus structural protein or polypeptide may include a signal peptide that is the same as, or heterologous with, the remainder of the polypeptide or protein. The term "signal peptide" is well known in the art and refers generally to a short (about 5-30 amino acids) sequence of amino acids, found generally at the N-terminus of a polypeptide that may direct translocation of the newly-translated polypeptide to a particular organelle, or aid in positioning of specific domains of the polypeptide chain relative to others. As a non-limiting example, the signal peptide may target the translocation of the protein into the endoplasmic reticulum and/or aid in positioning of the N-terminus proximal domain relative to a membrane-anchor domain of the nascent polypeptide to aid in cleavage and folding of the mature protein, for example which is not to be considered limiting, a rotavirus structural protein.

A signal peptide (SP) may be native to the protein or virus protein, or a signal peptide may be heterologous with respect to the primary sequence of the protein or virus protein being expressed. For example the native signal peptide of rotavirus structural protein may be used to express the rotavirus structural protein in a plant system.

A signal peptide may also be non-native, for example, from a protein, viral protein or native structural protein of a virus other than rotavirus protein, or from a plant, animal or bacterial polypeptide. A non limiting example of a signal peptide that may be used is that of alfalfa protein disulfide isomerase (PDISP) (nucleotides 32-103 of Accession No. Z11499). Furthermore, the signal peptide may be completely deleted or truncated. By truncation or truncated it is meant that 1%, 5%, 10%, 15%, 20%, 25%, 30%, 35%, 40%, 45%, 50%, 55%, 60%, 65%, 70%, 75%, 80%, 85%, 90%, 95%, 100% or any amount therebetween of amino acid residues are deleted from the signal peptide. Preferably, the truncated amino acid residues are continuous, and the truncation occurs from the second methionine onward

The present invention therefore provides for a rotavirus structural protein, such as for example VP2, VP4, VP6 and/or VP7, comprising a native, a non-native signal peptide or truncated signal peptide, and nucleic acids encoding such rotavirus structural proteins.

The one or more than one genetic constructs of the present invention may be expressed in any suitable plant host that is transformed by the nucleotide sequence, or constructs, or vectors of the present invention. Examples of suitable hosts include, but are not limited to, agricultural crops including alfalfa, canola, Brassica spp., maize, Nicotiana spp., potato, ginseng, pea, oat, rice, soybean, wheat, barley, sunflower, cotton and the like.

The nucleotide sequences encoding for the rotavirus structural proteins may be transferred into the plant host using 1, 2, 3, 4 or 5 binary plasmid vectors. Each binary plasmid vector may therefore contain 1, 2, 3, 4 or 5 nucleotide sequences encoding for a rotavirus structural protein.

The one or more genetic constructs of the present invention can further comprise a 3' untranslated region. A 3' untranslated region refers to that portion of a gene comprising a DNA segment that contains a polyadenylation signal and any other regulatory signals capable of effecting mRNA processing or gene expression. The polyadenylation signal is usually characterized by effecting the addition of polyadenylic acid tracks to the 3' end of the mRNA precursor. Polyadenylation signals are commonly recognized by the presence of homology to the canonical form 5' AATAAA-3' although variations are not uncommon. Non-limiting examples of suitable 3' regions are the 3' transcribed nontranslated regions containing a polyadenylation signal of Agrobacterium tumor inducing (Ti) plasmid genes, such as the nopaline synthase (NOS) gene, plant genes such as the soybean storage protein genes, the small subunit of the ribulose-I, 5-bisphosphate carboxylase gene (ssRUBISCO; US 4,962,028; which is incorporated herein by reference), the promoter used in regulating plastocyanin expression, described in US 7,125,978 (which is incorporated herein by reference).

One or more of the genetic constructs of the present invention may also include further enhancers, either translation or transcription enhancers, as may be required. Enhancers may be located 5' or 3' to the sequence being transcribed. Enhancer regions are well known to persons skilled in the art, and may include an ATG initiation codon, adjacent sequences or the like. The initiation codon, if present, may be in phase with the reading frame ("in frame") of the coding sequence to provide for correct translation of the transcribed sequence.

The constructs of the present invention can be introduced into plant cells using Ti plasmids, Ri plasmids, plant virus vectors, direct DNA transformation, micro-injection, electroporation, etc. For reviews of such techniques see for example Weissbach and Weissbach, Methods for Plant Molecular Biology, Academy Press, New York VIII, pp. 421-463 (1988); Geierson and Corey, Plant Molecular Biology, 2d Ed. (1988); and Miki and Iyer, Fundamentals of Gene Transfer in Plants. In Plant Metabolism, 2d Ed. DT. Dennis, DH Turpin, DD Lefebrve, DB Layzell (eds), Addison Wesly, Langmans Ltd. London, pp. 561-579 (1997). Other methods include direct DNA uptake, the use of liposomes, electroporation, for example using protoplasts, micro-injection, microprojectiles or whiskers, and vacuum infiltration. See, for example, Bilang, et al. (Gene 100: 247-250 (1991), Scheid et al. (Mol. Gen. Genet. 228: 104-112, 1991), Guerche et al. (Plant Science 52: 111-116, 1987), Neuhause et al. (Theor. Appl Genet. 75: 30-36, 1987), Klein et al., Nature 327: 70-73 (1987); Howell et al. (Science 208: 1265, 1980), Horsch et al. (Science 227: 1229-1231, 1985), DeBlock et al., Plant Physiology 91: 694-701, 1989), Methods for Plant Molecular Biology (Weissbach and Weissbach, eds., Academic Press Inc., 1988), Methods in Plant Molecular Biology (Schuler and Zielinski, eds., Academic Press Inc., 1989), Liu and Lomonossoff(J Virol Meth, 105:343-348, 2002,), U.S. Pat. Nos. 4,945,050; 5,036,006; and 5,100,792, U.S. patent application Ser. Nos. 08/438,666, filed May 10, 1995, and 07/951,715, filed Sep. 25, 1992, (all of which are hereby incorporated by reference).

### Transient expression

Without wishing to be bound by theory, the protein concentration and ratio of the different rotavirus structural proteins may be important for the assembly efficiency of RLPs. Therefore multiplicity and time of infection, may be important to manipulate protein concentration and the overall assembly efficiency of RLPs in plants.

The construct of the present invention may be transiently expressed in a plants or portion of a plant. A transient expression system relying on the epichromosomal expression of recombinant Agrobacterium tumefaciens in a plant, portion of a plant or plant cell may be used to express the rotavirus structural protein, targeted to various cell compartments or sub-compartments. A transient expression system allows for a high production speed. Furthermore, large amounts of protein can be attained within a few days after infiltration of recombinant Agrobacterium in plants (Rybicki, 2010; Fischer et al., 1999). It is also possible to express long gene sequences and have more than one gene simultaneously expressed in the same cell, allowing for efficient assembly of multimeric proteins (Lombardi et al., 2009).

The nucleotide sequences encoding for the rotavirus structural proteins may be transferred into the plant host into 1, 2, 3, 4 or 5 transformed Agrobacterium tumefaciens strain.

However, during transient expression post-transcriptional gene silencing may limit the expression of the heterologous proteins in plants. The co-expression of a suppressor of silencing, for example, but not limited to Nss from Tomato spotted wilt virus may be used to counteract the specific degradation of transgene mRNAs (Brigneti et al., 1998). Alternate suppressors of silencing are well known in the art and may be used as described herein (Chiba et al., 2006, Virology 346:7-14; which is incorporated herein by reference), for example but not limited to HcPro, TEV -p1/HC-Pro (Tobacco etch virus-pl/HC-Pro), BYV -p21, p19 of Tomato bushy stunt virus (TBSV p19), capsid protein of Tomato crinkle virus (TCV -CP), 2b of Cucumber mosaic virus; CMV-2b), p25 of Potato virus X (PVX-p25), p11 of Potato virus M (PVM-p11), p11 of Potato virus S (PVS-p11), p16 of Blueberry scorch virus, (BScV -p16), p23 of Citrus tristexa virus (CTV-p23), p24 of Grapevine leafroll-associated virus-2, (GLRaV-2 p24), p10 of Grapevine virus A, (GVA-p10), p14 of Grapevine virus B (GVB-p14), p10 of Heracleum latent virus (HLV-p10), or p16 of Garlic common latent virus (GCLV-p16). Therefore, a suppressor of silencing, for example HcPro, TEV -p1/HC-Pro, BYV-p21, TBSV p19, TCV-CP, CMV-2b, PVX-p25, PVM-p11, PVS-p11, BScV-p16, CTV-p23, GLRaV-2 p24, GBV-p14, HLV-p10, GCLV-p16or GVA-p10, may be co-expressed along with one or more rotavirus structural protein for example VP2, VP4, VP6, or a combination thereof, to further ensure high levels of protein production within a plant or portion of a plant.

The present invention also provides a methods as described above, wherein an additional (second, third, fourth, or fifth) nucleotide sequence is expressed within the plant, the additional (second, third, fourth, or fifth) nucleotide sequence encoding a suppressor of silencing is operatively linked with an additional (second, third, fourth, or fifth) regulatory region that is active in the plant. The nucleotide sequence encoding a suppressor of silencing may be, for example Nss, HcPro, TEV -p1/HC-Pro, BYV-p21, TBSV p19, TCV-CP, CMV-2b, PVX-p25, PVM-p11, PVS-p11, BScV-p16, CTV-p23, GLRaV-2 p24, GBV-p14, HLV-p10, GCLV-p16 or GVA-p10.

As described below, transient expression methods may be used to express the constructs of the present invention (see Liu and Lomonossoff, 2002, Journal of Virological Methods, 105:343-348; which is incorporated herein by reference). Alternatively, a vacuum-based transient expression method, as described by Kapila et al., 1997, which is incorporated herein by reference) may be used. These methods may include, for example, but are not limited to, a method of Agro-inoculation or Agro-infiltration, syringe infiltration, however, other transient methods may also be used as noted above. With Agro-inoculation, Agro-infiltration, or syringe infiltration, a mixture of Agrobacteria comprising the desired nucleic acid enter the intercellular spaces of a tissue, for example the leaves, aerial portion of the plant (including stem, leaves and flower), other portion of the plant (stem, root, flower), or the whole plant. After crossing the epidermis the Agrobacteria infect and transfer t-DNA copies into the cells. The t-DNA is episomally transcribed and the mRNA translated, leading to the production of the protein of interest in infected cells, however, the passage of t-DNA inside the nucleus is transient.

To aid in identification of transformed plant cells, the constructs of this invention may be further manipulated to include plant selectable markers. Useful selectable markers include enzymes that provide for resistance to chemicals such as an antibiotic for example, gentamycin, hygromycin, kanamycin, or herbicides such as phosphinothrycin, glyphosate, chlorosulfuron, and the like. Similarly, enzymes providing for production of a compound identifiable by colour change such as GUS (beta-glucuronidase), or luminescence, such as luciferase or GFP, may be used.

Also considered part of this invention are transgenic plants, plant cells or seeds containing the gene construct of the present invention. Methods of regenerating whole plants from plant cells are also known in the art. In general, transformed plant cells are cultured in an appropriate medium, which may contain selective agents such as antibiotics, where selectable markers are used to facilitate identification of transformed plant cells. Once callus forms, shoot formation can be encouraged by employing the appropriate plant hormones in accordance with known methods and the shoots transferred to rooting medium for regeneration of plants. The plants may then be used to establish repetitive generations, either from seeds or using vegetative propagation techniques. Transgenic plants can also be generated without using tissue cultures.

The use of the terms "regulatory region", "regulatory element" or "promoter" in the present application is meant to reflect a portion of nucleic acid typically, but not always, upstream of the protein coding region of a gene, which may be comprised of either DNA or RNA, or both DNA and RNA. When a regulatory region is active, and in operative association, or operatively linked, with a gene of interest, this may result in expression of the gene of interest. A regulatory element may be capable of mediating organ specificity, or controlling developmental or temporal gene activation. A "regulatory region" may includes promoter elements, core promoter elements exhibiting a basal promoter activity, elements that are inducible in response to an external stimulus, elements that mediate promoter activity such as negative regulatory elements or transcriptional enhancers. "Regulatory region", as used herein, may also includes elements that are active following transcription, for example, regulatory elements that modulate gene expression such as translational and transcriptional enhancers, translational and transcriptional repressors, upstream activating sequences, and mRNA instability determinants. Several of these latter elements may be located proximal to the coding region.

In the context of this disclosure, the term "regulatory element" or "regulatory region" typically refers to a sequence of DNA, usually, but not always, upstream (5') to the coding sequence of a structural gene, which controls the expression of the coding region by providing the recognition for RNA polymerase and/or other factors required for transcription to start at a particular site. However, it is to be understood that other nucleotide sequences, located within introns, or 3' of the sequence may also contribute to the regulation of expression of a coding region of interest. An example of a regulatory element that provides for the recognition for RNA polymerase or other transcriptional factors to ensure initiation at a particular site is a promoter element. Most, but not all, eukaryotic promoter elements contain a TATA box, a conserved nucleic acid sequence comprised of adenosine and thymidine nucleotide base pairs usually situated approximately 25 base pairs upstream of a transcriptional start site. A promoter element comprises a basal promoter element, responsible for the initiation of transcription, as well as other regulatory elements (as listed above) that modify gene expression.

There are several types of regulatory regions, including those that are developmentally regulated, inducible or constitutive. A regulatory region that is developmentally regulated, or controls the differential expression of a gene under its control, is activated within certain organs or tissues of an organ at specific times during the development of that organ or tissue. However, some regulatory regions that are developmentally regulated may preferentially be active within certain organs or tissues at specific developmental stages, they may also be active in a developmentally regulated manner, or at a basal level in other organs or tissues within the plant as well. Examples of tissue-specific regulatory regions, for example see-specific a regulatory region, include the napin promoter, and the cruciferin promoter (Rask et al., 1998, J. Plant Physiol. 152: 595-599; Bilodeau et al., 1994, Plant Cell 14: 125-130). An example of a leaf-specific promoter includes the plastocyanin promoter (see US 7,125,978, which is incorporated herein by reference).

An inducible regulatory region is one that is capable of directly or indirectly activating transcription of one or more DNA sequences or genes in response to an inducer. In the absence of an inducer the DNA sequences or genes will not be transcribed. Typically the protein factor that binds specifically to an inducible regulatory region to activate transcription may be present in an inactive form, which is then directly or indirectly converted to the active form by the inducer. However, the protein factor may also be absent. The inducer can be a chemical agent such as a protein, metabolite, growth regulator, herbicide or phenolic compound or a physiological stress imposed directly by heat, cold, salt, or toxic elements or indirectly through the action of a pathogen or disease agent such as a virus. A plant cell containing an inducible regulatory region may be exposed to an inducer by externally applying the inducer to the cell or plant such as by spraying, watering, heating or similar methods. Inducible regulatory elements may be derived from either plant or non-plant genes (e.g. Gatz, C. and Lenk, LR.P., 1998, Trends Plant Sci. 3, 352-358; which is incorporated by reference). Examples, of potential inducible promoters include, but not limited to, tetracycline-inducible promoter (Gatz, C.,1997, Ann. Rev. Plant Physiol. Plant Mol. BioI. 48,89-108; which is incorporated by reference), steroid inducible promoter (Aoyama. T. and Chua, N.H.,1997, Plant 1. 2, 397-404; which is incorporated by reference) and ethanol-inducible promoter (Salter, M.G., et aI, 1998, Plant 10urnal 16, 127-132; Caddick, M.X., et al,1998, Nature Biotech. 16, 177-180, which are incorporated by reference) cytokinin inducible IB6 and CKI 1 genes (Brandstatter, I. and K.ieber, 1.1.,1998, Plant Cell 10, 1009-1019; Kakimoto, T., 1996, Science 274,982-985; which are incorporated by reference) and the auxin inducible element, DR5 (Ulmasov, T., et aI., 1997, Plant Cell 9, 1963-1971; which is incorporated by reference).

A constitutive regulatory region directs the expression of a gene throughout the various parts of a plant and continuously throughout plant development. Examples of known constitutive regulatory elements include promoters associated with the CaMV 35S transcript (Odell et aI., 1985, Nature, 313: 810-812), the rice actin 1 (Zhang et aI, 1991, Plant Cell, 3: 1155-1165), actin 2 (An et al., 1996, Plant J., 10: 107-121), or tms 2 (U.S. 5,428,147, which is incorporated herein by reference), and triosephosphate isomerase 1 (Xu et. aI., 1994, Plant Physiol. 106: 459-467) genes, the maize ubiquitin 1 gene (Cornejo et ai, 1993, Plant Mol. BioI. 29: 637-646), the Arabidopsis ubiquitin 1 and 6 genes (Holtorf et aI, 1995, Plant Mol. BioI. 29: 637-646), and the tobacco translational initiation factor 4A gene (Mandel et aI, 1995, Plant Mol. BioI. 29: 995-1004).

The term "constitutive" as used herein does not necessarily indicate that a gene under control of the constitutive regulatory region is expressed at the same level in all cell types, but that the gene is expressed in a wide range of cell types even though variation in abundance is often observed. Constitutive regulatory elements may be coupled with other sequences to further enhance the transcription and/or translation of the nucleotide sequence to which they are operatively linked. For example, the CPMV-HT system is derived from the untranslated regions of the Cowpea mosaic virus (CPMV) and demonstrates enhanced translation of the associated coding sequence. By "native" it is meant that the nucleic acid or amino acid sequence is naturally occurring, or "wild type". By "operatively linked" it is meant that the particular sequences, for example a regulatory element and a coding region of interest, interact either directly or indirectly to carry out an intended function, such as mediation or modulation of gene expression. The interaction of operatively linked sequences may, for example, be mediated by proteins that interact with the operatively linked sequences.

The RLP produced within a plant may induce a rotavirus VP7 structural protein comprising plant-specific N-glycans. Therefore, this invention also provides for a RLP comprising VP7 having plant specific N-glycans.

Furthermore, modification of N-glycan in plants is known (see for example U.S. 60/944,344; which is incorporated herein by reference) and VP7 having modified N-glycans may be produced. VP7 comprising a modified glycosylation pattern, for example with reduced fucosylated, xylosylated, or both, fucosylated and xylosylated, N-glycans may be obtained, or VP7 having a modified glycosylation pattern may be obtained, wherein the protein lacks fucosylation, xylosylation, or both, and comprises increased galactosylation. Furthermore, modulation of post-translational modifications, for example, the addition of terminal galactose may result in a reduction of fucosylation and xylosylation of the expressed VP7 when compared to a wild-type plant expressing VP7.

For example, which is not to be considered limiting, the synthesis of VP7 having a modified glycosylation pattern may be achieved by co-expressing VP7 along with a nucleotide sequence encoding beta-1.4 galactosyltransferase (GalT), for example, but not limited to mammalian GalT, or human GalT however GalT from another sources may also be used. The catalytic domain of GalT may also be fused to a CTS domain (i.e. the cytoplasmic tail, transmembrane domain, stem region) of N-acetylglucosaminyl transferase (GNT1), to produce a GNT1-GalT hybrid enzyme, and the hybrid enzyme may be co-expressed with VP7. The VP7 may also be co-expressed along with a nucleotide sequence encoding N-acetylglucosaminyl transferase III (GnT-III), for example but not limited to mammalian GnT-III or human GnT-III, GnT-III from other sources may also be used. Additionally, a GNT1-GnT-III hybrid enzyme, comprising the CTS of GNT1 fused to GnT-III may also be used .

Therefore the present invention also provides RLPs comprising VP7 having modified N-glycans.

Without wishing to be bound by theory, the presence of plant N-glycans on VP7 may stimulate the immune response by promoting the binding of VP7 by antigen presenting cells. Stimulation of the immune response using plant N glycan has been proposed by Saint-Jore-Dupas et al. (2007).

The present invention will be further illustrated in the following examples.

### Examples

### Example 1

### Expression of rotavirus proteins and production of VLPs in N. benthamiana plant leaves

The following analysis utilized rotavirus capsid proteins from the G9 P[6] rotavirus strain, and assessed whether rotavirus-like particles were formed in the various compartments of tobacco N. benthamiana leaf cells. Co-expression of VP2 and VP6 as well as various combinations of VP2, VP6, VP7 and VP4 in tobacco plant leaves were investigated.

### Materials and Methods

### Plasmid construction

Plant codon optimized rotavirus cDNAs for VP2, VP4, VP6 and VP7 were supplied by Geneart, Germany. The plasmid DNA was transformed into DH5-α chemically competent E. coli cells (E. cloni™, Lucigen) as per the manufacturer's instructions. Novel binary Agrobacterium vectors pTRAc (cytoplasm), pTRAkc-rbcs1-cTP (chloroplast targeting) and pTRAkc-ERH (endoplasmic reticulum targeting) supplied by Rainer Fischer (Fraunhofer Institute for Molecular Biology and Applied Ecology, IME, Germany) were used in this study. An additional vector, pTRAkc-A (apoplast), was derived from the modification of pTRAkc-ERH by restriction enzyme (RE) digestion at sites NcoI and XhoI in the multiple cloning site (Figure 3). This removes the histidine tag and KDEL sequence which retain proteins in the ER. The proteins are instead targeted to the apoplast.

VP2, VP4 and VP6 cDNA was restriction enzyme (RE) digested with NcoI/XhoI while VP7 was cut with AflIII/XhoI. Restriction enzymes Afllll, NcoI and MluI have compatible sticky ends. For direct cloning of the DNA into pTRAc, pTRAkc-rbcs-cTP and pTRAkc-A, the vectors were each RE digested at sites AflIII/XhoI, MluI/XhoI and NcoI/XhoI, respectively. Cloning of DNA in the vectors was carried out as per standard protocol followed by transformation into chemically competent E. coli DH5-α cells (E. cloni™,Lucigen). Selected recombinant colonies were verified by colony PCR. For cloning in pTRAkc-ERH, a NotI restriction enzyme site was added to replace the stop codon of each the four rotavirus cDNA by PCR amplification. The cDNA was amplified with primers as detailed in Table 1. The PCR reaction conditions included denaturation at 95°C for 5 min, followed by five cycles of denaturation at 95°C for 30 sec, annealing at 52°C for 1 min, and elongation at 72°C for 1.5 min. A further 20 cycles were done as follows; 95°C for 30 sec, 57°C for 1 min, 72°C for 1.5 min and 72°C for 5 min. The amplified fragments were then cloned into pGEM-T-Easy (Promega) as per the manufacturer's instructions. Transformation was carried out in chemically competent E. coli DH5-α (E. cloni™, Lucigen). Colony PCR was then carried out on selected colonies as done for the other three constructs.

**Table 1: Rotavirus cDNA primers for ER vector cloning**

| **Primer** | **Sequence** | **R.E site added** | | **Orientation** |
|---|---|---|---|---|
| **VP2F** | **5'-TTCCATGGCTTACCGTAAAAGG-3'** | - | SEQ ID NO: 5 | Forward |
| **VP2R** | **5'-ATGCGGCCGCAAGCTCGTTCATAATCCTCATG-3'** | **Notl** | SEQ ID NO: 6 | Reverse |
| **VP4F** | **5-TTCCATGOCTTCCCTCATCTAC-3'** | **-** | SEQ ID NO: 7 | Forward |
| **VP4R** | **5'-ATGCGGCCGCAAGACGGCACTGGAGAATGAG-3'** | **Notl** | SEQ ID NO: 8 | Reverse |
| **VP6F** | **5'-TTCCATGGATGTGCTCTACTC-3'** | **-** | SEQ ID NO: 9 | Forward |
| **VP6R** | **5'-ATGCGGCCGCCTTCACGAGCATGGAACG-3'** | **Notl** | SEQ ID NO: 10 | Reverse |
| **VP7F** | **5'-GTACATGTACGGAATCGAGTAC-3'** | **-** | SEQ IDNO:11 | Forward |
| **VP7R** | **5'-ATGCGGCCGCCACACGGTAGTAGAAAGCAGC-3'** | **Notl** | SEQ ID NO: 12 | Reverse |

The pGEM-VP DNAs from positive colonies were sequenced to verify fidelity of the PCR. DNA was digested with NcoI/NotI and the appropriate DNA fragment cloned into pTRAkc-ERH at sites NcoI and NotI to form pTRAkc-ERH-VP. Transformation was then carried out into E. coli DH5-α cells as previously done. Colony PCR was also performed to check for rotavirus DNA in selected colonies.

### Agrobacterium transformation

Agrobacterium tumefaciens GV3101 strain was provided by Professor Rainer Fischer (Fraunhofer Institute for Molecular Biology and Applied Ecology IME, Aachen, Germany) and made electrocompetent as previously described (Shen and Forde, 1989). Three hundred nanograms of isolated rotavirus pTRA-VP constructs were mixed with 100 µl of electrocompetent GV3101 cells in a 0.1 cm electrogap cuvette (BioRadTM) then electroporated in a GenePulser (BioRad) under the following settings: 1.8 kV, 25 µF and 200 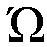 . Incubation was allowed for 1 hr at 27°C in 900 µl of LB before plating on LA plates containing 50 µg/ml carbenicillin (carb), 30 µg/ml kanamycin (kan) and 50 µg/ml rifampicin (rif). The plates were incubated at 27°C for 3 days. To check for positive transformants, plasmid DNA was isolated from the recombinant Agrobacterium colonies and back-transformed into E. coli competent DH5-α cells. These were then selected on 100 µg/ml ampicillin (amp) LA. Colony PCR and restriction enzyme digests on cDNA were done to verify successful transformants. Glycerol stocks of relevant recombinant Agrobacterium were made and stored at -70 °C.

### Recombinant Agrobacterium infiltration

A. tumefeciens LBA 4404 (pBIN-NSs) used in this study was obtained from Marcel Prins (Laboratory of Virology, Wageningen University, Binnenhaven, Netherlands). It contains the NSs silencing suppressor found in Tomato spotted wilt virus (TSWV). Recombinant Agrobacterium (pTRA-VPs) from glycerol stocks were grown at 27 °C overnight in LB with 50 µg/ml carb, 30 µg/ml kan and 50 µg/ml rif. The recombinant Agrobacterium and LBA4404 (pBIN-NSs) were then each inoculated in induction medium (LB, 10 mM 2-(N-morpholino) ethanesulphonic acid MES, 2 mM MgSO4, 20 µM acetosyringone, 50 µg/ml carb, 30 µg/ml kan and 50 µg/ml rif, and pH 5.6).

Cultures were incubated at 27°C overnight. Agrobacterium cells were collected by centrifugation at 4000 rpm for 5 min at 4°C and then resuspended in 2 ml infiltration medium (10 mM MES, 10 mM MgC12, 3 % sucrose, pH 5.6, 200 µM acetosyringone and sterile water). Optical density (OD600) of the cells was verified and diluted with infiltration medium to obtain an OD600 of 0.25. For each pTRA-VP construct, LBA4404 was mixed with recombinant Agrobacterium to final OD600 of 0.5. For co-expression studies, each construct was added to total an OD600 of 0.5, for example; VP2 - 0.25 and VP6 - 0.25, until the OD600 for mixture equalled 0.5. Acetosyringone used in the induction and infiltration medium helps in activation of vir genes in Agrobacterium.

Wounded plant cells release phenolic compounds which are detected by Vir A and Vir G genes in Agrobacterium subsequently leading to induction of protein expression in the host cells (Zupan, J. et al., 2000). The cells were then incubated at room temperature for 1 h to allow acetosyringone to induce vir genes. Three week old wild type N. benthamiana plants were infiltrated with recombinant Agrobacterium expressing the VP proteins. This involved either vacuum infiltration of whole plants or injection of recombinant Agrobacterium (pTRA-VP) into the abaxial air spaces on the ventral side of plant leaves. Recombinant agrobacterium was infiltrated either with or without the silencing suppressor LBA 4404 (pBIN-NSs).

Initially, 2 ml of Agrobacterium infiltration medium suspension was injected into each plant using a syringe per construct. One plant was used per construct over a seven day time trial. Co-expression of rotavirus proteins was also carried out in which VP2, VP6 and VP4 were simultaneously expressed in the cytoplasm of N. benthamiana plant leaves. Combinations investigated were VP2/6 and VP2/6/4. VP4 "spike" protein may bind to VP6, and thus there is a possibility that they could add to RLP structures. VP7 cloning was attempted but toxicity issues to host cells proved to be a problem. Recombinant VP7 Agrobacterium killed leaf cells within a day after infiltration. Several methods were tried to evade this such as infiltrating plants at a low temperature of 17° C and infiltrating after day 3 and/or day 5 of the time trials. As such, VP7 was omitted in the co-expression studies due to its toxic nature in the tobacco plants.

### Protein Extraction

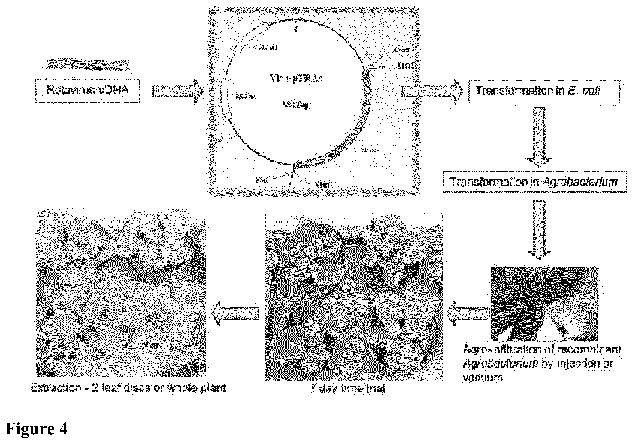

Whole leaves or two leaf discs per construct were harvested and ground in liquid nitrogen. Ground leaf matter was resuspended in sterile PBS containing Complete Protease Inhibitor (EDTA-free; Roche). This was then centrifuged for 5 min at 13000 rpm and the pellets (plant leaf matter) discarded. 100 µl of each construct were then mixed with 5X SDS-PAGE loading buffer and boiled for 2 min at 95 °C, ready for further analysis on SDS-PAGE gels and western blots. The rest of the samples were stored at -20 °C for future use. Figure 4 shows an overview of the cloning and infiltration procedure for the rotavirus cDNA.

### Apoplast protein extraction

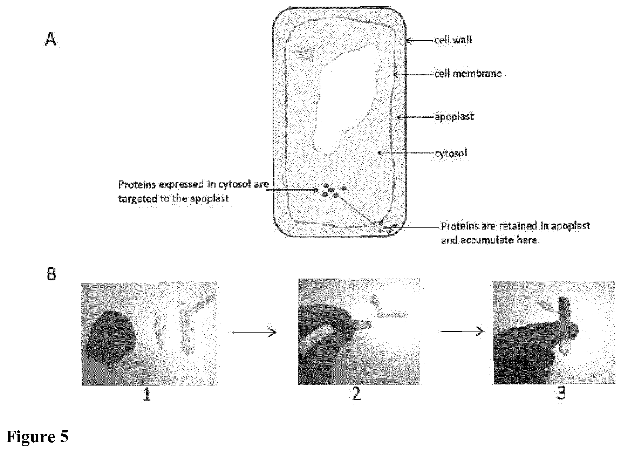

An additional extraction procedure was carried out on the pTRAkc-A, apoplast constructs. The apoplast is the free diffusional space between the plasma membrane and cell walls of plant cells (Figure 5a). Proteins expressed in the cytoplasm have an export sequence that targets them to the apoplast hence they accumulate here. In the extraction procedure that followed, whole leaves from each extraction day were either vacuum or injection infiltrated with sterile PBS containing Complete Protease Inhibitor. For vacuum infiltration, individual plant leaves were suspended in PBS and put under a vacuum at 100 mbar for 10 min in a vacuum tank. The leaves were then rolled and gently placed in spin columns (similar to Qiagen spin columns) with a hole at the bottom (Figure 5b2). The holes allow easy passage of fluid from the leaves without allowing solid leaf matter to pass through. The spin columns were placed in 2 ml Eppendorf tubes and centrifugation carried out at 4000 rpm for 15 min (Figure 5 b3). The filtrate was collected and protein loading dye for SDS-PAGE gels and western blot analysis was added to 100 µl of each filtrate sample.

### Western Blots and Coomassie stains

Western blots and Coomassie blue stained SDS-PAGE gels were used as previously described. Mouse anti-rotavirus VP6 antibody (US Biologicals) (1:5000), anti-mouse histidine tag antibody (Sigma®) (1:2000), chicken anti-VP2 and chicken anti-VP4 serum (1:2000) were used to probe each of the respective proteins in western blots. Coomassie blue stained SDS-PAGE gels were used to quantify proteins by density scanning of bands using a Syngene Gel Documentation System.

### Electron Microscopy

To determine whether expressed proteins assembled into RLPs, transmission electron microscopy (TEM) of immuno-trapped particles was performed on day 3 of expression of cytoplasm expressed VP6, VP2/6 and VP2/6/4, all in the presence of a silencing suppressor Nss. Glow discharged carbon/copper grids were placed on 20 µl of mouse anti-rotavirus VP6 antibody (1:5000) for 5 min and then washed 3 times with sterile distilled water. The grids were then placed on 10 µl of the protein extracts and left for 2 min before being washed 3 times again with sterile distilled water. Finally, the grids were floated on 20 µl of 2 % uranyl acetate for 1 min before viewing under a TEM (Zeiss 912 OMEGA Energy Filter Transmission Electron Microscope, University of Cape Town).

For samples isolated from sucrose gradients, the sucrose first had to be removed by dialysis before immune-trapping on the copper grids. If not removed, sucrose crystals inhibit definitive viewing of samples under the TEM as it forms crystals on the grids, disrupting the structure of bound carbon and material. The sucrose fractions were placed in 10 000 MW dialysis cassettes and dialyzed in sterile PBS containing 0.4 M NaCl for 4 hr before exchanging the buffer and leaving it overnight at 4 °C with stirring. Since volume increases with dialysis, the protein samples required concentrating. The samples were vacuum freeze dried for 3 hours and resuspended in 2 ml of sterile PBS, ready for further analysis.

### Sucrose gradient purification of RLPs

Plant protein extracts were initially filtered through miracloth to remove solid plant matter. Sucrose gradients from 10 to 60 % sucrose were set up in 40ml tubes each by creating six layers of 5 ml of sucrose dissolved in sterile PBS (pH 7.4). Clarified protein samples in 5 to 10 ml volumes were then loaded on top of each gradient column.

Ultracentrifugation at 150 000 g (SWTi28 swinging bucket rotor, Beckman Coulter) was carried out at 4 °C for 1 h 30 min. At the end of the centrifugation, 2 ml fractions were collected from the bottom of each column by tube puncture. Dot blots were then performed to determine fractions with proteins of interest. For each fraction, 1 µl of sample was loaded in a grid on a nitrocellulose membrane, which was then blocked with BSA blocking buffer. Western blot analysis was then performed as usual. Proteins were probed with mouse anti-VP6 antibody (1:5000) for VP6 or chicken anti-VP2 and VP4 serum (1:5000) for the other two proteins.

### Total Soluble Protein Assay

Total soluble protein (TSP) was determined by Bradford assays. This was carried out to compare the levels of accumulated proteins in cytoplasm co-expressed VP2/6. The protein IgG (1.43mg/ml stock) was used in a dilution series as a standard. 5 µl of standard and samples were each added to a clean dry microtitre plate. Total Soluble Protein Reagents A and B were added as per manufacturer's instructions (Bio-Rad Dc Protein Assay). All experiments were done in triplicate. Absorbance readings were recorded at 750 nm using a microplate reader (Bio-tek PowerWave XS).

### Results

### Expression of VP6 in plant leaf cell compartments

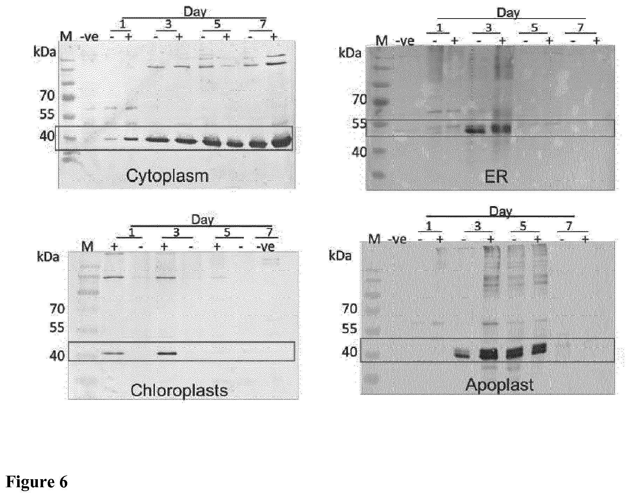

VP6 was expressed and targeted to all cellular compartments (Figure 6; (the line marks VP6, at ∼42 kDa)), with and without the silencing suppressor. In the cytoplasm, protein was expressed from day one of the time trial, with increasing protein accumulation in the cytoplasm during the week-long trial (Figure 6a). In the ER, protein accumulation was clearly seen at day 3 only (Figure 6b). The protein ran at a higher band size (approximately 11 kDa more) than the other proteins. This may be a result of the 6 histidine-tag added to the C-terminal end of the protein, as well as the cleavage site (refer to pProEx vector sequence)

Protein accumulation in the chloroplasts occurred between days 1 and 3 (Figure 6 "chloroplasts"). The silencing suppressor had an effect on the proteins as no proteins were detected in its absence. There was no protein expression at days 5 and 7. The apoplast, just as in the ER, had the best protein accumulation between days 3 and 5 of the time trial (Figure 6 "apoplast") and none at all at day 1 and day 7. The silencing suppressor had a positive effect especially on day 3, resulting in higher protein detection levels compared to when it was left out. It can also be seen that two bands are visible at the ~40 kDa mark probably as a result of cleavage of the signaling tag on the VP6 protein.

The ER, chloroplasts and apoplast all exhibited the highest protein expression on day 3, with the most protein accumulating in the presence of the silencing suppressor. The cytoplasm was the best in terms of protein accumulation as it exhibited high and increasing protein expression throughout the time-trial.

### Expression of histidine-tagged rotavirus proteins in the cytoplasm

The four rotavirus VPs were cloned into an additional vector, pTRAc-HT. This vector includes a 6-histidine tag to proteins targeted to the cytoplasm and makes detection easy by use of an anti-histidine tag antibody if the antibodies for the proteins of interest are unavailable. In our case, only VP6 has a commercially available antibody and hence we tried this procedure for early detection of all proteins while awaiting serum. The cytoplasm also worked well for VP6 expression and motivated us to try the other proteins.

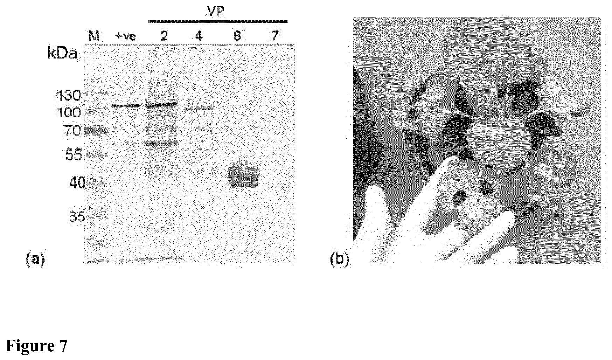

Western blot results of day 3 extracts from a 7 day time trial showed successful expression of VP2, VP4 and VP6 (Figure 7a). In order to obtain expression of VP7 in plants, various techniques were tried. However, plants infiltrated with VP7 exhibited yellowing leaves from day 1 and proceeded to wilt during the course of the time trial (Figure 7b). No expression of protein was detected under these conditions, even after day 1 of infiltration when the plant still looked reasonably good.

### Expression of VP2 and VP4 in plants

VP2 and VP4 were infiltrated in N. benthamiana plant leaves and targeted to the ER, chloroplast, cytoplasm and apoplast. We were unable to express VP2 targeted to the apoplast vector as we could not get any positive clones in E. coli. However, the protein was successfully expressed and targeted to all other 3 compartments (Figure 8a). Chicken anti-VP2 and anti-VP4 serum (1:2000) was used in western blot analysis of extracts. VP2 and VP4 bands were visible just below the 100 kDa mark (protein band indicated by arrow) as seen in Figure 8a and 8b respectively. Expression appeared to be best in the cytoplasm and ER for VP2 while for VP4, in the cytoplasm and apoplast. The silencing suppressor did not have a significant effect on the expression of the proteins. It only slightly increased expression in the VP2 ER construct and not so much in the rest, as can be seen from the western blot. VP4 constructs were all expressed in the presence of the silencing suppressor.

### Co-expression of VP2/6 and VP2/6/4 in the cytoplasm

The cytoplasm appeared to be best for rotavirus capsid protein expression and exhibited the highest extraction efficiencies. All further expression work was therefore done with proteins targeted to the cytoplasm.

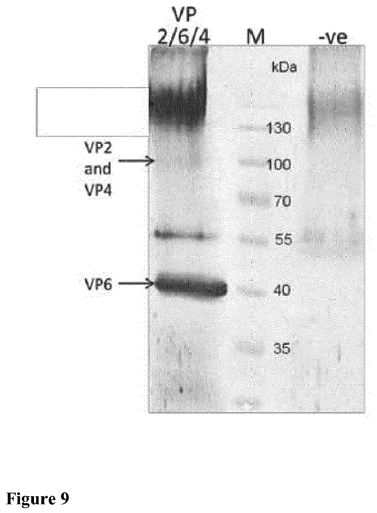

VP2 and VP6 have been shown to form RLPs with protective immunogenic responses in mice and therefore co-expression of VP2/6 and VP2/6/4 in the cytoplasm was investigated. Day 3 extracts of co-expressed VP2/6/4 were detected by western blot with anti-VP2 and VP4 serum (1/5000) and mouse anti-VP6 antibody (1:5000) (Figure 9). VP6 expression was very high as previously determined, but expression of VP2 and VP4 was very low as can be seen from the very faint band at the 100 kDa mark. This may have been attributed by the co-expression which resulted in more host cell resources being utilized in the over-expression of VP6, leaving less for VP2 and/or VP4. It was also not easy to determine if the detected band was both VP2 and VP4 or either of the 2 proteins. The very visible band running above 130 kDa may be dimerized VP6 proteins. The band visible at the 55 kDa mark is most likely the abundant plant enzyme Rubisco.

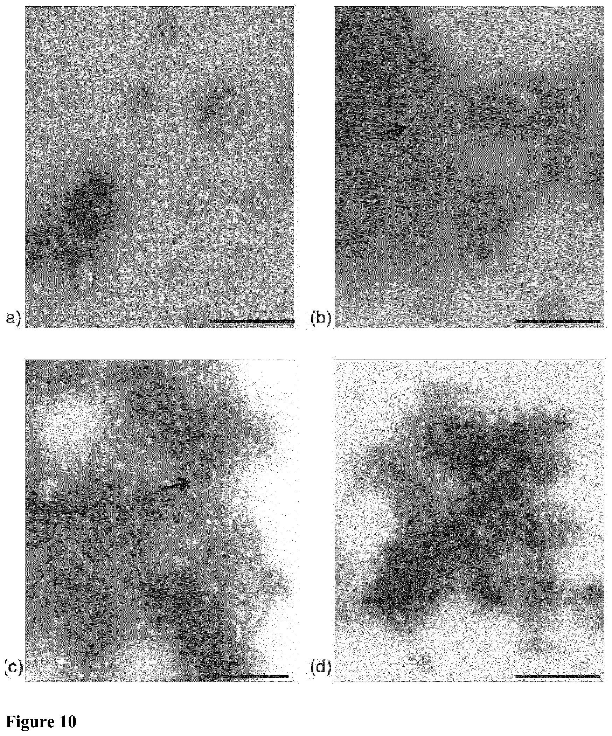

Transmission electron microscopic analysis on the cytoplasm-expressed VP6 as well as on co-expressed VP2/6 and VP2/6/4, were carried out to check for protein particles and assembled RLPs (Figure 10). This also determined if VP2 and/or VP4 were indeed co-expressed successfully. VP6 when expressed alone assembled to form sheaths of protein as indicated by the arrow in Figure 10b. On addition of VP2, the particles assembled to form RLPs (Figure 10c). VP2 acts as scaffolding protein that enables other proteins to assemble and ultimately form a complete rotavirus structure. VP6 as such bound to VP2 but it was still not easy to determine VP4 structures in co-expressed VP2/6/4. The electron micrograph in Figure 10d may be purely assembled VP2/6 particles. It has been shown however that VP4 binds to VP6 during protein assembly, and this occurs before VP7 binds. It is likely that these VP4 structures are not stable and may fall off the RLP structure during preparation procedures for electronic microscopy.

### Sucrose gradient purification of VP2/6 and VP2/6/4

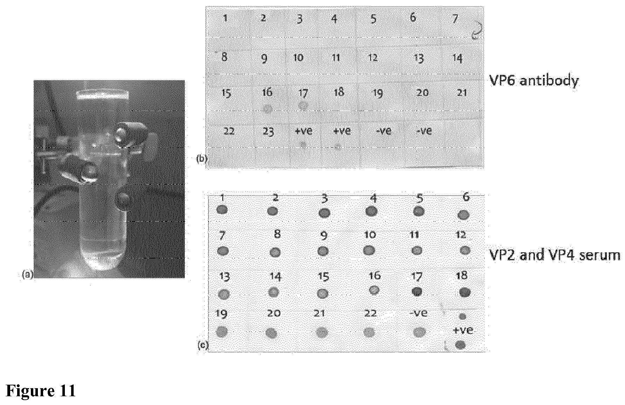

VP2/6 and VP2/6/4 were purified on a sucrose gradient ranging from 10 to 60 % sucrose (Figure 11a). 2 ml fractions were collected from the bottom of each of the tubes and probed with mouse anti-VP6 antibody and/or chicken anti-VP2 and VP4 serum to determine which fractions contained the proteins. For VP2/6, proteins were found in fractions 16 and 17 as these were positive for VP6 protein on the blot (Figure 11b). VP2/6/4 blot analysis with chicken anti-VP2 and VP4 serum showed positive results throughout all the fractions. This may have been as a result of the high levels of background protein detection by the chicken serum. However, intensity of dots was highest in fractions 17 and 18 as seen in Figure 11c, possibly due to higher concentration of the proteins of interest in these fractions.

Putting these results together (Figure 11b and 11c), the rotavirus proteins were in fractions ranging from 16 to 20.

### Western Blot and Coomassie stains of fractions

A western blot and SDS-PAGE of co-expressed VP2/6 were done to verify presence of VP2 and VP6 proteins in fractions 13 to 20. Western blot analysis for VP6 protein probed with mouse anti-VP6 antibody was positive in fractions 16 right through to 20 (Figure 12a, bottom arrow and Figure 12c). VP2 protein probed with chicken anti-VP2 serum was detected in fractions 17 to 20 (Figure 12a, top arrow). VP2 has been shown to express less than VP6 in past co-expression studies and this was also shown here Figure 12a in which VP2 protein bands have a lower intensity in comparison to VP6.

Fractions 16 and 17 of co-expressed VP2/6 previously determined to contain VP6 protein by dot blots (Figure 11b) were electrophoresed on an SDS-PAGE gel. A protein of known concentration, SF9 insect cell expressed VP6 (0.91 µg/µl) was included so as to determine the concentration of VP2/6 crude proteins (Figure 11b and c). This was done by density scanning of the crude protein band (lane labeled crude) using a Syngene Gel Documentation System and consequently allowed us to determine the amount of VP2/6 per kilogram of leaf matter. Protein yield was found to be approximately 1.54 g/kg fresh weight (FW). 1.1 mg of purified RLPs were obtained from 1 gram of plant material (1.1g/kg).

### Total Soluble Protein Assay of VP2/6

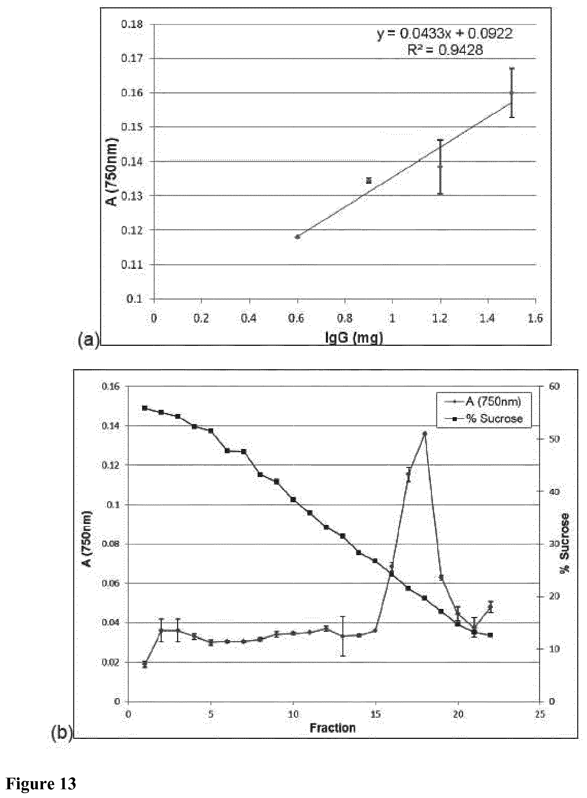

Total soluble protein (TSP) was determined on co-expressed VP2/6 fractions to determine the relative amounts of VP2/6 protein (Figure 13). Protein concentrations were calculated as 0.538 mg/ml and 1.012 mg/ml for fractions 17 and 18 respectively with the use of an IgG standard (Figure 13a). The protein bands corresponding to VP2/6 in these fractions were calculated by density scanning on a Syngene Gel Documentation System and found to be approximately 0.108 mg/ml and 0.202 mg/ml, respectively.

Thus, the TSP for VP2/6 in fractions 17 and 18 were both approximately 20 % TSP Most of the RLPs in the sucrose column were found to be between 15 and 25 % sucrose, corresponding with fractions 15 to approximately 20, where the graph is noted to suddenly peak and then subside. The differences in density of the various materials in the extract allowed me to separate and thereby purify the proteins of interest. SDS-PAGE gels stained with Coomassie blue showed only one prominent band indicating that the proteins are relatively pure (Figure 12b).

### TEM of purified VP2/6

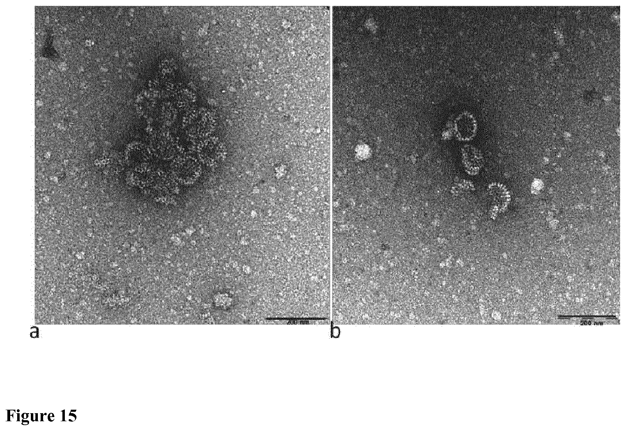

Purified VP2/6 fractions were pooled together and dialyzed in high salt PBS to remove sucrose before viewing on a transmission electron microscope. The TEM was done to determine purity and check if RLPs remained intact after the purification procedure. As can be seen in Figure 15, most of the background material which mainly consisted of the host cell's products (Figure 10b, c and d) was removed, leaving behind RLPs. Most of the RLPs remained intact but some appeared to have lost shape probably as a result of deformation due to the conditions on the EM grid.

### Preliminary analysis of expression of rotavirus structural proteins in N. benthamiana leaves

This preliminary analysis focused on the expression of rotavirus structural proteins VP2 (SEQ ID NO:1), VP4 (SEQ ID NO:2), VP6 (SEQ ID NO:3) and VP7 (SEQ ID NO:4) in N. benthamiana leaves as an example host expression system. The strain of rotavirus selected here was a G9 P[6] strain which circulates predominantly in South Africa and other sub-Saharan regions. A RLP vaccine targeting this strain would help in alleviating the burden of disease in sub-Saharan Africa.

A transient expression system mediated by Agrobacterium was used in this analysis. Transient expression, as opposed to transgenic expression, permits rapid expression of proteins in a relatively short time, without integration of the rotavirus capsid protein genes in the host's chromosome. Most proteins were expressed and accumulated to detectable amounts by day 3 of recombinant Agrobacterium infiltration in N. benthamiana leaves. As shown below successful expression of several rotavirus structural proteins was observed including VP2, VP4 and VP6 in plant leaf cell compartments as detailed in table 2:

**Table 2: Expression of rotavirus VP proteins in various leaf cell compartments**

| | **Leaf Cell Compartment** | | | |
|---|---|---|---|---|
| **Caplsd Protein** | **Apoplast** | **Chloroplast** | **Cytoplasm** | **ER** |
| **VP2** | 0 | 1 | **1** | **1** |
| **VP4** | 1 | 0 | **1** | **1** |
| **VP6** | 1 | 1 | **1** | **1** |
| **VP7** | 0 | 0 | 0 | 0 |

| | | | | |
|---|---|---|---|---|
| 0 = no expression 1 = expressed | | | | |

Expression of the glycoprotein VP7 was not observed possibly due to its toxic effects on plant cells. It is worth noting that for this preliminary study, a VP7 containing its native signal peptide had been used. Infiltrating at day 3 during co-expression trials was also tried. This was attempted to see if the protein was expressed and soon after assembled with VP2 and VP6 to form RLPs. The toxic nature of recombinant VP7 as observed in this study has been previously described (Williams et al., 1995; McCorquodale, 1987; Arias et al., 1986).

Past VP7 expression studies in transgenic potatoes has been reported (Li et al., 2006; Choi et al., 2005; Wu et al., 2003). Choi et. al. (2005) used a simian rotavirus VP7, and Li et. al. and Wu et. al. (Li et al., 2006; Wu et al., 2003) used human group A G1 VP7. The result described herein used human rotavirus G9 VP7.

VP2 was expressed and targeted to all compartments except the apoplast as we were unable to clone the appropriate cDNA, and time constraints only allowed us a few attempts before proceeding with the other constructs. Expression levels of VP2 were noted to be significantly low in all the compartments. In a past study quoted by Saldana et al. 2006, it was concluded that a VP2 having its sequence optimized for expression in the plant was impossible to express, despite mRNA being detected in the plant cells. They however managed to express it in tomato plant cells using synthetic DNA. The reason for the difficulty in VP2 expression is most likely as result of improper mRNA translation or that the mRNA contains some sequence motifs that destabilize the plant cells (Kawaguchi and Bailey-Serres, 2002). Evidence of low expression levels of VP2 in comparison to VP6 have been seen in both plant and insect-cell expression studies by Mena et al. (2006), Saldana et al. (2006), Vieira et al. (2005), and Labbé et al. (1991).

The outer capsid protein, VP4, which forms spikes on the surface of the virion structure, was expressed and targeted for accumulation in the cytoplasm, ER and apoplast. No protein accumulation was detected in chloroplasts. As observed for VP2, protein expression levels for VP4 were lower than seen for VP6 on western blots. The protein has a trypsin cleavage site which results in two proteins, VP5 and VP8. It may be possible that local trypsin in N. benthamiana leaves cleaves some of the proteins as they are produced resulting in lower concentration levels of accumulated, intact VP4 in the designated compartments. The protein has been shown to be a major neutralizing antigen but there have been a few attempts to clone the whole protein for vaccine development (Khodabandehloo et al., 2009; Mahajan et al., 1995; Nishikawa et al., 1989). There have been, however, several studies in the insect-cell and yeast expression system showing expression of either the VP5 or VP8 subunits of VP4 (Andrés et al., 2006; Favacho et al., 2006; Kovacs-Nolan et al., 2001). To date, the present study represents the first study showing expression of the whole protein in a plant expression system.

VP6 was expressed in all compartments with over-expression being observed in the cytoplasm with protein accumulation observed from day 1 to day 7 in this compartment. This is contrary to some literature which suggests that protease activity and gene silencing reduce or hinder the accumulation of foreign protein in the cytoplasm (Fischer et al., 2004). In addition, given the right pH conditions, VP6 is known to self-assemble into tubular or helical particles, much like the particles seen in our study (Figure 9b) (Estes et al., 1987). VP6 makes up about 50 % of the viral core and therefore is a major antigen in the development of a rotavirus vaccine. The result attained above enabled us to further investigate the co-expression of VP2, VP6 and VP4 in the cytoplasm.

When co-expressed in the cytoplasm, VP2 and VP6 assembled to form RLPs. Very high protein yields from transient expression system of between 1.27 - 1.54 g/kg FW were observed. When purified on a sucrose column, the amount of VPs retained was 1.1 g/kg FW. This yield is comparable to that obtained for the production of an antibody, IgG, using a transient expression system in N. benthamiana, with a yield of up to 1.5 g/kg FW (Vézina et al., 2009). Saldana et al. (2006) were until now the only group known to have successfully co-expressed rotavirus VP2 and VP6 in transgenic tomato plants and to levels of approximately 1 % total soluble protein. The assembly of VP2/6 in the insect-cell expression system has been well documented (Vieira et al., 2005;; O'Brien et al., 2000). These VP2/6 RLPs have also been shown to provide protective immunity against rotavirus infection (Zhou et al., 2011, Saldana et al., 2006). Thus the VP2/6 RLPs we produced in the plant expression system are suitable candidates for the development a subunit rotavirus vaccine.

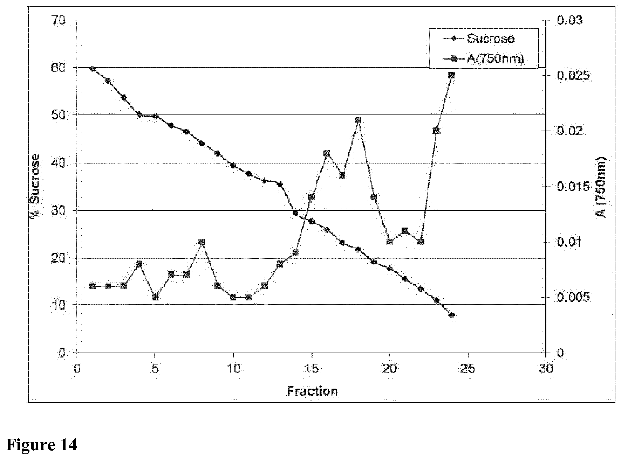

VP2/6/4 were also co-expressed and detected. The first peak visible (Figure 14, fraction 16) in the total protein absorbance reading of the co-expressed proteins might be assembled VP2/6/4, but on examination of this fraction under a TEM, no RLPs were detected. The protein peak observed may result from an accumulation of VP4 monomers or its respective VP5 and VP8 subunits. The second peak (fraction 18), when examined under a TEM showed RLP structures very much similar to the ones seen in the VP2/6 sample. However, Crawford et al have previously reported that VP4 could not be seen under TEM, and that VP2/6/4 and VP2/6/4/7 particles had similar structure and diameter under TEM (Crawford 1994). We made the same observation for VP2/6/7 RLPs, VP 2/6/4/7 RLPs and VP2/6 RLPs, which all look similar under regular TEM.

### Example 2

### Constructs

### A-2X35S/CPMV-HT/ RVA(WA) VP2(opt)/ NOS (Construct number 1710)

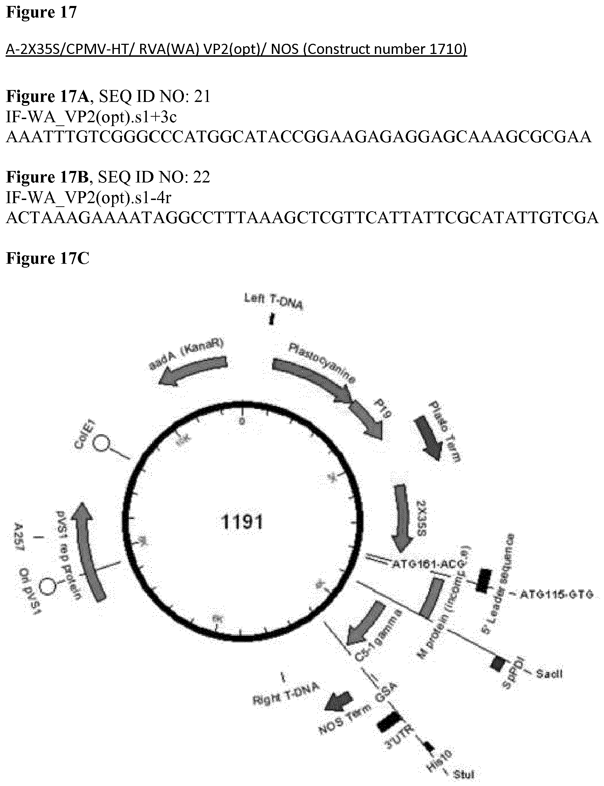

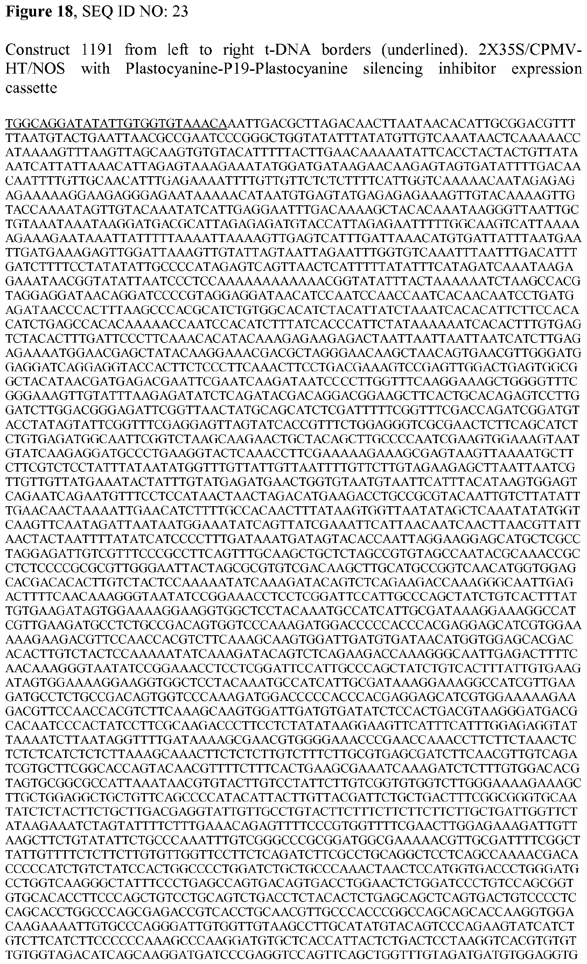

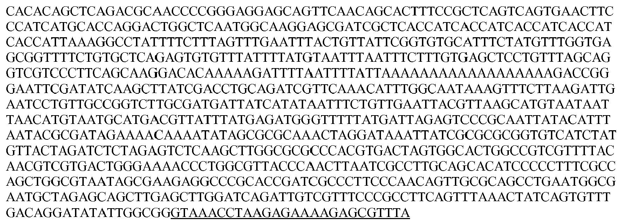

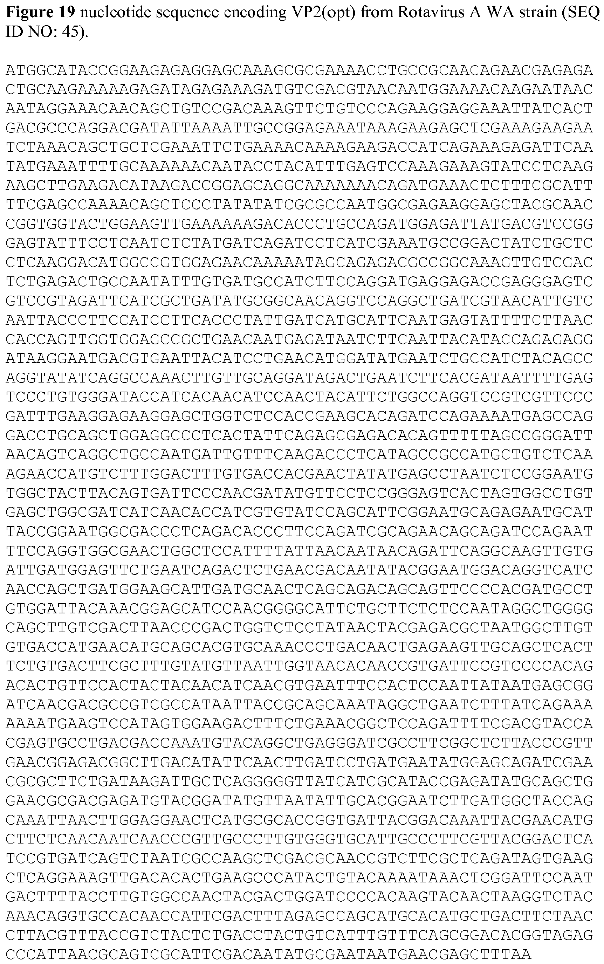

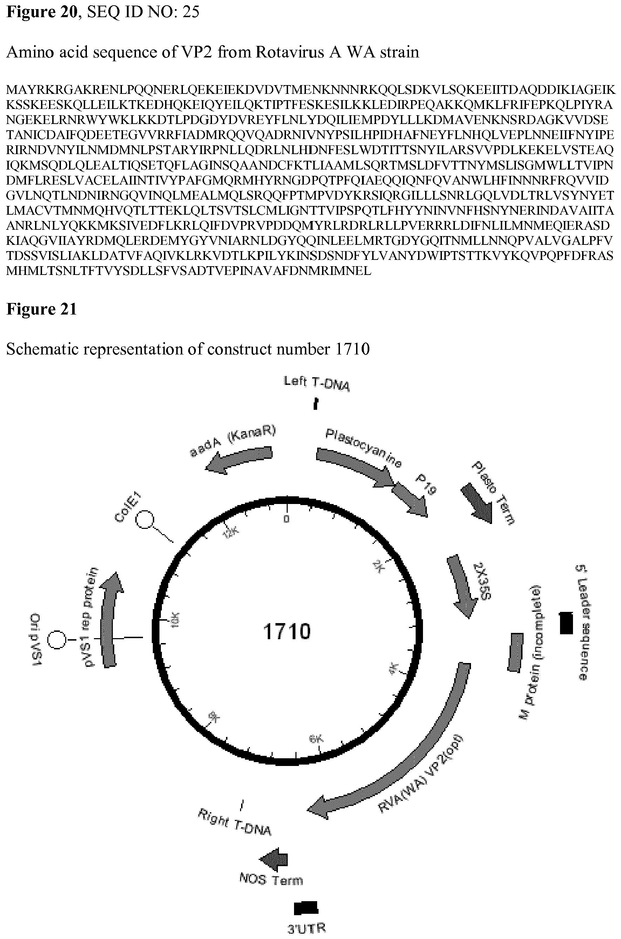

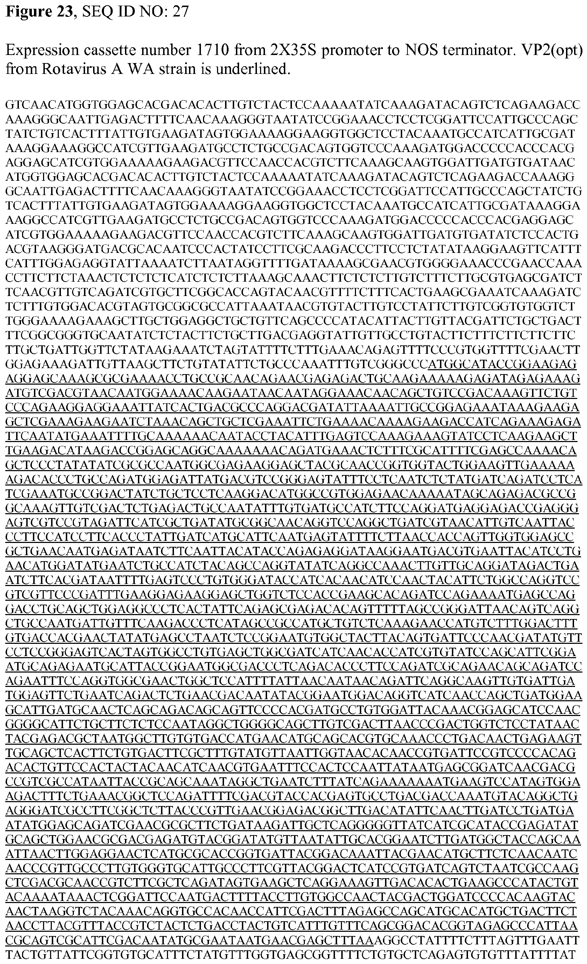

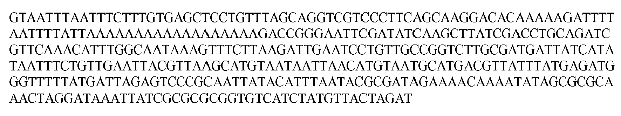

An optimized sequence encoding VP2 from Rotavirus A WA strain was cloned into 2X35S-CPMV-HT-NOS expression system in a plasmid containing Plasto_pro/P19/Plasto_ter expression cassette using the following PCR-based method. A fragment containing the VP2 coding sequence was amplified using primers IF-WA_VP2(opt).s1+3c (Figure 17A, SEQ ID NO: 21) and IF-WA_VP2(opt).s1-4r (Figure 17B, SEQ ID NO: 22), using optimized VP2 gene sequence (Figure 19, SEQ ID NO: 45) as template. For sequence optimization, VP2 protein sequence (Genbank accession number CAA33074) was backtranslated and optimized for human codon usage, GC content and mRNA structure. The PCR product was cloned in 2X35S/CPMV-HT/NOS expression system using In-Fusion cloning system (Clontech, Mountain View, CA). Construct number 1191 (Figure 17C) was digested with SacII and StuI restriction enzyme and the linearized plasmid was used for the In-Fusion assembly reaction. Construct number 1191 is an acceptor plasmid intended for "In Fusion" cloning of genes of interest in a CPMV-HT-based expression cassette. It also incorporates a gene construct for the co-expression of the TBSV P19 suppressor of silencing under the alfalfa Plastocyanin gene promoter and terminator. The backbone is a pCAMBIA binary plasmid and the sequence from left to right t-DNA borders is presented in Figure 18 (SEQ ID NO: 23). The resulting construct was given number 1710 (Figure 23, SEQ ID NO: 27). The amino acid sequence of VP2 from Rotavirus A strain WA is presented in Figure 20 (SEQ ID NO: 25). A representation of plasmid 1710 is presented in Figure 21.

### B-2X35S/CPMV-HT/RVA(WA) VP2(opt)/NOS into BeYDV(m)+Replicase amplification system (Construct number 1711)

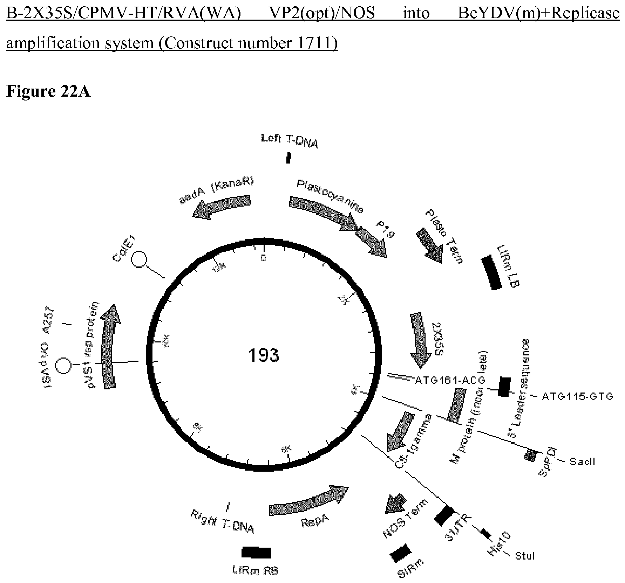

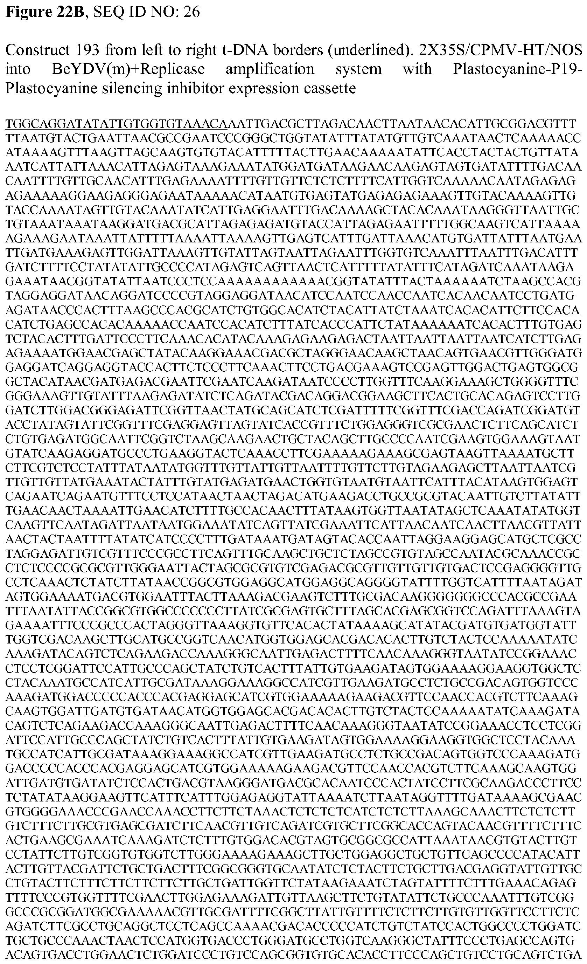

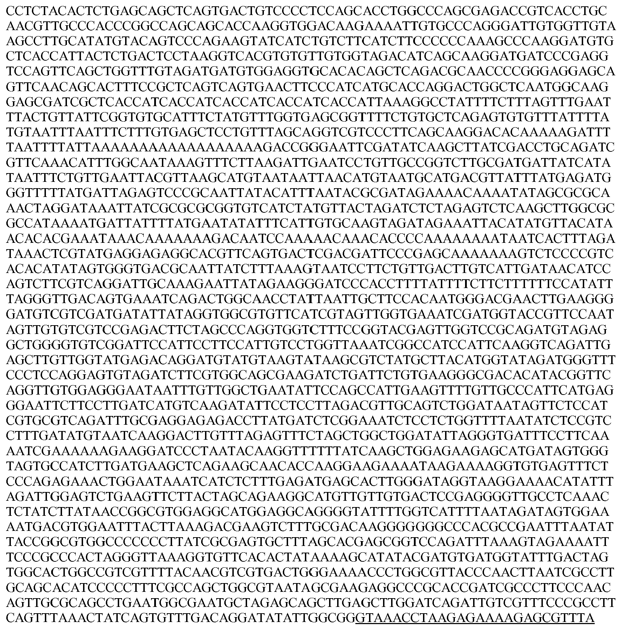

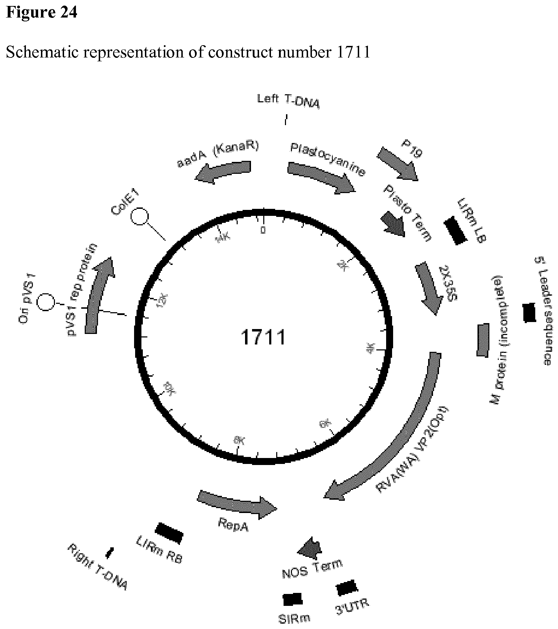

An optimized sequence encoding VP2 from Rotavirus A WA strain was cloned into 2X35S/CPMV-HT/NOS comprising the BeYDV(m)+replicase amplification system in a plasmid containing Plasto_pro/P19/Plasto_ter expression cassette using the following PCR-based method. A fragment containing the VP2 coding sequence was amplified using primers IF-WA_VP2(opt).s1+3c (Figure 17A, SEQ ID NO: 21) and IF-WA_VP2(opt).s1-4r (Figure 17B, SEQ ID NO: 22), using optimized VP2 gene sequence (SEQ ID NO: 45) as template. For sequence optimization, VP2 protein sequence (Genbank accession number CAA33074) was backtranslated and optimized for human codon usage, GC content and mRNA structure. The PCR product was cloned in 2X35S/CPMV-HT/NOS expression cassette into the BeYDV(m) amplification system using In-Fusion cloning system (Clontech, Mountain View, CA). Construct 193 (Figure 22A) was digested with SacII and StuI restriction enzyme and the linearized plasmid was used for the In-Fusion assembly reaction. Construct number 193 is an acceptor plasmid intended for "In Fusion" cloning of genes of interest in a CPMV-HT-based expression cassette into the BeYDV(m) amplification system. It also incorporates a gene construct for the co-expression of the TBSV P19 suppressor of silencing under the alfalfa Plastocyanin gene promoter and terminator. The backbone is a pCAMBIA binary plasmid and the sequence from left to right t-DNA borders is presented in Figure 22B (SEQ ID NO: 26). The resulting construct was given number 1711 (Figure 23, SEQ ID NO: 27). The amino acid sequence of VP2 from Rotavirus A strain WA is presented in Figure 20 (SEQ ID NO: 25). A representation of plasmid 1711 is presented in Figure 24.

### C-2X35S/CPMV-HT/RVA(WA) VP6(opt)/NOS (Construct number 1713)

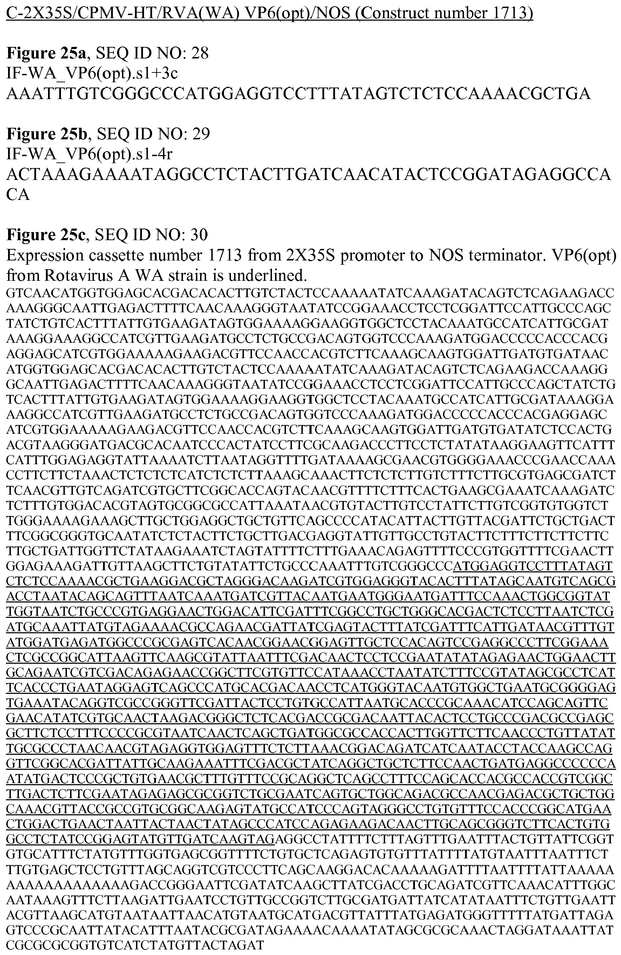

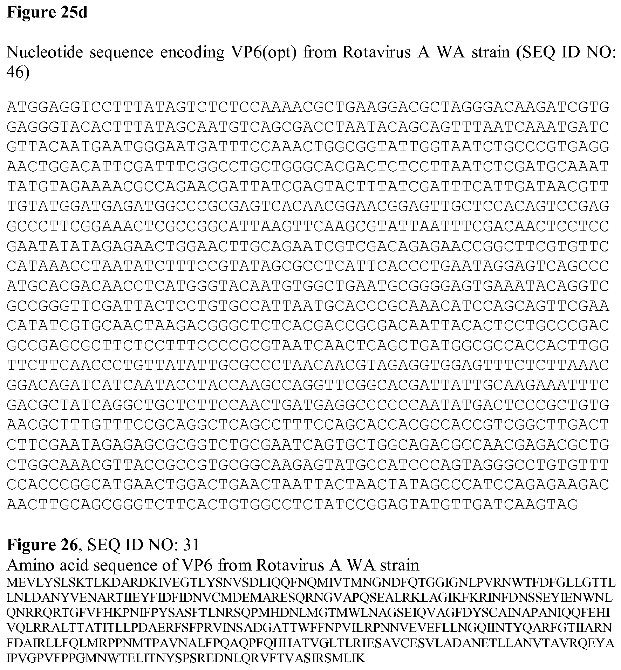

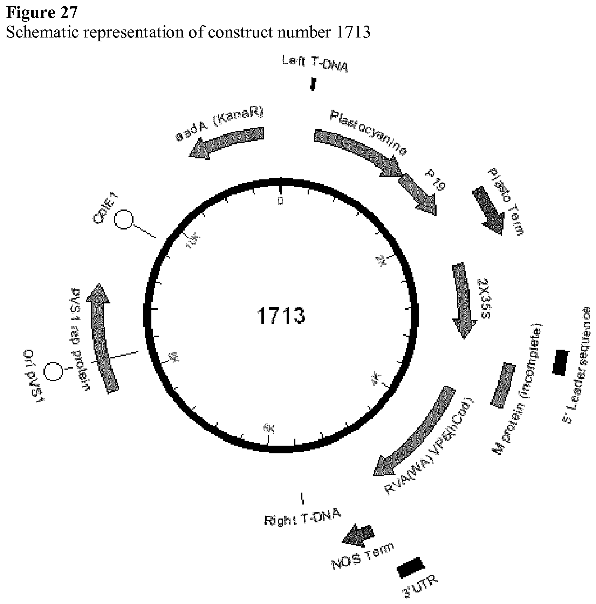

An optimized sequence encoding VP6 from Rotavirus A WA strain was cloned into 2X35S-CPMV-HT-NOS expression system in a plasmid containing Plasto_pro/P19/Plasto_ter expression cassette using the following PCR-based method. A fragment containing the VP6 coding sequence was amplified using primers IF-WA_VP6(opt).s1+3c (Figure 25a, SEQ ID NO: 28) and IF-WA_VP6(opt).s1-4r (Figure 25b, SEQ ID NO: 29), using optimized VP6 gene sequence (SEQ ID NO: 46) as template. For sequence optimization, VP6 protein sequence (Genbank accession number AAA47311) was backtranslated and optimized for human codon usage, GC content and mRNA structure. The PCR product was cloned in 2X35S/CPMV-HT/NOS expression system using In-Fusion cloning system (Clontech, Mountain View, CA). Construct number 1191 (Figure 17c) was digested with SacII and StuI restriction enzyme and the linearized plasmid was used for the In-Fusion assembly reaction. Construct number 1191 is an acceptor plasmid intended for "In Fusion" cloning of genes of interest in a CPMV-HT-based expression cassette. It also incorporates a gene construct for the co-expression of the TBSV P19 suppressor of silencing under the alfalfa Plastocyanin gene promoter and terminator. The backbone is a pCAMBIA binary plasmid and the sequence from left to right t-DNA borders is presented in Figure 18 (SEQ ID NO: 23). The resulting construct was given number 1713 (Figure 25c, SEQ ID NO: 30). The amino acid sequence of VP6 from Rotavirus A strain WA is presented in Figure 26 (SEQ ID NO: 31). A representation of plasmid 1713 is presented in Figure 27.

### D-2X35S/CPMV-HT/RVA(WA) VP6(opt)/NOS into BeYDV(m)+Replicase amplification system (Construct number 1714)

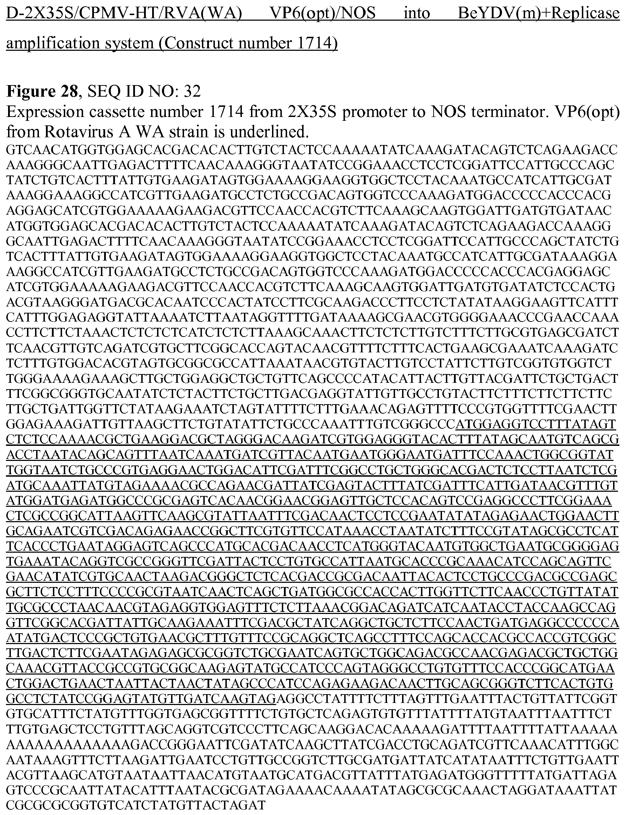

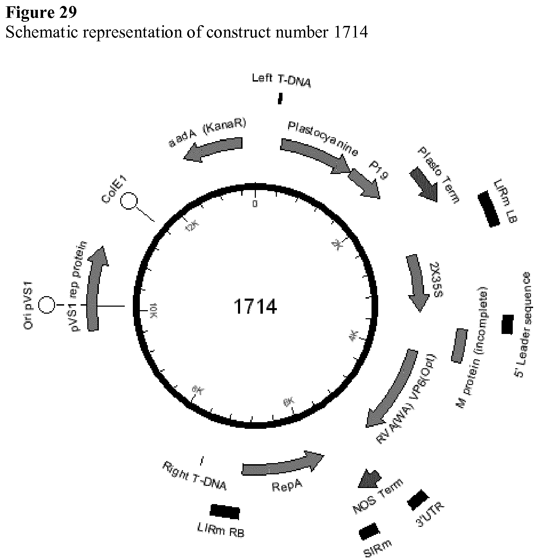

An optimized sequence encoding VP6 from Rotavirus A WA strain was cloned into 2X35S/CPMV-HT/NOS comprising the BeYDV(m)+replicase amplification system in a plasmid containing Plasto_pro/P19/Plasto_ter expression cassette using the following PCR-based method. A fragment containing the VP6 coding sequence was amplified using primers IF-WA_VP6(opt).s1+3c (Figure 25a, SEQ ID NO: 28) and IF-WA_VP6(opt).s1-4r (Figure 25b, SEQ ID NO: 29), using optimized VP6 gene sequence (SEQ ID NO: 46) as template. For sequence optimization, VP6 protein sequence (Genbank accession number AAA47311) was backtranslated and optimized for human codon usage, GC content and mRNA structure. The PCR product was cloned in 2X35S/CPMV-HT/NOS expression cassette into the BeYDV(m) amplification system using In-Fusion cloning system (Clontech, Mountain View, CA). Construct 193 (Figure 22A) was digested with SacII and StuI restriction enzyme and the linearized plasmid was used for the In-Fusion assembly reaction. Construct number 193 is an acceptor plasmid intended for "In Fusion" cloning of genes of interest in a CPMV-HT-based expression cassette into the BeYDV(m) amplification system. It also incorporates a gene construct for the co-expression of the TBSV P19 suppressor of silencing under the alfalfa Plastocyanin gene promoter and terminator. The backbone is a pCAMBIA binary plasmid and the sequence from left to right t-DNA borders is presented in Figure 22B (SEQ ID NO: 26). The resulting construct was given number 1714 (Figure 28, SEQ ID NO: 32). The amino acid sequence of VP6 from Rotavirus A strain WA is presented in Figure 26 (SEQ ID NO: 31). A representation of plasmid 1714 is presented in Figure 29.

### C-2X35S/CPMV-HT/RVA(Rtx) VP4(opt)/NOS (Construct number 1730)

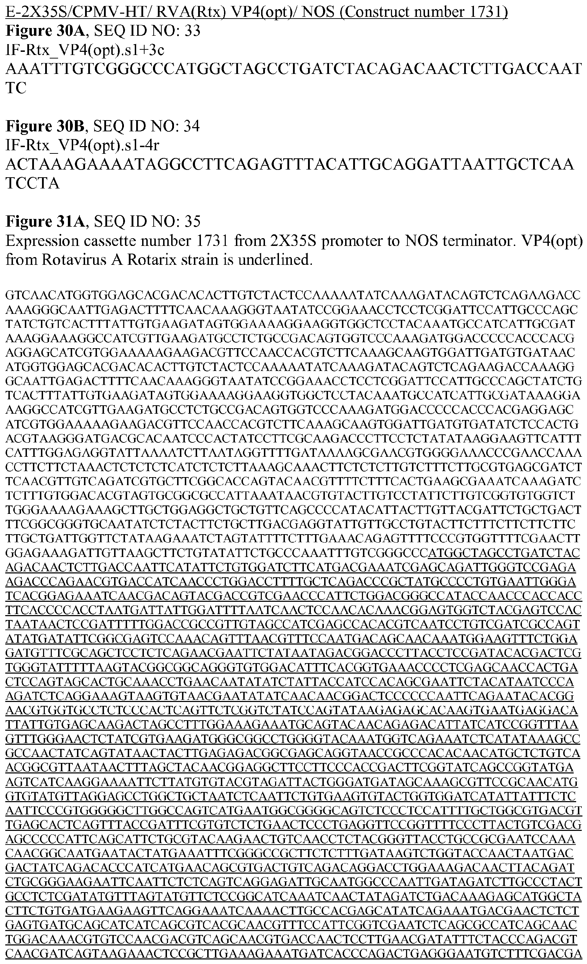

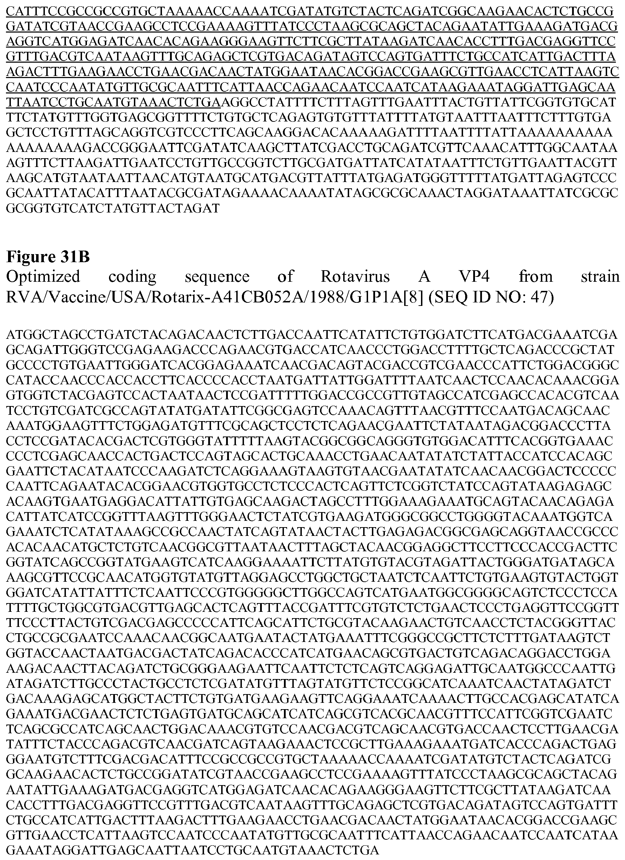

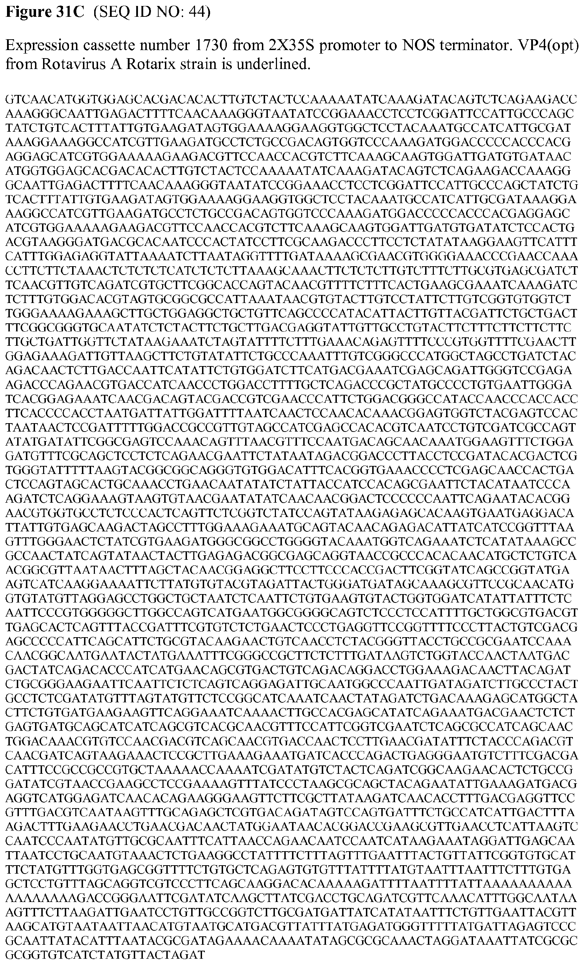

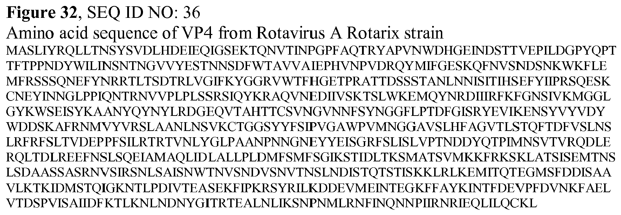

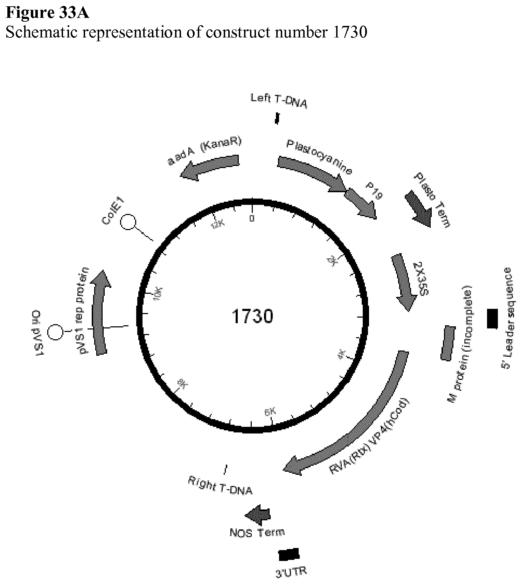

An optimized sequence encoding VP4 from Rotavirus A vaccine USA/Rotarix-A41CB052A/1988/G1P1A[8] strain was cloned into 2X35S/CPMV-HT/NOS in a plasmid containing Plasto_pro/P19/Plasto_ter expression cassette using the following PCR-based method. A fragment containing the VP4 coding sequence was amplified using primers IF-Rtx_VP4(opt).s1+3c (Figure 30A, SEQ ID NO: 33) and IF-Rtx_VP4(opt).s1-4r (Figure 30B, SEQ ID NO: 34), using optimized VP4 gene sequence (Figure 31B, (SEQ ID NO: 47) as template. For sequence optimization, VP4 protein sequence (Genbank accession number AEX30660) was backtranslated and optimized for human codon usage, GC content and mRNA structure. The PCR product was cloned in 2X35S/CPMV-HT/NOS expression cassette using In-Fusion cloning system (Clontech, Mountain View, CA). Construct number 1191 (Figure 18, SEQ ID NO: 23) was digested with SacII and StuI restriction enzyme and the linearized plasmid was used for the In-Fusion assembly reaction. Construct number 1191 is an acceptor plasmid intended for "In Fusion" cloning of genes of interest in a CPMV-HT-based expression cassette. It also incorporates a gene construct for the co-expression of the TBSV P19 suppressor of silencing under the alfalfa Plastocyanin gene promoter and terminator. The backbone is a pCAMBIA binary plasmid and the sequence from left to right t-DNA borders is presented in (Figure 18, SEQ ID NO: 23). The resulting construct was given number 1730 (Figure 31C, SEQ ID NO: 50). The amino acid sequence of VP4 from Rotavirus A vaccine USA/Rotarix-A41CB052A/1988/G1P1A[8] is presented in Figure 32 (SEQ ID NO: 36). A representation of plasmid 1730 is presented in Figure 33A.

### E-2X35S/CPMV-HT/RVA(Rtx) VP4(opt)/NOS into BeYDV(m)+Replicase amplification system (Construct number 1731)

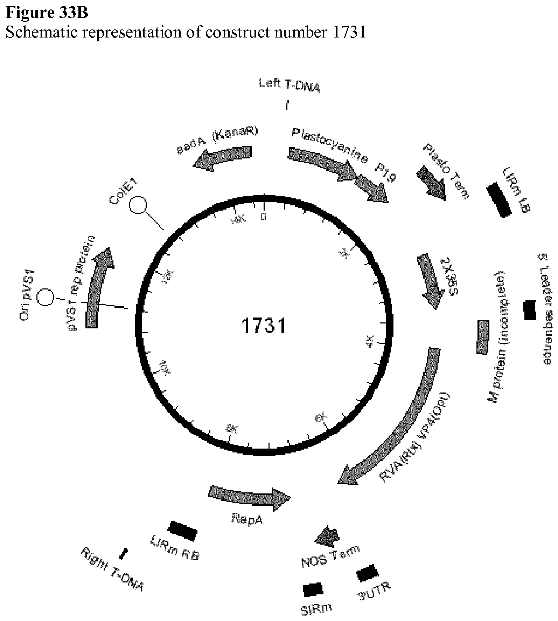

An optimized sequence encoding VP4 from Rotavirus A vaccine USA/Rotarix-A41CB052A/1988/G1P1A[8] strain was cloned into 2X35S/CPMV-HT/NOS comprising the BeYDV(m)+replicase amplification system in a plasmid containing Plasto_pro/P19/Plasto_ter expression cassette using the following PCR-based method. A fragment containing the VP4 coding sequence was amplified using primers IF-Rtx_VP4(opt).s1+3c (Figure 30A, SEQ ID NO: 33) and IF-Rtx_VP4(opt).s1-4r (Figure 30B, SEQ ID NO: 34), using optimized VP4 gene sequence (SEQ ID NO: 47) as template. For sequence optimization, VP4 protein sequence (Genbank accession number AEX30660) was backtranslated and optimized for human codon usage, GC content and mRNA structure. The PCR product was cloned in 2X35S/CPMV-HT/NOS expression cassette into the BeYDV(m) amplification system using In-Fusion cloning system (Clontech, Mountain View, CA). Construct 193 (Figure 22A) was digested with SacII and StuI restriction enzyme and the linearized plasmid was used for the In-Fusion assembly reaction. Construct number 193 is an acceptor plasmid intended for "In Fusion" cloning of genes of interest in a CPMV-HT-based expression cassette into the BeYDV(m) amplification system. It also incorporates a gene construct for the co-expression of the TBSV P19 suppressor of silencing under the alfalfa Plastocyanin gene promoter and terminator. The backbone is a pCAMBIA binary plasmid and the sequence from left to right t-DNA borders is presented in Figure 22B (SEQ ID NO: 26). The resulting construct was given number 1731 (Figure 31, SEQ ID NO: 35). The amino acid sequence of VP4 from Rotavirus A vaccine USA/Rotarix-A41CB052A/1988/G1P1A[8] is presented in Figure 32 (SEQ ID NO: 36). A representation of plasmid 1731 is presented in Figure 33B.

### F-2X35S/CPMV-HT/RVA(Rtx) VP7(opt)/NOS (Construct number 1733)

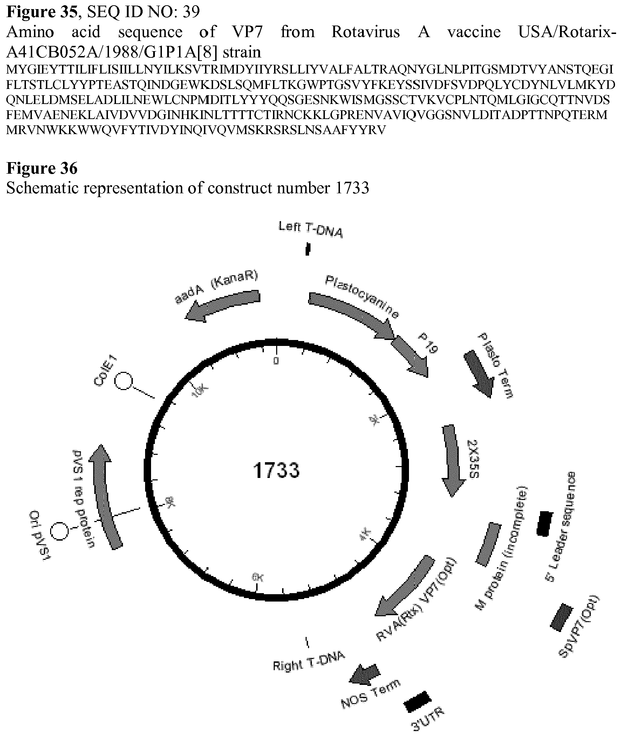

An optimized sequence encoding VP7 with is native signal peptide from Rotavirus A vaccine USA/Rotarix-A41CB052A/1988/G1P1A[8] strain was cloned into 2X35S-CPMV-HT-NOS expression system in a plasmid containing Plasto_pro/P19/Plasto_ter expression cassette using the following PCR-based method. A fragment containing the VP7 coding sequence was amplified using primers IF-Rtx_VP7(opt).s1+3c (Figure 34A, SEQ ID NO: 37) and IF-Rtx_VP7(opt).s1-4r (Figure 34B, SEQ ID NO: 38), using optimized VP7 gene sequence (SEQ ID NO: 54) as template. For sequence optimization, VP7 protein sequence (Genbank accession number AEX30682) was backtranslated and optimized for human codon usage, GC content and mRNA structure. The PCR product was cloned in 2X35S/CPMV-HT/NOS expression system using In-Fusion cloning system (Clontech, Mountain View, CA). Construct number 1191 (Figure 17C) was digested with SacII and StuI restriction enzyme and the linearized plasmid was used for the In-Fusion assembly reaction. Construct number 1191 is an acceptor plasmid intended for "In Fusion" cloning of genes of interest in a CPMV-HT-based expression cassette. It also incorporates a gene construct for the co-expression of the TBSV P19 suppressor of silencing under the alfalfa Plastocyanin gene promoter and terminator. The backbone is a pCAMBIA binary plasmid and the sequence from left to right t-DNA borders is presented in Figure 18 (SEQ ID NO: 23). The resulting construct was given number 1733 (Figure 34C, SEQ ID NO: 24). The amino acid sequence of VP7 with is native signal peptide from Rotavirus A vaccine USA/Rotarix-A41CB052A/1988/G1P1A[8] strain is presented in Figure 35 (SEQ ID NO: 39). A representation of plasmid 1733 is presented in Figure 36.

### D-2X35S/CPMV-HT/TrSp-RVA(Rtx) VP7(opt)/NOS (Construct number 1734)

An optimized sequence encoding VP7 with a truncated version of the native signal peptide from Rotavirus A vaccine USA/Rotarix-A41CB052A/1988/G1P1A[8] strain was cloned into 2X35S-CPMV-HT-NOS expression system in a plasmid containing Plasto_pro/P19/Plasto_ter expression cassette using the following PCR-based method. A fragment containing the VP7 coding sequence was amplified using primers IF-TrSP+Rtx_VP7(opt).s1+3c (Figure 44A, SEQ ID NO: 55) and IF-Rtx_VP7(opt).s1-4r (Figure 44B, SEQ ID NO: 56), using optimized VP7 gene sequence (corresponding to nt 88-891 from Figure 44C, SEQ ID NO: 57) as template. For sequence optimization, VP7 protein sequence (Genbank accession number AEX30682) was backtranslated and optimized for human codon usage, GC content and mRNA structure. The PCR product was cloned in 2X35S/CPMV-HT/NOS expression system using In-Fusion cloning system (Clontech, Mountain View, CA). Construct number 1191 (Figure 17C) was digested with SacII and StuI restriction enzyme and the linearized plasmid was used for the In-Fusion assembly reaction. Construct number 1191 is an acceptor plasmid intended for "In Fusion" cloning of genes of interest in a CPMV-HT-based expression cassette. It also incorporates a gene construct for the co-expression of the TBSV P19 suppressor of silencing under the alfalfa Plastocyanin gene promoter and terminator. The backbone is a pCAMBIA binary plasmid and the sequence from left to right t-DNA borders is presented in Figure 18 (SEQ ID NO: 23). The resulting construct was given number 1734 (Figure 44D, SEQ ID NO: 58). The amino acid sequence of VP7 with truncated signal peptide from Rotavirus A vaccine USA/Rotarix-A41CB052A/1988/G1P1A[8] strain is presented in Figure 44E (SEQ ID NO: 59). A representation of plasmid 1734 is presented in Figure 44F.

### G-2X35S/CPMV-HT/PDISP/RVA(WA) VP7(opt)/NOS into BeYDV(m)+Replicase amplification system (Construct number 1735)

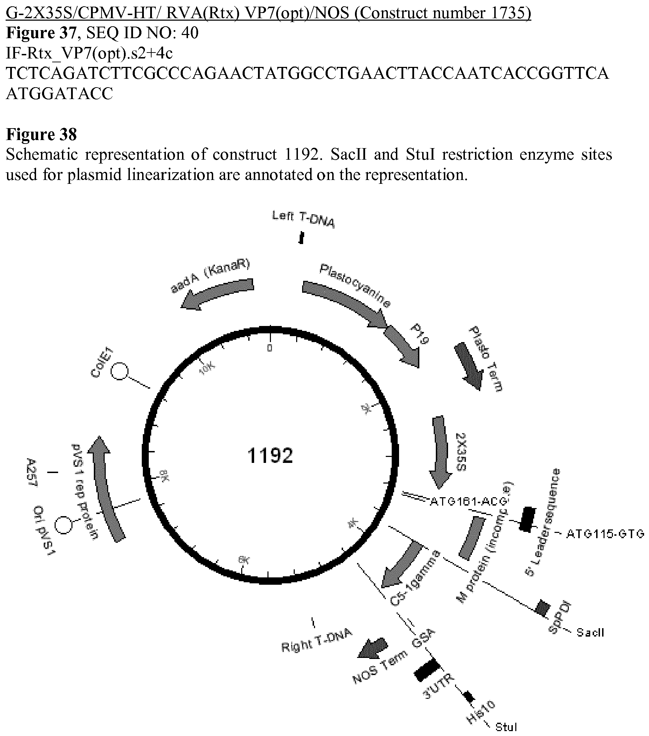

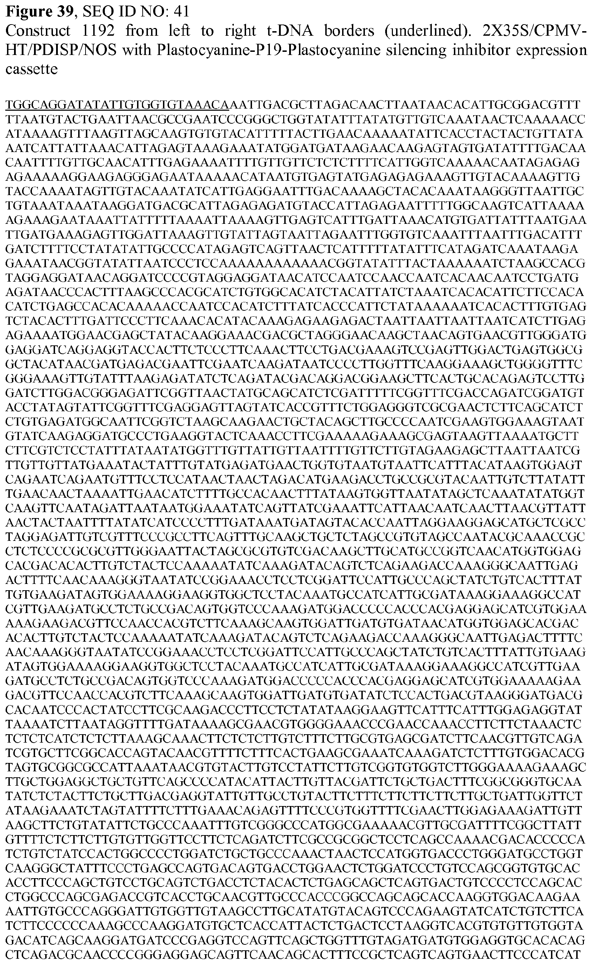

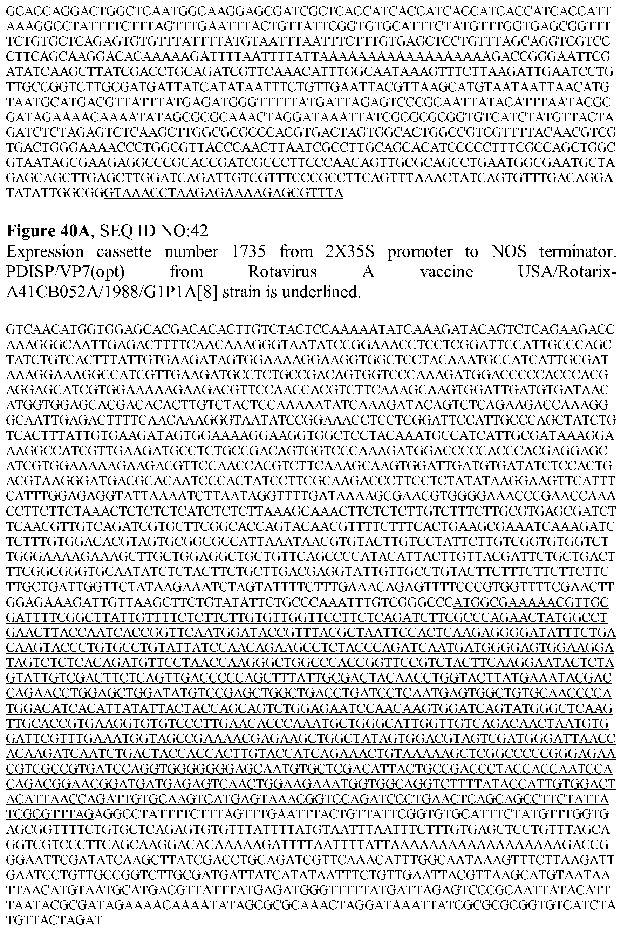

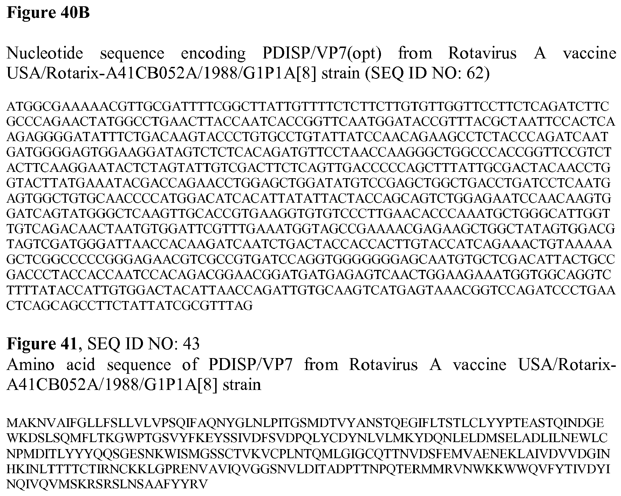

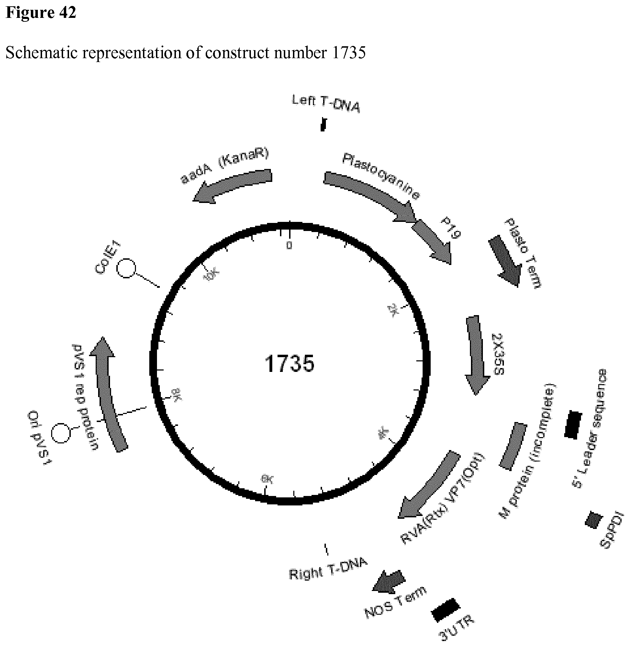

A sequence encoding VP7 from Rotavirus A vaccine USA/Rotarix-A41CB052A/1988/G1P1A[8] strain was cloned into 2X35S-CPMV-HT-PDISP-NOS expression system in a plasmid containing Plasto_pro/P19/Plasto_ter expression cassette using the following PCR-based method. A fragment containing the VP7 coding sequence without his wild type signal peptide was amplified using primers IF-Rtx_VP7(opt).s2+4c (Figure 37A, SEQ ID NO: 40) and IF-Rtx_VP7(opt).s1-4r (Figure 34B, SEQ ID NO: 38), using optimized VP7 gene sequence (SEQ ID NO: 54). For sequence optimization, VP7 protein sequence (Genbank accession number AEX30682) was backtranslated and optimized for human codon usage, GC content and mRNA structure. The PCR product was cloned in-frame with alfalfa PDI signal peptide in 2X35S/CPMV-HT/NOS expression system using In-Fusion cloning system (Clontech, Mountain View, CA). Construct 1192 (Figure 38) was digested with SacII and StuI restriction enzyme and the linearized plasmid was used for the In-Fusion assembly reaction. Construct number 1192 is an acceptor plasmid intended for "In Fusion" cloning of genes of interest in frame with an alfalfa PDI signal peptide in a CPMV-HT-based expression cassette. It also incorporates a gene construct for the co-expression of the TBSV P19 suppressor of silencing under the alfalfa Plastocyanin gene promoter and terminator. The backbone is a pCAMBIA binary plasmid and the sequence from left to right t-DNA borders is presented in Figure 39 (SEQ ID NO: 41). The resulting construct was given number 1735 (Figure 40, SEQ ID NO: 42). The amino acid sequence of PDISP/VP7 from Rotavirus A vaccine USA/Rotarix-A41CB052A/1988/G1P1A[8] strain is presented in Figure 41 (SEQ ID NO: 43). A representation of plasmid 1735 is presented in Figure 42.

**Table 3. Description of synthesized genes for the production of RLPs.**

| **SEQ ID No** | **Antigen** | **Strain of origin** | **Sequence type*** | **Figure in disclosure** |
|---|---|---|---|---|
| 45 | VP2 | WA | Optimized | 19B |
| 46 | VP6 | WA | Optimized | 25D |
| 47 | VP4 | Rotarix | Optimized | 31B |
| 50 | VP4 | SA11 | Wild-type | 43A |
| 51 | VP4 | SA11 | Optimized | 43B |
| 54 | VP7 | Rotarix | Optimized | 34E |
| 53 | VP7 | SA11 | Wild-type | 43D |
| 52 | VP7 | SA11 | Optimized | 43C |

| | | | | |
|---|---|---|---|---|
| * Optimized sequences were modified to favor the use of preferred human codons and increase GC content | | | | |

**Table 4. Description of assembled and tested construct for the production of RLPs.**

| **Expression system** | **Amplification system** | **Signal peptide^{†}** | **Antigen (strain)*^{,‡}** | **Gene SEQ ID used for PCR** | **Construct number** |
|---|---|---|---|---|---|
| CPMV HT | - | - | RVA(WA) VP2 [optimized] | SEQ ID NO: 45 | 1710 |
| CPMV HT | BeYDV(m)+rep | - | RVA(WA) VP2 [optimized] | SEQ ID NO: 45 | 1711 |
| CPMV HT | - | - | RVA(WA) VP6 [optimized] | SEQ ID NO: 46 | 1713 |
| CPMV HT | BeYDV(m)+rep | - | RVA(WA) VP6 [optimized] | SEQ ID NO: 46 | 1714 |
| CPMV HT | - | - | RVA(Rtx) VP4 [optimized] | SEQ ID NO: 47 | 1730 |
| CPMV HT | BeYDV(m)+rep | - | RVA(Rtx) VP4 [optimized] | SEQ ID NO: 47 | 1731 |
| CPMV HT | - | WtSp | RVA(Rtx) VP7 [optimized] | SEQ ID NO: 54 | 1733 |
| CPMV HT | - | TrSp | RVA(Rtx) VP7 [optimized] | SEQ ID NO: 54 | 1734 |
| CPMV HT | - | SpPDI | RVA(Rtx) VP7 [optimized] | SEQ ID NO: 54 | 1735 |
| CPMV HT | BeYDV(m)+rep | WtSp | RVA(Rtx) VP7 [optimized] | SEQ ID NO: 54 | 1736 |
| CPMV HT | BeYDV(m)+rep | TrSp | RVA(Rtx) VP7 [optimized] | SEQ ID NO: 54 | 1737 |
| CPMV HT | BeYDV(m)+rep | SpPDI | RVA(Rtx) VP7 [optimized] | SEQ ID NO: 54 | 1738 |
| CPMV HT | - | - | RVA(SA11) VP4 | SEQ ID NO: 50 | 1760 |
| CPMV HT | BeYDV(m)+rep | - | RVA(SA11) VP4 | SEQ ID NO: 50 | 1761 |
| CPMV HT | - | - | RVA(SA11) VP4 [optimized] | SEQ ID NO: 51 | 1770 |
| CPMV HT | BeYDV(m)+rep | - | RVA(SA11) VP4 [optimized] | SEQ ID NO: 51 | 1771 |
| CPMV HT | - | WtSp | RVA(SA11) VP7 | SEQ ID NO: 53 | 1763 |
| CPMV HT | - | TrSp | RVA(SA11) VP7 | SEQ ID NO: 53 | 1764 |
| CPMV HT | - | SpPDI | RVA(SA11) VP7 | SEQ ID NO: 53 | 1765 |
| CPMV HT | BeYDV(m)+rep | WtSp | RVA(SA11) VP7 | SEQ ID NO: 53 | 1766 |
| CPMV HT | BeYDV(m)+rep | TrSp | RVA(SA11) VP7 | SEQ ID NO: 53 | 1767 |
| CPMV HT | BeYDV(m)+rep | SpPDI | RVA(SA11) VP7 | SEQ ID NO: 53 | 1768 |
| CPMV HT | - | WtSp | RVA(SA11) VP7 [optimized] | SEQ ID NO: 52 | 1773 |
| CPMV HT | - | TrSp | RVA(SA11) VP7 [optimized] | SEQ ID NO: 52 | 1774 |
| CPMV HT | - | SpPDI | RVA(SA11) VP7 [optimized] | SEQ ID NO: 52 | 1775 |
| CPMV HT | BeYDV(m)+rep | WtSp | RVA(SA11) VP7 [optimized] | SEQ ID NO: 52 | 1776 |
| CPMV HT | BeYDV(m)+rep | TrSp | RVA(SA11) VP7[optimized] | SEQ ID NO: 52 | 1777 |
| CPMV HT | BeYDV(m)+rep | SpPDI | RVA(SA11) VP7 [optimized] | SEQ ID NO: 52 | 1778 |

| | | | | | |
|---|---|---|---|---|---|
| † WtSp: Wild type signal peptide, SpPDI: Signal peptide of plant origin, cloned form alfalfa protein disulfide isomerase gene, TrSp: truncated wild type signal peptide, TrSp start at 2nd Met in WtSp (M30). * [optimized] means that the sequence has been optimized based on codon usage, GC content and RNA structure. | | | | | |

### Example 3

### Assembly of gene constructs and Agrobacterium transformation

All plasmids, including plasmids 1710, 1713, 1730 and 1734, were used to transform Agrobacterium tumefaciens (AGL1; ATCC, Manassas, VA 20108, USA) by electroporation (Mattanovich et al., 1989, Nucleic Acid Res. 17:6747) alternatively, heat shock using CaC12-prepared competent cells (XU et al., 2008, Plant Methods 4) may be used. The integrity of the plasmids in the A. tumefaciens strains created was confirmed by restriction mapping. The A. tumefaciens strain transformed with a given binary plasmid is named AGL1/"plasmid number". For example, the A. tumefaciens strain transformed with construct number 1710 is termed "AGL1/1710".

### Preparation of plant biomass, inoculum, agroinfiltration, and harvesting

Nicotiana benthamiana plants were grown from seeds in flats filled with a commercial peat moss substrate. The plants were allowed to grow in the greenhouse under a 16/8 photoperiod and a temperature regime of 25°C day/20°C night. Three weeks after seeding, individual plantlets were picked out, transplanted in pots and left to grow in the greenhouse for three additional weeks under the same environmental conditions.

Agrobacteria transfected with each construct were grown in a LB medium from vegetal origin and supplemented with 10 mM 2-(N-morpholino)ethanesulfonic acid (MES) and 50 µg/ml kanamycin pH5.6 until they reached an OD600 between 0.6 and 2.5. Agrobacterium suspensions were mixed to reach appropriate ratio for each construct and brought to 2.5X OD600 with infiltration medium (10 mM MgCl2 and 10 mM MES pH 5.6). A. tumefaciens suspensions were stored overnight at 4°C. On the day of infiltration, culture batches were diluted with infiltration medium in 2.5 suspension volumes and allowed to warm before use. Whole plants of N. benthamiana were placed upside down in the bacterial suspension in an air-tight stainless steel tank under a vacuum of 20-40 Torr for 2-min. Following infiltration, plants were returned to the greenhouse for a 3-12 day incubation period until harvest. Harvested biomass was kept frozen (-80°C) until use for purification of particles.

### Extraction and purification of rotavirus-like particles

Proteins were extracted from frozen biomass by mechanical extraction in a blender with 3 volumes of extraction buffer (TNC: 10 mM Tris pH 7.4, 140 mM NaCl, 10 mM CaCl2). The slurry was filtered through a large pore nylon filter to remove large debris and centrifuged 5000 g for 5 min at 4°C. The supernatant was collected and centrifuged again at 5000 g for 30 min (4°C) to remove additional debris. The supernatant was depth-filtered and ultra-filtered and the filtrate was centrifuged at 75 000 g for 20 min (4°C) to concentrate the rotavirus-like particles. The pellet containing the particles was resuspended in 1/12 volume of TNC and the insoluble were remove with a centrifugation at 5000 g for 5 minutes. The supernatant was filtered on Miracloth before being loaded on iodixanol density gradients.

Density gradient centrifugation was performed as follows. Tubes containing step gradients from 5% to 45% of iodixanol were prepared and overlaid with the filtered extracts containing the rotavirus-like particles. The gradients were centrifuged at 120 000 g for 4 hours (4°C). After centrifugation, 1 ml fractions were collected from the bottom to the top and analysed by Coomassie-stained SDS-PAGE and Western blot. To remove iodixanol for the fractions selected for further analysis, selected fractions were centrifuged 75 000 g for 20 min (4°C) and the pelleted particles were resuspended in fresh TNC buffer.

### SDS-PAGE and immunoblotting

Protein concentrations were determined by the BCA protein assay (Pierce Biochemicals, Rockport IL). Proteins were separated by SDS-PAGE under reducing or non-reducing conditions and stained with Coomassie Blue. Stained gels were scanned and densitometry analysis performed using ImageJ Software (NIH).

For immunoblotting, electrophoresed proteins were electrotransferred onto polyvinylene difluoride (PVDF) membranes (Roche Diagnostics Corporation, Indianapolis, IN). Prior to immunoblotting, the membranes were blocked with 5% skim milk and 0.1% Tween-20 in Tris-buffered saline (TBS-T) for 16-18h at 4°C.

Immunoblotting was performed by incubation with a suitable antibody (Table 5), in 2 µg/ml in 2% skim milk in TBS-Tween 20 0.1%. Secondary antibodies used for chemiluminescence detection were as indicated in Table 5, diluted as indicated in 2% skim milk in TBS-Tween 20 0.1%. Immunoreactive complexes were detected by chemiluminescence using luminol as the substrate (Roche Diagnostics Corporation). Horseradish peroxidase-enzyme conjugation of human IgG antibody was carried out by using the EZ-Link Plus® Activated Peroxidase conjugation kit (Pierce, Rockford, IL).

**Table 5: Electrophoresis conditions, antibodies, and dilutions for immunoblotting of rotavirus antigens.**

| **Rotavirus antigen** | **Electrophore sis condition** | **Primary antibody** | **Dilution** | **Secondary antibody** | **Dilution** |
|---|---|---|---|---|---|
| VP2 | Reducing | Rabbit polyclonal anti-VP2 (kindly provided by professor Koki Taniguchi) | 1 µg/ml | Goat anti-rabbit (JIR 111-035-144) | 1:10 000 |
| VP6 | Reducing | ABIN 308233 | 1:20000 | Goat anti-rabbit (JIR 111-035-144) | 1:10 000 |
| VP7 | Non-Reducing | Rabbit polyclonal anti-VP7 (kindly provided by professor Koki Taniguchi) | 1:2000 | Goat anti-rabbit (JIR 111-035-144) | 1:10 000 |

### Anti-VP4 Enzyme-linked immuno sorbent assay (ELISA)

U-bottom 96-well microtiter plates were coated with a mouse monoclonal anti-VP4 (kindly provided by Professor Koki Taniguchi) diluted 1:100000 in 10 mM PBS pH7.4 (phosphate-buffered saline), 150 mM NaCl for 16-18 hours at 4°C. After incubation, plates were washed three times with 10 mM PBS pH7.4, 1 M NaCl containing 0.1% Tween-20 and blocked with 5% BSA in 10 mM PBS pH7.4, 150 mM NaCl containing 0.1% Tween-20 for 1 hour at 37°C. After the blocking step, plates were washed three times with 10 mM PBS pH7.4, 1 M NaCl containing 0.1% Tween-20. Samples were added and plates were incubated for 1 hour at 37°C. Plate were then washed 3 times with 10 mM PBS pH7.4, 1 M NaCl,1 mM CaCl2, 0.5 mM MgCl2 containing 0.1% Tween-20. For all remaining wash steps, washing buffer remain the same and during the third wash, plates were incubated 10 minutes at room temperature before completely removing washing solution. Rabbit polyclonal antibody raised against Rotavirus (kindly provided by Professor Koki Taniguchi) diluted 1:10000 with 3% BSA in 10 mM PBS pH7.4, 150 mM NaCl, 1 mM CaCl2, 0.5 mM MgCl2 containing 0.1% Tween-20 was added and plates were incubated for 1 hour at 37°C. Plates were then washed 3 times and horseradish peroxidase-conjugated goat anti-rabbit antibody (111-035-144, Jackson Immunoresearch, West Grove, PA) diluted 1:5000 with 3% BSA in 10 mM PBS pH7.4, 150 mM NaCl, 1 mM CaCl2, 0.5 mM MgCl2 containing 0.1% Tween-20 was added and plates were incubated for 1 hour at 37°C. Plates were washed 3 times. After final washes, plates were incubated with SureBlue TMB peroxidase substrate (KPL, Gaithersburg, MD) for 20 minutes at room temperature. Reaction was stopped by the addition of 1N HCl and A450 values were measured using a Multiskan Ascent plate reader (Thermo Scientific, Waltham, MA).

### Production of rotavirus-like particles comprising VP2 and VP6.

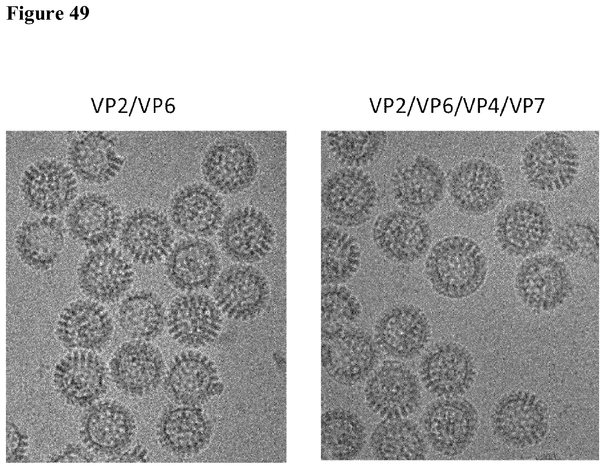

Rotavirus-like particles comprising VP2 and VP6 were produced by transient expression in Nicotiana benthamiana. Plants were agro-infiltrated with an inoculum of Agrobacteria containing a mixture of AGL1/1710 and AGL1/1713 in a 1:1 proportion and incubated for 7 days before harvest. Rotavirus-like particles were purified from the biomass using the methodology described in the materials and methods section. After centrifugation of the clarified extracts on iodixanol density gradient, the first ten fractions from the bottom of the tube were analyzed by Coomassie-stained SDS-PAGE. As shown in Figure 45A, the rotavirus antigens (VP2 and VP6) were mainly found in fractions 2 and 3 of the density gradient where the concentration of iodixanol is approximately 35%, a concentration where rotavirus-like particles are expected to be found. Very little contamination by plant proteins was found in these fractions. Western blot analysis of the fractions with anti-rotavirus hyperimmune rabbit serum and polyclonal rabbit anti-VP2 antibodies confirmed the identity of VP2 and VP6 in the density gradient fractions (Figures 45B and 45C). Fractions 2 and 3 were pooled and iodixanol was removed by high speed centrifugation and resuspension, and the purified particles were sent for cryo-electron microscopy analysis (NanoImaging Services Inc., La Jolla, CA) to confirm the assembly of the VP2 and VP6 into particles resembling the rotavirus particle. As shown in Figure 49 (left panel) the cryoEM images of VP2/VP6 particles confirmed the correct assembly of the antigens into rotavirus-like particles.

### Production of rotavirus-like particles comprising VP2, VP6 and VP7.

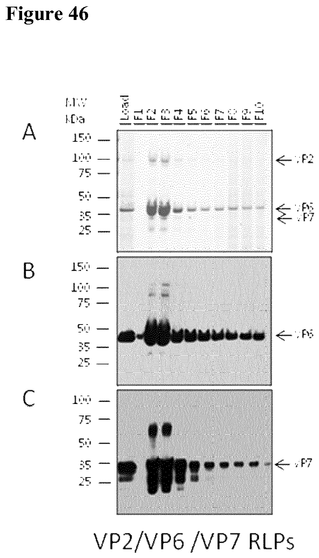

Rotavirus-like particles comprising VP2, VP6 and VP7 were produced by transient expression in Nicotiana benthamiana. Plants were agro-infiltrated with an inoculum of Agrobacteria containing a mixture of AGL1/1710 , AGL1/1713, AGL1/1734 in a 1:1:1 proportion and incubated for 7 days before harvest. Rotavirus-like particles were purified from the biomass using the methodology described in the materials and methods section. After centrifugation of the clarified extracts on iodixanol density gradient, the first ten fractions from the bottom of the tube were analyzed by Coomassie-stained SDS-PAGE. As shown in Figure 46A, the rotavirus antigens (VP2, VP6 and VP7) were mainly found in fractions 2 and 3 of the density gradient where the concentration of iodixanol is approximately 35%, a concentration where rotavirus-like particles are expected to be found. Very little contamination by plant proteins was found in these fractions. Western blot analysis of the fractions with anti-rotavirus hyperimmune rabbit serum and polyclonal rabbit anti-VP7 antibodies confirmed the identity of VP6 and VP7 in the density gradient fractions (Figures 46B and 46C).

### Production of rotavirus-like particles comprising VP2, VP4, VP6 and VP7.

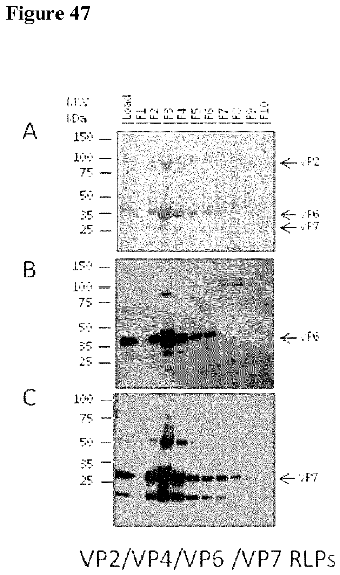

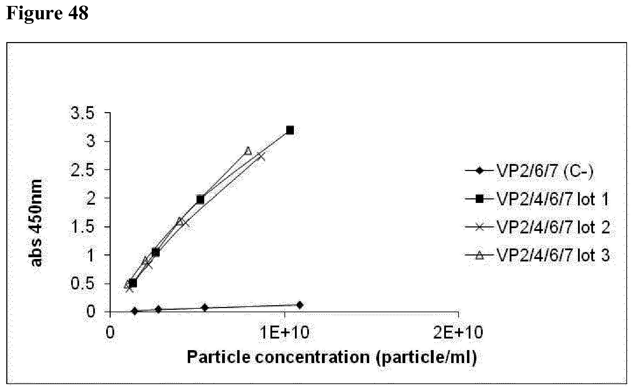

Rotavirus-like particles comprising VP2, VP4, VP6 and VP7 were produced by transient expression in Nicotiana benthamiana. Plants were agro-infiltrated with an inoculum of Agrobacteria containing a mixture of AGL1/1710, AGL1/1730 , AGL1/1713, AGL1/1734 in a 1:1:1:1 proportion and incubated for 7 days before harvest. Rotavirus-like particles were purified from the biomass using the methodology described in the materials and methods section. After centrifugation of the clarified extracts on iodixanol density gradient, the first ten fractions from the bottom of the tube were analyzed by Coomassie-stained SDS-PAGE. As shown in figure 47A, 3 of the 4 rotavirus antigens (VP2, VP6 and VP7) were visible and mainly found in fractions 3 of the density gradient where the concentration of iodixanol is approximately 35%, a concentration where rotavirus-like particles are expected to be found. Very little contamination by plant proteins was found in these fractions. The absence of detectable level of VP4 in the Coomassie-stained gel was expected since VP4 cannot be observed when the same analysis is performed on purified human rotavirus virion. Western blot analysis of the fractions with anti-rotavirus hyperimmune rabbit serum and polyclonal rabbit anti-VP7 antibodies confirmed the identity of VP6 and VP7 in the density gradient fractions (Figures 47B and 47C). Iodixanol was removed from fraction 3 by high speed centrifugation and resuspension and the purified particles were analyzed by ELISA to confirm the presence of VP4. The results presented in Figure 48 clearly show that the ELISA specifically recognizes VP4 as the negative control particles comprising VP2/VP6 and VP7 only resulted in background signal level. In contrast, the analysis of 3 different lots of purified particles comprising VP2, VP4, VP6 and VP7 antigens showed strong and uniform signals when assayed under the same conditions. Purified VP2/VP4/VP6/VP7 RLPs were sent for cryo-electron microscopy analysis (NanoImaging Services Inc., La Jolla, CA) to confirm the assembly of the four antigens into particles resembling the rotavirus particle. As shown in Figure 49 (right panel) the cryoEM images of VP2/VP4/VP6/VP7 particles confirmed the correct assembly of the antigens into rotavirus-like particles.

Table 6 lists sequences provided in various embodiments of the invention.

**Table 6: Sequence description for sequence identifiers.**

| **SEQ ID NO** | **Description** | **Page/Figure** |
|---|---|---|
| 1 | Amino acid sequence of optimized G9P6_ VP2 | Figure 16A |
| 2 | Amino acid sequence of optimized G9P6_ VP4 | Figure 16B |
| 3 | Amino acid sequence of optimized G9P6_ VP6 | Figure 16C |
| 4 | Amino acid of optimized G9P6_ VP7 | Figure 16D |
| 5 | Primer VP2F | Table 1 |
| 6 | Primer VP2R | Table 1 |
| 7 | Primer VP4F | Table 1 |
| 8 | Primer VP4R | Table 1 |
| 9 | Primer VP6F | Table 1 |
| 10 | Primer VP6R | Table 1 |
| 11 | Primer VP7F | Table 1 |
| 12 | Primer VP7R | Table 1 |
| 13 | Nucleotide sequence of G9P6_ VP2 | Figure 16A |
| 14 | Nucleotide sequence of optimized G9P6_ VP2 | Figure 16A |
| 15 | Nucleotide sequence of G9P6_ VP4 | Figure 16B |
| 16 | Nucleotide sequence of G9P6_ VP4 | Figure 16B |
| 17 | Nucleotide sequence of G9P6_ VP6 | Figure 16C |
| 18 | Nucleotide sequence of optimized G9P6_ VP6 | Figure 16C |
| 19 | Nucleotide sequence of G9P6_ VP7 | Figure 16D |
| 20 | Nucleotide sequence of G9P6_ VP7 | Figure 16D |
| 21 | Primer IF-WA VP2(opt).s1+3c | Figure 17A |
| 22 | Primer IF-WA VP2(opt).s1-4r | Figure 17B |
| 23 | Construct 1191 from left to right t-DNA borders (underlined). 2X35S/CPMV-HT/NOS with Plastocyanine-P19-Plastocyanine silencing inhibitor expression cassette | Figure 18 |
| 24 | Expression cassette number 1733 from 2X35S promoter to NOS terminator. VP7 from Rotavirus A vaccine USA/Rotarix-A41CB052A/1988/G1P1A[8] strain is underlined. | Figure 34C |
| 25 | Amino acid sequence of VP2 from Rotavirus A WA strain | Figure 20 |
| 26 | Construct 193 from left to right t-DNA borders (underlined). 2X35S/CPMV-HT/NOS into BeYDV(m)+Replicase amplification system with Plastocyanine-P19-Plastocyanine silencing inhibitor expression cassette | Figure 22B |
| 27 | Expression cassette number 1710 from 2X35S promoter to NOS terminator. VP2(opt) from Rotavirus A WA strain is underlined. | Figure 23 |
| 28 | Primer IF-WA VP6(opt).s1+3c | Figure 25a |
| 29 | Primer IF-WA VP6(opt).s1-4r | Figure 25b |
| 30 | Expression cassette number 1713 from 2X35S promoter to NOS terminator. VP6(opt) from Rotavirus A WA strain is underlined. | Figure 25c |
| 31 | Amino acid sequence of VP6 from Rotavirus A WA strain | Figure 26 |
| 32 | Expression cassette number 1714 from 2X35S promoter to NOS terminator. VP6(opt) from Rotavirus A WA strain is underlined. | Figure 28 |
| 33 | Primer IF-Rtx_VP4(opt).s1+3c | Figure 30A |
| 34 | Primer IF-Rtx_VP4(opt).s1-4r | Figure 30B |
| 35 | Expression cassette number 1731 from 2X35S promoter to NOS terminator. VP4(opt) from Rotavirus A Rotarix strain is underlined. | Figure 31A |
| 36 | Amino acid sequence of VP4 from rotavirus A Rotarix strain | Figure 32 |
| 37 | Primer IF-Rtx_VP7(opt).s1+3c | Figure 34A |
| 38 | Primer IF-Rtx_VP7(opt).s1-4r | Figure 34B |
| 39 | Amino acid sequence of VP7 from Rotavirus A vaccine USA/Rotarix-A41CB052A/1988/G1P1A[8] strain | Fig 35 |
| 40 | Primer IF-Rtx_VP7(opt).s2+4c | Figure 37A |
| 41 | Construct 1192 from left to right t-DNA borders (underlined). 2X35S/CPMV-HT/PDISP/NOS with Plastocyanine-P19-Plastocyanine silencing inhibitor expression cassette | Figure 39 |
| 42 | Expression cassette number 1735 from 2X35S promoter to NOS terminator. PDISP/VP7(opt) from Rotavirus A vaccine USA/Rotarix-A41CB052A/1988/G1P1A[8] strain is underlined. | Figure 40A |
| 43 | Amino acid sequence of PDISP/VP7 from Rotavirus A vaccine USA/Rotarix-A41CB052A/1988/G1P1A[8] strain | 41 |
| 44 | Expression cassette number 1730 from 2X35S promoter to NOS terminator. VP4(opt) from Rotavirus A Rotarix strain is underlined. | Figure 31C |
| 45 | Nucleotide sequence encoding VP2(opt) from Rotavirus A WA strain | Figure 19 |
| 46 | Nucleotide sequence encoding VP6(opt) from Rotavirus A WA strain | Figure 25d |
| 47 | Optimized coding sequence of Rotavirus A VP4 from strain RVA/Vaccine/USA/Rotarix-A41CB052A/1988/G1P1A[8] | Figure 31B |
| 48 | Nucleotide sequence encoding VP7 from Rotavirus A vaccine USA/Rotarix-A41CB052A/1988/G1P1A[8] strain | Figure 34D |
| 49 | Nucleotide sequence encoding PDISP/VP7(opt) from Rotavirus A vaccine USA/Rotarix-A41CB052A/1988/G1P1A[8] strain | Figure 40B |
| 50 | Coding sequence of Rotavirus A VP4 from strain RVA/Simian-tc/ZAF/SA1 1-H96/1958/G3P5B[2] | Figure 43A |
| 51 | Optimized coding sequence of Rotavirus A VP4 from strain RVA/Simian-tc/ZAF/SA1 1-H96/1958/G3P5B[2] | Figure 43B |
| 52 | Optimized coding sequence of Rotavirus A VP7 from strain RVA/Simian-tc/ZAF/SA1 1-H96/1958/G3P5B[2] | Figure 43C |
| 53 | Coding sequence of Rotavirus A VP7 from strain RVA/Simian-tc/ZAF/SA1 1-H96/1958/G3P5B[2] | Figure 43D |
| 54 | Optimized coding sequence of Rotavirus A VP7 from strain RVA/Vaccine/USA/Rotarix-A41CB052A/1988/G1P1A[8] | Figure 34E |
| 55 | Primer IF-TrSP+Rtx_VP7(opt).s1+3c | Figure 44A |
| 56 | Primer IF-Rtx_VP7(opt).s1-4r | Figure 44B |
| 57 | Nucleotide sequence of Optimized coding sequence of Rotavirus A VP7 from strain RVA/Vaccine/USA/Rotarix-A41 CB052A/1988/G 1P1A[8] | Figure 44C |
| 58 | Expression cassette number 1734 from 2X35S promoter to NOS terminator. VP7 from Rotavirus A vaccine USA/Rotarix-A41CB052A/1988/G1P1A[8] strain is underlined. | Figure 44D |
| 59 | Amino acid sequence of TrSp-VP7 from Rotavirus A vaccine USA/Rotarix-A41CB052A/1988/G1P1A[8] strain | Figure 44E |

### References

Yang Y M, Li X, Yang H, et al. Immunogenicity and virus-like particle formation of rotavirus capsid produced in transgenic plants. Sci China Life Sci, 2011, 54: 82-89
Angel, J., Franco, M.A. and Greenberg, H.B. (2007). Rotavirus vaccines: recent developments and future considerations. Nature reviews: Microbiology 5, 529-539
Araujo, I.T., Ferreira, M.S.R. and Failho, A.M. (2001). Rotavirus genotypes [4]G9, P[6]G9, and P[8]G9 in hospitalized children with acute gastroenteritis in Rio de Janeiro, Brazil. J Clin Microbiol 39 1999-2001.
Arias, C.F., Ballado, T. and Plebafiski, M. (1986). Synthesis of the outer-capsid glycoprotein of the simian rotavirus SA11 in Escherichia coli. Gene 47, 211-219
Au K.S., Mattion N.M., Estes M.K., (1993). A Subviral Particle Binding Domain on the Rotavirus Nonstructural Glycoprotein NS28. Virology 194, 665-67
Balen B, Krsnik-Rasol M, (2007). N-glycosylation of recombinant therapeutic glycoproteins in plant systems. Food Technology and Biotechnology 45 1-10.
Bardor, M., Faveeuw, C., Fitchette, A.C., Gilbert,D., Galas, L., Trottein, F., Faye, L. and Lerouge P. (2003). Immunoreactivity in mammals of two typical plant glycoepitopes, core alpha (1,3)-fucose and core xylose. Glycobiology 13 427-434
Berois, M., Sapin, C., Erk, I., Poncet, D. and Cohen, J. (2003). Rotavirus Nonstructural Protein NSP5 Interacts with Major Core Protein VP2. Journal of virology 77, 1757
Bertolotti-Ciarlet, A., Ciarlet, M., Crawford, S.E., Conner, M.E. and Estes, M.K. (2003). Immunogenicity and protective efficacy of rotavirus 2/6-virus-like particles produced by a dual baculovirus expression vector and administered intramuscularly, intranasally, or orally to mice. Vaccine 21, 3885-3900
Chen, J.Z., Settembre, E.C., Aoki, A.T., Zhang, X., Bellamy, A.R., Dormitzer, P.R., Harrison, S.C. and Grigorieff, N. (2009). Molecular interactions in rotavirus assembly and uncoating seen by high-resolution cryo-EM. PNAS 106, 10644-10648
Crawford, S.E., Estes, M.K., Ciarlet, M., Barone, C., O'Neal, C.M., Cohen, J. and Conner, M.E. (1999). Heterotypic protection and induction of a broad heterotypic neutralization response by rotavirus-like particles. Journal of Virology 73, 4813- 4822
Crawford, S.E., Labbe, M., Cohen, J., Burroughs, M.H., Zhou, Y.J. and Estes, M.K. (1994). Characterization of virus-like particles produced by the expression of rotavirus capsid proteins in insect cells. Journal of virology 68, 5945-5952
Denisova, E.R., Dowling, W., LaMonica, R., Shaw, R., Scarlata, S., Ruggeri, F. and Mackow, E.R. (1999). Rotavirus Capsid Protein VP5* Permeabilizes Membranes. Journal of Virology 73 3147-3153
Dennehy, P.H. (2007). Rotavirus vaccines - An update. Vaccine 25, 3137-3141
Estes M.K (1996). Rotavirus and their replication. Fields Virology 2, 1625-1655
Fabbretti, E., Afrikanova, I., Vascotto, F. and Burrone, O.R. (1999). Two nonstructural rotavirus proteins, NSP2 and NSP5, form viroplasm-like structures in vivo. J Gen Virol 80 333-9.
Favacho, A.R., Kurtenbach, E., Sardi, S.I. and Gouvea, V.S. (2006). Cloning, expression, and purification of recombinant bovine rotavirus hemagglutinin, VP8*, in Escherichia coli. Protein Expression and Purification 46, 196-203
Gentsch, J.R., Laird, A.R., Bielfelt, B., Griffin, D.D., Bányai, K., Ramachandran, M., Jain, V., Cunliffe, N.A., Nakagomi, O., Kirkwood, C.D., Fischer, T.K., Parashar, U.D., Bresee, J.S., Jiang, B. and Glass, R.I. (2005). Serotype Diversity and Reassortment between Human and Animal Rotavirus Strains: Implications for Rotavirus Vaccine Programs J Infect Dis. 192 , S146-59
Glass, R.I., Parashar, U.D., Bresee, J.S., Turcios, R., Fischer, T.K., Widdowson, MA., Jiang, B. amd Gentsch, J.R. (2006). Rotavirus vaccines: current prospects and future challenges. Lancet 368, 323-32
Gonzalez, A.M., Nguyen, T.V., Azevedo, M.S. P., Jeong, K., Agarib, F., Iosef, C., Chang, K., Lovgren-Bengtsson, K., Morein, B. and Saif, L.J. (2004). Antibody responses to human rotavirus (HRV) in gnotobiotic pigs following a new prime/boost vaccine strategy using oral attenuated HRV priming and intranasal VP2/6 rotavirus-like particle (VLP) boosting with ISCOM. Clinical & Experimental Immunology 135 361-372
Gonzalez, R.A., Espinosa, R., Romero, P., López, S. and Arias C.F. (2000). Relative localization of viroplasmic and endoplasmic reticulum-resident rotavirus proteins in infected cells. Archives of Virology 145, 1963-1973
Greenberg, H.B. and Estes, M.K. (2009). Rotaviruses: From Pathogenesis to Vaccination. Gastroenterology 136, 1939-1951
Hoshino, Y., Jones, R.W. and Kapikian, A.Z. (1998). Serotypic characterization of outer capsid spike protein VP4 of vervet monkey rotavirus SA11 strain. Archives of Virology 143, 1233-1244
Istrate, C., Hinkula, J., Charpilienne, A., Poncet, D., Cohen, J., Svensson, L. and Johansen, K. (2008). Parenteral administration of RF 8-2/6/7 rotavirus-like particles in a one-dose regimen induce protective immunity in mice. Vaccine 26, 4594-4601
Khodabandehloo, M., Shamsi, S.M., Shahrabadi, Keyvani, H. and Bambai, B. (2009). Cloning and Expression of Simian Rotavirus Spike Protein (VP4) in Insect Cells by Baculovirus Expression System. Iranian Biomedical Journal 13 9-18
Kim, Y., Nielsen, P.R., Hodgins, D., Chang, K.O. and Saif, L.J. (2002). Lactogenic antibody responses in cows vaccinated with recombinant bovine rotavirus-like particles (VLPs) of two serotypes or inactivated bovine rotavirus vaccines. Vaccine 20, 1248-1258
Kovacs-Nolan J., Erika Sasaki, Dongwan Yoo, Yoshinori Mine, Cloning and Expression of Human Rotavirus Spike Protein, VP8*, in Escherichia coli. Biochemical and Biophysical Research Communications, 282, 1183-1188
Lawton, J.A., Estes, M.K. and Venkataram, P.B.V. (2000). Mechanism of genome transcription in segmented dsRNA viruses. Advances in Virus Research 55, 185-214
Lopez, T., Camacho M., Zayas M., Najera R., Sanchez R., Arias C. F. and Lopez S. (2005). Silencing the Morphogenesis of Rotavirus. Journal of Virology 79, 184-92
Lundgren, O. and Svensson, L. (2001). Pathogenesis of Rotavirus diarrhoea. Microbes and Infection 3 1145-1156
Madore, H.P., Estes, M.K., Zarley, C.D., Hu, B., Parsons, S., Digravio, D., Greiner, S., Smith, R., Jiang, B., Corsaro, B., Barniak, V., Crawford, S. and Conner, M.E. (1999). Biochemical and immunologic comparison of virus-like particles for a rotavirus subunit vaccine. Vaccine 17, 2461-2471
Marusic, C., Rizza, P., Lattanzi, L., Mancini, C., Spada, M., Belardelli, F., Benvenuto, E. and Capone, I. (2001). Chimeric plant virus particles as immunogens for inducing murine and human immune responses against human immunodeficiency virus type 1. Journal of Virology 75, 8434-8439.
Matsumura, T., Itchoda, N. and Tsunemitsu, H. (2002). Production of immunogenic VP6 protein of bovine group A rotavirus in transgenic potato plants. Archives of Virology 147, 1263-1270
Mena, J.A., Ramírez, O.T. and Palomares, L.A. (2006). Intracellular distribution of rotavirus structural proteins and virus-like particles expressed in the insect cellbaculovirus system. Journal of Biotechnology 122, 443-452
Meyer, J.C., Bergmann, C.C. and Bellamy, A.R. (1989). Interaction of rotavirus cores with the nonstructural glycoprotein NS28. Virology 171, 98-107
Molinari, P., Peralta, A. and Taboga, O. (2008). Production of rotavirus-like particles in Spodoptera frugiperda larvae. Journal of Virological Methods 147, 364-367
Nilsson, M., von Bonsdorff, C.H., Weclewicz, K., Cohen, J. and Svensson, L. (1998). Assembly of viroplasm and virus-like particles of rotavirus by a Semliki Forest virus replicon. Virology 242, 255-265
Nishikawa, K., Fukuhara, N., Liprandi, F., Green, K., Kapikian, A., Chanock, R. and Gorziglia, M. (1989). VP4 protein of porcine rotavirus strain OSU expressed by a baculovirus recombinant induces neutralizing antibodies. Virology 173, 631-637
O'Brien, G.J., Bryant, C.J., Voogd, C., Greenberg, H.B., Gardner, R.C. and Bellamy, A.R. (2000). Rotavirus VP6 expressed by PVX vectors in Nicotiana benthamiana coats PVX rods and also assembles into virus-like particles. Virology 270, 444-453
Palombo, E.A. (1999). Genetic and antigenic diversity of human rotaviruses: potential impact on the success of candidate vaccines. FEMS Microbiology Letters 181, 1-8
Palomares, L.A. and Ramirez, O.T. (2009). Challenges for the production of virus-like particles in insect cells: The case of rotavirus-like particles. BiochemicalEngineering Journal 45(3), 158-167
Peralta, A., Molinari, P. and Taboga, O. (2009). Chimeric recombinant rotavirus-like particles as a vehicle for the display of heterologous epitopes. Virology Journal 6, 192
Ramachandran, M., Kirkwood, C.D., Unicomb, L., Cunliffe, N.A., Ward, R.L., Bhan, M.K., Clark, H.F., Glass, R.I. and Gentsch, J.R. (2000). Molecular characterization of serotype G9 rotavirus strains from a global collection. Virology 278, 436-444
Ribes, J.M., Ortego, J., Ceriani, J., Montava, R., Enjuanes, L. and Buesa, J. (2011). Transmissible gastroenteritis virus (TGEV)-based vectors with engineered murine tropism express the rotavirus VP7 protein and immunize mice against rotavirus. Virology 410 107-118
Rodríguez-Limas, W.A., Tyo, K.E.J., Nielsen, J., Ramírez, O.T. and Palomares, L.A. (2011). Molecular and process design for rotavirus-like particle production in Saccharomyces cerevisiae. Microb Cell Fact. 10, 33
Saldana, S., Esquivel Guadarrama, F., Olivera Flores Tde, J., Arias, N., Lopez, S., Arias, C., Ruiz-Medrano, R., Mason, H., Mor, T., Richter, L., Arntzen, C.J. and Gomez Lim, M.A. (2006). Production of rotavirus-like particles in tomato (Lycopersicon esculentum L.) fruit by expression of capsid proteins VP2 and VP6 and immunological studies. Viral Immunology 19, 42-53
Sanchez-Padilla, E., Grais, R.F., Guerin, P.J., Steele, A.D., Burny, M.E. and Luquero, F.J. (2009). Burden of disease and circulating serotypes of rotavirus infection in sub-Saharan Africa: systematic review and meta-analysis. Lancet Infectious Diseases 9, 567-576.
Steele, A.D., Ivanoff, B. and African Rotavirus Network (2003). Rotavirus strains circulating in Africa during 1996-1999: emergence of G9 strains and P[6] strains. Vaccine 21, 361-367
Tian, P., Ball, J.M., Zeng, C.Q.Y. and Estes, M.K. (1996). The Rotavirus Nonstructural Glycoprotein NSP4 Possesses Membrane Destabilization Activity. Journal of Vriology 70, 6973-6981
Thongprachum, A., Chaimongkol, N., Khamrin, P., Pantip, C., Mizuguchi, M., Ushijima, H. and Maneekarn, N. (2010). A novel multiplex RT-PCR for identification of VP6 subgroups of human and porcine rotaviruses, Journal of Virological Methods, 168, 191-196
Trabelsi, A., Peenze, I., Pager, C., Jeddi, M. and Steele, D. (2000). Distribution of Rotavirus VP7 Serotypes and VP4 Genotypes Circulating in Sousse, Tunisia, from 1995 to 1999: Emergence of Natural Human Reassortants. Journal of Clinical Microbiology 38, 3415-3419
Varani, G. and Allain, F.H-T. (2002). How a rotavirus hijacks the human protein synthesis machinery. Nature Structural Biology 9, 158 - 160.
Vende, P., Taraporewala, Z.F. and Patton, J.T. (2002). RNA-Binding Activity of the Rotavirus Phosphoprotein NSP5 Includes Affinity for Double-Stranded RNA. Journal of Virology 76, 5291-5299.
Vesikari, T., Karvonen, A., Korhonen, T., Espo, M., Lebacq, E., Forster, J., Zepp, F., Delem, A. and De Vos, B. (2004). Safety and immunogenicity of RIX4414 live attenuated human rotavirus vaccine in adults, toddlers and previously uninfected infants. Vaccine 22, 2836-2842
Vézina, L.-P., Faye, L., Lerouge, P., D'Aoust, M.-A., Marquet-Blouin, E., Burel, C., Lavoie, P.-O., Bardor, M. and Gomord, V. (2009), Transient co-expression for fast and high-yield production of antibodies with human-like N-glycans in plants. Plant Biotechnology Journal 7, 442-455.
Zhou, B., Zhang, Y., Wang, X., Dong, J., Wang, B., Han, C., Yu, J., Li, D. (2010). Oral administration of plant-based rotavirus VP6 induces antigen-specific IgAs, IgGs and passive protection in mice. Vaccine 28, 6021-6027
Zhou, H., Guo, L., Wang, M., Qu, J., Zhao, Z., Wang, J. and Hung, T. (2011). Prime immunization with rotavirus VLP 2/6 followed by boosting with an adenovirus expressing VP6 induces protective immunization against rotavirus in mice. Virology Journal 8, 3

All citations are hereby incorporated by reference.

The present invention has been described with regard to one or more embodiments. However, it will be apparent to persons skilled in the art that a number of variations and modifications can be made without departing from the scope of the invention as defined in the claims.

In the following clauses, preferred embodiments of the invention are described:
1. A method of producing a rotavirus like particle (RLP) in a plant, portion of a plant or plant cell, comprising:
   a) introducing a first nucleic acid comprising a first regulatory region active in the plant operatively linked to a first nucleotide sequence encoding a first rotavirus structural protein, a second nucleic acid comprising a second regulatory region active in the plant operatively linked to a second nucleotide sequence encoding a second rotavirus structural protein and a third nucleic acid comprising a third regulatory region active in the plant operatively linked to a third nucleotide sequence encoding a third rotavirus structural protein into the plant, portion of a plant or plant cell,
   b) incubating the plant, portion of a plant or plant cell under conditions that permit the transient expression of the first, second and third nucleic acid, thereby producing the RLP.
2. The method of clause 1, wherein a fourth nucleic acid comprising a fourth regulatory region active in the plant and operatively linked to a fourth nucleotide sequence encoding a fourth rotavirus structural protein is introduced into the plant, portion of a plant or plant cell in step a), and is expressed when incubating the plant, portion of a plant or plant cell in step b).
3. The method of clause 1, wherein the first rotavirus structural protein is VP2, the second rotavirus structural protein is VP6 and the third rotavirus structural protein is VP4.
4. The method of clause 1, wherein the first rotavirus structural protein is VP2, the second rotavirus structural protein is VP6 and the third rotavirus structural protein is VP7.
5. The method of clause 2, wherein the first rotavirus structural protein is VP2, the second rotavirus structural protein is VP6, the third rotavirus structural protein is VP4 and the fourth rotavirus structural protein is VP7.
6. The method of clause 1 or 2, wherein an additional nucleic acid is expressed in the plant, portion of a plant or plant cell, and wherein the additional nucleic acid comprises a regulatory region active in the plant operatively linked to a nucleotide sequence encoding a suppressor of silencing.
7. The method of clause 1 or 2, wherein the codon usage of the nucleotide sequence is adjusted to preferred human codon usage, increased GC content or a combination thereof.
8. The method of clause 1 or 2, wherein the rotavirus structural protein comprises a truncated, native or a non-native signal peptide.
9. The method of clause 8, wherein the non-native signal peptide is a protein disulfide isomerase signal (PDI) peptide.
10. The method of clause 1, wherein the first, second or third nucleotide sequence or a combination thereof is operatively linked to a Cowpea Mosaic Virus (CPMV) regulatory region.
11. The method of clause 2, wherein the first, second, third or fourth nucleotide sequence or a combination thereof is operatively linked to a Cowpea Mosaic Virus (CPMV) regulatory region.
12. The method of clause 1 or 2, further comprising the steps of:
   c) harvesting the plant, portion of a plant or plant cell, and
   d) purifying the RLPs from the plant, portion of a plant or plant cell, wherein the RLPs range in size from 70-100 nm.
13. The method of clause 12, wherein the RLP is purified in the presence of calcium.
14. A RLP produced by the method of clause 1 or 2, wherein the RLP comprising at least aVP4 rotavirus structural protein.
15. A RLP produced by the method of clause 3, wherein the RLP is a double layered RLP.
16. A RLP produced by the method of clause 4 or 5, wherein the RLP is a triple layered RLP.
17. The method of clause 1 or 2, wherein the rotavirus structural protein is selected from rotavirus strain G9 P[6], rotavirus A WA strain, rotavirus A vaccine USA/Rotarix-A41CB052A/1988/G1P1A[8] strain and rotavirus SA11 strain.
18. The method of any one of clauses 3, 4 and 5, wherein the nucleotide sequence encoding VP2 comprises from 80% to 100% identity with a nucleotide sequence as defined by SEQ ID NO:13, SEQ ID NO:14, or SEQ ID NO: 45.
19. The method of any one of clauses 3, 4 and 5, wherein the nucleotide sequence encoding VP6 comprises from 80% to 100% identity with a nucleotide sequence as defined by SEQ ID NO:17, SEQ ID NO:18 or SEQ ID NO:46.
20. The method of any one of clauses 4 and 5, wherein the nucleotide sequence encoding VP7 comprises from 80% to 100% identity with a nucleotide sequence as defined by SEQ ID NO: 19, 20, 48, 49, 52, 53, 54 or 57.
21. The method of any one of clauses 3, and 5, wherein the nucleotide sequence encoding VP4 comprises from 80% to 100% identity with a nucleotide sequence as defined by SEQ ID NO: 15, 16, 47, 50, or 51.
22. The method of any one of clauses 3, 4 and 5, wherein VP2 is encoded by an amino acid sequence comprising from 80% to 100% identity with the amino acid sequence defined by SEQ ID NO:1 or SEQ ID NO: 25.
23. The method of any one of clauses 3, 4 and 5, wherein VP6 is encoded by an amino acid sequence comprising from 80% to 100% identity with the amino acid sequence defined by SEQ ID NO:3 or SEQ ID NO: 31.
24. The method any one of clauses 4 and 5, wherein VP7 is encoded by an amino acid sequence comprising from 80% to 100% identity with the amino acid sequence defined by SEQ ID NO: 4, 39, 43 or 59.
25. The method of any one of clauses 3 and 5, wherein VP4 is encoded by an amino acid sequence comprising from 80% to 100% identity with the amino acid sequence defined by SEQ ID NO: 2 or SEQ ID NO: 36.
26. The method of clause 1, wherein the first, second or third nucleic acid sequence or a combination thereof comprises a regulatory region active in the plant operatively linked to one or more than one comovirus enhancer, to one or more than one amplification element and to a nucleotide sequence encoding a rotavirus structural protein, and wherein a fourth nucleic acid encoding a replicase is introduced into the plant, portion of a plant or plant cell.
27. The method of clause 2, wherein the first, second, third or fourth nucleic acid sequence or a combination thereof comprises a regulatory region active in the plant operatively linked to one or more than one comovirus enhancer, to one or more than one amplification element and to a nucleotide sequence encoding a rotavirus structural protein, and wherein a fifth nucleic acid encoding a replicase is introduced into the plant, portion of a plant or plant cell.
28. The method of clauses 1 , wherein at the first, second or third nucleotide sequence or a combination thereof is further operatively linked to a compartment targeting sequence.
29. The method of clauses 2, wherein the first, second, third or fourth nucleotide sequence or a combination thereof is further operatively linked to a compartment targeting sequence.
30. A method of producing a rotavirus like particle (RLP) in a plant, portion of a plant or plant cell, comprising:
   a) introducing a nucleic acid comprising a regulatory region active in the plant operatively linked to a first nucleotide sequence encoding one or more rotavirus structural protein, into the plant, portion of a plant or plant cell,
   b) incubating the plant, portion of a plant or plant cell under conditions that permit the transient expression of the first nucleic acid, thereby producing the RLP.
31. The method of clause 30, wherein a second nucleic acid comprising a second regulatory region active in the plant and operatively linked to a second nucleotide sequence encoding one or more rotavirus structural protein is introduced into the plant, portion of a plant or plant cell in step a), and is expressed when incubating the plant, portion of a plant or plant cell in step b).
32. The method of clause 31, wherein a third nucleic acid comprising a third regulatory region active in the plant and operatively linked to a third nucleotide sequence encoding one or more rotavirus structural protein is introduced into the plant, portion of a plant or plant cell in step a), and is expressed when incubating the plant, portion of a plant or plant cell in step b).
33. The method of any one of clauses 30- 32, wherein the one or more rotavirus structural protein is selected from the group comprising VP2, VP4, VP6 and VP7.
34. The method of any one of clause 30-33, wherein an additional nucleotide sequence is expressed within the plant, portion of a plant or plant cell, the additional nucleotide sequence encoding a suppressor of silencing, the additional nucleotide sequence operatively linked with a regulatory region that is active in the plant.
35. The methods of any one of clauses 30-34, wherein the one or more rotavirus structural protein is from rotavirus strain G9 P[6].
36. The method of any one of clauses 30-35, wherein the nucleotide sequence is further operatively linked to a compartment targeting sequence.
37. The method of any one of clause 28, 29 and 36, wherein the compartment targeting sequence directs the one or more rotavirus structural protein to the endoplasmatic reticulum (ER), chloroplast, plastid or apoplast of the plant cell.
38. The method of clause 37, wherein the compartment targeting sequence encodes an apoplast signal peptide or a plastid signal peptide.
39. The method of any one of clause 30-38, further comprising the steps of:
   c) harvesting the plant, portion of a plant or plant cell, and
   d) purifying the RLPs from the plant, portion of a plant or plant cell, wherein the RLPs range in size from 70-100 nm.
40. A method of producing a rotavirus like particle (RLP) in a plant, portion of a plant or plant cell comprising:
   a) providing a plant, portion of a plant or plant cell comprising a nucleic acid comprising a regulatory region active in the plant operatively linked to a nucleotide sequence encoding one or more a rotavirus structural protein;
   b) incubating the plant, portion of a plant or plant cell under conditions that permit the transient expression of the nucleic acid, thereby producing the RLP.
41. A method of producing a rotavirus like particle (RLP) in a plant, portion of a plant or plant cell comprising:
   a) providing a plant, portion of a plant or plant cell comprising a first nucleic acid comprising a first regulatory region active in the plant operatively linked to a first nucleotide sequence encoding a first rotavirus structural protein, a second nucleic acid comprising a second regulatory region active in the plant operatively linked to a second nucleotide sequence encoding a second rotavirus structural protein and a third nucleic acid comprising a third regulatory region active in the plant operatively linked to a third nucleotide sequence encoding a third rotavirus structural protein into the plant, portion of a plant or plant cell; and
   b) incubating the plant, portion of a plant or plant cell under conditions that permit the transient expression of the first, second and third nucleic acid, thereby producing the RLP.
42. The method of clause 41, wherein the plant, portion of a plant or plant cell is provided with a fourth nucleic acid comprising a fourth regulatory region active in the plant and operatively linked to a fourth nucleotide sequence encoding a fourth rotavirus structural protein is introduced into the plant, portion of a plant or plant cell in step a), and the fourth rotavirus structural protein is expressed when incubating the plant, portion of a plant or plant cell in step b).
43. A RLP produced by the method of any one of clauses 30 to 42, wherein the RLP comprising at least aVP4 rotavirus structural protein.
44. A composition comprising an effective dose of the RLP of any one of clauses 14, 15, 16 and 43 for inducing an immune response in a subject, and a pharmaceutically acceptable carrier.
45. A method of inducing immunity to a rotavirus infection in a subject, comprising administering the composition of clause 44.
46. The method of clause 45, wherein the composition is administered to a subject orally, intradermally, intranasally, intramusclarly, intraperitoneally, intravenously, or subcutaneously.
47. A plant matter comprising a RLP produced by the method of any one of clause 1 to 13 and 17 to 42.
48. A method of producing rotavirus structural protein in plant, portion of a plant or plant cell comprising
   a) introducing a nucleic acid comprising a regulatory region active in the plant operatively linked to a nucleotide sequence encoding one or more rotavirus structural protein, into the plant, portion of a plant or plant cell;
   b) incubating the plant, portion of a plant or plant cell under conditions that permit the transient expression of the nucleic acid, thereby producing the one or more rotavirus structural protein.
49. The method of clause 48, wherein the nucleotide sequence is further operatively linked to a compartment targeting sequence.
50. The method of clause 48, wherein the one or more rotavirus structural protein is VP2, VP4, VP6 or VP7.

## Claims

1. A rotavirus like particle (RLP) produced by a method in a plant, portion of a plant or plant cell, comprising:
a) introducing a first nucleic acid comprising a first regulatory region active in the plant operatively linked to a first nucleotide sequence encoding a first rotavirus structural protein, a second nucleic acid comprising a second regulatory region active in the plant operatively linked to a second nucleotide sequence encoding a second rotavirus structural protein and a third nucleic acid comprising a third regulatory region active in the plant operatively linked to a third nucleotide sequence encoding a third rotavirus structural protein into the plant, portion of a plant or plant cell,
b) incubating the plant, portion of a plant or plant cell under conditions that permit the transient expression of the first, second and third nucleic acid, thereby producing the RLP.

2. The RLP of claim 1, wherein a fourth nucleic acid comprising a fourth regulatory region active in the plant and operatively linked to a fourth nucleotide sequence encoding a fourth rotavirus structural protein is introduced in to the plant, portion of a plant or plant cell in the step a), and is expressed when incubating the plant, portion of a plant or plant cell in the step b).

3. The RLP of claim 1, wherein the first rotavirus structural protein is VP2, the second rotavirus structural protein is VP6, the third rotavirus structural protein is VP4 and the RLP is a double layered RLP.

4. The RLP of claim 1, wherein, wherein the first rotavirus structural protein is VP2, the second rotavirus structural protein is VP6, the third rotavirus structural protein is VP7 and the RLP is a triple layered RLP.

5. The RLP of claim 2, wherein the first rotavirus structural protein is VP2, the second rotavirus structural protein is VP6, the third rotavirus structural protein is VP4, the fourth rotavirus structural protein is VP7 and the RLP is a triple layered RLP.

6. A composition comprising an effective dose of the RLP of any one of claims 1 to 5 for inducing an immune response in a subject, and a pharmaceutically acceptable carrier.

7. A plant matter comprising the RLP of any one of claims 1 to 5.
